# EUROPEAN PATENT SPECIFICATION

(11) **EP 2 931 880 B1**
(45) Date of publication and mention of the grant of the patent: **20.03.2019**
(21) Application number: 12889818.6
(22) Date of filing: 12.12.2012
(51) Int. Cl.: C12N 7/01, A61K 35/12, A61K 35/76, A61P 35/00, C07K 14/08, C07K 14/145, C12N 15/40, C12N 15/47, C12N 15/62, C12N 15/86

(54) **COMPOSITIONS AND METHODS FOR THE TREATMENT OF BRAIN CANCERS**
ZUSAMMENSETZUNGEN UND VERFAHREN ZUR BEHANDLUNG VON HIRNTUMOREN
COMPOSITIONS ET PROCÉDÉS PERMETTANT DE TRAITER LES CANCERS DU CERVEAU

(43) Date of publication of application: 21.10.2015
(73) Proprietor: Turnstone Limited Partnership, Toronto ON M5X 1B8 (CA)
(72) Inventor: STOJDL, David, Ottawa, Ontario K1G 6P7 (CA); BELL, John, Cameron, Ottawa, Ontario K1G 0K8 (CA)
(74) Representative: Müller Fottner Steinecke
(86) International application number: PCT/CA2012/050893
(87) International publication number: WO 2014/089668

(56) References cited:
- CA-A1- 2 739 963
- GUIDO WOLLMANN ET AL: "Oncolytic Virus Therapy for Glioblastoma Multiforme", THE CANCER JOURNAL, vol. 18, no. 1, 1 January 2012 (2012-01-01), pages 69-81, XP055173700, ISSN: 1528-9117, DOI: 10.1097/PPO.0b013e31824671c9
- ALEXANDER MUIK ET AL: "Pseudotyping vesicular stomatitis virus with lymphocytic choriomeningitis virus glycoproteins enhances infectivity for glioma cells and minimizes neurotropism", JOURNAL OF VIROLOGY, THE AMERICAN SOCIETY FOR MICROBIOLOGY, US , vol. 85, no. 11 1 June 2011 (2011-06-01), pages 5679-5684, XP002695439, ISSN: 0022-538X, DOI: 10.1128/JVI.02511-10 Retrieved from the Internet: URL:http://jvi.asm.org/content/85/11/5679 [retrieved on 2011-03-30]
- J. ZHANG ET AL: "Oncolytic therapeutic potency of Farmington and modified maraba virus in immunocompetent intracranial glioma models and in mice bearing human brain tumor initiating cells models", NEURO-ONCOLOGY, vol. 13, no. suppl 3, 21 October 2011 (2011-10-21), pages iii107-iii120, XP055258745, US ISSN: 1522-8517, DOI: 10.1093/neuonc/nor158
- BEYER, R. J. ET AL.: 'Oncoretrovirus and Lentivirus Vectors Pseudotyped with Lymphocytic Choriomeningitis Virus Glycoprotein: Generation, Concentration, and Broad Host Range' JOURNAL OF VIROLOGY. vol. 76, no. 3, 2002, pages 1488 - 1495, XP002323020
- WATSON, D. J. ET AL.: 'Targeted Transduction Patterns in the Mouse Brain by Lentivirus Vectors Pseudotyped with VSV, Ebola, Mokola, LCMV, or MuLVEnvelope Proteins' MOLECULAR THERAPY. vol. 5, no. 5, 2002, pages 537 - 1495, XP002323022
- BRUN, J. ET AL.: 'Identification of Genetically Modified Maraba Virus as an Oncolytic Rhabdovirus' MOLECULAR THERAPY vol. 18, no. 8, 2010, pages 1440 - 1449, XP055005870

## Description

### FIELD

The present disclosure relates to rhabdovirus chimeras and their use as an oncolytic treatment. More specifically, the present disclosure relates to Maraba rhabdovirus chimeras and its use in the treatment of primary and secondary brain cancers.

### BACKGROUND

Brain tumours are composed of cells that exhibit unrestrained growth in the brain. They can be benign (that is, noncancerous) or malignant (that is, cancerous). Cancerous brain tumours are further classified as either primary or secondary tumours.

Primary tumours start in the brain, whereas secondary tumours spread to the brain from another site such as breast or lung. Secondary tumours may also be referred to as metastatic. A secondary (that is, metastatic) brain tumour occurs when cancer cells spread to the brain from a primary cancer in another part of the body. Secondary tumours are three times more common than primary tumours of the brain. All metastatic brain tumours are malignant.

Brain tumours are generally named and classified according to the following: the type of brain cells from which they originate, or the location in which the cancer develops. The biological diversity of these tumours, makes classification difficult. About 80% of malignant primary brain tumours are known collectively as gliomas (that is, they originate in glial cells) and are classified into 4 grades reflecting the degree of malignancy.

Brain cancer is the leading cause of cancer-related death in patients younger than age 35 and accounts for roughly 10% of all cancers diagnosed in North America. Treatment of brain tumours is complicated by the fact that there are more than 120 different types, which range from low grade astrocytomas to grade 4 glioblastoma multiforme (GBM).

Malignant gliomas, such as GBM, are by far the most common brain cancer found in adults but are the fastest growing and most malignant of the primary brain tumours and therefore are the most difficult to treat. Even with aggressive single and multimodal treatment options such as surgery, chemotherapy, radiation and small molecule inhibitors, the survival has remained unchanged over the past three decades with a median survival of less than one year after diagnosis.

Reasons for the failure of conventional treatments is multifactorial including the highly infiltrative/invasive nature of GBM, limitation of drug delivery through the blood brain barrier and neural parenchyma, and genetic heterogeneity resulting in intrinsic resistance to available treatments and the rise of aggressive resistant clones. Therefore, there is a dire requirement for new treatment options, which has led to the renaissance of oncolytic viral therapy for brain cancers in general and GBM in particular.

Vesicular stomatitis virus (VSV) is a potent oncolytic rhabdovirus that infects and kills a broad range of tumour cell types (Brun et al., Mol Ther 18:1440-1449, 2010). As with other rhabdoviruses, neurotropism with subsequent neurovirulence, as well as a highly potent nAb response remain problems (Diallo et al., Methods Mol Biol 797:127-140, 2011). Although VSV is known to be effective by systemic delivery in neurological tumour models (Cary et al., J Virol 85:5708-5717, 2011; Lun et al., J Natl Cancer Inst 98: 1546-1547, 2006; Wollmann et al., J Virol 84:1563-1573, 2010), its inherent neurotoxicity has hindered its consideration as a clinical candidate (Hoffmann et al., J Gen Virol 91:2782-2793, 2010; Sur et al., Vet Pathol 40:512-520, 2003).

A VSV pseudotyped with the G-protein of LCMV and its enhanced infectivity for brain cancer cells *in vitro* while sparing normal neurons has been disclosed (Muik et al., J Virol 85(11):5679-5684, 2011).

Maraba is a recently characterized oncolytic rhabdovirus that shares some sequence similarity, a similar yet more potent oncolytic spectrum, and similar neurotoxicity profile to VSV (Brun et al., Mol Ther 18:1440-1449, 2010). The rhabdoviruses VSV and Maraba constitute some of the most efficacious viruses being tested preclinically. However, a desired route of viral administration for brain cancer is intracerebral delivery, which is not currently possible with either VSV or Maraba due to their inherent neurotoxicity.

It is desirable to provide an oncolytic viral therapy for the treatment of cancers, and more specifically for the treatment of brain cancers, that obviates or mitigates at least one disadvantage of previous oncolytic viral therapies.

### SUMMARY

It is an object of the present disclosure to obviate or mitigate at least one disadvantage of previous oncolytic viral therapies. In some examples, the oncolytic viral therapy may exhibit reduced levels of neurotoxicity.

According to one aspect of the present invention, there is provided an isolated viral particle having a genome that includes open reading frames that encode: a protein having a sequence comprising SEQ ID NO: 1, or a variant thereof; a protein having a sequence comprising SEQ ID NO: 2, or a variant thereof; a protein having a sequence comprising SEQ ID NO: 3, or a variant thereof; a protein having a sequence comprising SEQ ID NO: 4 or 5, or a variant thereof; and a protein having a sequence comprising SEQ ID NO: 6, 7 or 8; and wherein the viral genome preferably comprises open reading frames that encode: a protein having a sequence comprising SEQ ID NO: 1; a protein having a sequence comprising SEQ ID NO: 2; a protein having a sequence comprising SEQ ID NO: 3; a protein having a sequence comprising SEQ ID NO: 5; and a protein having a sequence comprising SEQ ID NO: 7; and wherein the variant of a reference protein is a protein having a sequence which is at least 75%, at least 80%, at least 85%, at least 90%, or at least 95% identical to the sequence of the reference protein, and the variant protein maintains the same biological function as the reference protein.

The genome may include an open reading frame that encodes a protein having a sequence comprising SEQ ID NO: 6. Alternatively, the genome may include an open reading frame that encodes a protein having a sequence comprising SEQ ID NO: 7. Alternatively, the genome may include an open reading frame that encodes a protein having a sequence comprising SEQ ID NO: 8.

The viral genome may include open reading frames that encode: a protein having a sequence comprising SEQ ID NO: 1; a protein having a sequence comprising SEQ ID NO: 2; a protein having a sequence comprising SEQ ID NO: 3; a protein having a sequence comprising SEQ ID NO: 5; and a protein having a sequence comprising SEQ ID NO: 7.

According to another aspect of the present invention, there is provided an isolated viral particle that includes an RNA polynucleotide which has a sequence that includes: the reverse complement of the sequence defined by position 64 to position 1332 of SEQ ID NO: 10, or a conservative variant thereof; the reverse complement of the sequence defined by position 1393 to position 2190 of SEQ ID NO: 10, or a conservative variant thereof; the reverse complement of the sequence defined by position 4943 to position 11272 of SEQ ID NO: 10, or a conservative variant thereof; the reverse complement of the sequence defined by position 2256 to position 2945 of SEQ ID NO: 10, or a conservative variant thereof; the reverse complement of the sequence defined by position 3041 to position 4816 of SEQ ID NO: 10; and the reverse complements of promoters thereof; and wherein a conservative variant of a sequence of nucleotides is a sequence that is at least 75%, at least 80%, at least 85%, at least 90%, or at least 95% identical to the reference sequence of nucleotides. The conservative variant may be a sequence that includes one or more silent substitutions.

The isolated viral particle may be an isolated viral particle that produces a cDNA polynucleotide that includes a sequence according to SEQ ID NO: 9 when the virus is in a host cell.

The isolated viral particle may be an isolated viral particle that includes an RNA polynucleotide which includes a sequence according to SEQ ID NO: 10.

According to yet another aspect of the present invention, there is provided an isolated viral particle that includes an RNA polynucleotide which has a sequence that includes: the reverse complement of the sequence defined by position 64 to position 1332 of SEQ ID NO: 12, or a conservative variant thereof; the reverse complement of the sequence defined by position 1393 to position 2190 of SEQ ID NO: 12, or a conservative variant thereof; the reverse complement of the sequence defined by position 4664 to position 10993 of SEQ ID NO: 12, or a conservative variant thereof; the reverse complement of the sequence defined by position 2256 to position 2945 of SEQ ID NO: 12, or a conservative variant thereof; the reverse complement of the sequence defined by position 3041 to position 4537 of SEQ ID NO: 12; and the reverse complements of promoters thereof; and wherein a conservative variant of a sequence of nucleotides is a sequence that is at least 75%, at least 80%, at least 85%, at least 90%, or at least 95% identical to the reference sequence of nucleotides. The conservative variant may be a sequence comprising one or more silent substitutions.

The isolated viral particle may be an isolated viral particle that produces a cDNA polynucleotide that includes a sequence according to SEQ ID NO: 11 when the virus is in a host cell.

The isolated viral particle may be an isolated viral particle that includes an RNA polynucleotide which includes a sequence according to SEQ ID NO: 12.

According to still another aspect of the present invention, there is provided an isolated viral particle that includes an RNA polynucleotide which has a sequence that includes: the reverse complement of the sequence defined by position 64 to position 1332 of SEQ ID NO: 14, or a conservative variant thereof; the reverse complement of the sequence defined by position 1393 to position 2190 of SEQ ID NO: 14, or a conservative variant thereof; the reverse complement of the sequence defined by position 5195 to position 11524 of SEQ ID NO: 14, or a conservative variant thereof; the reverse complement of the sequence defined by position 2256 to position 2942 of SEQ ID NO: 14, or a conservative variant thereof; the reverse complement of the sequence defined by position 3038 to position 5068 of SEQ ID NO: 14; and the reverse complements of promoters thereof; and wherein a conservative variant of a sequence of nucleotides is a sequence that is at least 75%, at least 80%, at least 85%, at least 90%, or at least 95% identical to the reference sequence of nucleotides. The conservative variant may be a sequence comprising one or more silent substitutions.

The isolated viral particle may be an isolated viral particle that produces a cDNA polynucleotide that includes a sequence according to SEQ ID NO: 13 when the virus is in a host cell.

The isolated viral particle may be an isolated viral particle that includes an RNA polynucleotide which includes a sequence according to SEQ ID NO: 14.

According to an additional aspect of the present invention, there is provided an isolated viral particle according to the present invention for use in the treatment of cancer in a person administered the virus. The cancer may be a brain cancer. The brain cancer may be a glioblastoma.

The isolated viral particle may be used to infect a cell where the infected cell is used for the treatment of cancer.

It is also disclosed the use of an isolated viral particle according to the present disclosure for inducing a cytotoxic response in a person administered the virus. The cytotoxic response may be an anti-cancer response.

The isolated viral particle may be formulated for direct delivery to the central nervous system, outside the blood/brain barrier, inside the blood/brain barrier, or any combination thereof. The isolated viral particle may be formulated for administration via intrathecal injection, intravenous injection, intracranial injection, or any sequential or simultaneous combination thereof.

The isolated viral particle may be used to infect a cell where the infected cell is used to generate the cytotoxic response. The infected cell maybe formulated for direct delivery to the central nervous system, outside the blood/brain barrier, inside the blood/brain barrier, or any combination thereof. The infected cell may be formulated for administration via intrathecal injection, intravenous injection, intracranial injection, or any sequential or simultaneous combination thereof.

The isolated viral particle may be administered to the patient directly. The isolated viral particle may be administered directly to the central nervous system, outside the blood/brain barrier, inside the blood/brain barrier, or any combination thereof. The isolated viral particle may be administered to the patient via intrathecal injection, intravenous injection, intracranial injection, or any combination thereof sequentially or simultaneously.

The use may include infecting a cell with the isolated viral particle and administering the infected cell to the patient. The infected cell may be administered directly to the central nervous system, outside the blood/brain barrier, inside the blood/brain barrier, or any combination thereof. The infected cell may be administered to the patient intrathecally, intravenously, via intracranial injection, or any combination thereof sequentially or simultaneously.

It is also disclosed a method for inducing a cytotoxic response in a patient. The method includes administering an isolated viral particle according to the present disclosure to the patient.

The isolated viral particle may be administered to the patient directly. The isolated viral particle may be administered directly to the central nervous system, outside the blood/brain barrier, inside the blood/brain barrier, or any combination thereof. The isolated viral particle may be administered to the patient intrathecally, intravenously, via intracranial injection, or any combination thereof sequentially or simultaneously.

The method disclosed herein may include infecting a cell with the isolated viral particle and administering the infected cell to the patient. The infected cell may be administered directly to the central nervous system, outside the blood/brain barrier, inside the blood/brain barrier, or any combination thereof. The infected cell may be administered to the patient intrathecally, intravenously, via intracranial injection, or any combination thereof sequentially or simultaneously.

According to still another aspect of the present invention, there is provided a kit for the treatment of cancer in a patient. The kit includes: the isolated viral particle according to the present invention; and instructions for administration of the isolated viral particle to the patient.

The cancer maybe a brain cancer. The brain cancer may be a glioblastoma.

The isolated viral particle may be formulated for direct delivery to the central nervous system, outside the blood/brain barrier, inside the blood/brain barrier, or any combination thereof. The isolated viral particle may be formulated for administration via intrathecal injection, intravenous injection, intracranial injection, or any sequential or simultaneous combination thereof.

The isolated viral particle may be formulated for infection of a cell and the cell maybe formulated for delivery to the central nervous system, outside the blood/brain barrier, inside the blood/brain barrier, or any combination thereof. The cell may be for administration via intrathecal injection, intravenous injection, intracranial injection, or any sequential or simultaneous combination thereof.

Other aspects and features of the present disclosure will become apparent to those ordinarily skilled in the art upon review of the following description of specific examples in conjunction with the accompanying figures.

### BRIEF DESCRIPTION OF THE DRAWINGS

Embodiments of the present disclosure will now be described, by way of example only, with reference to the attached Figures.
Figure 1 is a graph illustrating the identification of non-neurotoxic rhabdoviruses based on survival of Balb/C mice after a single intracerebral dose of the indicated virus (1e7 pfu). Animals were monitored for weight loss, piloerection, hind leg paralysis, morbidity and mortality.
Figure 2A is a schematic illustration of G swapping MRB G with BG G or EB G.
Figures 2B and 2C are graphs illustration results from viability assays demonstrating viral attenuation in normal human astrocytes (NHA) and GM38 skin fibroblasts. Error bars represent standard error of the mean (SEM) of 4 biological replicates.
Figures 2D through 2K are graphs illustrating results from viability assays demonstrating MRBGG are cytolytic on human brain cancer cell lines. Viability was assayed using Alamar blue 72h post treatment. Error bars represent SEM of 4 biological replicates.
Figure 3A is a summary of the intracerebral toxicity of wild type FMT, BG, MS, MRB, and several engineered rhabdovirus strains in VSV and MRB vector backbones. MRBGG and Maraba EbG Δ51 are viruses according to the present invention.
Figure 3B is a summary of the viral load in brain homogenates of animals sacrificed 3 months post intracerebral inoculation. Limit of detection is 10¹.
Figure 3C shows pathology photos. Photos of FMT and MRBGG pathology of acutely infected Balb/C mice are indistinguishable from saline injected animals. Balb/C mice were inoculated intracerebrally with the indicated viruses (1e7 pfu) and sacrificed 48 hours post inoculation.
Figure 3D is a graph illustrating the motor function of mice treated with non-neurotoxic rhabdoviruses and control mice. Motor function is not compromised after intracerebral injection of non-neurotoxic rhabdoviruses. Motor function was assessed by rotorod analysis measuring the latency to fall off an accelerating rod.
Figure 3E is a graph illustrating the toxicity profile after a single IV injection of either FMT or MRBGG chimera at varying doses. Maximum tolerated dose (MTD) is equal to the highest dose not resulting in durable morbidity as measured by behaviour and weight.
Figure 4A is an IVIS image of U87MG tumours post MRBGG or EbG IV treatment (3 doses 1e9 pfu) vs. control treatment with PBS. Systemic delivery of these viruses enhances efficacy in a human U87MG xenograft model.
Figure 4B is a graph illustrating the flux plot, demonstrating a significant tumour regression in response to three IV doses (1e9 pfu) of MRBGG or EbG. Error bars represent SEM.
Figure 4C is a Kaplan Meir survival plot of MRBGG (Log rank test P=0.01) and EbG (Log rank test P=0.01) IV treated animals.
Figure 5 is a graph illustrating the oncolytic activity of a variety of viruses on a panel of human glioblastoma cells.
Figure 6A is a graph illustrating the in vivo neurotoxicity of Maraba chimeric viruses according to the present disclosure vs. control viruses. The graph shows Kaplan Meir survival plots of Balb/C mice after a single intracerebral dose of the indicated virus (1e6 pfu).
Figure 6B is a graph showing the weight variation of the animals of Figure 6A.
Figure 7A is a graph illustrating in vivo efficacy of maraba chimeras according to the present disclosure versus control viruses. The graph shows Kaplan Meir survival plots of CD-1 nude mice with U87MG tumors post treatment.
Figure 7B is a graph showing the weight variation of the animals of Figure 7A.
Figure 8A is an IVIS image of U87MG tumours illustrating in vivo efficacy of PBS control in a human U87MG xenograft model. The image shows tumours pre and post (1 week, 2 weeks, 3 weeks, 4 weeks) treatment.
Figure 8B is a flux plot illustrating a significant increase in tumour burden over time in untreated control animals.
Figure 9A is an IVIS image of U87MG tumours illustrating in vivo efficacy of BG wild type (BG-WT) virus treatment in a human U87MG xenograft model. The image shows U87MG tumours post BG (1 week, 2 weeks, 3 weeks, 4weeks) treatment (1 dose 1e7 pfu: IC).
Figure 9B is a flux plot illustrating an initial moderate tumour regression in response to IC dose (1e7 pfu) of BG followed by a recurrence in tumour burden.
Figure 10A is an IVIS image of U87MG tumours illustrating in vivo efficacy of FMT wild type (FMT-WT) virus treatment in a human U87MG xenograft model. The image shows U87MG tumours post FMT-WT (1 week, 2 weeks, 3 weeks, 4weeks) treatment (1 dose 1e7 pfu: IC).
Figure 10B is a flux plot demonstrating a significant tumour regression in response to IC dose (1e7 pfu) of FMT-WT.
Figure 11A is an IVIS image of U87MG tumours illustrating in vivo efficacy of MRB BG(G) treatment in a human U87MG xenograft model. The image shows U87MG tumours post MRB BG(G) (1 week, 2 weeks, 3 weeks, 4 weeks) treatment (1 dose 1e7 pfu: IC).
Figure 11B is a flux plot illustrating moderate tumour regression in response to IC dose (1e7 pfu) of MRB BGG.
Figure 12A is an IVIS image of U87MG tumours illustrating in vivo efficacy of MRB FMT(G) treatment in a human U87MG xenograft model. The image shows U87MG tumours post MRB FMT(G) (1 weeks, 2 weeks, 3 weeks) treatment (1 dose 1e7pfu).
Figure 12B is a flux plot demonstrating a significant tumour regression in response to IC dose (1e7 pfu) of MRB FMT G. However, all animals succumbed to neurotoxic effects of MRB FMT(G) treatment prior to 4 weeks post treatment.
Figure 13A is an IVIS image of U87MG tumours illustrating in vivo efficacy of FMT MRB(G) treatment in a human U87MG xenograft model. The image shows U87MG tumours post FMT MRB(G) (1 week, 2 weeks, 3 weeks) treatment (1 dose 1e7 pfu: IC).
Figure 13B is a flux plot illustrating a significant tumour regression in response to IC dose (1e7 pfu) of FMT MRB(G). However, all animals succumbed to neurotoxic effects of FMT MRB G treatment prior to 4 weeks post treatment.
Figure 14A is an IVIS image of U87MG tumours illustrating in vivo efficacy of VSV-LCMV(G) treatment in a human U87MG xenograft model. The image shows U87MG tumours post VSV LCMV G (1 week, 2 weeks, 3 weeks, 4 weeks) treatment (1 dose 1e7 pfu: IC).
Figure 14B is a flux plot illustrating a significant tumour regression in response to IC dose (1e7 pfu) of VSV-LCMV(G).
Figure 15A is an IVIS image of U87MG tumours illustrating in vivo efficacy of MRB LCMV(G) treatment in a human U87MG xenograft model. The image shows U87MG tumours post MRB LCMV G (1 week, 2 weeks, 3 weeks, 4 weeks) treatment (1 dose 1e7 pfu: IC).
Figure 15B is a flux plot illustrating a significant tumour regression in response to IC dose (1e7 pfu) of MRB-LCMV(G).
Figure 16 is a graph illustrating the neutralizing antibody titres in Balb/C mice treated with wild type Maraba virus, attenuated VSV (VSV-Δ51), Maraba LCMV(G) chimera or VSV-LCMV(G) chimera.

### DESCRIPTION

### Definitions

Throughout the present disclosure, several terms are employed that are defined in the following paragraphs.

As used herein, the words "desire" or "desirable" refer to embodiments of the technology that afford certain benefits, under certain circumstances. However, other embodiments may also be desirable, under the same or other circumstances. Furthermore, the recitation of one or more desired embodiments does not imply that other embodiments are not useful, and is not intended to exclude other embodiments from the scope of the technology.

As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features.

Although the open-ended term "comprising," as a synonym of non-restrictive terms such as including, containing, or having, is used herein to describe and claim embodiments of the present technology, embodiments may alternatively be described using more limiting terms such as "consisting of" or "consisting essentially of." Thus, for any given embodiment reciting materials, components or process steps, the present technology also specifically includes embodiments consisting of, or consisting essentially of, such materials, components or processes excluding additional materials, components or processes (for consisting of) and excluding additional materials, components or processes affecting the significant properties of the embodiment (for consisting essentially of), even though such additional materials, components or processes are not explicitly recited in this application. For example, recitation of a composition or process reciting elements A, B and C specifically envisions embodiments consisting of, and consisting essentially of, A, B and C, excluding an element D that may be recited in the art, even though element D is not explicitly described as being excluded herein.

As referred to herein, all compositional percentages are by weight of the total composition, unless otherwise specified. Disclosures of ranges are, unless specified otherwise, inclusive of endpoints and include all distinct values and further divided ranges within the entire range. Thus, for example, a range of "from A to B" or "from about A to about B" is inclusive of A and of B. Disclosure of values and ranges of values for specific parameters (such as temperatures, molecular weights, weight percentages, etc.) are not exclusive of other values and ranges of values useful herein. It is envisioned that two or more specific exemplified values for a given parameter may define endpoints for a range of values that may be claimed for the parameter. For example, if Parameter X is exemplified herein to have value A and also exemplified to have value Z, it is envisioned that Parameter X may have a range of values from about A to about Z. Similarly, it is envisioned that disclosure of two or more ranges of values for a parameter (whether such ranges are nested, overlapping or distinct) subsume all possible combination of ranges for the value that might be claimed using endpoints of the disclosed ranges. For example, if Parameter X is exemplified herein to have values in the range of 1-10, or 2-9, or 3-8, it is also envisioned that Parameter X may have other ranges of values including 1-9, 1-8, 1-3, 1-2, 2-10, 2-8, 2-3, 3-10, and 3-9.

"A" and "an" as used herein indicate "at least one" of the item is present; a plurality of such items may be present, when possible.

"About" when applied to values indicates that the calculation or the measurement allows some slight imprecision in the value (with some approach to exactness in the value; approximately or reasonably close to the value; nearly). If, for some reason, the imprecision provided by "about" is not otherwise understood in the art with this ordinary meaning, then "about" as used herein indicates at least variations that may arise from ordinary methods of measuring or using such parameters.

As used herein, the term "and/or" includes any and all combinations of one or more of the associated listed items.

As used herein, a virus that has "reduced levels of neurotoxicity" or "reduced neurotoxicity" would be understood to refer to a virus that, when injected into the right striatum of a mouse brain at a given dose, results in a mouse with fewer signs of neurotoxicity (for example, weight loss, piloerection, hind leg paralysis, morbidity and mortality) than a mouse which is injected with the corresponding wild-type virus.

As used herein, a virus having "substantially no level of neurotoxicity" or "substantially no neurotoxicity" would be understood to refer to a virus that, when injected into a patient at an efficacious dose, results in no detectable signs of reduced motor function compared to the patient before injection with the virus using a standard protocol for that a patient of that species. For example, a virus having "substantially no neurotoxicity" would be understood to refer to a virus that, when injected into a mouse at 1e7 pfu results in a mouse with no detectable signs of reduced motor function as measured by time on a rotorod, compared to the mouse before injection with the virus.

### Detailed Description

Of the more than 250 currently identified rhabdoviruses, the authors of the present disclosure tested several wild type rhabdoviruses and determined many to be effective at killing CNS tumour cell lines. Several of these potent viral isolates were also determined to demonstrate remarkable attenuation, resulting in 100% survival after intracerebral inoculation. This is in striking contrast to previously tested Maraba and VSV viruses. The authors of the present disclosure subsequently sequenced and engineered chimeric viruses to test alongside known non-neurotoxic wild type isolates.

Generally, the present disclosure provides systems, methods, uses, processes, articles, and compositions that relate to engineered chimeric Maraba rhabdoviruses, and related nucleotide and protein sequences thereof. For example, the present disclosure provides the use of chimeric Maraba rhabdovirus in oncolytic treatments, for example treatment of primary or secondary brain cancers.

Contemplated oncolytic viruses may be used to treat cancer by directly administering the virus to a patient, or by infecting a cell with the virus and administering the infected cell to the patient to deliver the virus. The cell to be infected by the virus may be a cancer cell from the patient, a normal immune cell, or a stem cell. In some examples, the cancer to be treated is brain cancer, such as malignant glioma. One example of a malignant glioma is glioblastoma.

Viral particles according to the present disclosure may contain no wild type plasmid, may contain no sequences which encode a wild-type Maraba G-protein, or both.

In one example of viral particles according to the present invention, there is provided an isolated viral particle having a genome that includes open reading frames that encode: Maraba proteins N, P, and L, or any variants thereof; as well as Maraba protein M or protein Δ51M, or any variants thereof; and a Bahia Grande G protein, a LCMV G protein, or an Ebola G protein.

Maraba protein N may have a sequence which includes SEQ ID NO: 1. Maraba protein P may have a sequence which includes SEQ ID NO: 2. Maraba protein L may have a sequence which includes SEQ ID NO: 3. Maraba proteins M and Δ51M may have sequence which include SEQ ID NO: 4 and 5, respectively. Bahia Grande G protein may have a sequence which includes SEQ ID NO: 6. LCMV G protein may have a sequence which includes SEQ ID NO: 7. Ebola G protein may have a sequence which includes SEQ ID NO: 8.

A variant of a reference protein is be a protein having a sequence which is at least 75%, at least 80%, at least 85%, at least 90%, or at least 95% identical to the sequence of the reference protein, and the variant protein maintains the same biological function as the reference protein. For example, a variant protein would be considered to maintain the same biological function as the reference protein if a viral particle which has been modified with the variant protein had the same cytotoxicity and neurotoxicity as a viral particle with the reference protein.

In a particular example, the isolated viral particle has a genome which includes open reading frames that encode proteins having sequences that include SEQ ID NOs: 1, 2, 3, 4, and 6.

In another example, the isolated viral particle has a genome which includes open reading frames that encode proteins having sequences that include SEQ ID NOs: 1, 2, 3, 4, and 7.

In still another example, the isolated viral particle has a genome which includes open reading frames that encode proteins having sequences that include SEQ ID NOs: 1, 2, 3, 4, and 8.

In a further example, the isolated viral particle has a genome which includes open reading frames that encode proteins having sequences that include SEQ ID NOs: 1, 2, 3, 5, and 6.

In still yet another example, the isolated viral particle has a genome which includes open reading frames that encode proteins having sequences that include SEQ ID NOs: 1, 2, 3, 5, and 7.

In still a further example, the isolated viral particle has a genome which includes open reading frames that encode proteins having sequences that include SEQ ID NOs: 1, 2, 3, 5, and 8.

In another example of viral particles according to the present invention, there is provided an isolated viral particle comprising an RNA polynucleotide which has a sequence that includes: the reverse complement of the sequence defined by position 64 to position 1332 of SEQ ID NO: 10, or a conservative variant thereof; the reverse complement of the sequence defined by position 1393 to position 2190 of SEQ ID NO: 10, or a conservative variant thereof; the reverse complement of the sequence defined by position 4943 to position 11272 of SEQ ID NO: 10, or a conservative variant thereof; the reverse complement of the sequence defined by position 2256 to position 2945 of SEQ ID NO: 10, or a conservative variant thereof; the reverse complement of the sequence defined by position 3041 to position 4816 of SEQ ID NO: 10; and the reverse complements of promoters thereof.

A conservative variant is a sequence that is at least 75%, at least 80%, at least 85%, at least 90%, or at least 95% identical to the reference sequence of nucleotides. A conservative variant may be a sequence comprising one or more silent substitutions.

A particular example of a viral particle according to the present invention is an isolated viral particle that produces a cDNA polynucleotide comprising a sequence according to SEQ ID NO: 9 when the virus is in a host cell.

A particular example of a viral particle according to the present invention is an isolated viral particle comprising an RNA polynucleotide comprising a sequence according to SEQ ID NO: 10.

In another example of viral particles according to the present invention, there is provided an isolated viral particle comprising an RNA polynucleotide which has a sequence that includes: the reverse complement of the sequence defined by position 64 to position 1332 of SEQ ID NO: 12, or a conservative variant thereof; the reverse complement of the sequence defined by position 1393 to position 2190 of SEQ ID NO: 12, or a conservative variant thereof; the reverse complement of the sequence defined by position 4664 to position 10993 of SEQ ID NO: 12, or a conservative variant thereof; the reverse complement of the sequence defined by position 2256 to position 2945 of SEQ ID NO: 12, or a conservative variant thereof; the reverse complement of the sequence defined by position 3041 to position 4537 of SEQ ID NO: 12; and the reverse complements of promoters thereof.

A conservative variant is a sequence that is at least 75%, at least 80%, at least 85%, at least 90%, or at least 95% identical to the reference sequence of nucleotides. A conservative variant may be a sequence comprising one or more silent substitutions.

A particular example of a viral particle according to the present invention is an isolated viral particle that produces a cDNA polynucleotide comprising a sequence according to SEQ ID NO: 11 when the virus is in a host cell.

A particular example of a viral particle according to the present invention is an isolated viral particle comprising an RNA polynucleotide comprising a sequence according to SEQ ID NO: 12.

In another example of viral particles according to the present invention, there is provided an isolated viral particle comprising an RNA polynucleotide which has a sequence that includes: the reverse complement of the sequence defined by position 64 to position 1332 of SEQ ID NO: 14, or a conservative variant thereof; the reverse complement of the sequence defined by position 1393 to position 2190 of SEQ ID NO: 14, or a conservative variant thereof; the reverse complement of the sequence defined by position 5195 to position 11524 of SEQ ID NO: 14, or a conservative variant thereof; the reverse complement of the sequence defined by position 2256 to position 2942 of SEQ ID NO: 14, or a conservative variant thereof; the reverse complement of the sequence defined by position 3038 to position 5068 of SEQ ID NO: 14; and the reverse complements of promoters thereof.

A conservative variant is a sequence that is at least 75%, at least 80%, at least 85%, at least 90%, or at least 95% identical to the reference sequence of nucleotides. A conservative variant may be a sequence comprising one or more silent substitutions.

A particular example of a viral particle according to the present invention is an isolated viral particle that produces a cDNA polynucleotide comprising a sequence according to SEQ ID NO: 13 when the virus is in a host cell.

A particular example of a viral particle according to the present invention is an isolated viral particle comprising an RNA polynucleotide comprising a sequence according to SEQ ID NO: 14.

According to another aspect of the present invention, an isolated viral particle according to the present invention may be used for the treatment of cancer. The cancer may be a brain cancer, for example a glioblastoma.

The isolated viral particle maybe used to infect a cell and the infected cell may be used for the treatment of cancer.

It is also disclosed an isolated viral particle according to the present disclosure to induce a cytotoxic response in a person administered the virus. The cytotoxic response may be an anti-cancer response. The isolated viral particle may be used to infect a cell and the infected cell maybe used to generate the cytotoxic response.

The isolated viral particle may be formulated for direct delivery to the central nervous system, outside the blood/brain barrier, inside the blood/brain barrier, or any combination thereof. The isolated viral particle may be formulated for administration via intrathecal injection, intravenous injection, intracranial injection, or any sequential or simultaneous combination thereof.

The infected cell may be formulated for direct delivery to the central nervous system, outside the blood/brain barrier, inside the blood/brain barrier, or any combination thereof. The infected cell may be formulated for administration via intrathecal injection, intravenous injection, intracranial injection, or any sequential or simultaneous combination thereof.

The isolated viral particle may be administered to the patient directly. The isolated viral particle may be administered directly to the central nervous system, outside the blood/brain barrier, inside the blood/brain barrier, or any combination thereof. The isolated viral particle may be administered to the patient intrathecally, intravenously, via intracranial injection, or any combination thereof sequentially or simultaneously.

The use may include infecting a cell with the isolated viral particle and administering the infected cell to the patient. The infected cell may be administered directly to the central nervous system, outside the blood/brain barrier, inside the blood/brain barrier, or any combination thereof. The infected cell may be administered to the patient intrathecally, intravenously, via intracranial injection, or any combination thereof sequentially or simultaneously.

It is also disclosed a method for inducing a cytotoxic response in a patient which includes administering an isolated viral particle according to the present disclosure to the patient.

The isolated viral particle may be administered to the patient directly. The isolated viral particle may be administered directly to the central nervous system, outside the blood/brain barrier, inside the blood/brain barrier, or any combination thereof. The isolated viral particle may be administered to the patient intrathecally, intravenously, via intracranial injection, or any combination thereof sequentially or simultaneously.

The method disclosed herein may include infecting a cell with the isolated viral particle and administering the infected cell to the patient. The infected cell may be administered directly to the central nervous system, outside the blood/brain barrier, inside the blood/brain barrier, or any combination thereof. The infected cell may be administered to the patient intrathecally, intravenously, via intracranial injection, or any combination thereof sequentially or simultaneously.

According to another aspect of the present invention, there is provided a kit for the treatment of cancer in a patient. The kit includes an isolated viral particle according to the present invention and instructions for administration of the isolated viral particle to the patient.

The cancer may be a brain cancer, for example a glioblastoma.

The isolated viral particle may be formulated for direct delivery to the central nervous system, outside the blood/brain barrier, inside the blood/brain barrier, or any combination thereof. The isolated viral particle maybe formulated for administration via intrathecal injection, intravenous injection, intracranial injection, or any sequential or simultaneous combination thereof.

The isolated viral particle may be formulated for infection of a cell and the cell is for delivery to the central nervous system, outside the blood/brain barrier, inside the blood/brain barrier, or any combination thereof. The cell may be formulated for administration via intrathecal injection, intravenous injection, intracranial injection, or any sequential or simultaneous combination thereof.

In any of the above aspects, administration via one route may be combined with one or more other routes of administration. Administration of the viral particle via the different routes may be sequential and/or simultaneous. The route or mode of administration of a virus according to the present disclosure is not expected to affect the ability of the virus to infect and kill cancerous cells, regardless of whether the virus is administered directly or by first infecting a cell and administering the infected cell to the patient. Viruses according to the present disclosure, when administered either inside or outside the blood/brain, are expected to be able to cross the blood/brain barrier and infect cancerous cells on the other side of the blood/brain barrier.

Techniques for infecting a cell with a virus and using the infected cell to deliver the virus are discussed in, for example: Power AT, et al. Carrier cell-based delivery of an oncolytic virus circumvents antiviral immunity. Mol Ther. 2007 Jan;15(1):123-30; and Tyler MA, et al. Neural stem cells target intracranial glioma to deliver an oncolytic adenovirus in vivo. Gene Ther. 2009 Feb;16(2):262-78.

### Polynucleotide and Amino Acid Sequences

Polynucleotides comprising nucleic acid sequences (e.g., DNA and RNA) and amino acid (e.g., protein) sequences are provided that may be used in a variety of methods and techniques known to those skilled in the art of molecular biology. These include isolated, purified, and recombinant forms of the listed sequences and further include complete or partial forms of the listed sequences. Non-limiting uses for amino acid sequences include making antibodies to proteins or peptides comprising the disclosed amino acid sequences. Non-limiting uses for the polynucleotide sequences include making hybridization probes, as primers for use in the polymerase chain reaction (PCR), for chromosome and gene mapping, and the like. Complete or partial amino acid or polynucleotide sequences can be used in such methods and techniques.

The present disclosure features the identification of polynucleotide sequences, including gene sequences and coding nucleic acid sequences, and amino acid sequences. In addition to the sequences expressly provided in the accompanying sequence listing, also included are polynucleotide sequences that are related structurally and/or functionally. Also included are polynucleotide sequences that hybridize under stringent conditions to any of the polynucleotide sequences in the sequence listing, or a subsequence thereof (e.g., a subsequence comprising at least 100 contiguous nucleotides). Polynucleotide sequences also include sequences and/or subsequences configured for RNA production and/or translation, e.g., mRNA, antisense RNA, sense RNA, RNA silencing and interference configurations, etc.

Polynucleotide sequences that are substantially identical to those provided in the sequence listing can be used in the compositions and methods disclosed herein. Substantially identical or substantially similar polynucleotide sequences are defined as polynucleotide sequences that are identical, on a nucleotide by nucleotide basis, with at least a subsequence of a reference polynucleotide. Such polynucleotides can include, e.g., insertions, deletions, and substitutions relative to any of those listed in the sequence listing. For example, such polynucleotides are typically at least about 75% identical to a reference polynucleotide selected from those in the sequence listing, or a subsequence thereof. Furthermore, such sequences can be at least about 75%, at least about 80%, at least about 85%, at least about 90%, at least about 95%, at least about 98%, at least about 99%, or at least about 99.5%, identical to the reference sequence. Subsequences of these polynucleotides can include at least about 5, at least about 10, at least about 15, at least about 20, at least about 25, at least about 50, at least about 75, at least about 100, at least about 500, about 1000 or more, contiguous nucleotides or complementary subsequences. Such subsequences can be, e.g., oligonucleotides, such as synthetic oligonucleotides, isolated oligonucleotides, or full-length genes or cDNAs. Polynucleotide sequences complementary to any of the described sequences are included.

Amino acid sequences include the amino acid sequences represented in the sequence listing, and subsequences thereof. Also included are amino acid sequences that are highly related structurally and/or functionally. For example, in addition to the amino acid sequences in the sequence listing, amino acid sequences that are substantially identical can be used in the disclosed compositions and methods. Substantially identical or substantially similar amino acid sequences are defined as amino acid sequences that are identical, on an amino acid by amino acid basis, with at least a subsequence of a reference amino acid sequence. Such amino acid sequences can include, e.g., insertions, deletions, and substitutions relative to any of the amino acid sequences in the sequence listing. For example, such amino acids are typically at least about 75% identical to a reference amino acid sequence, or a subsequence thereof. Frequently, such amino acid sequences are at least about 75%, at least about 80%, at least about 85%, at least about 90%, at least about 95%, at least about 98%, at least about 99%, or at least about 99.5%, identical to the reference sequence. Subsequences of the amino acid sequences can include at least about 5, at least about 10, at least about 15, at least about 20, at least about 25, at least about 50, at least about 75, at least about 100, at least about 500, about 1000 or more, contiguous amino acids. Conservative variants of amino acid sequences or subsequences are also possible. Amino acid sequences can be cytotoxic, enzymatically active, enzymatically inactive, and the like.

Where the polynucleotide sequences are translated to form a polypeptide or subsequence of a polypeptide, nucleotide changes can result in either conservative or non-conservative amino acid substitutions. Conservative amino acid substitutions refer to the interchangeability of residues having functionally similar side chains. Conservative substitution tables providing functionally similar amino acids are well-known in the art. Table 1 sets forth examples of six groups containing amino acids that are "conservative substitutions" for one another. Other conservative substitution charts are available in the art, and can be used in a similar manner.

**Table 1**

| Conservative Substitution Group | | | | |
|---|---|---|---|---|
| 1 | Alanine (A) | Serine (S) | Threonine (T) | |
| 2 | Aspartic acid (D) | Glutamic acid(E) | | |
| 3 | Asparagine (N) | Glutamine (Q) | | |
| 4 | Arginine (R) | Lysine (K) | | |
| 5 | Isoleucine (I) | Leucine (L) | Methionine (M) | Valine (V) |
| 6 | Phenylalanine (F) | Tyrosine (Y) | Tryptophan (W) | |

One of skill in the art will appreciate that many conservative substitutions yield functionally identical constructs. For example, as discussed above, owing to the degeneracy of the genetic code, "silent substitutions" (i.e., substitutions in a polynucleotide sequence which do not result in an alteration in an encoded polypeptide) are an implied feature of every polynucleotide sequence which encodes an amino acid. Similarly, "conservative amino acid substitutions," in one or a few amino acids in an amino acid sequence (e.g., about 1%, 2%, 3%, 4%, 5%, 6%, 7%, 8%, 9%, 10% or more) are substituted with different amino acids with highly similar properties, are also readily identified as being highly similar to a disclosed construct. Such conservative variations of each disclosed sequence are also contemplated.

Methods for obtaining conservative variants, as well as more divergent versions of the polynucleotide and amino acid sequences, are widely known in the art. In addition to naturally occurring homologues which can be obtained, e.g., by screening genomic or expression libraries according to any of a variety of well-established protocols, see, e.g., Ausubel et al. Current Protocols in Molecular Biology (supplemented through 2004) John Wiley & Sons, New York ("Ausubel"); Sambrook et al. Molecular Cloning - A Laboratory Manual (2nd Ed.), Vol. 1-3, Cold Spring Harbor Laboratory, Cold Spring Harbor, N.Y., 1989 ("Sambrook"), and Berger and Kimmel Guide to Molecular Cloning Techniques, Methods in Enzymology volume 152 Academic Press, Inc., San Diego, Calif. ("Berger"), additional variants can be produced by any of a variety of mutagenesis procedures. Many such procedures are known in the art, including site directed mutagenesis, oligonucleotide-directed mutagenesis, and many others. For example, site directed mutagenesis is described, e.g., in Smith (1985) "In vitro mutagenesis" Ann. Rev. Genet. 19:423-462, and references therein, Botstein & Shortle (1985) "Strategies and applications of in vitro mutagenesis" Science 229:1193-1201; and Carter (1986) "Site-directed mutagenesis" Biochem. J. 237:1-7. Oligonucleotide-directed mutagenesis is described, e.g., in Zoller & Smith (1982) "Oligonucleotide-directed mutagenesis using M13-derived vectors: an efficient and general procedure for the production of point mutations in any DNA fragment" Nucleic Acids Res. 10:6487-6500). Mutagenesis using modified bases is described e.g., in Kunkel (1985) "Rapid and efficient site-specific mutagenesis without phenotypic selection" Proc. Natl. Acad. Sci. USA 82:488-492, and Taylor et al. (1985) "The rapid generation of oligonucleotide-directed mutations at high frequency using phosphorothioate-modified DNA" Nucl. Acids Res. 13: 8765-8787. Mutagenesis using gapped duplex DNA is described, e.g., in Kramer et al. (1984) "The gapped duplex DNA approach to oligonucleotide-directed mutation construction" Nucl. Acids Res. 12: 9441-9460). Point mismatch mutagenesis is described, e.g., by Kramer et al. (1984) "Point Mismatch Repair" Cell 38:879-887). Double-strand break mutagenesis is described, e.g., in Mandecki (1986) "Oligonucleotide-directed double-strand break repair in plasmids of Escherichia coli: a method for site-specific mutagenesis" Proc. Natl. Acad. Sci. USA, 83:7177-7181, and in Arnold (1993) "Protein engineering for unusual environments" Current Opinion in Biotechnology 4:450-455). Mutagenesis using repair-deficient host strains is described, e.g., in Carter et al. (1985) "Improved oligonucleotide site-directed mutagenesis using M13 vectors" Nucl. Acids Res. 13: 4431-4443. Mutagenesis by total gene synthesis is described e.g., by Nambiar et al. (1984) "Total synthesis and cloning of a gene coding for the ribonuclease S protein" Science 223: 1299-1301. DNA shuffling is described, e.g., by Stemmer (1994) "Rapid evolution of a protein in vitro by DNA shuffling" Nature 370:389-391, and Stemmer (1994) "DNA shuffling by random fragmentation and reassembly: In vitro recombination for molecular evolution," Proc. Natl. Acad. Sci. USA 91:10747-10751.

Many of the above methods are further described in Methods in Enzymology Volume 154, which also describes useful controls for trouble-shooting problems with various mutagenesis methods. Kits for mutagenesis, library construction and other diversity generation methods are also commercially available. For example, kits are available from, e.g., Amersham International pic (Piscataway, N.J.) (e.g., using the Eckstein method above), Bio/Can Scientific (Mississauga, Ontario, CANADA), Bio-Rad (Hercules, Calif.) (e.g., using the Kunkel method described above), Boehringer Mannheim Corp. (Ridgefield, Conn.), Clonetech Laboratories of BD Biosciences (Palo Alto, Calif.), DNA Technologies (Gaithersburg, Md.), Epicentre Technologies (Madison, Wis.) (e.g., the 5 prime 3 prime kit); Genpak Inc. (Stony Brook, N.Y.), Lemargo Inc (Toronto, CANADA), Invitrogen Life Technologies (Carlsbad, Calif.), New England Biolabs (Beverly, Mass.), Pharmacia Biotech (Peapack, N.J.), Promega Corp. (Madison, Wis.), QBiogene (Carlsbad, Calif.), and Stratagene (La Jolla, Calif.) (e.g., QuickChange™ site-directed mutagenesis kit and Chameleon™ double-stranded, site-directed mutagenesis kit).

### Determining Sequence Relationships

Similar sequences can be objectively determined by any number of methods, e.g., percent identity, hybridization, immunologically, and the like. A variety of methods for determining relationships between two or more sequences (e.g., identity, similarity and/or homology) are available and well-known in the art. Methods include manual alignment, computer assisted sequence alignment, and combinations thereof, for example. A number of algorithms (which are generally computer implemented) for performing sequence alignment are widely available or can be produced by one of skill. These methods include, e.g., the local homology algorithm of Smith and Waterman (1981) Adv. Appl. Math. 2:482; the homology alignment algorithm of Needleman and Wunsch (1970) J. Mol. Biol. 48:443; the search for similarity method of Pearson and Lipman (1988) Proc. Natl. Acad. Sci. (USA) 85:2444; and/or by computerized implementations of these algorithms (e.g., GAP, BESTFIT, FASTA, and TFASTA in the Wisconsin Genetics Software Package Release 7.0, Genetics Computer Group, 575 Science Dr., Madison, Wis.).

For example, software for performing sequence identity (and sequence similarity) analysis using the BLAST algorithm is described in Altschul et al. (1990) J. Mol. Biol. 215:403-410. This software is publicly available, e.g., through the National Center for Biotechnology Information on the internet at ncbi.nlm.nih.gov. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold. These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP (BLAST Protein) program uses as defaults a wordlength (W) of 3, an expectation (E) of 10, and the BLOSUM62 scoring matrix (see, Henikoff & Henikoff (1989) Proc. Natl. Acad. Sci. USA 89:10915).

Additionally, the BLAST algorithm performs a statistical analysis of the similarity between two sequences (see, e.g., Karlin & Altschul (1993) Proc. Nat'l. Acad. Sci. USA 90:5873-5787). One measure of similarity provided by the BLAST algorithm is the smallest sum probability (p(N)), which provides an indication of the probability by which a match between two nucleotide or amino acid sequences would occur by chance. For example, a nucleic acid is considered similar to a reference sequence (and, therefore, in this context, homologous) if the smallest sum probability in a comparison of the test nucleic acid to the reference nucleic acid is less than about 0.1, or less than about 0.01, and or even less than about 0.001.

Another example of a sequence alignment algorithm is PILEUP, which creates a multiple sequence alignment from a group of related sequences using progressive, pairwise alignments. It can also plot a tree showing the clustering relationships used to create the alignment. PILEUP uses a simplification of the progressive alignment method of Feng & Doolittle (1987) J. Mol. Evol. 35:351-360. The method used is similar to the method described by Higgins & Sharp (1989) CABIOS5:151-153. The program can align, e.g., up to 300 sequences of a maximum length of 5,000 letters. The multiple alignment procedure begins with the pairwise alignment of the two most similar sequences, producing a cluster of two aligned sequences. This cluster can then be aligned to the next most related sequence or cluster of aligned sequences. Two clusters of sequences can be aligned by a simple extension of the pairwise alignment of two individual sequences. The final alignment is achieved by a series of progressive, pairwise alignments. The program can also be used to plot a dendogram or tree representation of clustering relationships. The program is run by designating specific sequences and their amino acid or nucleotide coordinates for regions of sequence comparison.

An additional example of an algorithm that is suitable for multiple DNA, or amino acid, sequence alignments is the CLUSTALW program (Thompson, J. D. et al. (1994) Nucl. Acids. Res. 22: 4673-4680). CLUSTALW performs multiple pairwise comparisons between groups of sequences and assembles them into a multiple alignment based on homology. Gap open and Gap extension penalties can be, e.g., 10 and 0.05 respectively. For amino acid alignments, the BLOSUM algorithm can be used as a protein weight matrix. See, e.g., Henikoff and Henikoff (1992) Proc. Natl. Acad. Sci. USA 89: 10915-10919.

Polynucleotide hybridization similarity can also be evaluated by hybridization between single stranded (or single stranded regions of) nucleic acids with complementary or partially complementary polynucleotide sequences. Hybridization is a measure of the physical association between nucleic acids, typically, in solution, or with one of the nucleic acid strands immobilized on a solid support, e.g., a membrane, a bead, a chip, a filter, etc. Nucleic acid hybridization occurs based on a variety of well characterized physico-chemical forces, such as hydrogen bonding, solvent exclusion, base stacking, and the like. Numerous protocols for nucleic acid hybridization are well-known in the art. An extensive guide to the hybridization of nucleic acids is found in Tijssen (1993) Laboratory Techniques in Biochemistry and Molecular BiologyHybridization with Nucleic Acid Probes, part I, chapter 2, "Overview of principles of hybridization and the strategy of nucleic acid probe assays," (Elsevier, N.Y.), as well as in Ausubel et al. Current Protocols in Molecular Biology (supplemented through 2004) John Wiley & Sons, New York ("Ausubel"); Sambrook et al. Molecular Cloning - A Laboratory Manual (2nd Ed.), Vol. 1-3, Cold Spring Harbor Laboratory, Cold Spring Harbor, N.Y., 1989 ("Sambrook"), and Berger and Kimmel Guide to Molecular Cloning Techniques, Methods in Enzymology volume 152 Academic Press, Inc., San Diego, Calif. ("Berger"). Hames and Higgins (1995) Gene Probes 1, IRL Press at Oxford University Press, Oxford, England (Hames and Higgins 1) and Hames and Higgins (1995) Gene Probes 2, IRL Press at Oxford University Press, Oxford, England (Hames and Higgins 2) provide details on the synthesis, labeling, detection and quantification of DNA and RNA, including oligonucleotides.

Conditions suitable for obtaining hybridization, including differential hybridization, are selected according to the theoretical melting temperature (Tm) between complementary and partially complementary nucleic acids. Under a given set of conditions, e.g., solvent composition, ionic strength, etc., the. Tm is the temperature at which the duplex between the hybridizing nucleic acid strands is 50% denatured. That is, the Tm corresponds to the temperature corresponding to the midpoint in transition from helix to random coil; it depends on the length of the polynucleotides, nucleotide composition, and ionic strength, for long stretches of nucleotides.

After hybridization, unhybridized nucleic acids can be removed by a series of washes, the stringency of which can be adjusted depending upon the desired results. Low stringency washing conditions (e.g., using higher salt and lower temperature) increase sensitivity, but can produce nonspecific hybridization signals and high background signals. Higher stringency conditions (e.g., using lower salt and higher temperature that is closer to the T.sub.m) lower the background signal, typically with primarily the specific signal remaining, See, also, Rapley, R. and Walker, J. M. eds., Molecular Biomethods Handbook (Humana Press, Inc. 1998).

"Stringent hybridization wash conditions" or "stringent conditions" in the context of nucleic acid hybridization experiments, such as Southern and northern hybridizations, are sequence dependent, and are different under different environmental parameters. An extensive guide to the hybridization of nucleic acids is found in Tijssen (1993), supra, and in Hames and Higgins 1 and Hames and Higgins 2, supra.

An example of stringent hybridization conditions for hybridization of complementary nucleic acids which have more than 100 complementary residues on a filter in a Southern or northern blot is 2×SSC, 50% formamide at 42°C, with the hybridization being carried out overnight (e.g., for approximately 20 hours). An example of stringent wash conditions is a 0.2×SSC wash at 65°C for 15 minutes (see Sambrook, supra for a description of SSC buffer). Often, the wash determining the stringency is preceded by a low stringency wash to remove signal due to residual unhybridized probe. An example low stringency wash is 2×SSC at room temperature (e.g., 20° C for 15 minutes).

In general, a signal to noise ratio of at least 2.5×-5× (and typically higher) than that observed for an unrelated probe in the particular hybridization assay indicates detection of a specific hybridization. Detection of at least stringent hybridization between two sequences indicates relatively strong structural similarity to those provided in the sequence listings herein.

Generally, "highly stringent" hybridization and wash conditions are selected to be about 5°C or less lower than the thermal melting point (Tm) for the specific sequence at a defined ionic strength and pH (as noted below, highly stringent conditions can also be referred to in comparative terms). Target sequences that are closely related or identical to the nucleotide sequence of interest (e.g., "probe") can be identified under stringent or highly stringent conditions. Lower stringency conditions are appropriate for sequences that are less complementary.

For example, in determining stringent or highly stringent hybridization (or even more stringent hybridization) and wash conditions, the stringency of the hybridization and wash conditions is gradually increased (e.g., by increasing temperature, decreasing salt concentration, increasing detergent concentration, and/or increasing the concentration of organic solvents, such as formamide, in the hybridization or wash), until a selected set of criteria are met. For example, the stringency of the hybridization and wash conditions is gradually increased until a probe comprising one or more of the present polynucleotide sequences, or a subsequence thereof, and/or complementary polynucleotide sequences thereof, binds to a perfectly matched complementary target, with a signal to noise ratio that is at least 2.5×, and optionally 5×, or 10×, or 100× or more, as high as that observed for hybridization of the probe to an unmatched target, as desired.

Using subsequences derived from the nucleic acids listed in the sequence listing, target nucleic acids can be obtained; such target nucleic acids are also a feature of the current disclosure. For example, such target nucleic acids include sequences that hybridize under stringent conditions to an oligonucleotide probe that corresponds to a unique subsequence of any of the polynucleotides in the sequence listing, or a complementary sequence thereof; the probe optionally encodes a unique subsequence in any of the amino acid sequences of the sequence listing.

For example, hybridization conditions are chosen under which a target oligonucleotide that is perfectly complementary to the oligonucleotide probe hybridizes to the probe with at least about a 5-10× higher signal to noise ratio than for hybridization of the target oligonucleotide to a negative control non-complimentary nucleic acid. Higher ratios of signal to noise can be achieved by increasing the stringency of the hybridization conditions such that ratios of about 15×, 20×, 30×, 50× or more are obtained. The particular signal will depend on the label used in the relevant assay, e.g., a fluorescent label, a calorimetric label, a radioactive label, or the like.

### Vectors, Promoters and Expression Systems

Polynucleotide sequences of the present disclosure can be in any of a variety of forms, e.g., expression cassettes, vectors, plasmids, viral particles, or linear nucleic acid sequences. For example, vectors, plasmids, cosmids, bacterial artificial chromosomes (BACs), YACs (yeast artificial chromosomes), phage, viruses and nucleic acid segments can comprise the present nucleic acid sequences or subsequences thereof. These nucleic acid constructs can further include promoters, enhancers, polylinkers, regulatory genes, etc. Thus, the present disclosure also relates, e.g., to vectors comprising the polynucleotides disclosed herein, host cells that incorporate these vectors, and the production of the various disclosed polypeptides (including those in the sequence listing) by recombinant techniques.

In accordance with these aspects, the vector may be, for example, a plasmid vector, a single or double-stranded phage vector, or a single or double-stranded RNA or DNA viral vector. Such vectors may be introduced into cells as polynucleotides, preferably DNA, by well-known techniques for introducing DNA and RNA into cells. The vectors, in the case of phage and viral vectors, also may be and preferably are introduced into cells as packaged or encapsidated virus by well-known techniques for infection and transduction. Viral vectors may be replication competent or replication defective. In the latter case, viral propagation generally will occur only in complementing host cells.

In some examples, vectors include those useful for expression of polynucleotides and polypeptides of the present disclosure. Generally, such vectors comprise cis-acting control regions effective for expression in a host, operably linked to the polynucleotide to be expressed. Appropriate trans-acting factors are supplied by the host, supplied by a complementing vector or supplied by the vector itself upon introduction into the host.

In certain examples in this regard, the vectors provide for protein expression. Such preferred expression may be inducible expression, temporally limited expression, or expression restricted to predominantly certain types of cells, or any combination of the above. Some embodiments of inducible vectors can be induced for expression by environmental factors that are easy to manipulate, such as temperature and nutrient additives. A variety of vectors suitable to this aspect, including constitutive and inducible expression vectors for use in prokaryotic and eukaryotic hosts, are well-known and employed routinely by those of skill in the art. Such vectors include, among others, chromosomal, episomal and virus-derived vectors, e.g., vectors derived from bacterial plasmids, from bacteriophage, from transposons, from yeast episomes, from insertion elements, from yeast chromosomal elements, from viruses such as rhabdoviruses, baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids and binaries used for Agrobacterium-mediated transformations.

Vectors can include a selectable marker and a reporter gene. For ease of obtaining sufficient quantities of vector, a bacterial origin that allows replication in E. coli can be used. The following vectors, which are commercially available, are provided by way of example. Among vectors preferred for use in bacteria are pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia. Among preferred eukaryotic vectors are pWLNEO, pSV2CAT, pOG44, pXT1 and pSG available from Stratagene; and pSVK3, pBPV, pMSG and pSVL available from Pharmacia. Useful plant binary vectors include BIN19 and its derivatives available from Clontech. These vectors are listed solely by way of illustration of the many commercially available and well-known vectors that are available to those of skill in the art. It will be appreciated that any other plasmid or vector suitable for, for example, introduction, maintenance, propagation or expression of one or more polynucleotides and/or polypeptides as provided in the present sequence listing, including variants thereof as described, in a host may be used.

In general, expression constructs will contain sites for transcription initiation and termination, and, in the transcribed region, a ribosome-binding site for translation when the construct encodes a polypeptide. The coding portion of the mature transcripts expressed by the constructs will include a translation-initiating AUG at the beginning and a termination codon appropriately positioned at the end of the polypeptide to be translated. In addition, the constructs may contain control regions that regulate as well as engender expression. Generally, in accordance with many commonly practiced procedures, such regions will operate by controlling transcription, such as transcription factors, repressor binding sites and termination signals, among others. For secretion of a translated protein into the lumen of the endoplasmic reticulum, into the periplasmic space or into the extracellular environment, appropriate secretion signals may be incorporated into the expressed polypeptide. These signals may be endogenous to the polypeptide or they may be heterologous signals.

Transcription of the DNA (e.g., encoding the polypeptides) of the present disclosure by higher eukaryotes may be increased by inserting an enhancer sequence into the vector. Enhancers are cis-acting elements of DNA, usually about from 10 to 300 bp that act to increase transcriptional activity of a promoter in a given host cell-type. Examples of enhancers include the SV40 enhancer, which is located on the late side of the replication origin at bp 100 to 270, the cytomegalovirus early promoter enhancer, the polyoma enhancer on the late side of the replication origin, and adenovirus enhancers. Additional enhancers useful in the disclosure to increase transcription of the introduced DNA segment, include, inter alia, viral enhancers like those within the 35S promoter, as shown by Odell et al., Plant Mol. Biol. 10:263-72 (1988), and an enhancer from an opine gene as described by Fromm et al., Plant Cell 1:977 (1989). The enhancer may affect the tissue-specificity and/or temporal specificity of expression of sequences included in the vector.

Termination regions also facilitate effective expression by ending transcription at appropriate points. Useful terminators include, but are not limited to, pinll (see An et al., Plant Cell 1(1):115-122 (1989)), glb1 (see Genbank Accession #L22345), gz (see gzw64a terminator, Genbank Accession #S78780), and the nos terminator from Agrobacterium. The termination region can be native with the promoter nucleotide sequence, can be native with the DNA sequence of interest, or can be derived from another source. For example, other convenient termination regions are available from the Ti-plasmid of A. tumefaciens, such as the octopine synthase and nopaline synthase termination regions. See also: Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. 1989) Nucleic Acids Res. 17:7891-7903; and Joshi et al. (1987) Nucleic Acid Res. 15:9627 -9639.

Among known eukaryotic promoters suitable for generalized expression are the CMV immediate early promoter, the HSV thymidine kinase promoter, the early and late SV40 promoters, the promoters of retroviral LTRs, such as those of the Rous sarcoma virus ("RSV"), metallothionein promoters, such as the mouse metallothionein-I promoter and various plant promoters, such as globulin-1. The native promoters of the polynucleotide sequences listing in the sequence listing may also be used. Representatives of prokaryotic promoters include the phage lambda PL promoter, the E. coli lac, trp and tac promoters to name just a few of the well-known promoters.

Isolated or recombinant viruses, virus infected cells, or cells including one or more portions of the present polynucleotide sequences and/or expressing one or more portions of the present amino acid sequences are also contemplated.

A polynucleotide, optionally encoding the heterologous structural sequence of an amino acid sequence as disclosed, generally will be inserted into a vector using standard techniques so that it is operably linked to a promoter for expression. Operably linked, as used herein, includes reference to a functional linkage between a promoter and a second sequence, wherein the promoter sequence initiates and mediates transcription of the DNA corresponding to the second sequence. Generally, operably linked means that the polynucleotide sequence being linked is contiguous and, where necessary to join two protein coding regions, contiguous and in the same reading frame. When the polynucleotide is intended for expression of a polypeptide, the polynucleotide will be positioned so that the transcription start site is located appropriately 5' to a ribosome binding site. The ribosome-binding site will be 5' to the AUG that initiates translation of the polypeptide to be expressed. Generally, there will be no other open reading frames that begin with an initiation codon, usually AUG, and lie between the ribosome binding site and the initiation codon. Also, generally, there will be a translation stop codon at the end of the polypeptide and there will be a polyadenylation signal in constructs for use in eukaryotic hosts. Transcription termination signals appropriately disposed at the 3' end of the transcribed region may also be included in the polynucleotide construct.

For nucleic acid constructs designed to express a polypeptide, the expression cassettes can additionally contain 5' leader sequences. Such leader sequences can act to enhance translation. Translation leaders are known in the art and include: picornavirus leaders, for example: EMCV leader (Encephalomyocarditis 5' noncoding region), Elroy-Stein et al. (1989) Proc. Nat. Acad. Sci. USA 86:6126-6130; potyvirus leaders, for example, TEV leader (Tobacco Etch Virus), Allison et al. (1986); MDMV leader (Maize Dwarf Mosaic Virus), Virology 154:9-20; human immunoglobulin heavy-chain binding protein (BiP), Macejak et al. (1991) Nature 353:90-94; untranslated leader from the coat protein mRNA of alfalfa mosaic virus (AMV RNA 4), Jobling et al. (1987) Nature 325:622-625); tobacco mosaic virus leader (TMV), Gallie et al. (1989) Molecular Biology of RNA, pages 237-256; and maize chlorotic mottle virus leader (MCMV) Lommel et al. (1991) Virology 81:382-385. See also Della-Cioppa et al. (1987) Plant Physiology 84:965-968. The cassette can also contain sequences that enhance translation and/or mRNA stability such as introns. The expression cassette can also include, at the 3' terminus of the isolated nucleotide sequence of interest, a translational termination region.

In those instances where it is desirable to have the expressed product of the polynucleotide sequence directed to a particular organelle or secreted at the cell's surface the expression cassette can further comprise a coding sequence for a transit peptide. Such transit peptides are well-known in the art and include, but are not limited to: the transit peptide for the acyl carrier protein, the small subunit of RUBISCO, plant EPSP synthase, and the like.

In making an expression cassette, the various DNA fragments can be manipulated so as to provide for the polynucleotide sequences in the proper orientation and, as appropriate, in the proper reading frame. Toward this end, adapters or linkers can be employed to join DNA fragments or other manipulations can be involved to provide for convenient restriction sites, removal of superfluous DNA, removal of restriction sites, or the like. For this purpose, in vitro mutagenesis, primer repair, restriction digests, annealing, and resubstitutions such as transitions and transversions, can be employed.

Introduction of a construct into a host cell can be effected by calcium phosphate transfection, DEAE-dextran mediated transfection, microinjection, cationic lipid-mediated transfection, electroporation, transduction, scrape loading, ballistic introduction, infection or other methods. Such methods are described in many standard laboratory manuals, such as Davis et al., Basic Methods in Molecular Biology, (1986) and Sambrook et al., Molecular Cloning - A Laboratory Manual, 2nd Ed., Cold Spring Harbor Laboratory Press, Cold Spring Harbor, N.Y. (1989).

Representative examples of appropriate hosts include bacterial cells, such as streptococci, staphylococci, E. coli, streptomyces and Salmonella typhimurium cells; fungal cells, such as yeast cells and Aspergillus cells; insect cells such as Drosophila S2 and Spodoptera Sf9 cells; animal cells such as CHO, COS and Bowes melanoma cells; and plant cells.

The host cells can be cultured in conventional nutrient media, which may be modified as appropriate for, inter alia, activating promoters, selecting transformants or amplifying genes. Culture conditions, such as temperature, pH and the like, previously used with the host cell selected for expression generally will be suitable for expression of nucleic acids and/or polypeptides, as will be apparent to those of skill in the art. Mature proteins can be expressed in mammalian cells, yeast, bacteria, or other cells under the control of appropriate promoters. Cell-free translation systems can also be employed to produce such proteins using RNAs derived from the polynucleotides disclosed herein.

Following transformation of a suitable host strain and growth of the host strain to an appropriate cell density, where the selected promoter is inducible it is induced by appropriate means (e.g., temperature shift or exposure to chemical inducer) and cells are cultured for an additional period. Cells typically then are harvested by centrifugation, disrupted by physical or chemical means, and the resulting crude extract retained for further purification. Microbial cells employed in expression of proteins can be disrupted by any convenient method, including freeze-thaw cycling, sonication, mechanical disruption, or use of cell lysing agents; such methods are well-known to those skilled in the art.

Compositions and methods of the present disclosure can include administering the polynucleotides and/or amino acids as provided herein. For example, treatments for glioblastoma can include administering one or more of the polynucleotides and/or amino acids. The one or more polynucleotides and/or amino acids may be in an isolated form or may be part of a composition, including a viral particle. In various embodiments, the administering can take the following forms: intradermal, transdermal, parenteral, intravascular, intravenous, intramuscular, intranasal, subcutaneous, regional, percutaneous, intratracheal, intraperitoneal, intraarterial, intravesical, intratumoral, inhalation, perfusion, lavage, direct injection, alimentary, oral, or intracranial administration. The mode of administration may depend on

### Examples

### Example 1: Identification of Non-Neurotoxic Rhabdoviruses and In Vitro Cytotoxicity.

To determine in vivo neurotoxicity: groups of 6-8 weeks old female BALB//c mice (n = 3/group) received a single intracranial (IC) injection of the indicated viruses at 1e7 pfu. Following IC injection, mice were monitored daily for signs of distress including weight loss, piloerection, hind-limb paralysis and respiratory distress.

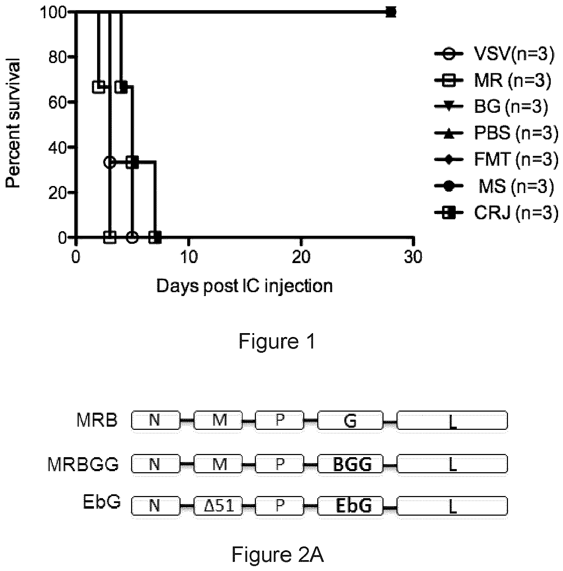

Figure 1 shows the survival of BALB/c mice after a single IC dose of the indicated virus (1e7 pfu). Animals treated with IC injection of VSV, Maraba Virus (MR) or Carajas Virus (CRJ) survived less than 10 days while control animals (PBS) and all other animals injected IC with Farmington (FMT), Bahia Grande (BG) and Muir Springs (MS) showed 100% survival out to 30 days post IC injection indicative of their non-neurotoxic potential. Kaplan Meier survival plots were compared using Mantel-Cox Log rank analysis (Graphpad Prism).

In addition to exploring the oncolytic potential of wild-type FMT, BG and MS, the authors of the present disclosure reasoned that generating chimeric viruses of maraba virus (MRB) and a non-neurotoxic virus (for example, BG) would result in a virus with both desirable properties. The glycoprotein from BG was swapped into MRB, creating a chimeric Maraba virus with BG glycoprotein, termed "Maraba BGG" or "MRBGG" or "MRB-BG(G) or variations thereof, and including the RNA sequence which is the reverse complement of SEQ ID NO: 10. Rhabdoviruses, such as Maraba virus, carry their genetic material in the form of negative-sense single-stranded RNA. The RNA sequences disclosed herein correspond to RNA strands which encode the viral genetic material and are, therefore, the reverse complement of the genetic RNA which are carried by the rhabdoviruses.

The genome of the Maraba MGG viral particle has open reading frames that encode Maraba proteins N, P, and L; as well as Maraba protein M; and Bahia Grande G protein. The Maraba protein N has a sequence which corresponds to SEQ ID NO: 1. The Maraba protein P has a sequence which corresponds to SEQ ID NO: 2. The Maraba protein L has a sequence which corresponds to SEQ ID NO: 3. The Maraba protein M has a sequence which corresponds to SEQ ID NO: 4. The Bahia Grande G protein has a sequence which corresponds to SEQ ID NO: 6.

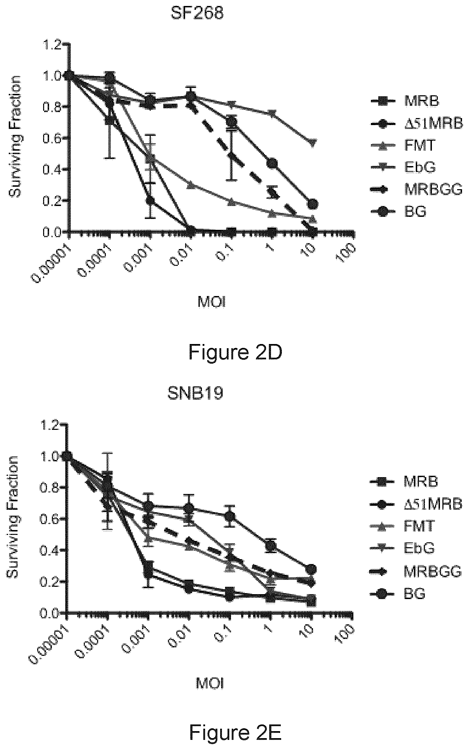

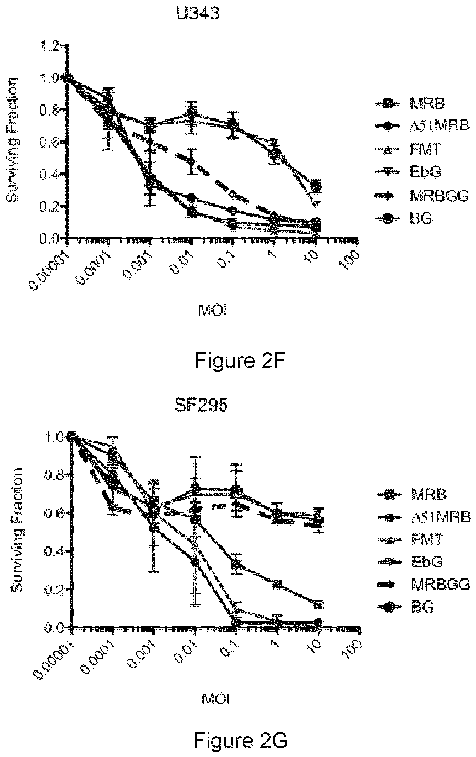

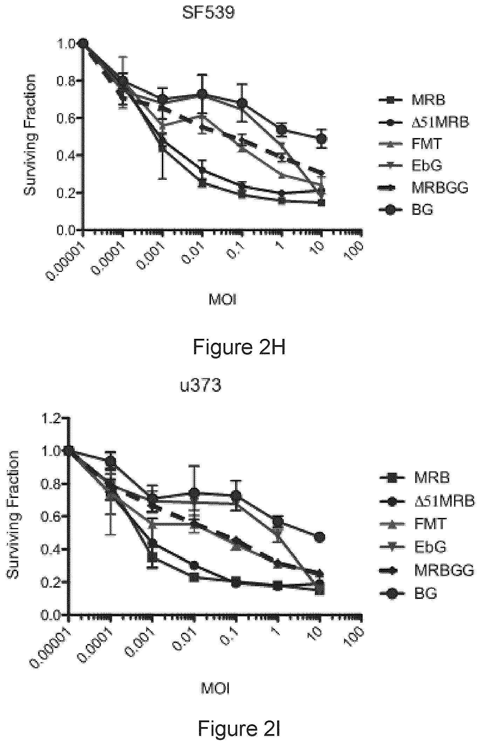

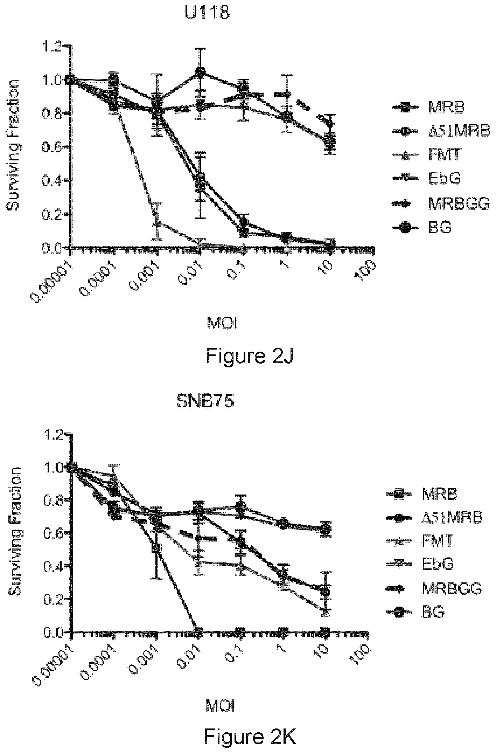

Another chimeric virus was produced by swapping out the MRB G glycoprotein for the Ebola glycoprotein, this time into the more attenuated Maraba vector (Δ51MRB) to create a chimeric virus, termed "Maraba EbG" or "EbG" or variations thereof, and including the RNA sequence which is the reverse complement of SEQ ID NO: 14 (see Figure 2A). The genome of the Maraba EbG viral particle has open reading frames that encode Maraba proteins N, P, and L; as well as Maraba protein Δ51M; and Ebola G protein. The Maraba protein N has a sequence which corresponds to SEQ ID NO: 1. The Maraba protein P has a sequence which corresponds to SEQ ID NO: 2. The Maraba protein L has a sequence which corresponds to SEQ ID NO: 3. The Maraba protein Δ51M has a sequence which corresponds to SEQ ID NO: 5. The Ebola G protein has a sequence which corresponds to SEQ ID NO: 8.

The authors of the present disclosure hypothesized that the Maraba EbG variant would increase the therapeutic window for the chimeric virus in a replicating oncolytic rhabdovirus (Figure 2A) since it has been previously demonstrated that a lentiviral vector pseudotyped with Ebola-Zaire glycoprotein resulted in no viral transduction of the mouse CNS while retaining the ability to transduce 293T cancer cell line (see Watson, D.J., Kobinger, G.P., Passini, M.A., Wilson, J.M. & Wolfe, J.H. Targeted transduction patterns in the mouse brain by lentivirus vectors pseudotyped with VSV, Ebola, Mokola, LCMV, or MuLV envelope proteins. Mol Ther 5, 528-537 (2002); and Watson, D.J., Passini, M.A. & Wolfe, J.H. Transduction of the choroid plexus and ependyma in neonatal mouse brain by vesicular stomatitis virus glycoprotein-pseudotyped lentivirus and adeno-associated virus type 5 vectors. Hum Gene Ther 16, 49-56 (2005)).

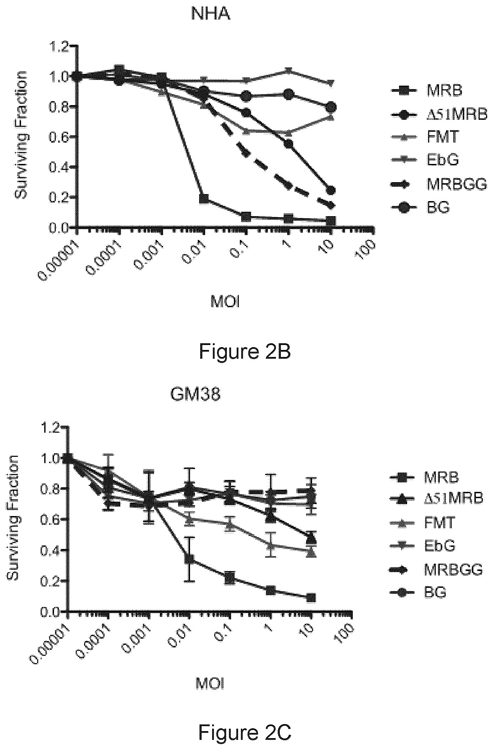

To test the killing capacity of these chimeric viruses, as compared to wild type isolates, cell killing assays were performed on 2 normal human diploid cell lines primary normal human astrocytes (NHA) and primary fibroblasts (GM38) (Figures 2B and 2C) and a panel of 8 CNS tumour cell lines SF268, SNB19, U118, U343, SF295, SNB75, SF539 and U373 (Figures 2D through 2K).

Cells were acquired from the National Institute of General Medical Sciense Mutant Cell Repository, Camden, NJ and were propagated in Dulbecco's modified Eagle's medium (Hyclone, Logan, UT) supplemented with 10% fetal calf serum (Cansera, Etobicoke, Ontario, Canada). Viability Assays were performed with the indicated cell lines as follows: Cells were plated at a density of 10 000 cells/well into 96 well plates and infected the next day with either: wild-type Maraba, wild type FMT, wild type BG, attenuated Maraba, Maraba EbG, or Maraba BGG at various multiplicity of infections (0.0001-10 pfu/cell).

Following a 48 hour incubation, Alamar Blue (Resazurin sodium salt (Sigma-Aldrich) was added to a final concentration of 20 µg/ml. After a 6 hour incubation the absorbance was read at a wavelength of 573 nm. While wild type Maraba was very potent against all of the GBM cell lines, it was also highly lytic against both NHA and GM38. In contrast, Maraba EbG and wild-type BG demonstrated significant selective killing of tumour cell lines at MOIs (10 pfu) that were innocuous to normal cells (NHA and GM38). The chimeric virus "MRBGG", demonstrated greater potency than Maraba EbG or wild-type BG against the majority of GBM cell lines, while remaining very safe in normal fibroblasts. Wild type FMT demonstrated the greatest therapeutic index, with potency rivaling MRB in the majority of GBM lines while remaining highly attenuated in NHA and GM38 primary cell lines. This demonstrates that wild type FMT, and Maraba viruses engineered to be chimeric for BG or Ebola glycoproteins, show potent and selective oncolytic activity when tested against brain cancer cell lines.

### Example 2: In Vivo Safety of two Maraba Virus Chimeras

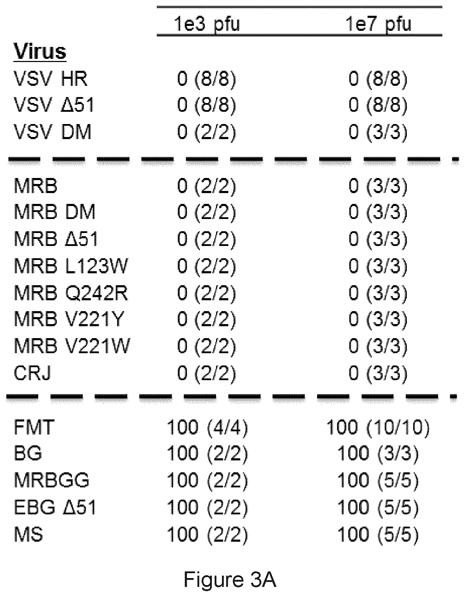

The wild type isolates (FMT, BG and MS) and the two chimeric viruses (EbG and MRBGG) which demonstrated attenuation in non-transformed cells *in vitro* (see Example 1), were tested to ascertain whether the observed attenuation translates to safety *in vivo.* Animals were administered two doses intracerebrally, a low (1e3 pfu) and high dose (1e7 pfu) of these viruses (Figure 3A).

All 5 viruses were found to be safe, with 100% of the animals surviving 100 days post treatment with no persistent infection. At these doses, animals displayed transient weight loss and piloerection which is consistent with viral infection, but these symptoms resolved within 5-7 days post inoculation. In contrast, all animals that received similar IC doses of wild type or attenuated Maraba and VSV strains succumbed to infection within a week (Figure 3A). These animals displayed clinical signs of a CNS infection with rapid and progressive weight loss, hind leg paralysis and had significant titres of virus in their brain just prior to death (data not shown).

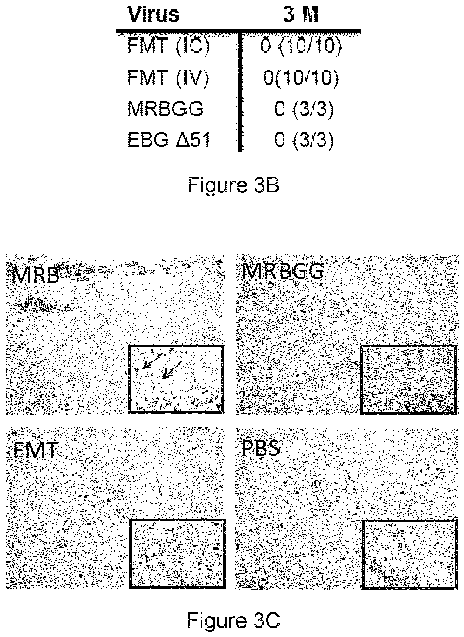

Viral titres were determined by plaque assay on animal brains 3 months after treatment with wild type FMT (IC and IV) and the chimeric Maraba viruses (EbG and MRBGG). Plaque assays were performed with Vero cells plated at a density of 5e5 cells per/well of a 6 well dish. The next day 100 µl of serial viral dilutions were prepared and added for 1 hour to Vero cells. After viral adsorption, 2 ml of agarose overlay was added (1:1 1% agarose: 2X DMEM and 20% FCS) and plaques were counted the following day. No virus was detected in animal brains 3 months post IC infection (Figure 3B).

In addition, following administration of high doses of FMT (1e7 pfu) and MRBGG (1e7 pfu) in the brain, no signs of cell death or inflammatory responses were found comparable to those of saline injected control mice (Figure 3C). This differed dramatically from wild-type MRB injected animals, which displayed a striking increase in inflammatory cells, condensed nuclei, and a perforated morphology.

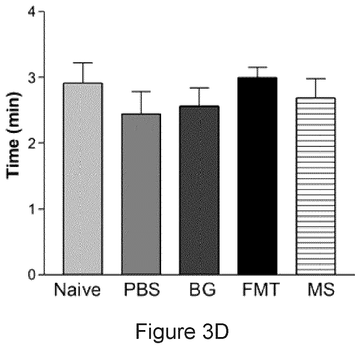

Although no acute neurotoxicity resulted from IC treatment with FMT, BG, or MS, an assessment of their cognitive and motor function was performed several days after virus infection. Motor function was assessed before and after treatment with these 3 wild type viruses (Figure 3D). Balb/C mice were tested for motor function/performance on a rotating rod apparatus prior to IC viral administration. Mice were placed on a rotorod for 3 trials per day for 4 consecutive days. After allowing the animals 0.5 min to adjust to the apparatus, the rod was accelerated in a linear fashion at 0.1 rpm/s. Latency to fall was measured in minutes and animals were divided into groups of 3. Motor function was assessed one week post injection in Naive (uninjected), PBS, FMT, Maraba EbG, BG, MRBGG and MS IC treated animals. Standard error of the mean was calculated. Specifically, there is no significant difference in the latency to fall between the mock-infected animals or virus infected animals, 1 week prior and 1 week post injection (Figure 3D).

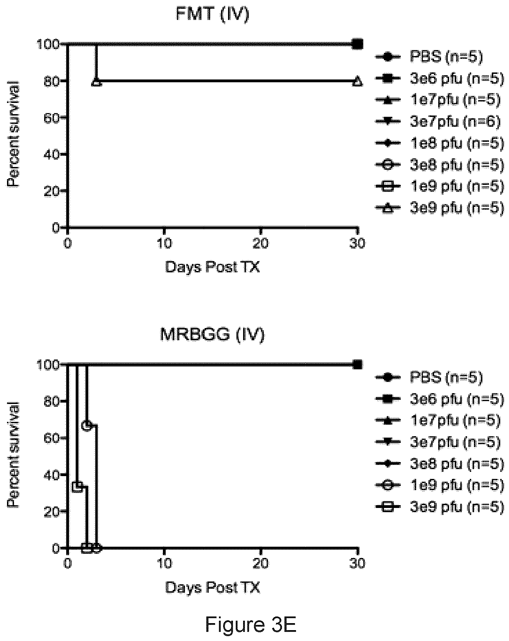

In addition to intracranial toxicity, the toxicity of FMT and MRBGG was evaluated when administered intravenously (IV) in immunocompetent mice with escalating doses of virus (Figure 3E). MRBGG is tolerated up to a dose of 3e8 pfu, which demonstrates IV safety that is one order of magnitude safer than published results of wild type Maraba. FMT is well tolerated IV and never reaches an LD50 even at our highest dose 3e9 pfu which is comparable to an attenuated version of Maraba as previously described (Brun, J. et al. Identification of Genetically Modified Maraba Virus as an Oncolytic Rhabdovirus. Mol Ther 18, 1440 (2010)). FMT animals IV dosed at greater than 3e8 pfu displayed transient weight loss and moderate piloerection, which resolved 5-7 days post treatment (data not shown).

### Example 3: In Vivo Efficacy of Maraba Virus Chimeras

The *in vivo* efficacy of chimeric Maraba viruses was also determined in mouse models of glioblastoma. The sensitivity of the human glioblastoma cell line U87MG to viral infection in vitro was determined. FMT and wild type Maraba were equally potent at killing U87MG cells with an EC50 score of less than 0.001 multiplicities of infection (data not shown). Maraba virus chimeras (Maraba EbG, Maraba BGG) and BG wild-type were also potent at killing U87MG cells in vitro with an EC50 score of less than 0.1 multiplicities of infection (data not shown).

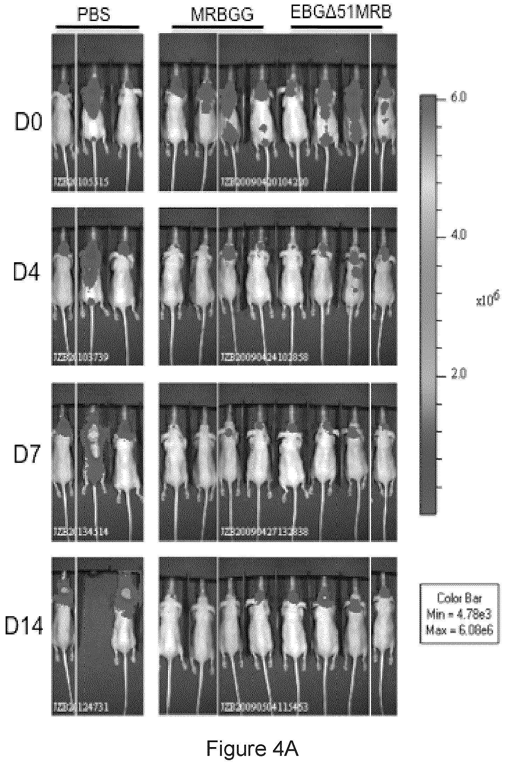

After adapting human U87MG glioma cells for bioluminescent imaging, an intracerebral U87MG glioma model in athymic mice was established and IV efficacy of Maraba virus chimeras according to the present invention was examined in this model (Figure 4 A-C). In the human glioblastoma xenograft model human, glioblastoma U87MG cells were adapted for bioluminescent imaging by transducing with lentivirus containing firefly luciferase (FLUC) and transfecting FLUC plasmid respectively. U87MG FLUC cells were injected IC into CD1 nude mice. Untreated CD-1 animals develop tumours at about day 15-21.

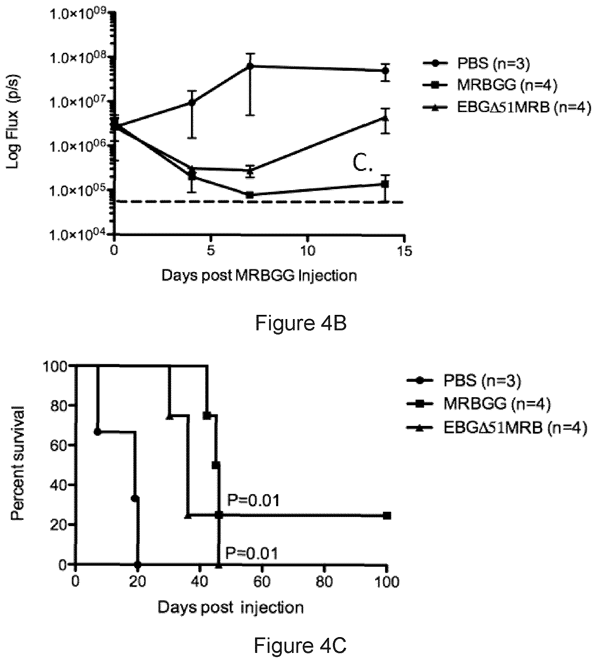

Animals with FLUC expressing tumours were monitored for tumour progression using the live imaging IVIS Xenogen 200 system after an IP injection of luciferin (Gold Biotechnology Inc). The animals were monitored for signs of distress including survival, weight loss, morbidity, piloerection, hind-limb paralysis and respiratory distress. Three days after the first treatment a significant decrease in tumour burden was observed with a maximal effect observed by day 7 (Figure 4 A & B). However by day 14 tumors were starting to recur. Also observed was a delay in time to death following intravenous treatment with Maraba virus chimeras (Figure 4C). Interestingly, the spinal metastases in Maraba virus chimera treated animals in this model are completely cleared in all tumour bearing animals. In contrast, animals treated with UV inactivated virus had a significant increase in tumour burden by day 7 at which point they started exhibiting neurological symptoms from their brain tumours. All IV treated animals responded to treatment with 3 of 8 durably cured and surviving beyond 100 days post treatment.

### Example 4: Exploring Other Maraba Virus Chimeras

Vesicular stomatitis virus (VSV) is a potent oncolytic rhabdovirus. However, neurotropism with subsequent neurovirulence, as well as a highly potent nAb response are problems associated with VSV treatment. The inherent neurotoxicity has hindered its consideration as a clinical candidate.

The inherent neurotoxicity is thought to be mediated by its glycoprotein (VSV-G). However, lentiviral vectors that typically use VSV-G have had their neurotoxicity attenuated through pseudotyping with the lymphocytic choriomeningitis virus G protein (LCMV-G) (Beyer et al., J Virol 76:1488-1495, 2002; and U.S. Patent Publication No. 2011/0250188 to Von Laer). LCMV is a prototypical member of the arenavirus family of enveloped negative sense RNA viruses. The authors of the present disclosure hypothesized that the neurotoxicity of the Maraba virus may be attenuated through replacement of its glycoprotein (Maraba-G protein) with LCMV-G protein. A chimeric Maraba virus having LCMV-G protein was produced by swapping out the MRB G glycoprotein for the LCMV glycoprotein to create a chimeric virus, termed "Maraba LCMV-G" or "Maraba LCMV(G)", and including the RNA sequence which is the reverse complement of SEQ ID NO: 12 (see Figure 2A).

The genome of the Maraba LCMV-G viral particle has open reading frames that encode Maraba proteins N, P, and L; as well as Maraba protein M; and LCMV-G protein. The Maraba protein N has a sequence which corresponds to SEQ ID NO: 1. The Maraba protein P has a sequence which corresponds to SEQ ID NO: 2. The Maraba protein L has a sequence which corresponds to SEQ ID NO: 3. The Maraba protein M has a sequence which corresponds to SEQ ID NO: 4. The LCMV-G protein has a sequence which corresponds to SEQ ID NO: 7.

Manufacturing and rescuing Maraba chimeric viruses was performed as follows: A plasmid encoding the wildtype recombinant Maraba virus genome (Brun et al., 2010) was modified by standard DNA cloning methods so that the Maraba glycoprotein sequence was replaced with the glycoprotein sequences from Bahia Grande Virus, Leukocytic Choriomenigitis Virus (LCMV) or Farmington Virus. Briefly, a NotI restriction site was introduced by PCR-based mutageneis directly after the stop codon in the Maraba G sequence. Using this newly introduced NotI site and existing KpnI site between the M and G protein sequences, Maraba G was removed by restriction digest to generate pMRB(-G)-KpnI/NotI. Primers to amplify the glycoprotein sequences of both Farmington and Bahia Grande were designed to introduce 5' KpnI and 3' NotI restriction sites. These sequences were amplified by PCR and ligated into pMRB(-G)-KpnI/NotI. The LCMV glycoprotein precursor sequence (GenBank EF164923.1) was synthesized with 5' KpnI and 3' NotI sites introduced (Integrated DNA Technologies, Coralville, IA). This DNA fragment was ligated into the above-described pMRB(-G)-KpnI/NotI, becoming pMRB-LCMV-G, pMRB-BG-G and pMRB-FMT-G.

Additionally, the recombinant genome of the Farmington Virus was modified, replacing wild-type Farmington glycoprotein with the Maraba glycoprotein, as described in PCT Application No. PCT/CA2012/050385 and in a similar manner to creating the Maraba glycoprotein variants described above.

Recombinant Maraba virus particles [MRBGG, MRB FMTG, MRB LCMVG] were generated using techniques described previously (Brun et al., 2010) from the modified Maraba genomic plasmids described above. Briefly, A549 cells were infected at an MOI of 10 with T7 RNA polymerase-expressing vaccinia virus for 1.5 h. Cells were subsequently transfected by lipofectamine 2000 with above-described modified recombinant Maraba genomic plasmids together with pCI-Neo constructs encoding the Maraba N, P and L proteins. Forty-eight hours after transfection the media was removed, filtered through a 0.2 µm filter and the filtrate used to infect SNB19 cells. Cytopathic effect was observed in successful rescues after forty-eight hours and the virus was then plaque purified three times on Vero cells. FMT-MRB-G virus was generated in a similar fashion as above except that the initial transfection contained pFMT-MRB-G and PCI-Neo constructs encoding Farmington N, P and L proteins.

Recombinant viruses underwent three rounds of plaque purification (on SNB19 cells), before scale up, purification on sucrose cushion, and resuspension in PBS containing 15% glucose.

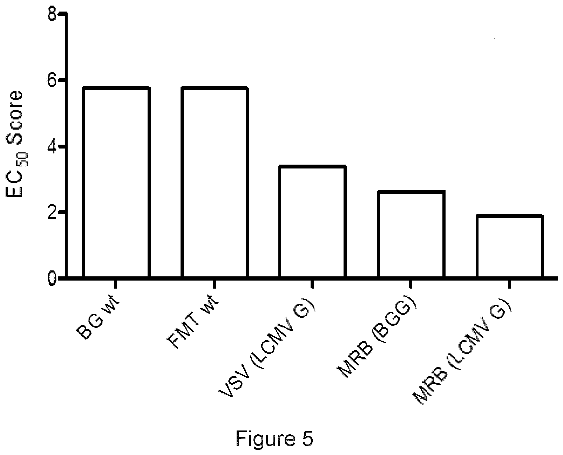

The relative cytotoxicity of a variety of viruses on a panel of human glioblastoma (Astrocytoma) cells (U87MG, SF268, U118, U373, U343, SNB19, 2 primary patient GBM cell samples) was determined. The indicated cell lines were seeded into 96 well plates (1e4 cells/well). The next day cells were infected with the indicated viruses: wild type BG, wild type FMT, VSV LCMVG ("VSV (LCMV G)"), MRB BGG ("MRB (BGG)"), or MRB LCMVG ("MRB (LCMV G)") at various MOIs (0.0001-10 pfu/cell). Following a 96 hour incubation, Alamar Blue (Resazurin sodium salt (Sigma-Aldrich)) was added to a final concentration of 20 µg/ml. After a 6 hour incubation the absorbance was read at a wavelength of 573 nm. Cell metabolic viability was plotted and the multiplicity of infection (MOI) EC50 values were determined and then scored in ranges as follows: 1= MOI <0.01; 2= MOI <0.1; 3= MOI <1; 4=MOI <10; 5= MOI >10; 6=resistant. The average of the EC50 score for all 8 glioma lines was plotted for each virus (Figure 5). The MRB-LCMVG chimera displayed the lowest EC50 value (and therefore highest potency with respect to oncolytic activity against brain cancer cell lines) versus MRBBG and VSV-LCMVG chimeras or wild type non-neurotoxic BG and FMT viruses.

### Example 5: In Vivo Safety of Other Rhabdovirus Chimeras

To determine *in vivo* neurotoxicity: groups of 6-8 weeks old female BALB//c mice (n = 2 to 10/group) received a single intracranial (IC) injection of the indicated viruses at 1e7 pfu. After administration of general anaesthetic (isoflurane), mice were prepared for surgery by shaving heads, applying chlorhexidine disinfectant to scalp, covering eyes with antibiotic ointment and applying a topical anaesthetic to ears. Mice were then placed onto a stereotaxic mount and immobilized using ear bars. With a scalpel blade, a 0.5 cm incision down the midline of the scalp was made to expose the top of the skull. Using a disposable 23G needle, a hole on the right side of the skull, approximately 0.5 mm above the coronal suture and 2mm from the sagittal suture, was made. A 10 µL glass Hamilton syringe was loaded with virus diluted in phosphate buffered saline (PBS) and mounted on the stereotaxic syringe pump. The needle was inserted to a depth of 4 mm and after 30 seconds was withdrawn by 0.5mm. The virus (dose 1e7 pfu) was then infused into the brain at a rate of 5 µL/minute. After a subsequent 30 second wait time, the needle was withdrawn, the scalp glued together with veterinary adhesive and the animal was allowed to recover from general anaesthetic in an infant incubator. Mice received follow-up pain control (buprenorphine) for 72 h post surgery during which time body mass was measured and wellness assessments were made every 12 h.

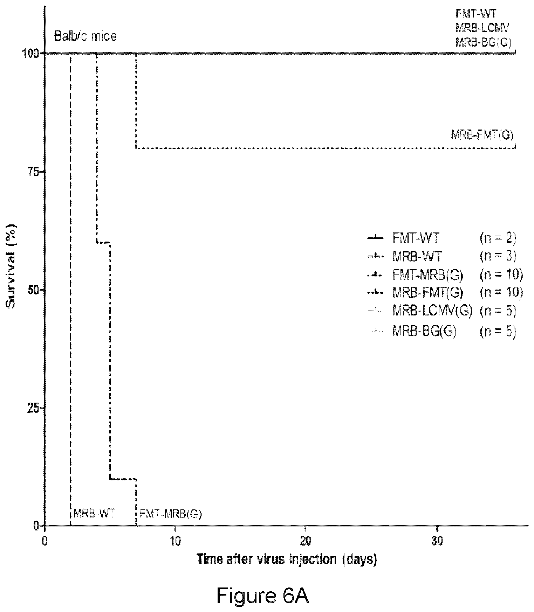

Figure 6A shows Kaplan Meier survival plots of BALB/c mice after a single IC dose of the indicated virus (1e7 pfu). The survival plots were compared using Mantel-Cox Log rank analysis (Graphpad Prism). Animals treated with IC injection of wild-type Maraba Virus (MRB-WT) or chimeric Farmington virus having Maraba-G protein (FMT-MRB(G)) survived less than 10 days while animals injected IC with wild-type Farmington (FMT-WT), Maraba LCMV-G, and Maraba BGG showed 100% survival out to 30 days post IC injection indicative of their non-neurotoxic potential. Chimeric Maraba virus having Farmington G protein (MRB-FMT(G)) showed less than 100% survival at 30 days post IC injection, but increased survival vs. control. Animals treated with chimera MRB-FMT(G) showed an intermediate survival rate due to two mice being euthanized early due to loss of body mass.

The MRB-FMT(G) viral particle produces a cDNA polynucleotide which includes SEQ ID NO: 15 when the virus is in a host cell. The MRB-FMT(G) viral particle includes the RNA sequence which is the reverse complement of SEQ ID NO: 16. The genome of the MRB-FMT(G) virus has open reading frames that encode Maraba proteins N, P, and L; as well as Maraba protein M; and Farmington G protein. The Maraba protein N has a sequence which corresponds to SEQ ID NO: 1. The Maraba protein P has a sequence which corresponds to SEQ ID NO: 2. The Maraba protein L has a sequence which corresponds to SEQ ID NO: 3. The Maraba protein M has a sequence which corresponds to SEQ ID NO: 4. The Farmington-G protein has a sequence which corresponds to SEQ ID NO: 17.

The FMT-MRB(G) viral particle produces a cDNA polynucleotide which includes SEQ ID NO: 18 when the virus is in a host cell. The FMT-MRB(G) viral particle includes the RNA sequence which is the reverse complement of SEQ ID NO: 19. The genome of the FMT-MRB(G) virus has open reading frames that encode Farmington proteins N, P, and L; as well as Farmington protein M; and Maraba G protein. The Farmington protein N has a sequence which corresponds to SEQ ID NO: 20. The Farmington protein P has a sequence which corresponds to SEQ ID NO: 21. The Farmington protein L has a sequence which corresponds to SEQ ID NO: 22. The Farmington protein M has a sequence which corresponds to SEQ ID NO: 23. The Maraba-G protein has a sequence which corresponds to SEQ ID NO: 24.

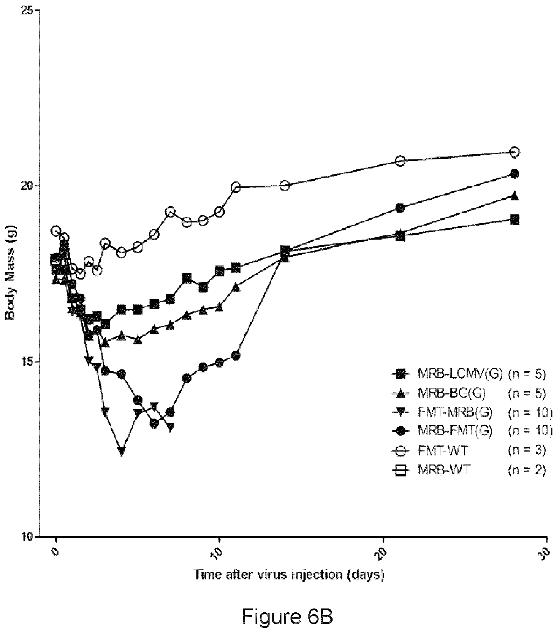

Figure 6B shows the corresponding body mass variations. All animals showed an initial drop in body mass 3-5 days after treatment. In animals treated with an IC injection of wild type FMT, or chimeras MRBGG or MRB LCMVG the drop in body mass was temporary and animals recovered initial body mass between 20-25 days following treatment. The three animals that remained from the group treated with chimera MRB FMTG showed a moderate recovery of body mass in the same time period.

Figures 6A and 6B indicate (i) that Farmington virus, a non-neurotoxic virus, may be made neurotoxic by replacement of its G-protein with the wild type G-protein from Maraba virus, a neurotoxic virus; and (ii) Maraba virus, a neurotoxic virus, is not made non-neurotoxic by replacement of its G-protein with *any* G-protein from a non-neurotoxic virus since replacement with the G-protein from the Farmington virus did not confer non-neurotoxicity (to be clear, Maraba virus is made non-neurotoxic by replacement of its G-protein with *specific* non-neurotoxic G-proteins).

### Example 6: In Vivo Efficacy of Maraba Chimeric Viruses According to the Present Disclosure and Control Viruses

The *in vivo* efficacy of chimeric viruses was also determined in mouse models of glioblastoma. Six to eight week old CD-1 nude mice were injected intracranially with 1e6 U87MG-Fluc cells (human glioblastoma cells transduced with lentivirus to express firefly luciferase), as described above. One week later, mice were imaged using an in-vivo imaging system (Xenogen IVIS 200 Imaging System, Caliper Life Sciences) and sorted so that groups of five had similar levels of firefly luciferase expression from the established tumours in their brains. Briefly, mice were anaesthetized using isoflurane, injected with luciferin solution (2 mg/mouse) and placed into the IVIS machine. Images were taken and luminescence quantified using manufacturers' software (Living Image®, Caliper Life Sciences). The tumour signal from each mouse was normalized to the background signal from that exposure. This pre-treatment value was assigned a value of 100% and all subsequent values were compared to this starting point. The next day, mice were again stereotaxically injected with the indicated virus (dose 1e7 pfu, or phosphate buffered saline as a control), as described previously. Mice were imaged by IVIS at one week intervals for five weeks and during this time, as tumour-related health indicators warranted, mice were humanely euthanized as per institutional guidelines.

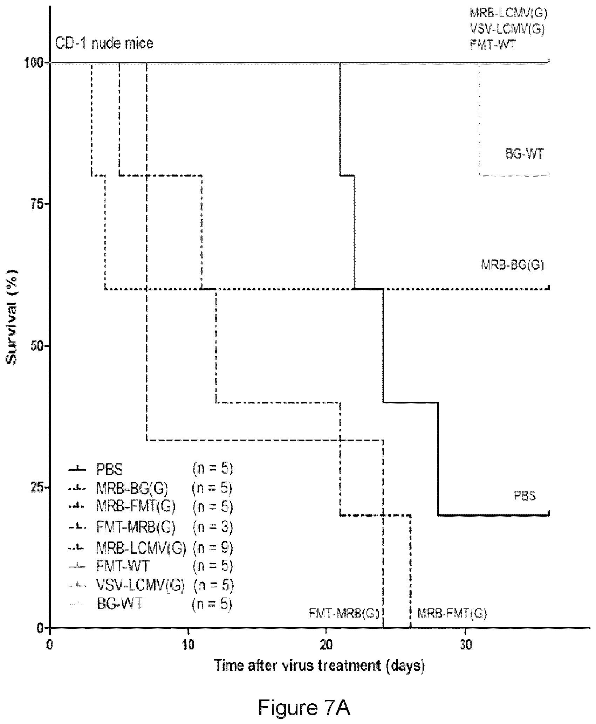

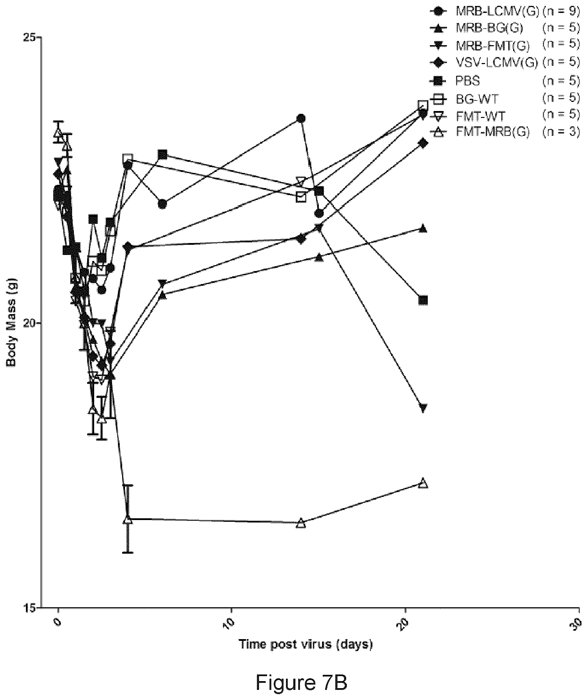

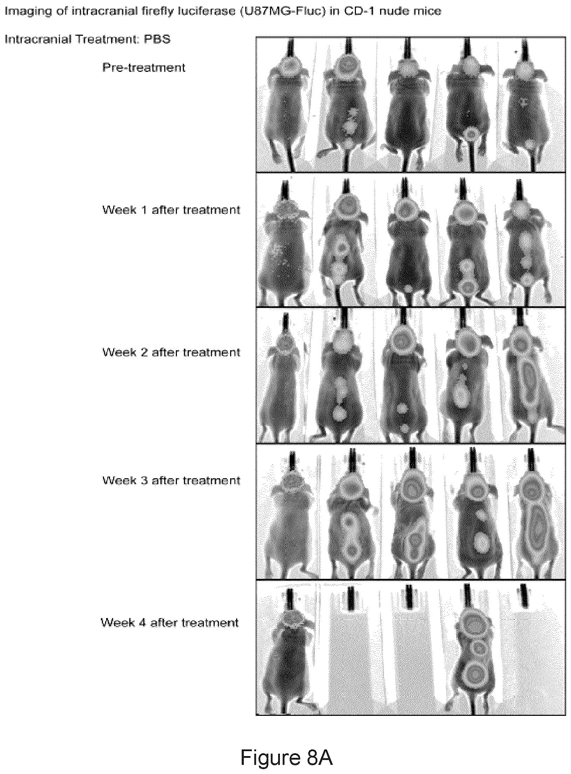

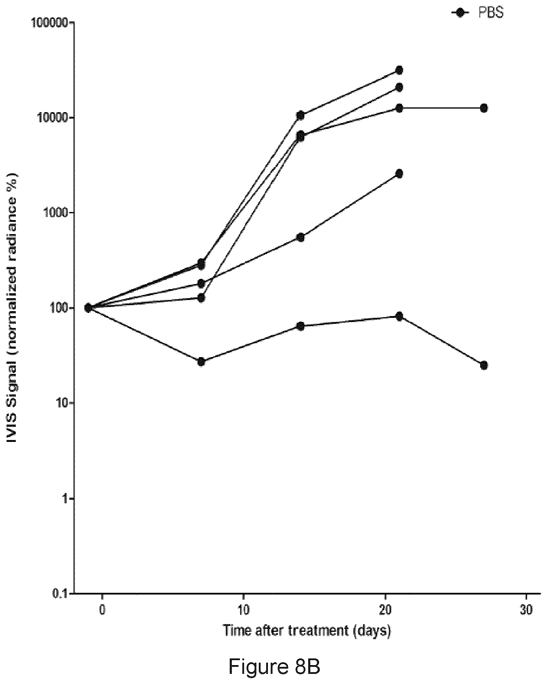

Figure 7A is a graph illustrating in vivo efficacy of maraba chimeras according to the present disclosure versus control viruses. The graph shows Kaplan Meir survival plots of CD-1 nude mice with U87MG tumors post treatment. Animals treated with FMT-MRB(G) and MRB-FMT(G) survived more than 20 days but less than 30 days post IC injection. Animals treated with PBS survived to approximately 30 days post IC injection before succumbing to their tumors. Animals treated with MRB-BGG showed over 50% survival at 30 days post IC injection. Treatment with wild-type BG showed over 75% survival at 30 days post IC injection. Treatment with MRB-LCMV(G), VSV-LCMV(G) and FMT-WT showed 100% survival out to 30 days post IC injection. Figure 7B is a graph showing the weight variation of the animals of Figure 7A. All animals showed an initial drop in body mass 3-5 days after treatment. In animals treated with an IC injection of wild type FMT, BG or chimeras MRB LCMVG or VSV LCMVG the drop in body mass was temporary and animals recovered initial body mass by 20 days following treatment. Animals treated with chimera MRB FMTG or FMT MRBG or PBS controls did not show any recovery of body mass in the same time period. Detailed results are illustrated in Figures 8-15.

Figure 8A is an IVIS image of U87MG tumours illustrating in vivo efficacy of PBS control in a human U87MG xenograft model. The image shows tumours pre and post (1 week, 2 weeks, 3 weeks, 4 weeks) treatment. Figure 8B is a flux plot illustrating a significant increase in tumour burden over time in untreated control animals.

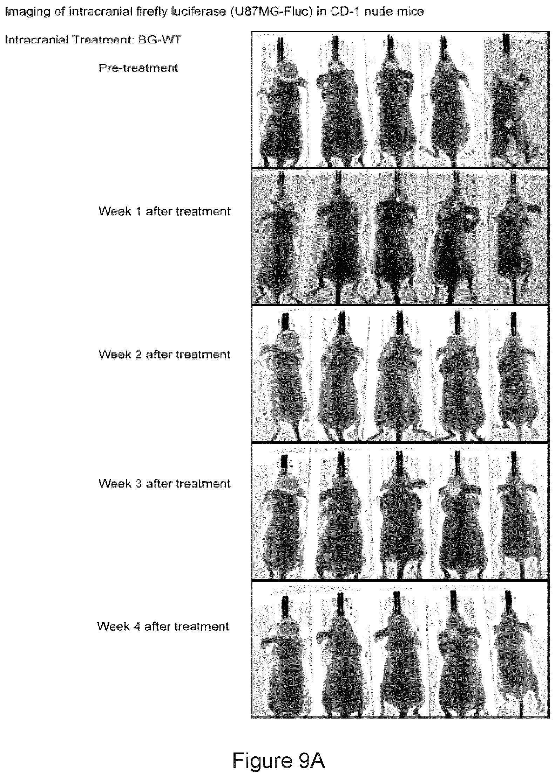

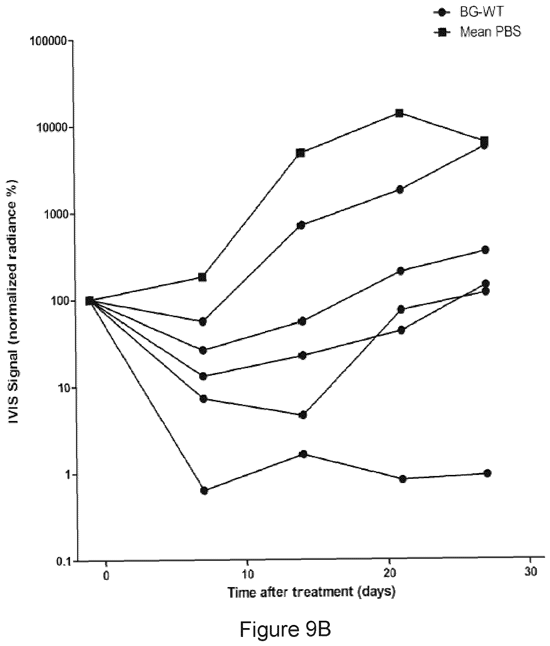

Figure 9A is an IVIS image of U87MG tumours illustrating in vivo efficacy of BG wild type (BG-WT) virus treatment in a human U87MG xenograft model. The image shows U87MG tumours post BG (1 week, 2 weeks, 3 weeks, 4 weeks) treatment (1 dose 1e7 pfu: IC). Figure 9B is a flux plot illustrating an initial moderate tumour regression in response to IC dose (1e7 pfu) of BG followed by a recurrence in tumour burden.

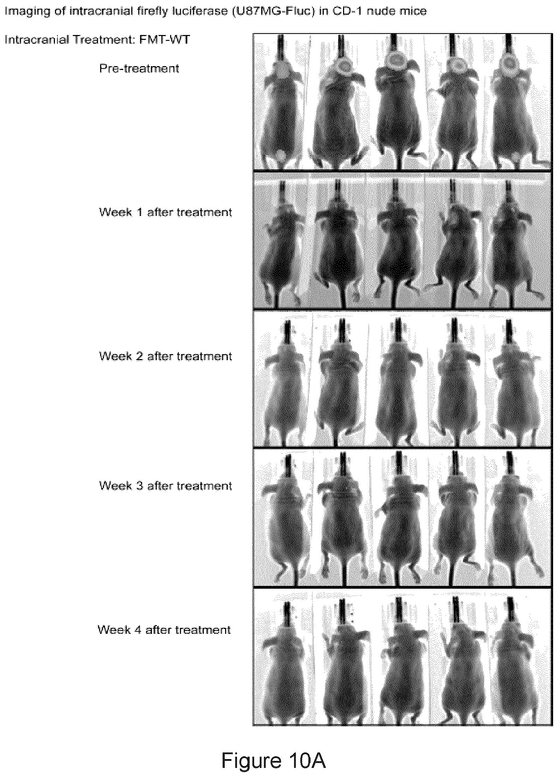

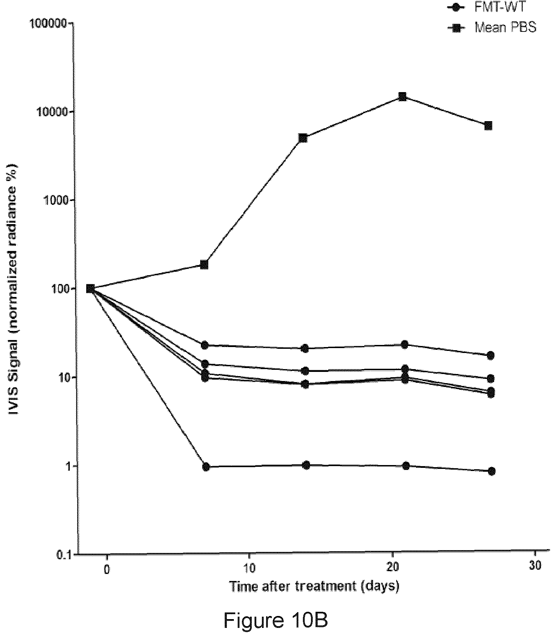

Figure 10A is an IVIS image of U87MG tumours illustrating in vivo efficacy of FMT wild type (FMT-WT) virus treatment in a human U87MG xenograft model. The image shows U87MG tumours post FMT-WT (1 week, 2 weeks, 3 weeks, 4 weeks) treatment (1 dose 1e7 pfu: IC). Figure 10B is a flux plot demonstrating a significant tumour regression in response to IC dose (1e7 pfu) of FMT-WT.

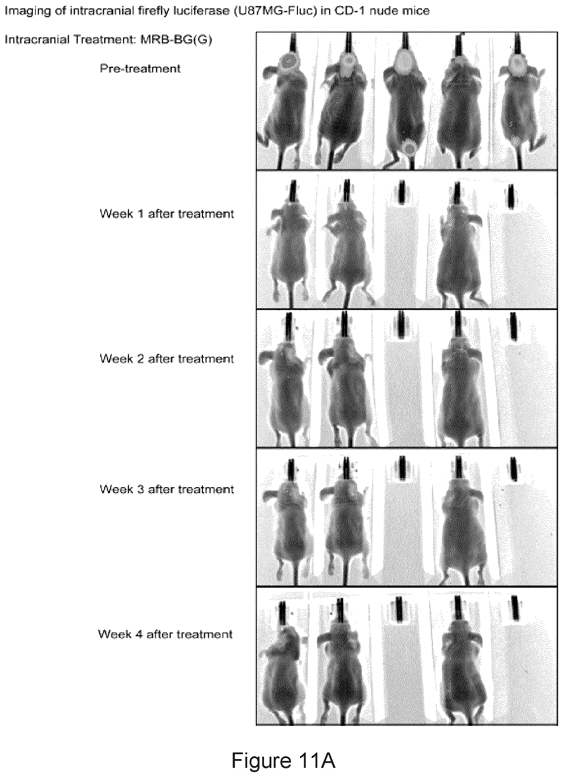

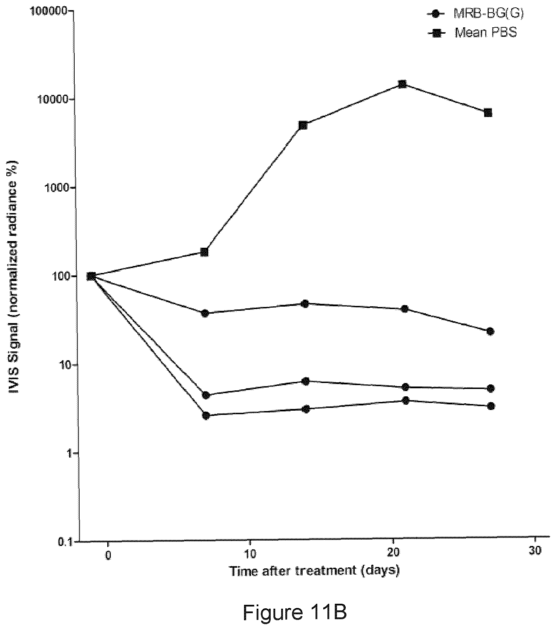

Figure 11A is an IVIS image of U87MG tumours illustrating in vivo efficacy of MRB BG(G) treatment in a human U87MG xenograft model. The image shows U87MG tumours post MRB BG(G) (1 week, 2 weeks, 3 weeks, 4 weeks) treatment (1 dose 1e7 pfu: IC). Figure 11B is a flux plot illustrating moderate tumour regression in response to IC dose (1e7 pfu) of MRB BGG.

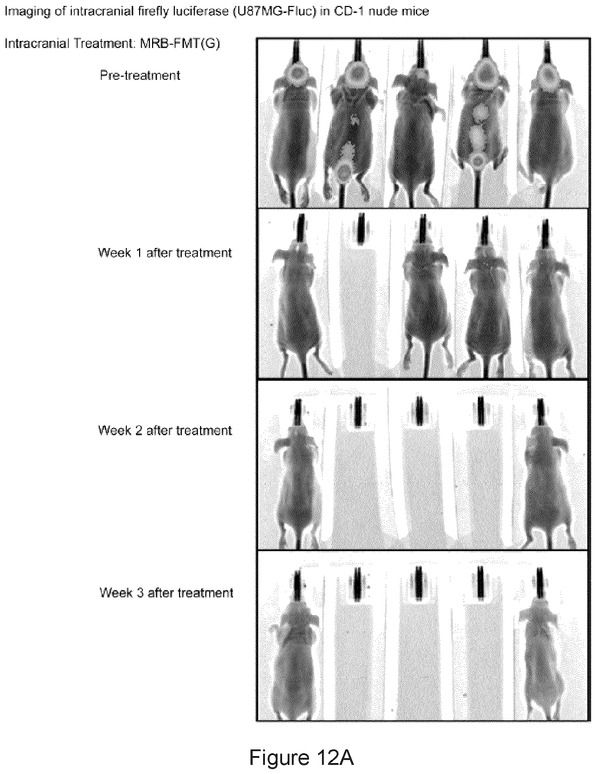

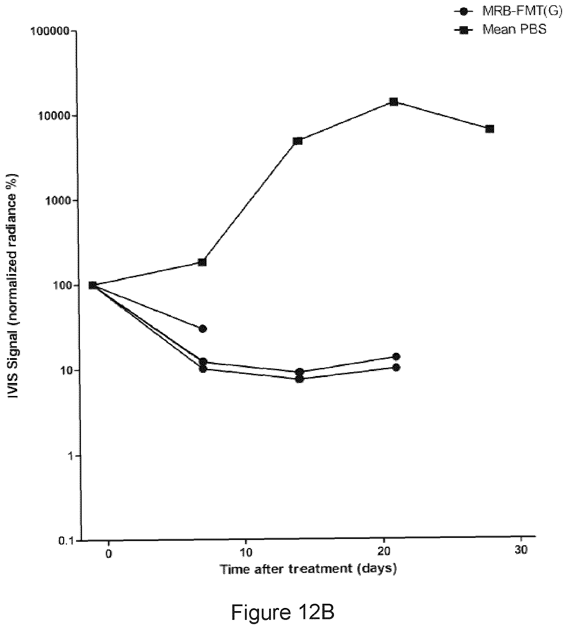

Figure 12A is an IVIS image of U87MG tumours illustrating in vivo efficacy of MRB FMT(G) treatment in a human U87MG xenograft model. The image shows U87MG tumours post MRB FMT(G) (1 weeks, 2 weeks, 3 weeks) treatment (1 dose 1e7 pfu: IC). Figure 12B is a flux plot demonstrating a significant tumour regression in response to IC dose (1e7 pfu: IC) of MRB FMT G. However, all animals succumbed to neurotoxic effects of MRB FMT(G) treatment prior to 4 weeks post treatment.

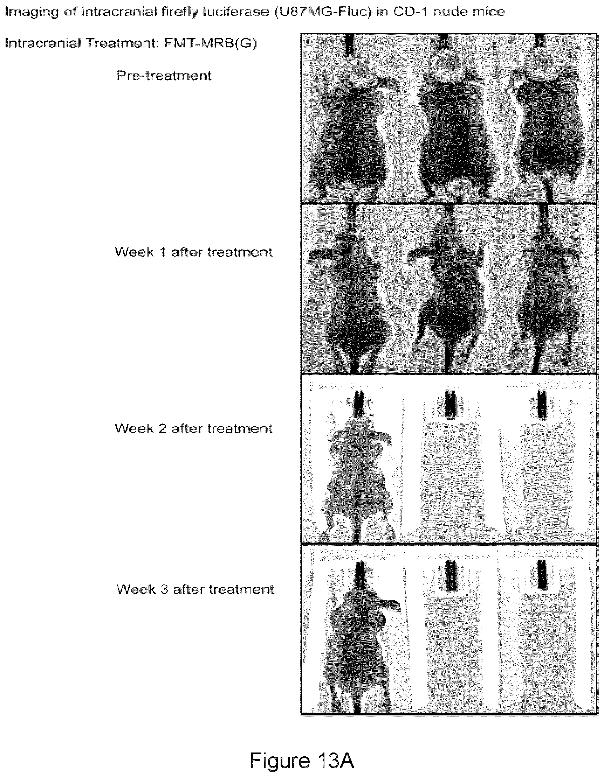

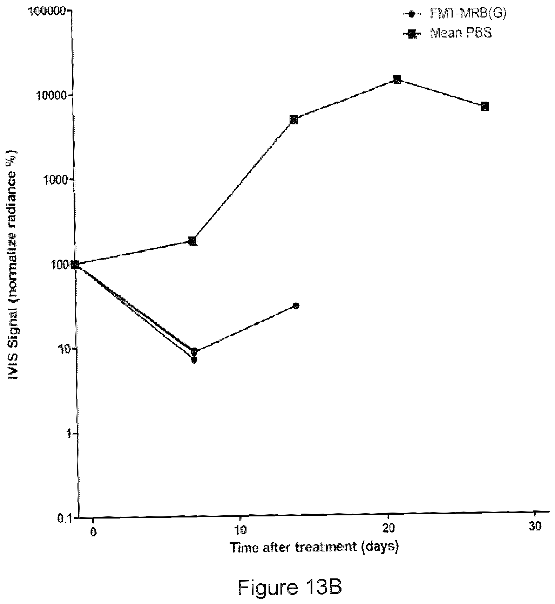

Figure 13A is an IVIS image of U87MG tumours illustrating in vivo efficacy of FMT MRB(G) treatment in a human U87MG xenograft model. The image shows U87MG tumours post FMT MRB(G) (1 week, 2 weeks, 3 weeks) treatment (1 dose 1e7 pfu: IC). Figure 13B is a flux plot illustrating a significant tumour regression in response to IC dose (1e7 pfu) of FMT MRB(G). However, all animals succumbed to neurotoxic effects of FMT MRB G treatment prior to 4 weeks post treatment.

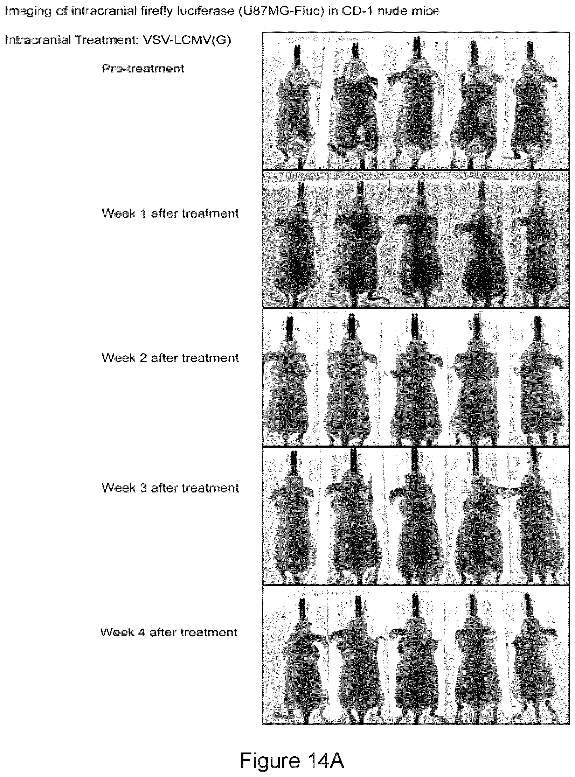

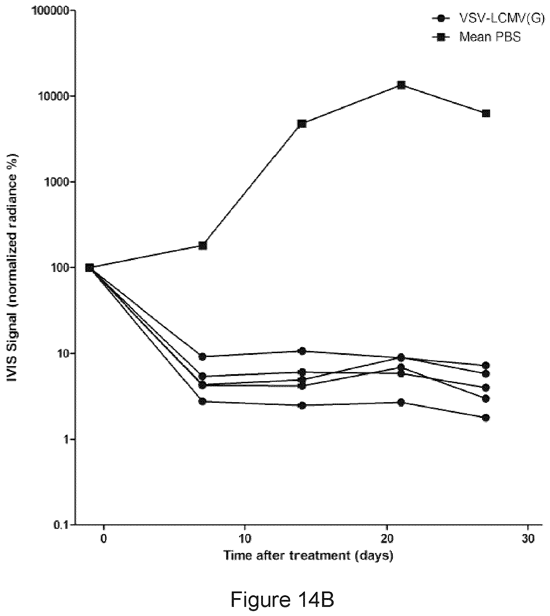

Figure 14A is an IVIS image of U87MG tumours illustrating in vivo efficacy of VSV-LCMV(G) treatment in a human U87MG xenograft model. The image shows U87MG tumours post VSV LCMV G (1 week, 2 weeks, 3 weeks, 4 weeks) treatment (1 dose 1e7 pfu: IC). Figure 14B is a flux plot illustrating a significant tumour regression in response to IC dose (1e7 pfu) of VSV-LCMV(G).

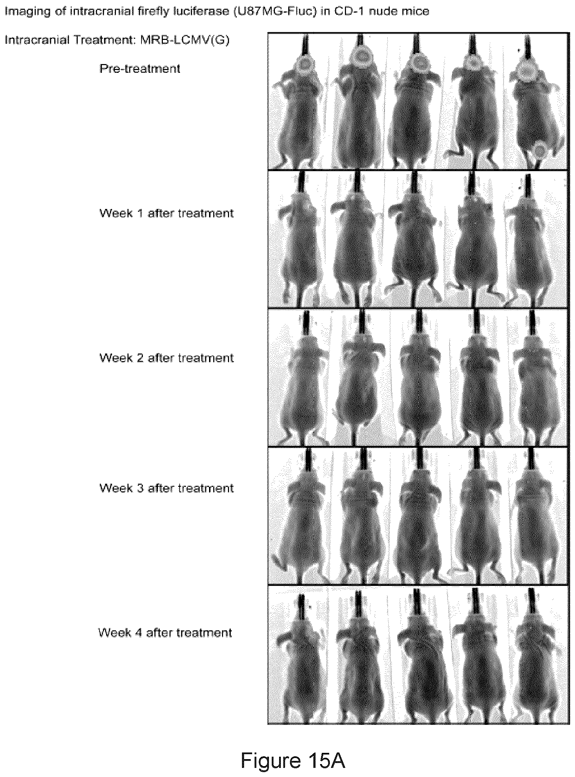

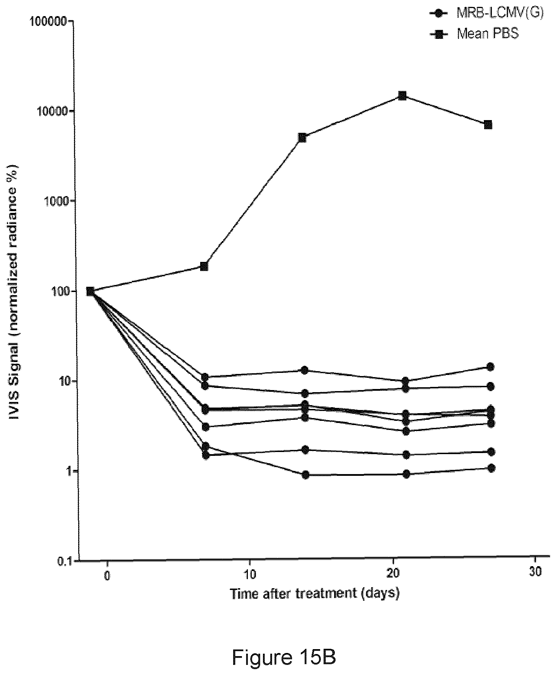

Figure 15A is an IVIS image of U87MG tumours illustrating in vivo efficacy of MRB LCMV(G) treatment in a human U87MG xenograft model. The image shows U87MG tumours post MRB LCMV G (1 week, 2 weeks, 3 weeks, 4 weeks) treatment (1 dose 1e7 pfu: IC). Figure 15B is a flux plot illustrating a significant tumour regression in response to IC dose (1e7 pfu) of MRB LCMV(G).

### Example 7: Neutralizing Antibody Responses to Maraba Chimera Viruses

Assays to quantify the presence of neutralizing antibodies to indicated viruses were performed as previously described (Propagation, Purification, and In Vivo Testing of Oncolytic Vesicular Stomatitis Virus Strains, J-S Diallo et al., Oncolytic Viruses: Methods and Protocols, Methods in Molecular Biology Vol 797 (2012)).

Briefly, on day 0, 50 µL of saphenous vein blood from 6-8 week old female Balb/c mice was collected into heparin coated tubes, centrifuged and serum removed. Subsequently, three animals per group were injected intravenously by tail vein injection with 1e7 pfu of the indicated virus. Mice were again bled on day 7, then injected in the same manner as on day 0. Mice were bled a final time on day 14 by terminal cardiac puncture. Serum from each animal, from each of the three time points (day 0, 7, 14) was serially diluted at 1:2 across a 96 well plate, starting with an initial dilution of 1/50. Each serum-containing well was incubated with 2.5e4 pfu/well of the injected virus for one hour, giving an initial serum dilution of 1/100. The serum and virus mixture was then added to 96 well plates seeded the day before with 1.25e4 Vero cells/well. Two days later, monolayers were assessed by microscopy for evidence of cytopathic effect (CPE). The lowest dilution at which 50 percent CPE was evident determined the neutralizing antibody titer for a particular sample.

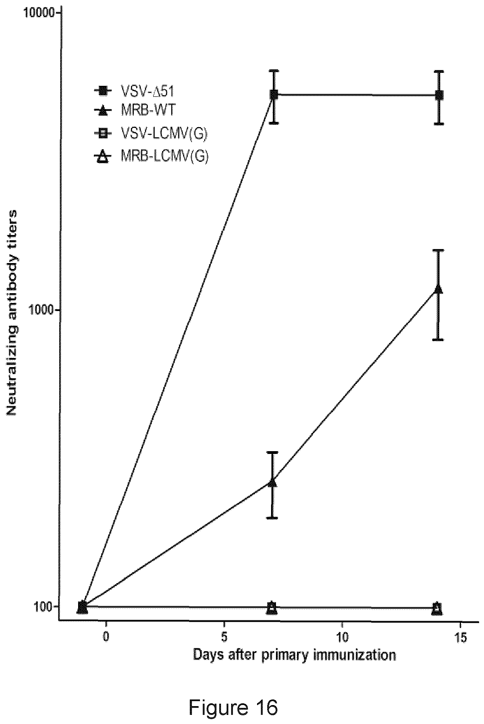

Figure 16 is a graph illustrating the neutralizing antibody titres in Balb/C mice treated with attenuated VSV (VSV-Δ51) or wild type Maraba virus (MRB-WT) versus VSV-LCMV(G) or Maraba-LCMV(G) chimera viruses. The wild type MRB and VSV Δ51 (attenuated) induced significant neutralizing antibody titres while the corresponding chimeras VSV-LCMV(G) and MRB-LCMV(G) did not induce neutralizing antibody response. Reciprocal challenges of serum derived from day 14 mice were also performed. Serum collected from each of wild type MRB, VSV Δ51 (attenuated), VSV-LCMV(G), MRB-LCMV(G) was challenged with MRB-LCMV(G), VSV-LCMV(G), VSV-Δ51 (attenuated) and wild type MRB, respectively. In all cases a neutralizing antibody response was not evident.

### Example 8: Chimera Virus Titres in Production Cells

To manufacture the indicated viruses, each were inoculated into forty 15 cm plastic tissue culture plates with subconfluent monolayers of Vero cells at a multiplicity of infection of 0.01. Twenty hours later, media was collected and virus was purified and titred as per Diallo et al 2012. Yield was calculated and each LCMV(G) chimera was compared to its parent wildtype. When compared to its parental strain, the MRB-LCMV(G) virus yielded over 2-fold more virus than VSV-LCMV(G (titre ratio VSV-LCMVG to wild type VSV is 0.028; in comparison, the titre ratio MRB-LCMV(G) to wild type MRB is 0.067).

In the preceding description, for purposes of explanation, numerous details are set forth in order to provide a thorough understanding of the examples. However, it will be apparent to one skilled in the art that these specific details are not required. The above-described examples are intended to be exemplary only. Alterations, modifications and variations can be effected to the particular embodiments by those of skill in the art without departing from the scope, which is defined solely by the claims appended hereto.

## Claims

1. An isolated viral particle having a genome comprising open reading frames that encode:
a protein having a sequence comprising SEQ ID NO: 1, or a variant thereof;
a protein having a sequence comprising SEQ ID NO: 2, or a variant thereof;
a protein having a sequence comprising SEQ ID NO: 3, or a variant thereof;
a protein having a sequence comprising SEQ ID NO: 5 or 4, or a variant thereof; and
a protein having a sequence comprising SEQ ID NO: 7, 6, or 8;
and wherein the viral genome preferably comprises open reading frames that encode: a protein having a sequence comprising SEQ ID NO: 1; a protein having a sequence comprising SEQ ID NO: 2; a protein having a sequence comprising SEQ ID NO: 3; a protein having a sequence comprising SEQ ID NO: 5; and a protein having a sequence comprising SEQ ID NO: 7;
and wherein the variant of a reference protein is a protein having a sequence which is at least 75%, at least 80%, at least 85%, at least 90%, or at least 95% identical to the sequence of the reference protein, and the variant protein maintains the same biological function as the reference protein.

2. An isolated viral particle comprising an RNA polynucleotide which has a sequence that includes:
the reverse complement of the sequence defined by position 64 to position 1332 of SEQ ID NO: 10, or a conservative variant thereof;
the reverse complement of the sequence defined by position 1393 to position 2190 of SEQ ID NO: 10, or a conservative variant thereof;
the reverse complement of the sequence defined by position 4943 to position 11272 of SEQ ID NO: 10, or a conservative variant thereof;
the reverse complement of the sequence defined by position 2256 to position 2945 of SEQ ID NO: 10, or a conservative variant thereof;
the reverse complement of the sequence defined by position 3041 to position 4816 of SEQ ID NO: 10; and
the reverse complements of promoters thereof;
and wherein a conservative variant of a sequence of nucleotides is a sequence that is at least 75%, at least 80%, at least 85%, at least 90%, or at least 95% identical to the reference sequence of nucleotides, and wherein the conservative variant is preferably a sequence comprising one or more silent substitutions.

3. An isolated viral particle that produces a cDNA polynucleotide comprising a sequence according to SEQ ID NO: 9 when the virus is in a host cell.

4. An isolated viral particle comprising an RNA polynucleotide comprising a sequence according to SEQ ID NO: 10.

5. An isolated viral particle comprising an RNA polynucleotide which has a sequence that includes:
the reverse complement of the sequence defined by position 64 to position 1332 of SEQ ID NO: 12, or a conservative variant thereof;
the reverse complement of the sequence defined by position 1393 to position 2190 of SEQ ID NO: 12, or a conservative variant thereof;
the reverse complement of the sequence defined by position 4664 to position 10993 of SEQ ID NO: 12, or a conservative variant thereof;
the reverse complement of the sequence defined by position 2256 to position 2945 of SEQ ID NO: 12, or a conservative variant thereof;
the reverse complement of the sequence defined by position 3041 to position 4537 of SEQ ID NO: 12; and
the reverse complements of promoters thereof;
and wherein a conservative variant of a sequence of nucleotides is a sequence that is at least 75%, at least 80%, at least 85%, at least 90%, or at least 95% identical to the reference sequence of nucleotides, and wherein the conservative variant is preferably a sequence comprising one or more silent substitutions.

6. An isolated viral particle that produces a cDNA polynucleotide comprising a sequence according to SEQ ID NO: 11 when the virus is in a host cell.

7. An isolated viral particle comprising an RNA polynucleotide comprising a sequence according to SEQ ID NO: 12.

8. An isolated viral particle comprising an RNA polynucleotide which has a sequence that includes:
the reverse complement of the sequence defined by position 64 to position 1332 of SEQ ID NO: 14, or a conservative variant thereof;
the reverse complement of the sequence defined by position 1393 to position 2190 of SEQ ID NO: 14, or a conservative variant thereof;
the reverse complement of the sequence defined by position 5195 to position 11524 of SEQ ID NO: 14, or a conservative variant thereof;
the reverse complement of the sequence defined by position 2256 to position 2942 of SEQ ID NO: 14, or a conservative variant thereof;
the reverse complement of the sequence defined by position 3038 to position 5068 of SEQ ID NO: 14; and
the reverse complements of promoters thereof;
and wherein a conservative variant of a sequence of nucleotides is a sequence that is at least 75%, at least 80%, at least 85%, at least 90%, or at least 95% identical to the reference sequence of nucleotides, and wherein the conservative variant is preferably a sequence comprising one or more silent substitutions.

9. An isolated viral particle that produces a cDNA polynucleotide comprising a sequence according to SEQ ID NO: 13 when the virus is in a host cell.

10. An isolated viral particle comprising an RNA polynucleotide comprising a sequence according to SEQ ID NO: 14.

11. An isolated viral particle according to any one of claims 1 to 10 for use in the treatment of cancer, preferably a brain cancer, such as a glioblastoma, in a person administered the virus;
wherein the isolated viral particle is preferably used to infect a cell and the infected cell is used for the treatment of cancer;
wherein the isolated viral particle is preferably formulated:
for direct delivery to the central nervous system, outside the blood/brain barrier, inside the blood/brain barrier, or any combination thereof; or
for infection of the cell and the infected cell is for delivery to the central nervous system, outside the blood/brain barrier, inside the blood/brain barrier, or any combination thereof;
wherein the isolated viral particle or the infected cell is preferably formulated for administration via intrathecal injection, intravenous injection, intracranial injection, or any combination thereof simultaneously or sequentially.

12. A kit for the treatment of cancer, preferably a brain cancer, such as a glioblastoma, in a patient, the kit comprising:
the isolated viral particle according to any one of claims 1 to 10; and
instructions for administration of the isolated viral particle to the patient;
wherein the isolated viral particle is preferably formulated for:
direct delivery to the central nervous system, outside the blood/brain barrier, inside the blood/brain barrier, or any combination thereof; or
infection of a cell and the cell is for delivery to the central nervous system, outside the blood/brain barrier, inside the blood/brain barrier, or any combination thereof;
wherein the isolated viral particle or cell is preferably formulated for administration via intrathecal injection, intravenous injection, intracranial injection, or any combination thereof simultaneously or sequentially.

## Patentansprüche

1. Isoliertes Viruspartikel, das ein Genom hat, umfassend offene Leserahmen, kodierend:
ein Protein, das eine Sequenz hat, umfassend SEQ ID NO: 1 oder eine Variante davon;
ein Protein, das eine Sequenz hat, umfassend SEQ ID NO: 2 oder eine Variante davon;
ein Protein, das eine Sequenz hat, umfassend SEQ ID NO: 3 oder eine Variante davon;
ein Protein, das eine Sequenz hat, umfassend SEQ ID NO: 5 oder 4 oder eine Variante davon; und
ein Protein, das eine Sequenz hat, umfassend SEQ ID NO: 7, 6 oder 8;
und wobei das virale Genom vorzugsweise offene Leserahmen umfasst, kodierend:
ein Protein, das eine Sequenz hat, umfassend SEQ ID NO: 1; ein Protein, das eine Sequenz hat, umfassend SEQ ID NO: 2; ein Protein, das eine Sequenz hat, umfassend SEQ ID NO: 3; ein Protein, das eine Sequenz hat, umfassend SEQ ID NO: 5; und ein Protein, das eine Sequenz hat umfassend SEQ ID NO: 7;
und wobei die Variante eines Referenzproteins ein Protein ist, das eine Sequenz hat, die mindestens 75%, mindestens 80%, mindestens 85%, mindestens 90% oder mindestens 95% identisch zu der Sequenz des Referenzproteins ist und das Variantenprotein die gleiche biologische Funktion wie das Referenzprotein behält.

2. Isoliertes Viruspartikel, umfassend ein RNA-Polynukleotid, das eine Sequenz hat, enthaltend:
das reverse Komplement der Sequenz, die durch die Position 64 bis Position 1332 von SEQ ID NO: 10 definiert ist oder eine konservative Variante davon;
das reverse Komplement der Sequenz, die durch die Position 1393 bis Position 2190 von SEQ ID NO: 10 definiert ist oder eine konservative Variante davon;
das reverse Komplement der Sequenz, die durch die Position 4943 bis Position 11272 von SEQ ID NO: 10 definiert ist oder eine konservative Variante davon;
das reverse Komplement der Sequenz, die durch die Position 2256 bis Position 2945 von SEQ ID NO: 10 definiert ist oder eine konservative Variante davon;
das reverse Komplement der Sequenz, die durch die Position 3041 bis Position 4816 von SEQ ID NO: 10 definiert ist; und
die reversen Komplemente der Promoteren davon;
und wobei eine konservative Variante einer Nukleotidsequenz eine Sequenz ist, die mindestens 75%, mindestens 80%, mindestens 85%, mindestens 90% oder mindestens 95% identisch zu der Referenznukleotidsequenz ist und wobei die konservative Variante vorzugsweise eine Sequenz ist, umfassend eine oder mehrere stille Substitutionen.

3. Isoliertes Viruspartikel, das ein cDNA-Polynukleotid bildet, umfassend eine Sequenz nach SEQ ID NO: 9, wenn das Virus in einer Wirtszelle ist.

4. Isoliertes Viruspartikel, umfassend ein RNA-Polynukleotid, umfassend eine Sequenz nach SEQ ID NO: 10.

5. Isoliertes Viruspartikel, umfassend ein RNA-Polynukleotid, das eine Sequenz hat enthaltend:
das reverse Komplement der Sequenz, die durch Position 64 bis Position 1332 von SEQ ID NO: 12 definiert ist oder eine konservative Variante davon;
das reverse Komplement der Sequenz, die durch Position 1393 bis Position 2190 von SEQ ID NO: 12 definiert ist oder eine konservative Variante davon;
das reverse Komplement der Sequenz, die durch Position 4664 bis Position 10993 von SEQ ID NO: 12 definiert ist oder eine konservative Variante davon;
das reverse Komplement der Sequenz, die durch Position 2256 bis Position 2945 von SEQ ID NO: 12 definiert ist oder eine konservative Variante davon;
das reverse Komplement der Sequenz, die durch Position 3041 bis Position 4537 von SEQ ID NO: 12 definiert ist; und
die reversen Komplemente der Promotoren davon;
und wobei eine konservative Variante einer Nukleotidsequenz eine Sequenz ist, die mindestens 75%, mindestens 80%, mindestens 85%, mindestens 90% oder mindestens 95% identisch ist zu der Referenznukleotidsequenz und wobei die konservative Variante vorzugsweise eine Sequenz ist, umfassend eine oder mehrere stille Substitutionen.

6. Isoliertes Viruspartikel, das ein cDNA-Polynukleotid bildet, umfassend eine Sequenz nach SEQ ID NO: 11, wenn das Virus in einer Wirtszelle ist.

7. Isoliertes Viruspartikel, umfassend ein RNA-Polynukleotid umfassend eine Sequenz nach SEQ ID NO: 12.

8. Isoliertes Viruspartikel, umfassend ein RNA-Polynukleotid, dass eine Sequenz hat enthaltend:
das reverse Komplement der Sequenz, die durch Position 64 bis Position 1332 von SEQ ID NO: 14 definiert ist oder eine konservative Variante davon;
das reverse Komplement der Sequenz, die durch Position 1393 bis Position 2190 von SEQ ID NO: 14 definiert ist oder eine konservative Variante davon;
das reverse Komplement der Sequenz, die durch Position 5195 bis Position 11524 von SEQ ID NO: 14 definiert ist oder eine konservative Variante davon;
das reverse Komplement der Sequenz, die durch Position 2256 bis Position 2942 von SEQ ID NO: 14 definiert ist oder eine konservative Variante davon;
das reverse Komplement der Sequenz, die durch Position 3038 bis Position 5068 von SEQ ID NO: 14 definiert ist; und
die reversen Komplemente der Promotoren davon;
und wobei eine konservative Variante einer Nukleotidsequenz eine Sequenz ist, die mindestens 75%, mindestens 80%, mindestens 85%, mindestens 90% oder
mindestens 95% identisch ist zu der Referenznukleotidsequenz und wobei die konservative Variante vorzugsweise eine Sequenz ist, umfassend eine oder mehrere stille Substitutionen.

9. Isoliertes Viruspartikel, das ein cDNA-Polynukleotid bildet, umfassend eine Sequenz nach SEQ ID NO: 13, wenn das Virus in einer Wirtszelle ist.

10. Isoliertes Viruspartikel, umfassend ein RNA-Polynukleotid umfassend eine Sequenz nach SEQ ID NO: 14.

11. Isoliertes Viruspartikel nach einem der Ansprüche 1 bis 10 zur Verwendung in der Behandlung von Krebs, vorzugsweise einem Gehirntumor wie einem Glioblastom in einer Person, der das Virus verabreicht wurde; wobei das isolierte Viruspartikel vorzugsweise verwendet wird, um eine Zelle zu infizieren und die infizierte Zelle zur Behandlung von Krebs verwendet wird;
wobei das isolierte Viruspartikel vorzugsweise formuliert ist:
zur direkten Abgabe an das zentrale Nervensystem, außerhalb der Blut-Hirn-Schranke, innerhalb der Blut-Hirn-Schranke oder jegliche Kombination davon; oder
zur Infektion der Zelle und die infizierte Zelle für die Abgabe an das zentrale Nervensystem, außerhalb der Blut-Hirn-Schranke, innerhalb der Blut-Hirn-Schranke ist oder jegliche Kombination davon;
wobei das isolierte Viruspartikel oder die infizierte Zelle vorzugsweise zur Verabreichung über intratekale Injektion, intravenöse Injektion, intrakraniale Injektion oder jede Kombination davon simultan oder nacheinander formuliert ist.

12. Kit zur Behandlung von Krebs, vorzugsweise einem Gehirntumor wie einem Glioblastom in einem Patienten, das Kit, umfassend:
das isolierte Viruspartikel nach einem der Ansprüche 1 bis 10; und
Anweisungen zur Verabreichung des isolierten Viruspartikels an den Patienten;
wobei das isolierte Viruspartikel vorzugsweise formuliert ist zur:
direkten Abgabe an das zentrale Nervensystem, außerhalb der Blut-Hirn-Schranke, innerhalb der Blut-Hirn-Schranke oder jegliche Kombination davon; oder
Infektion einer Zelle und die Zelle für die Abgabe an das zentrale Nervensystem, außerhalb der Blut-Hirn-Schranke, innerhalb der Blut-Hirn-Schranke ist oder jede Kombination davon;
wobei das isolierte Viruspartikel oder die Zelle vorzugsweise zur Verabreichung über intratekale Injektion, intravenöse Injektion, intrakraniale Injektion oder jede Kombination davon simultan oder nacheinander formuliert ist.

## Revendications

1. Particule virale isolée ayant un génome comprenant des cadres de lecture ouverts qui codent :
une protéine ayant une séquence comprenant SEQ ID NO: 1, ou un variant de celle-ci ;
une protéine ayant une séquence comprenant SEQ ID NO: 2, ou un variant de celle-ci ;
une protéine ayant une séquence comprenant SEQ ID NO: 3, ou un variant de celle-ci ;
une protéine ayant une séquence comprenant SEQ ID NO: 5 ou 4, ou un variant de celle-ci ;
et
une protéine ayant une séquence comprenant SEQ ID NO: 7, 6 ou 8 ;
et où le génome viral comprend de préférence des cadres de lecture ouverts qui codent : une protéine ayant une séquence comprenant SEQ ID NO: 1; une protéine ayant une séquence comprenant SEQ ID NO: 2 ; une protéine ayant une séquence comprenant SEQ ID NO: 3 ; une protéine ayant une séquence comprenant SEQ ID NO: 5 ; et une protéine ayant une séquence comprenant SEQ ID NO: 7 ;
et où le variant d'une protéine de référence est une protéine ayant une séquence qui est identique à raison d'au moins 75 %, d'au moins 80 %, d'au moins 85 %, d'au moins 90 % ou d'au moins 95 % à la séquence de la protéine de référence, et la protéine variante maintient la même fonction biologique que la protéine de référence.

2. Particule virale isolée comprenant un polynucléotide d'ARN qui a une séquence qui inclut :
le complément inversé de la séquence définie par la position 64 à la position 1332 de SEQ ID NO: 10, ou un variant conservatif de celui-ci ;
le complément inversé de la séquence définie par la position 1393 à la position 2190 de SEQ ID NO: 10, ou un variant conservatif de celui-ci ;
le complément inversé de la séquence définie par la position 4943 à la position 11272 de SEQ ID NO: 10, ou un variant conservatif de celui-ci ;
le complément inversé de la séquence définie par la position 2256 à la position 2945 de SEQ ID NO: 10, ou un variant conservatif de celui-ci ;
le complément inversé de la séquence définie par la position 3041 à la position 4816 de SEQ ID NO: 10 ; et
les compléments inversés de promoteurs de ceux-ci ;
et où un variant conservatif d'une séquence de nucléotides est une séquence qui est identique à raison d'au moins 75 %, d'au moins 80 %, d'au moins 85 %, d'au moins 90 % ou d'au moins 95 % à la séquence de nucléotides de référence, et où le variant conservatif est de préférence une séquence comprenant une ou plusieurs substitutions silencieuses.

3. Particule virale isolée qui produit un polynucléotide d'ADNc comprenant une séquence selon SEQ ID NO: 9 quand le virus est dans une cellule hôte.

4. Particule virale isolée comprenant un polynucléotide d'ARN comprenant une séquence selon SEQ ID NO: 10.

5. Particule virale isolée comprenant un polynucléotide d'ARN qui a une séquence qui inclut :
le complément inversé de la séquence définie par la position 64 à la position 1332 de SEQ ID NO: 12, ou un variant conservatif de celui-ci ;
le complément inversé de la séquence définie par la position 1393 à la position 2190 de SEQ ID NO: 12, ou un variant conservatif de celui-ci ;
le complément inversé de la séquence définie par la position 4664 à la position 10993 de SEQ ID NO: 12, ou un variant conservatif de celui-ci ;
le complément inversé de la séquence définie par la position 2256 à la position 2945 de SEQ ID NO: 12, ou un variant conservatif de celui-ci ;
le complément inversé de la séquence définie par la position 3041 à la position 4537 de SEQ ID NO: 12 ; et
les compléments inversés de promoteurs de ceux-ci ;
et où un variant conservatif d'une séquence de nucléotides est une séquence qui est identique à raison d'au moins 75 %, d'au moins 80 %, d'au moins 85 %, d'au moins 90 % ou d'au moins 95 % à la séquence de nucléotides de référence, et où le variant conservatif est de préférence une séquence comprenant une ou plusieurs substitutions silencieuses.

6. Particule virale isolée qui produit un polynucléotide d'ADNc comprenant une séquence selon SEQ ID NO: 11 quand le virus est dans une cellule hôte.

7. Particule virale isolée comprenant un polynucléotide d'ARN comprenant une séquence selon SEQ ID NO: 12.

8. Particule virale isolée comprenant un polynucléotide d'ARN qui a une séquence qui inclut :
le complément inversé de la séquence définie par la position 64 à la position 1332 de SEQ ID NO: 14, ou un variant conservatif de celui-ci ;
le complément inversé de la séquence définie par la position 1393 à la position 2190 de SEQ ID NO: 14, ou un variant conservatif de celui-ci ;
le complément inversé de la séquence définie par la position 5195 à la position 11524 de SEQ ID NO: 14, ou un variant conservatif de celui-ci ;
le complément inversé de la séquence définie par la position 2256 à la position 2942 de SEQ ID NO: 14, ou un variant conservatif de celui-ci ;
le complément inversé de la séquence définie par la position 3038 à la position 5068 de SEQ ID NO: 14 ; et
les compléments inversés de promoteurs de ceux-ci ;
et où un variant conservatif d'une séquence de nucléotides est une séquence qui est identique à raison d'au moins 75 %, d'au moins 80 %, d'au moins 85 %, d'au moins 90 % ou d'au moins 95 % à la séquence de nucléotides de référence, et où le variant conservatif est de préférence une séquence comprenant une ou plusieurs substitutions silencieuses.

9. Particule virale isolée qui produit un polynucléotide d'ADNc comprenant une séquence selon SEQ ID NO: 13 quand le virus est dans une cellule hôte.

10. Particule virale isolée comprenant un polynucléotide d'ARN comprenant une séquence selon SEQ ID NO: 14.

11. Particule virale isolée selon l'une quelconque des revendications 1 à 10 destinée à être utilisée dans le traitement du cancer, de préférence d'un cancer du cerveau, comme un glioblastome, chez une personne à laquelle le virus a été administré ;
où la particule virale isolée est de préférence utilisée pour infecter une cellule et la cellule infectée est utilisée pour le traitement du cancer ;
où la particule virale isolée est de préférence formulée :
pour la fourniture directe au système nerveux central, à l'extérieur de la barrière hémato-encéphalique, à l'intérieur de la barrière hémato-encéphalique, ou toute combinaison de ceux-ci ; ou
pour l'infection de la cellule et la cellule infectée est destinée à la fourniture au système nerveux central, à l'extérieur de la barrière hémato-encéphalique, à l'intérieur de la barrière hémato-encéphalique, ou toute combinaison de ceux-ci ;
où la particule virale isolée ou la cellule infectée est de préférence formulée pour l'administration via injection intrathécale, injection intraveineuse, injection intracrânienne, ou toute combinaison de celles-ci simultanément ou successivement.

12. Kit pour le traitement du cancer, de préférence d'un cancer du cerveau, comme un glioblastome, chez un patient, le kit comprenant :
la particule virale isolée selon l'une quelconque des revendications 1 à 10 ; et
des instructions pour l'administration de la particule virale isolée au patient ;
où la particule virale isolée est de préférence formulée pour :
la fourniture directe au système nerveux central, à l'extérieur de la barrière hémato-encéphalique, à l'intérieur de la barrière hémato-encéphalique, ou toute combinaison de ceux-ci ; ou
l'infection d'une cellule et la cellule est destinée à la fourniture au système nerveux central, à l'extérieur de la barrière hémato-encéphalique, à l'intérieur de la barrière hémato-encéphalique, ou toute combinaison de ceux-ci ;
où la particule virale isolée ou la cellule est de préférence formulée pour l'administration via injection intrathécale, injection intraveineuse, injection intracrânienne, ou toute combinaison de celles-ci simultanément ou successivement.


### SEQUENCE LISTING

<110> Children's Hospital of Eastern Ontario Research Institute Inc. Stojdl, David F
<120> COMPOSITIONS AND METHODS FOR THE TREATMENT OF BRAIN CANCERS
<130> PAT 7139W-90
<140> UNKNOWN
   <141> 2012-12-12
<160> 24
<170> PatentIn version 3.5
<210> 1
   <211> 422
   <212> PRT
   <213> Maraba virus
<400> 1
   Met Ser Val Thr Val Lys Arg Val Ile Asp Asp Ser Leu Ile Thr Pro
   1               5                   10                  15
   Lys Leu Pro Ala Asn Glu Asp Pro Val Glu Tyr Pro Ala Asp Tyr Phe
               20                  25                  30
   Lys Lys Ser Arg Asp Ile Pro Val Tyr Ile Asn Thr Thr Lys Ser Leu
           35                  40                  45
   Ser Asp Leu Arg Gly Tyr Val Tyr Gln Gly Leu Lys Ser Gly Asn Ile
       50                  55                  60
   Ser Ile Ile His Val Asn Ser Tyr Leu Tyr Ala Ala Leu Lys Glu Ile
   65                  70                  75                  80
   Arg Gly Lys Leu Asp Arg Asp Trp Ile Thr Phe Gly Ile Gln Ile Gly
                   85                  90                  95
   Lys Thr Gly Asp Ser Val Gly Ile Phe Asp Leu Leu Thr Leu Lys Pro
               100                 105                 110
   Leu Asp Gly Val Leu Pro Asp Gly Val Ser Asp Ala Thr Arg Thr Ser
           115                 120                 125
   Ser Asp Asp Ala Trp Leu Pro Leu Tyr Leu Leu Gly Leu Tyr Arg Val
       130                 135                 140
   Gly Arg Thr Gln Met Pro Glu Tyr Arg Lys Lys Leu Met Asp Gly Leu
   145                 150                 155                 160
   Ile Asn Gln Cys Lys Met Ile Asn Glu Gln Phe Glu Pro Leu Leu Pro
                   165                 170                 175
   Glu Gly Arg Asp Val Phe Asp Val Trp Gly Asn Asp Ser Asn Tyr Thr
               180                 185                 190
   Lys Ile Val Ala Ala Val Asp Met Phe Phe His Met Phe Lys Lys His
           195                 200                 205
   Glu Lys Ala Ser Phe Arg Tyr Gly Thr Ile Val Ser Arg Phe Lys Asp
       210                 215                 220
   Cys Ala Ala Leu Ala Thr Phe Gly His Leu Cys Lys Ile Thr Gly Met
   225                 230                 235                 240
   Ser Thr Glu Asp Val Thr Thr Trp Ile Leu Asn Arg Glu Val Ala Asp
                   245                 250                 255
   Glu Met Val Gln Met Met Tyr Pro Gly Gln Glu Ile Asp Lys Ala Asp
               260                 265                 270
   Ser Tyr Met Pro Tyr Leu Ile Asp Leu Gly Leu Ser Ser Lys Ser Pro
           275                 280                 285
   Tyr Ser Ser Val Lys Asn Pro Ala Phe His Phe Trp Gly Gln Leu Thr
       290                 295                 300
   Ala Leu Leu Leu Arg Ser Thr Arg Ala Arg Asn Ala Arg Gln Pro Asp
   305                 310                 315                 320
   Asp Ile Glu Tyr Thr Ser Leu Thr Thr Ala Gly Leu Leu Tyr Ala Tyr
                   325                 330                 335
   Ala Val Gly Ser Ser Ala Asp Leu Ala Gln Gln Phe Tyr Val Gly Asp
               340                 345                 350
   Asn Lys Tyr Val Pro Glu Thr Gly Asp Gly Gly Leu Thr Thr Asn Ala
           355                 360                 365
   Pro Pro Gln Gly Arg Asp Val Val Glu Trp Leu Ser Trp Phe Glu Asp
       370                 375                 380
   Gln Asn Arg Lys Pro Thr Pro Asp Met Leu Met Tyr Ala Lys Arg Ala
   385                 390                 395                 400
   Val Ser Ala Leu Gln Gly Leu Arg Glu Lys Thr Ile Gly Lys Tyr Ala
                   405                 410                 415
   Lys Ser Glu Phe Asp Lys
               420
<210> 2
   <211> 265
   <212> PRT
   <213> Maraba virus
<400> 2
   Met Asp Gln Leu Ser Lys Val Lys Glu Phe Leu Lys Thr Tyr Ala Gln
   1               5                   10                  15
   Leu Asp Gln Ala Val Gln Glu Met Asp Asp Ile Glu Ser Gln Arg Glu
               20                  25                  30
   Glu Lys Thr Asn Phe Asp Leu Phe Gln Glu Glu Gly Leu Glu Ile Lys
           35                  40                  45
   Glu Lys Pro Ser Tyr Tyr Arg Ala Asp Glu Glu Glu Ile Asp Ser Asp
       50                  55                  60
   Glu Asp Ser Val Asp Asp Ala Gln Asp Leu Gly Ile Arg Thr Ser Thr
   65                  70                  75                  80
   Ser Pro Ile Glu Gly Tyr Val Asp Glu Glu Gln Asp Asp Tyr Glu Asp
                   85                  90                  95
   Glu Glu Val Asn Val Val Phe Thr Ser Asp Trp Lys Gln Pro Glu Leu
               100                 105                 110
   Glu Ser Asp Gly Asp Gly Lys Thr Leu Arg Leu Thr Ile Pro Asp Gly
           115                 120                 125
   Leu Thr Gly Glu Gln Lys Ser Gln Trp Leu Ala Thr Ile Lys Ala Val
       130                 135                 140
   Val Gln Ser Ala Lys Tyr Trp Asn Ile Ser Glu Cys Ser Phe Glu Ser
   145                 150                 155                 160
   Tyr Glu Gln Gly Val Leu Ile Arg Glu Arg Gln Met Thr Pro Asp Val
                   165                 170                 175
   Tyr Lys Val Thr Pro Val Leu Asn Ala Pro Pro Val Gln Met Thr Ala
               180                 185                 190
   Asn Gln Asp Val Trp Ser Leu Ser Ser Thr Pro Phe Thr Phe Leu Pro
           195                 200                 205
   Lys Lys Gln Gly Val Thr Pro Leu Thr Met Ser Leu Glu Glu Leu Phe
       210                 215                 220
   Asn Thr Arg Gly Glu Phe Ile Ser Leu Gly Gly Asn Gly Lys Met Ser
   225                 230                 235                 240
   His Arg Glu Ala Ile Ile Leu Gly Leu Arg His Lys Lys Leu Tyr Asn
                   245                 250                 255
   Gln Ala Arg Leu Lys Tyr Asn Leu Ala
               260                 265
<210> 3
   <211> 2109
   <212> PRT
   <213> Maraba virus
<400> 3
   Met Asp Val Asn Asp Phe Glu Leu His Glu Asp Phe Ala Leu Ser Glu
   1               5                   10                  15
   Asp Asp Phe Val Thr Ser Glu Phe Leu Asn Pro Glu Asp Gln Met Thr
               20                  25                  30
   Tyr Leu Asn His Ala Asp Tyr Asn Leu Asn Ser Pro Leu Ile Ser Asp
           35                  40                  45
   Asp Ile Asp Phe Leu Ile Lys Lys Tyr Asn His Glu Gln Ile Pro Lys
       50                  55                  60
   Met Trp Asp Val Lys Asn Trp Glu Gly Val Leu Glu Met Leu Thr Ala
   65                  70                  75                  80
   Cys Gln Ala Ser Pro Ile Leu Ser Ser Thr Met His Lys Trp Val Gly
                   85                  90                  95
   Lys Trp Leu Met Ser Asp Asp His Asp Ala Ser Gln Gly Phe Ser Phe
               100                 105                 110
   Leu His Glu Val Asp Lys Glu Ala Asp Leu Thr Phe Glu Val Val Glu
           115                 120                 125
   Thr Phe Ile Arg Gly Trp Gly Gly Arg Glu Leu Gln Tyr Lys Arg Lys
       130                 135                 140
   Asp Thr Phe Pro Asp Ser Phe Arg Val Ala Ala Ser Leu Cys Gln Lys
   145                 150                 155                 160
   Phe Leu Asp Leu His Lys Leu Thr Leu Ile Met Asn Ser Val Ser Glu
                   165                 170                 175
   Val Glu Leu Thr Asn Leu Ala Lys Asn Phe Lys Gly Lys Asn Arg Lys
               180                 185                 190
   Ala Lys Ser Gly Asn Leu Ile Thr Arg Leu Arg Val Pro Ser Leu Gly
           195                 200                 205
   Pro Ala Phe Val Thr Gln Gly Trp Val Tyr Met Lys Lys Leu Glu Met
       210                 215                 220
   Ile Met Asp Arg Asn Phe Leu Leu Met Leu Lys Asp Val Ile Ile Gly
   225                 230                 235                 240
   Arg Met Gln Thr Ile Leu Ser Met Ile Ser Arg Asp Asp Asn Leu Phe
                   245                 250                 255
   Ser Glu Ser Asp Ile Phe Thr Val Leu Lys Ile Tyr Arg Ile Gly Asp
               260                 265                 270
   Lys Ile Leu Glu Arg Gln Gly Thr Lys Gly Tyr Asp Leu Ile Lys Met
           275                 280                 285
   Ile Glu Pro Ile Cys Asn Leu Lys Met Met Asn Leu Ala Arg Lys Tyr
       290                 295                 300
   Arg Pro Leu Ile Pro Thr Phe Pro His Phe Glu Lys His Ile Ala Asp
   305                 310                 315                 320
   Ser Val Lys Glu Gly Ser Lys Ile Asp Lys Gly Ile Glu Phe Ile Tyr
                   325                 330                 335
   Asp His Ile Met Ser Ile Pro Gly Val Asp Leu Thr Leu Val Ile Tyr
               340                 345                 350
   Gly Ser Phe Arg His Trp Gly His Pro Phe Ile Asn Tyr Tyr Glu Gly
           355                 360                 365
   Leu Glu Lys Leu His Lys Gln Val Thr Met Pro Lys Thr Ile Asp Arg
       370                 375                 380
   Glu Tyr Ala Glu Cys Leu Ala Ser Asp Leu Ala Arg Ile Val Leu Gln
   385                 390                 395                 400
   Gln Gln Phe Asn Glu His Lys Lys Trp Phe Val Asp Val Asp Lys Val
   405 410 415
   Pro Gln Ser His Pro Phe Lys Ser His Met Lys Glu Asn Thr Trp Pro
   420 425 430
   Thr Ala Ala Gln Val Gln Asp Tyr Gly Asp Arg Trp His Gln Leu Pro
   435 440 445
   Leu Ile Lys Cys Phe Glu Ile Pro Asp Leu Leu Asp Pro Ser Ile Ile
   450 455 460
   Tyr Ser Asp Lys Ser His Ser Met Asn Arg Ser Glu Val Leu Arg His
   465 470 475 480
   Val Arg Leu Thr Pro His Val Pro Ile Pro Ser Arg Lys Val Leu Gln
   485 490 495
   Thr Met Leu Glu Thr Lys Ala Thr Asp Trp Lys Glu Phe Leu Lys Lys
   500 505 510
   Ile Asp Glu Glu Gly Leu Glu Asp Asp Asp Leu Val Ile Gly Leu Lys
   515 520 525
   Gly Lys Glu Arg Glu Leu Lys Ile Ala Gly Arg Phe Phe Ser Leu Met
   530 535 540
   Ser Trp Lys Leu Arg Glu Tyr Phe Val Ile Thr Glu Tyr Leu Ile Lys
   545 550 555 560
   Thr His Phe Val Pro Met Phe Lys Gly Leu Thr Met Ala Asp Asp Leu
   565 570 575
   Thr Ala Val Ile Lys Lys Met Met Asp Thr Ser Ser Gly Gln Gly Leu
   580 585 590
   Asp Asn Tyr Glu Ser Ile Cys Ile Ala Asn His Ile Asp Tyr Glu Lys
   595 600 605
   Trp Asn Asn His Gln Arg Lys Glu Ser Asn Gly Pro Val Phe Lys Val
   610 615 620
   Met Gly Gln Phe Leu Gly Tyr Pro Arg Leu Ile Glu Arg Thr His Glu
   625 630 635 640
   Phe Phe Glu Lys Ser Leu Ile Tyr Tyr Asn Gly Arg Pro Asp Leu Met
   645 650 655
   Arg Val Arg Gly Asn Ser Leu Val Asn Ala Ser Ser Leu Asn Val Cys
               660                 665                 670
   Trp Glu Gly Gln Ala Gly Gly Leu Glu Gly Leu Arg Gln Lys Gly Trp
           675                 680                 685
   Ser Ile Leu Asn Leu Leu Val Ile Gln Arg Glu Ala Lys Ile Arg Asn
       690                 695                 700
   Thr Ala Val Lys Val Leu Ala Gln Gly Asp Asn Gln Val Ile Cys Thr
   705                 710                 715                 720
   Gln Tyr Lys Thr Lys Lys Ser Arg Asn Asp Ile Glu Leu Lys Ala Ala
                   725                 730                 735
   Leu Thr Gln Met Val Ser Asn Asn Glu Met Ile Met Ser Ala Ile Lys
               740                 745                 750
   Ser Gly Thr Glu Lys Leu Gly Leu Leu Ile Asn Asp Asp Glu Thr Met
           755                 760                 765
   Gln Ser Ala Asp Tyr Leu Asn Tyr Gly Lys Val Pro Ile Phe Arg Gly
       770                 775                 780
   Val Ile Arg Gly Leu Glu Thr Lys Arg Trp Ser Arg Val Thr Cys Val
   785                 790                 795                 800
   Thr Asn Asp Gln Ile Pro Thr Cys Ala Asn Ile Met Ser Ser Val Ser
                   805                 810                 815
   Thr Asn Ala Leu Thr Val Ala His Phe Ala Glu Asn Pro Val Asn Ala
               820                 825                 830
   Ile Ile Gln Tyr Asn Tyr Phe Gly Thr Phe Ala Arg Leu Leu Leu Met
           835                 840                 845
   Met His Asp Pro Ala Leu Arg Ile Ser Leu Tyr Glu Val Gln Ser Lys
       850                 855                 860
   Ile Pro Gly Leu His Ser Leu Thr Phe Lys Tyr Ser Met Leu Tyr Leu
   865                 870                 875                 880
   Asp Pro Ser Ile Gly Gly Val Ser Gly Met Ser Leu Ser Arg Phe Leu
                   885                 890                 895
   Ile Arg Ser Phe Pro Asp Pro Val Thr Glu Ser Leu Ala Phe Trp Lys
               900                 905                 910
   Phe Ile His Ser His Ala Arg Ser Asp Ser Leu Lys Glu Ile Cys Ala
           915                 920                 925
   Val Phe Gly Asn Pro Glu Ile Ala Arg Phe Arg Leu Thr His Val Asp
       930                 935                 940
   Lys Leu Val Glu Asp Pro Thr Ser Leu Asn Ile Ala Met Gly Met Ser
   945                 950                 955                 960
   Pro Ala Asn Leu Leu Lys Thr Glu Val Lys Lys Cys Leu Leu Glu Ser
                   965                 970                 975
   Arg Gln Ser Ile Lys Asn Gln Ile Val Arg Asp Ala Thr Ile Tyr Leu
               980                 985                 990
   His His Glu Glu Asp Lys Leu Arg  Ser Phe Leu Trp Ser  Ile Thr Pro
           995                 1000                1005
   Leu Phe  Pro Arg Phe Leu Ser  Glu Phe Lys Ser Gly  Thr Phe Ile
       1010                 1015                 1020
   Gly Val  Ala Asp Gly Leu Ile  Ser Leu Phe Gln Asn  Ser Arg Thr
       1025                 1030                 1035
   Ile Arg  Asn Ser Phe Lys Lys  Arg Tyr His Arg Glu  Leu Asp Asp
       1040                 1045                 1050
   Leu Ile  Ile Lys Ser Glu Val  Ser Ser Leu Met His  Leu Gly Lys
       1055                 1060                 1065
   Leu His  Leu Arg Arg Gly Ser  Val Arg Met Trp Thr  Cys Ser Ser
       1070                 1075                 1080
   Thr Gln  Ala Asp Leu Leu Arg  Phe Arg Ser Trp Gly  Arg Ser Val
       1085                 1090                 1095
   Ile Gly  Thr Thr Val Pro His  Pro Leu Glu Met Leu  Gly Gln His
       1100                 1105                 1110
   Phe Lys  Lys Glu Thr Pro Cys  Ser Ala Cys Asn Ile  Ser Gly Leu
       1115                 1120                 1125
   Asp Tyr  Val Ser Val His Cys  Pro Asn Gly Ile His  Asp Val Phe
       1130                 1135                 1140
   Glu Ser  Arg Gly Pro Leu Pro  Ala Tyr Leu Gly Ser  Lys Thr Ser
       1145                 1150                 1155
   Glu Ser  Thr Ser Ile Leu Gln  Pro Trp Glu Arg Glu  Ser Lys Val
       1160                 1165                 1170
   Pro Leu  Ile Lys Arg Ala Thr  Arg Leu Arg Asp Ala  Ile Ser Trp
       1175                 1180                 1185
   Phe Val  Ser Pro Asp Ser Asn  Leu Ala Ser Thr Ile  Leu Lys Asn
       1190                 1195                 1200
   Ile Asn  Ala Leu Thr Gly Glu  Glu Trp Ser Lys Lys  Gln His Gly
       1205                 1210                 1215
   Phe Lys  Arg Thr Gly Ser Ala  Leu His Arg Phe Ser  Thr Ser Arg
       1220                 1225                 1230
   Met Ser  His Gly Gly Phe Ala  Ser Gln Ser Thr Ala  Ala Leu Thr
       1235                 1240                 1245
   Arg Leu  Met Ala Thr Thr Asp  Thr Met Arg Asp Leu  Gly Glu Gln
       1250                 1255                 1260
   Asn Tyr  Asp Phe Leu Phe Gln  Ala Thr Leu Leu Tyr  Ala Gln Ile
       1265                 1270                 1275
   Thr Thr  Thr Val Val Arg Asn  Gly Ser Phe His Ser  Cys Thr Asp
       1280                 1285                 1290
   His Tyr  His Ile Thr Cys Lys  Ser Cys Leu Arg Ala  Ile Asp Glu
       1295                 1300                 1305
   Ile Thr  Leu Asp Ser Ala Met  Glu Tyr Ser Pro Pro  Asp Val Ser
       1310                 1315                 1320
   Ser Val  Leu Gln Ser Trp Arg  Asn Gly Glu Gly Ser  Trp Gly His
       1325                 1330                 1335
   Glu Val  Lys Gln Ile Tyr Pro  Val Glu Gly Asp Trp  Arg Gly Leu
       1340                 1345                 1350
   Ser Pro  Val Glu Gln Ser Tyr  Gln Val Gly Arg Cys  Ile Gly Phe
       1355                 1360                 1365
   Leu Phe  Gly Asp Leu Ala Tyr  Arg Lys Ser Ser His  Ala Asp Asp
       1370                 1375                 1380
   Ser Ser  Met Phe Pro Leu Ser  Ile Gln Asn Lys Val  Arg Gly Arg
   1385 1390 1395
   Gly Phe Leu Lys Gly Leu Met Asp Gly Leu Met Arg Ala Ser Cys
   1400 1405 1410
   Cys Gln Val Ile His Arg Arg Ser Leu Ala His Leu Lys Arg Pro
   1415 1420 1425
   Ala Asn Ala Val Tyr Gly Gly Leu Ile Tyr Leu Ile Asp Lys Leu
   1430 1435 1440
   Ser Ala Ser Ala Pro Phe Leu Ser Leu Thr Arg His Gly Pro Leu
   1445 1450 1455
   Arg Glu Glu Leu Glu Thr Val Pro His Lys Ile Pro Thr Ser Tyr
   1460 1465 1470
   Pro Thr Ser Asn Arg Asp Met Gly Val Ile Val Arg Asn Tyr Phe
   1475 1480 1485
   Lys Tyr Gln Cys Arg Leu Val Glu Lys Gly Arg Tyr Lys Thr His
   1490 1495 1500
   Tyr Pro Gln Leu Trp Leu Phe Ser Asp Val Leu Ser Ile Asp Phe
   1505 1510 1515
   Leu Gly Pro Leu Ser Ile Ser Ser Thr Leu Leu Gly Ile Leu Tyr
   1520 1525 1530
   Lys Gln Thr Leu Ser Ser Arg Asp Lys Asn Glu Leu Arg Glu Leu
   1535 1540 1545
   Ala Asn Leu Ser Ser Leu Leu Arg Ser Gly Glu Gly Trp Glu Asp
   1550 1555 1560
   Ile His Val Lys Phe Phe Ser Lys Asp Thr Leu Leu Cys Pro Glu
   1565 1570 1575
   Glu Ile Arg His Ala Cys Lys Phe Gly Ile Ala Lys Glu Ser Ala
   1580 1585 1590
   Val Leu Ser Tyr Tyr Pro Pro Trp Ser Gln Glu Ser Tyr Gly Gly
   1595 1600 1605
   Ile Thr Ser Ile Pro Val Tyr Phe Ser Thr Arg Lys Tyr Pro Lys
   1610 1615 1620
   Ile Leu  Asp Val Pro Pro Arg  Val Gln Asn Pro Leu  Val Ser Gly
       1625                 1630                 1635
   Leu Arg  Leu Gly Gln Leu Pro  Thr Gly Ala His Tyr  Lys Ile Arg
       1640                 1645                 1650
   Ser Ile  Val Lys Asn Lys Asn  Leu Arg Tyr Arg Asp  Phe Leu Ser
       1655                 1660                 1665
   Cys Gly  Asp Gly Ser Gly Gly  Met Thr Ala Ala Leu  Leu Arg Glu
       1670                 1675                 1680
   Asn Arg  Gln Ser Arg Gly Ile  Phe Asn Ser Leu Leu  Glu Leu Ala
       1685                 1690                 1695
   Gly Ser  Leu Met Arg Gly Ala  Ser Pro Glu Pro Pro  Ser Ala Leu
       1700                 1705                 1710
   Glu Thr  Leu Gly Gln Glu Arg  Ser Arg Cys Val Asn  Gly Ser Thr
       1715                 1720                 1725
   Cys Trp  Glu Tyr Ser Ser Asp  Leu Ser Gln Lys Glu  Thr Trp Asp
       1730                 1735                 1740
   Tyr Phe  Leu Arg Leu Lys Arg  Gly Leu Gly Leu Thr  Val Asp Leu
       1745                 1750                 1755
   Ile Thr  Met Asp Met Glu Val  Arg Asp Pro Asn Thr  Ser Leu Met
       1760                 1765                 1770
   Ile Glu  Lys Asn Leu Lys Val  Tyr Leu His Gln Ile  Leu Glu Pro
       1775                 1780                 1785
   Thr Gly  Val Leu Ile Tyr Lys  Thr Tyr Gly Thr His  Ile Ala Thr
       1790                 1795                 1800
   Gln Thr  Asp Asn Ile Leu Thr  Ile Ile Gly Pro Phe  Phe Glu Thr
       1805                 1810                 1815
   Val Asp  Leu Val Gln Ser Glu  Tyr Ser Ser Ser Gln  Thr Ser Glu
       1820                 1825                 1830
   Val Tyr  Phe Val Gly Arg Gly  Leu Arg Ser His Val  Asp Glu Pro
       1835                 1840                 1845
   Trp Val  Asp Trp Pro Ser Leu  Met Asp Asn Trp Arg  Ser Ile Tyr
       1850                 1855                 1860
   Ala Phe His Asp Pro Thr Thr Glu Phe Ile Arg Ala Lys Lys Val
       1865                1870                1875
   Cys Glu Ile Asp Ser Leu Ile Gly Ile Pro Ala Gln Phe Ile Pro
       1880                1885                1890
   Asp Pro Phe Val Asn Leu Glu Thr Met Leu Gln Ile Val Gly Val
       1895                1900                1905
   Pro Thr Gly Val Ser His Ala Ala Ala Leu Leu Ser Ser Gln Tyr
       1910                1915                1920
   Pro Asn Gln Leu Val Thr Thr Ser Ile Phe Tyr Met Thr Leu Val
       1925                1930                1935
   Ser Tyr Tyr Asn Val Asn His Ile Arg Arg Ser Pro Lys Pro Phe
       1940                1945                1950
   Ser Pro Pro Ser Asp Gly Val Ser Gln Asn Ile Gly Ser Ala Ile
       1955                1960                1965
   Val Gly Leu Ser Phe Trp Val Ser Leu Met Glu Asn Asp Leu Gly
       1970                1975                1980
   Leu Tyr Lys Gln Ala Leu Gly Ala Ile Lys Thr Ser Phe Pro Ile
       1985                1990                1995
   Arg Trp Ser Ser Val Gln Thr Lys Asp Gly Phe Thr Gln Glu Trp
       2000                2005                2010
   Arg Thr Lys Gly Asn Gly Ile Pro Lys Asp Cys Arg Leu Ser Asp
       2015                2020                2025
   Ser Leu Ala Gln Ile Gly Asn Trp Ile Arg Ala Met Glu Leu Val
       2030                2035                2040
   Arg Asn Lys Thr Arg Gln Ser Gly Phe Ser Glu Thr Leu Phe Asp
       2045                2050                2055
   Gln Phe Cys Gly Leu Ala Asp His His Leu Lys Trp Arg Lys Leu
       2060                2065                2070
   Gly Asn Arg Thr Gly Ile Ile Asp Trp Leu Asn Asn Arg Ile Ser
       2075                2080                2085
   Ser Ile Asp Lys Ser Ile Leu Val Thr Lys Ser Asp Leu His Asp
       2090                2095                2100
   Glu Asn Ser Trp Arg Glu
   2105
<210> 4
   <211> 229
   <212> PRT
   <213> Maraba virus
<400> 4
   Met Ser Ser Leu Lys Lys Ile Leu Gly Ile Lys Gly Lys Gly Lys Lys
   1 5 10 15
   Ser Lys Lys Leu Gly Met Ala Pro Pro Pro Tyr Glu Glu Glu Thr Pro
   20 25 30
   Met Glu Tyr Ser Pro Ser Ala Pro Tyr Asp Lys Ser Leu Phe Gly Val
   35 40 45
   Glu Asp Met Asp Phe His Asp Gln Arg Gln Leu Arg Tyr Glu Lys Phe
   50 55 60
   His Phe Ser Leu Lys Met Thr Val Arg Ser Asn Lys Pro Phe Arg Asn
   65 70 75 80
   Tyr Asp Asp Val Ala Ala Ala Val Ser Asn Trp Asp His Met Tyr Ile
   85 90 95
   Gly Met Ala Gly Lys Arg Pro Phe Tyr Lys Ile Leu Ala Phe Met Gly
   100 105 110
   Ser Thr Leu Leu Lys Ala Thr Pro Ala Val Leu Ala Asp Gln Gly Gln
   115 120 125
   Pro Glu Tyr His Ala His Cys Glu Gly Arg Ala Tyr Leu Pro His Arg
   130 135 140
   Leu Gly Pro Thr Pro Pro Met Leu Asn Val Pro Glu His Phe Arg Arg
   145 150 155 160
   Pro Phe Asn Ile Gly Leu Phe Arg Gly Thr Ile Asp Ile Thr Leu Val
   165 170 175
   Leu Phe Asp Asp Glu Ser Val Asp Ser Ala Pro Val Ile Trp Asp His
   180 185 190
   Phe Asn Ala Ser Arg Leu Ser Ser Phe Arg Glu Lys Ala Leu Leu Phe
   195 200 205
   Gly Leu Ile Leu Glu Lys Lys Ala Thr Gly Asn Trp Val Leu Asp Ser
       210                 215                 220
   Ile Ser His Phe Lys
   225
<210> 5
   <211> 228
   <212> PRT
   <213> Artificial
<220>
   <223> mutant of Maraba protein M
<400> 5
   Met Ser Ser Leu Lys Lys Ile Leu Gly Ile Lys Gly Lys Gly Lys Lys
   1               5                   10                  15
   Ser Lys Lys Leu Gly Met Ala Pro Pro Pro Tyr Glu Glu Glu Thr Pro
               20                  25                  30
   Met Glu Tyr Ser Pro Ser Ala Pro Tyr Asp Lys Ser Leu Phe Gly Val
           35                  40                  45
   Glu Asp Asp Phe His Asp Gln Arg Gln Leu Arg Tyr Glu Lys Phe His
       50                  55                  60
   Phe Ser Leu Lys Met Thr Val Arg Ser Asn Lys Pro Phe Arg Asn Tyr
   65                  70                  75                  80
   Asp Asp Val Ala Ala Ala Val Ser Asn Trp Asp His Met Tyr Ile Gly
                   85                  90                  95
   Met Ala Gly Lys Arg Pro Phe Tyr Lys Ile Leu Ala Phe Met Gly Ser
               100                 105                 110
   Thr Leu Leu Lys Ala Thr Pro Ala Val Leu Ala Asp Gln Gly Gln Pro
           115                 120                 125
   Glu Tyr His Ala His Cys Glu Gly Arg Ala Tyr Leu Pro His Arg Leu
       130                 135                 140
   Gly Pro Thr Pro Pro Met Leu Asn Val Pro Glu His Phe Arg Arg Pro
   145                 150                 155                 160
   Phe Asn Ile Gly Leu Phe Arg Gly Thr Ile Asp Ile Thr Leu Val Leu
                   165                 170                 175
   Phe Asp Asp Glu Ser Val Asp Ser Ala Pro Val Ile Trp Asp His Phe
   180 185 190
   Asn Ala Ser Arg Leu Ser Ser Phe Arg Glu Lys Ala Leu Leu Phe Gly
   195 200 205
   Leu Ile Leu Glu Lys Lys Ala Thr Gly Asn Trp Val Leu Asp Ser Ile
   210 215 220
   Ser His Phe Lys
   225
<210> 6
   <211> 591
   <212> PRT
   <213> Bahia Grande virus
<400> 6
   Met Ile Ser Asn Met Phe Phe Leu Phe Gln Leu Ser Leu Phe Leu Gln
   1 5 10 15
   Phe Ile Ala Gly Asp Glu Ser Leu Glu Thr Ile Thr Ala Pro Glu Thr
   20 25 30
   Pro Asp Pro Ile Leu Leu Lys Gly Asp Thr Lys Tyr Leu Phe Leu Val
   35 40 45
   Pro Ser Ser Val Lys Asn Trp Lys Pro Ala Asp Leu Asn Glu Leu Thr
   50 55 60
   Cys Pro Pro Leu Ile Ser Lys Pro Asp Thr Ser Glu Met Thr Tyr Phe
   65 70 75 80
   Ser Thr Asp Val Met Glu Leu Gln Lys His His Glu Leu Ala Pro Val
   85 90 95
   Glu Gly Tyr Leu Cys Ser Gly Leu Arg Tyr Lys Val Ile Cys Ser Glu
   100 105 110
   Gly Phe Phe Gly Gln Lys Thr Ile Ala Lys Lys Ile Glu Asn Ile Glu
   115 120 125
   Pro Asp Ser Lys Gln Cys Leu Asp Asp Leu Ser Lys Phe Lys Asn Asp
   130 135 140
   Asp Tyr Leu Leu Pro Tyr Phe Pro Ser Glu Asp Cys Asn Trp Met Lys
   145 150 155 160
   Glu Thr Pro Thr His Lys Asp Phe Ile Val Phe Gln Lys His Phe Val
                   165                 170                 175
   Lys Tyr Asp Pro Tyr Asn Asn Gly Phe Tyr Asp Pro Leu Leu Lys Lys
               180                 185                 190
   Asp Tyr Cys Asp Thr Gln Val Cys Glu Thr Glu His Asp Gln Thr Ile
           195                 200                 205
   Trp Ile Thr Glu Lys Ser Ile Glu Asn Glu Cys Ile Phe Asn Tyr Pro
       210                 215                 220
   Ile Lys Lys His Ile Phe His Thr Ala Asp Phe Gly Lys Met Ile Ile
   225                 230                 235                 240
   Asp Tyr Glu Leu Asn Gln Trp Thr Ser Val Glu Asp Gly Cys Leu Ile
                   245                 250                 255
   Asn Tyr Cys Gly Arg Glu Gly Ile Arg Leu Ser Asn Gly Met Phe Phe
               260                 265                 270
   Val Gly Lys Phe Tyr Lys Asn Leu Asn Asn Leu Gln Thr Cys Ser Ala
           275                 280                 285
   Gly Thr Lys Val Ser Tyr Lys Pro Leu Thr Ser Lys Leu Glu Glu Ile
       290                 295                 300
   Glu Asn Glu Ile Ile Leu Asp Gln Glu Arg Leu Leu Cys Leu Asp Ser
   305                 310                 315                 320
   Ile Arg Gln Met Thr Ala Thr Lys Lys Leu Ser Phe Tyr Ser Leu Ser
                   325                 330                 335
   Phe Leu Glu Pro Lys Ser Ser Ser Arg His Lys Val Phe Arg Ile His
               340                 345                 350
   Asn Lys Thr Leu Glu Tyr Thr Glu Thr Glu Trp His Pro Ile Met Ser
           355                 360                 365
   Phe Asn Phe Asp Glu Pro Asn Lys Ile Gly Ile Asp Lys Asn Gly Lys
       370                 375                 380
   Ser Val Tyr Trp Asn Glu Trp Val Pro Ser Gly Ile Ser Gly Leu Leu
   385                 390                 395                 400
   Ser Gly Phe Asn Gly Val Tyr Lys Lys Glu Asn Glu Thr Lys Val Thr
                   405                 410                 415
   Ile Ala Arg Leu Glu Thr Ile Lys Glu Asp Tyr Asp Arg Glu Met Met
               420                 425                 430
   Ile Asp His Glu Leu Val Glu Val Glu His Pro Lys Ile Val His Leu
           435                 440                 445
   Lys Arg Glu Asn Ile Thr Gly Ser Arg Val Glu Ile Val Asn Lys Glu
       450                 455                 460
   His Ser Asp Val Ser Gly Trp Leu Ser Ser Val Leu Ser Ser Phe Trp
   465                 470                 475                 480
   Gly Lys Ile Met Met Thr Ile Ile Ser Ile Ile Leu Ile Val Ile Ile
                   485                 490                 495
   Gly Leu Val Leu Ile Asn Cys Cys Pro Ile Ile Cys Lys Ser Cys Ile
               500                 505                 510
   Lys Arg Tyr Lys Thr Lys Glu Glu Ser Arg Asn Arg His Arg Leu Asp
           515                 520                 525
   Arg Glu Asp Asn Gly Arg Leu Arg Arg Gln His Arg Val Ile Phe Asn
       530                 535                 540
   Asn Gln Ser Asn Asp Glu Glu Asn Ala Ile Glu Met Val Glu Tyr Thr
   545                 550                 555                 560
   Asp Thr Pro Arg Pro Leu Arg Pro Ile Pro Asp Ala Thr Thr Ser Asp
                   565                 570                 575
   Thr Glu Ser Arg Ser Pro Thr Thr Ala His Ser Phe Phe Asn Arg
               580                 585                 590
<210> 7
   <211> 498
   <212> PRT
   <213> Lymphocytic choriomeningitis virus
<400> 7
   Met Gly Gln Ile Val Thr Met Phe Glu Ala Leu Pro His Ile Ile Asp
   1               5                   10                  15
   Glu Val Ile Asn Ile Val Ile Ile Val Leu Ile Ile Ile Thr Ser Ile
               20                  25                  30
   Lys Ala Val Tyr Asn Phe Ala Thr Cys Gly Ile Leu Ala Leu Val Arg
           35                  40                  45
   Phe Leu Phe Leu Ala Gly Arg Ser Cys Gly Met Tyr Gly Leu Asn Gly
       50                  55                  60
   Pro Asp Ile Tyr Lys Gly Val Tyr Gln Phe Lys Ser Val Glu Phe Asp
   65                  70                  75                  80
   Met Ser His Leu Asn Leu Thr Met Pro Asn Ala Cys Ser Ala Asn Asn
                   85                  90                  95
   Ser His His Tyr Ile Ser Met Gly Ser Ser Gly Leu Glu Leu Thr Phe
               100                 105                 110
   Thr Asn Asp Ser Ile Leu Asn His Asn Phe Cys Asn Leu Thr Ser Ala
           115                 120                 125
   Phe Asn Lys Lys Thr Phe Asp His Thr Leu Met Ser Ile Val Ser Ser
       130                 135                 140
   Leu His Leu Ser Ile Arg Gly Asn Ser Asn His Lys Ala Val Ser Cys
   145                 150                 155                 160
   Asp Phe Asn Asn Gly Ile Thr Ile Gln Tyr Asn Leu Ser Phe Ser Asp
                   165                 170                 175
   Pro Gln Ser Ala Ile Ser Gln Cys Arg Thr Phe Arg Gly Arg Val Leu
               180                 185                 190
   Asp Met Phe Arg Thr Ala Phe Gly Gly Lys Tyr Met Arg Ser Gly Trp
           195                 200                 205
   Gly Trp Ala Gly Ser Asp Gly Lys Thr Thr Trp Cys Ser Gln Thr Ser
       210                 215                 220
   Tyr Gln Tyr Leu Ile Ile Gln Asn Arg Thr Trp Glu Asn His Cys Arg
   225                 230                 235                 240
   Tyr Ala Gly Pro Phe Gly Met Ser Arg Ile Leu Phe Ala Gln Glu Lys
                   245                 250                 255
   Thr Lys Phe Leu Thr Arg Arg Leu Ala Gly Thr Phe Thr Trp Thr Leu
               260                 265                 270
   Ser Asp Ser Ser Gly Val Glu Asn Pro Gly Gly Tyr Cys Leu Thr Lys
           275                 280                 285
   Trp Met Ile Leu Ala Ala Glu Leu Lys Cys Phe Gly Asn Thr Ala Val
   290 295 300
   Ala Lys Cys Asn Val Asn His Asp Glu Glu Phe Cys Asp Met Leu Arg
   305 310 315 320
   Leu Ile Asp Tyr Asn Lys Ala Ala Leu Ser Lys Phe Lys Gln Asp Val
   325 330 335
   Glu Ser Ala Leu His Val Phe Lys Thr Thr Val Asn Ser Leu Ile Ser
   340 345 350
   Asp Gln Leu Leu Met Arg Asn His Leu Arg Asp Leu Met Gly Val Pro
   355 360 365
   Tyr Cys Asn Tyr Ser Lys Phe Trp Tyr Leu Glu His Ala Lys Thr Gly
   370 375 380
   Glu Thr Ser Val Pro Lys Cys Trp Leu Val Thr Asn Gly Ser Tyr Leu
   385 390 395 400
   Asn Glu Thr His Phe Ser Asp Gln Ile Glu Gln Glu Ala Asp Asn Met
   405 410 415
   Ile Thr Glu Met Leu Arg Lys Asp Tyr Ile Lys Arg Gln Gly Ser Thr
   420 425 430
   Pro Leu Ala Leu Met Asp Leu Leu Met Phe Ser Thr Ser Ala Tyr Leu
   435 440 445
   Ile Ser Ile Phe Leu His Leu Val Lys Ile Pro Thr His Arg His Ile
   450 455 460
   Lys Gly Gly Ser Cys Pro Lys Pro His Arg Leu Thr Asn Lys Gly Ile
   465 470 475 480
   Cys Ser Cys Gly Ala Phe Lys Val Pro Gly Val Lys Thr Ile Trp Lys
   485 490 495
   Arg Arg
<210> 8
   <211> 676
   <212> PRT
   <213> Ebola virus
<400> 8
   Met Gly Val Thr Gly Ile Leu Gln Leu Pro Arg Asp Arg Phe Lys Arg
   1               5                   10                  15
   Thr Ser Phe Phe Leu Trp Val Ile Ile Leu Phe Gln Arg Thr Phe Ser
               20                  25                  30
   Ile Pro Leu Gly Val Ile His Asn Ser Thr Leu Gln Val Ser Asp Val
           35                  40                  45
   Asp Lys Leu Val Cys Arg Asp Lys Leu Ser Ser Thr Asn Gln Leu Arg
       50                  55                  60
   Ser Val Gly Leu Asn Leu Glu Gly Asn Gly Val Ala Thr Asp Val Pro
   65                  70                  75                  80
   Ser Ala Thr Lys Arg Trp Gly Phe Arg Ser Gly Val Pro Pro Lys Val
                   85                  90                  95
   Val Asn Tyr Glu Ala Gly Glu Trp Ala Glu Asn Cys Tyr Asn Leu Glu
               100                 105                 110
   Ile Lys Lys Pro Asp Gly Ser Glu Cys Leu Pro Ala Ala Pro Asp Gly
           115                 120                 125
   Ile Arg Gly Phe Pro Arg Cys Arg Tyr Val His Lys Val Ser Gly Thr
       130                 135                 140
   Gly Pro Cys Ala Gly Asp Phe Ala Phe His Lys Glu Gly Ala Phe Phe
   145                 150                 155                 160
   Leu Tyr Asp Arg Leu Ala Ser Thr Val Ile Tyr Arg Gly Thr Thr Phe
                   165                 170                 175
   Ala Glu Gly Val Val Ala Phe Leu Ile Leu Pro Gln Ala Lys Lys Asp
               180                 185                 190
   Phe Phe Ser Ser His Pro Leu Arg Glu Pro Val Asn Ala Thr Glu Asp
           195                 200                 205
   Pro Ser Ser Gly Tyr Tyr Ser Thr Thr Ile Arg Tyr Gln Ala Thr Gly
       210                 215                 220
   Phe Gly Thr Asn Glu Thr Glu Tyr Leu Phe Glu Val Asp Asn Leu Thr
   225                 230                 235                 240
   Tyr Val Gln Leu Glu Ser Arg Phe Thr Pro Gln Phe Leu Leu Gln Leu
                   245                 250                 255
   Asn Glu Thr Ile Tyr Thr Ser Gly Lys Arg Ser Asn Thr Thr Gly Lys
               260                 265                 270
   Leu Ile Trp Lys Val Asn Pro Glu Ile Asp Thr Thr Ile Gly Glu Trp
           275                 280                 285
   Ala Phe Trp Glu Thr Lys Lys Asn Leu Thr Arg Lys Ile Arg Ser Glu
       290                 295                 300
   Glu Leu Ser Phe Thr Val Val Ser Asn Gly Ala Lys Asn Ile Ser Gly
   305                 310                 315                 320
   Gln Ser Pro Ala Arg Thr Ser Ser Asp Pro Gly Thr Asn Thr Thr Thr
                   325                 330                 335
   Glu Asp His Lys Ile Met Ala Ser Glu Asn Ser Ser Ala Met Val Gln
               340                 345                 350
   Val His Ser Gln Gly Arg Glu Ala Ala Val Ser His Leu Thr Thr Leu
           355                 360                 365
   Ala Thr Ile Ser Thr Ser Pro Gln Ser Leu Thr Thr Lys Pro Gly Pro
       370                 375                 380
   Asp Asn Ser Thr His Asn Thr Pro Val Tyr Lys Leu Asp Ile Ser Glu
   385                 390                 395                 400
   Ala Thr Gln Val Glu Gln His His Arg Arg Thr Asp Asn Asp Ser Thr
                   405                 410                 415
   Ala Ser Asp Thr Pro Ser Ala Thr Thr Ala Ala Gly Pro Pro Lys Ala
               420                 425                 430
   Glu Asn Thr Asn Thr Ser Lys Ser Thr Asp Phe Leu Asp Pro Ala Thr
           435                 440                 445
   Thr Thr Ser Pro Gln Asn His Ser Glu Thr Ala Gly Asn Asn Asn Thr
       450                 455                 460
   His His Gln Asp Thr Gly Glu Glu Ser Ala Ser Ser Gly Lys Leu Gly
   465                 470                 475                 480
   Leu Ile Thr Asn Thr Ile Ala Gly Val Ala Gly Leu Ile Thr Gly Gly
                   485                 490                 495
   Arg Arg Thr Arg Arg Glu Ala Ile Val Asn Ala Gln Pro Lys Cys Asn
               500                 505                 510
   Pro Asn Leu His Tyr Trp Thr Thr Gln Asp Glu Gly Ala Ala Ile Gly
           515                 520                 525
   Leu Ala Trp Ile Pro Tyr Phe Gly Pro Ala Ala Glu Gly Ile Tyr Ile
       530                 535                 540
   Glu Gly Leu Met His Asn Gln Asp Gly Leu Ile Cys Gly Leu Arg Gln
   545                 550                 555                 560
   Leu Ala Asn Glu Thr Thr Gln Ala Leu Gln Leu Phe Leu Arg Ala Thr
                   565                 570                 575
   Thr Glu Leu Arg Thr Phe Ser Ile Leu Asn Arg Lys Ala Ile Asp Phe
               580                 585                 590
   Leu Leu Gln Arg Trp Gly Gly Thr Cys His Ile Leu Gly Pro Asp Cys
           595                 600                 605
   Cys Ile Glu Pro His Asp Trp Thr Lys Asn Ile Thr Asp Lys Ile Asp
       610                 615                 620
   Gln Ile Ile His Asp Phe Val Asp Lys Thr Leu Pro Asp Gln Gly Asp
   625                 630                 635                 640
   Asn Asp Asn Trp Trp Thr Gly Trp Arg Gln Trp Ile Pro Ala Gly Ile
                   645                 650                 655
   Gly Val Thr Gly Val Ile Ile Ala Val Ile Ala Leu Phe Cys Ile Cys
               660                 665                 670
   Lys Phe Val Phe
           675
<210> 9
   <211> 11380
   <212> DNA
   <213> Artificial
<220>
   <223> DNA encoded by chimeric virus that contains RNA from Maraba and Bahia Grande viruses
<400> 9
   acgaagacaa acaaaccatt gatagaatta agaggctcat gaaaatcctt aacagcgttc      60
   aaaatgtctg ttacagtcaa gagagtcatt gatgattcac tcatcacccc caaattgcct     120
   gcgaatgagg accctgtgga gtaccctgct gattatttca aaaagtcccg tgatattccg     180
   gtgtacataa acacgaccaa aagtttgtct gatttgcggg gctatgttta tcaaggccta     240
   aagtcaggca acatctctat aattcatgtc aacagttatc tgtatgcagc attaaaagag 300
   atcagaggaa aattggacag agattggatc acctttggta tccaaatcgg aaaaacagga 360
   gatagcgtgg ggatattcga tttactgacc ctaaaacctc tagatggtgt tttaccagat 420
   ggggtgtctg atgctactcg aactagctca gacgatgcat ggcttccact gtatctattg 480
   gggttataca gagttggtcg aacacagatg ccagaataca ggaagaagct gatggatggt 540
   ctgattaatc aatgtaagat gatcaatgag cagtttgaac cactgttgcc agaaggaaga 600
   gatgtctttg atgtctgggg aaatgacagc aattacacaa agattgtggc cgctgtagat 660
   atgttcttcc atatgttcaa aaagcatgag aaggcctctt tcaggtatgg cacaatagtg 720
   tcaagattta aggattgtgc agcattggct acatttggtc atctgtgtaa gatcactggt 780
   atgtccactg aagatgtgac aacttggatt ctaaacaggg aggtggctga tgagatggtt 840
   caaatgatgt acccaggaca ggagatagat aaggctgatt cttacatgcc ttatctaatc 900
   gacttaggtc tgtcctcaaa atctccatat tcatcagtta aaaatccagc tttccatttt 960
   tggggtcaat tgaccgcatt gttactgaga tcaaccagag ccagaaatgc acgtcagccg 1020
   gatgacatcg agtatacatc cctgaccact gctgggctgt tgtatgcata tgccgttggt 1080
   tcgtctgcag acctggctca acaattctac gttggggaca acaagtatgt gccagaaact 1140
   ggagatggag gattaaccac caatgcaccg ccacaagggc gagatgtggt cgagtggctt 1200
   agttggtttg aagatcaaaa cagaaaacct accccagaca tgctcatgta tgctaagaga 1260
   gctgtcagtg ctttacaagg attgagggag aagacgattg gcaagtacgc caagtcagag 1320
   tttgacaaat gacaactcac tcaccatatg tattactacc tttgcttcat atgaaaaaaa 1380
   ctaacagcga tcatggatca gctatcaaag gtcaaggaat tccttaagac ttacgcgcag 1440
   ttggatcaag cagtacaaga gatggatgac attgagtctc agagagagga aaagactaat 1500
   tttgatttgt ttcaggaaga aggattggag attaaggaga agccttccta ttatcgggca 1560
   gatgaagaag agattgattc agatgaagac agcgtggatg atgcacaaga cttagggata 1620
   cgtacatcaa caagtcccat cgaggggtat gtggatgagg agcaggatga ttatgaggat 1680
   gaggaagtga acgtggtgtt tacatcggac tggaaacagc ctgagctgga atccgacggg 1740
   gatgggaaaa ctctccgatt gacgatacca gatggattga ctggggagca gaagtcgcaa 1800
   tggcttgcca cgattaaggc agttgttcag agtgctaaat attggaacat ctcagaatgt 1860
   tcatttgaga gttatgagca aggggttttg attagagaga gacaaatgac tcctgatgtc 1920
   tacaaagtca ctcctgtttt aaatgctcca ccggttcaaa tgacagctaa tcaagatgtt 1980
   tggtctctca gcagcactcc atttacattt ttgcccaaga aacaaggtgt gactccattg 2040
   accatgtcct tagaagaact cttcaacacc cgaggtgaat tcatatctct gggaggaaac 2100
   gggaaaatga gtcaccggga ggccatcatt ctagggttga gacacaagaa gctctataat 2160
   caagccagac taaagtataa cttagcttga atatgaaaaa aactaacaga tatcaaaaga 2220
   tatctctaac tcagtccatt gtgttcagtt caatcatgag ctctctcaag aaaattttgg 2280
   gtattaaagg gaaagggaag aaatctaaga aattaggtat ggctccccca ccctatgaag 2340
   aagagactcc aatggaatat tctccaagtg caccttatga taagtcattg tttggagtcg 2400
   aagatatgga tttccatgat caacgtcaac tccgatatga gaaatttcac ttctcattga 2460
   agatgactgt gagatcaaac aaaccatttc gaaattatga tgacgttgca gcagcggtgt 2520
   ccaattggga tcatatgtac atcggcatgg caggaaaacg tcctttttat aagatattag 2580
   cattcatggg ttctactcta ttgaaggcta caccagctgt cttggctgac caaggacagc 2640
   cagaatatca tgctcactgt gagggacgag cttacttgcc gcatcggtta gggccgaccc 2700
   ctccgatgtt gaatgtccct gaacattttc gccgtccatt taacatcgga ttattcagag 2760
   ggacaatcga cataaccctg gtacttttcg atgatgaatc tgtagattct gccccggtca 2820
   tatgggatca ttttaatgca tccagattga gcagcttcag agaaaaggct ttgttgtttg 2880
   gtttgattct agaaaagaaa gccactggga attgggtatt ggactctatt agtcatttca 2940
   agtaattatc acaagtgttg aggtgatggg cagactatga aaaaaactaa cagggttcaa 3000
   acactcttga tcgaggtacc cagttatatt tgttacaaca atgatttcga atatgttttt 3060
   cttgtttcaa ctctcattat ttctacagtt tatagcagga gatgagtcat tagaaacaat 3120
   aacagcccct gaaactcctg accctatact cttaaaagga gatacaaaat atctgttctt 3180
   agtcccttct tctgtcaaaa attggaaacc agctgacctg aatgaattaa catgcccccc 3240
   cctaatctcg aaaccagata cttctgaaat gacttatttt tccacagatg tgatggagtt 3300
   acaaaaacat catgaattgg caccagtaga agggtattta tgttcgggtt tgcgttacaa 3360
   agtaatatgt tctgaaggat tttttggaca aaaaacaata gcaaaaaaga ttgagaacat 3420
   tgaacctgat agtaaacaat gccttgatga cttgtcaaaa tttaagaatg atgattacct 3480
   actcccatat ttcccttctg aagattgtaa ttggatgaaa gagactccca cccataaaga 3540
   ttttatagtt tttcaaaaac attttgttaa atatgaccca tacaataatg gtttttatga 3600
   tcctttactt aaaaaagact actgtgatac tcaagtctgt gagacagaac atgatcaaac 3660
   tatttggata acagaaaaga gtattgaaaa tgaatgcatc ttcaattatc cgattaaaaa 3720
   gcatatattc catacagctg actttgggaa aatgataata gattacgaat taaatcaatg 3780
   gacttcagtg gaagatgggt gtttaattaa ctattgtgga agagagggaa taaggttatc 3840
   taatgggatg ttctttgtag gtaagttcta taaaaatctc aataatttac agacctgtag 3900
   tgctggaaca aaggtcagtt acaagccttt aacctccaag ctggaagaaa ttgaaaatga 3960
   aatcattcta gatcaggaaa gattattatg tcttgattca attaggcaaa tgacagcaac 4020
   aaaaaaatta tcattttatt ctttatcctt tctagaacca aaatcttcta gtaggcacaa 4080
   ggtctttaga attcataata aaacactaga atataccgaa accgaatggc atccaatcat 4140
   gtcgtttaat tttgatgaac caaacaaaat tggaattgac aagaatggta aatcagttta 4200
   ttggaatgaa tgggttccta gtggaatatc tgggctgtta tcagggttca atggagtcta 4260
   caaaaaagaa aatgaaacta aagtaactat tgcccgatta gaaacaataa aagaagatta 4320
   tgatagggag atgatgatag atcacgagtt ggtagaggta gaacatccta aaattgtaca 4380
   cttaaaaaga gagaacatca caggatctag agtcgaaatt gttaataaag aacattctga 4440
   tgtgagtggt tggctgtcat cagtattgag tagtttttgg ggaaaaatca tgatgacaat 4500
   aataagtata atcttaatcg taataatagg attagtttta ataaactgct gcccaattat 4560
   atgcaaatca tgtattaaac gttataaaac aaaggaagaa tcccgcaata gacatagatt 4620
   ggatagagaa gataacggta gattgaggag gcaacatcga gttattttta acaatcaatc 4680
   caatgatgaa gaaaatgcca ttgaaatggt agaatatact gacactccca ggccattgcg 4740
   accgattcct gatgccacaa catcagacac tgagtcaaga tcccccacaa cagcccatag 4800
   ttttttcaac cgttaagcgg ccgccagatg acgcatgagg gtcagatcag atttacagcg 4860
   taagtgtgat atttaggatt ataaaggttc cttcatttta atttgttaca gactgtatga 4920
   aaaaaactca tcaacagcca tcatggatgt taacgatttt gagttgcatg aggactttgc 4980
   attgtctgaa gatgactttg tcacttcaga atttctcaat ccggaagacc aaatgacata 5040
   cctgaatcat gccgattata atttgaattc tcccttaatc agcgatgata ttgatttcct 5100
   gatcaagaaa tataatcatg agcaaattcc gaaaatgtgg gatgtcaaga attgggaggg 5160
   agtgttagag atgttgacag cctgtcaagc cagtccaatt ttatctagca ctatgcataa 5220
   gtgggtggga aagtggctca tgtctgatga tcatgacgca agccaaggct tcagttttct 5280
   tcatgaagtg gacaaagaag ctgatctgac gtttgaggtg gtggagacat tcattagagg 5340
   atggggaggt cgagaattgc agtacaagag gaaagacaca tttccggact cctttagagt 5400
   tgcagcctca ttgtgtcaaa aattccttga tttgcacaaa ctcactctga taatgaattc 5460
   agtctctgaa gtcgaactta ccaacctagc aaagaatttt aaaggaaaaa acaggaaagc 5520
   aaaaagcgga aatctgataa ccagattgag ggttcccagt ttaggtcctg cttttgtgac 5580
   tcagggatgg gtgtacatga agaagttgga aatgattatg gatcggaatt ttttgttgat 5640
   gttgaaagac gttatcatcg ggaggatgca gacgatcctg tccatgatct caagagatga 5700
   taatctcttc tccgagtctg atatctttac tgtattaaag atataccgga taggggataa 5760
   gatattagaa aggcaaggga caaagggtta cgacttgatc aaaatgattg agcctatttg 5820
   taacttaaag atgatgaatc tggcacgtaa atatcgtcct ctcatcccta catttcctca 5880
   ttttgaaaaa catattgctg actctgttaa ggaaggatcg aaaatagaca aagggattga 5940
   gtttatatat gatcacatta tgtcaatccc tggtgtggac ttgaccttag ttatttacgg 6000
   atcatttcgg cactggggtc atccttttat caactactat gagggcttag agaagctaca 6060
   caagcaggtt acaatgccca agactattga cagagaatat gcagaatgtc ttgctagtga 6120
   tctggcaaga atcgttcttc agcaacaatt caatgaacat aagaaatggt ttgttgatgt 6180
   agataaagtc ccacaatccc atcctttcaa aagccatatg aaagagaata cttggcctac 6240
   tgcagcccaa gttcaggatt acggcgatcg ctggcatcag ctcccactca tcaaatgctt 6300
   cgaaatccca gatttgttag atccatcgat catctactca gacaaaagtc attccatgaa 6360
   ccggtctgaa gtactacgac atgtaagact tacacctcat gtgcccattc caagcaggaa 6420
   agtattgcag acaatgttgg agactaaggc aacagactgg aaagagtttt taaagaaaat 6480
   tgacgaagag gggttagagg atgatgatct tgtcatagga ctcaaaggga aagagagaga 6540
   attaaaaatt gcgggaagat tcttttcttt gatgtcctgg aagctcagag agtattttgt 6600
   catcactgag tatttgatta agacgcactt tgtcccgatg tttaaagggt tgaccatggc 6660
   ggatgacttg acagcggtga taaagaagat gatggacaca tcttcaggac aaggcttaga 6720
   taattatgaa tccatttgta tagccaacca tattgactat gagaagtgga acaatcatca 6780
   aagaaaagag tcgaacgggc ccgtgttcaa ggtgatgggt caattcttgg gatatccacg 6840
   tctgattgag agaactcatg aattttttga gaagagtctg atatattaca atggacgacc 6900
   agatctgatg cgggttcgag gaaattctct agtcaacgcc tcatctttaa atgtctgctg 6960
   ggagggtcaa gctgggggat tagaaggact gcgacagaag ggatggagta ttctaaattt 7020
   gcttgtcatt cagagagaag caaaaataag gaacaccgcc gtgaaagtgc tagctcaagg 7080
   tgacaatcag gtgatatgta ctcagtataa aacgaagaaa tcccggaatg atattgagct 7140
   taaggcagct ctaacacaga tggtatctaa taatgagatg attatgtctg cgattaaatc 7200
   aggcaccgag aaactgggtc ttttgattaa tgatgatgag acaatgcaat ctgctgatta 7260
   cctcaattac gggaaggttc ccattttcag aggagtaatc agaggccttg agacaaaaag 7320
   atggtcacgc gtgacctgtg tgacaaatga tcagattcca acgtgtgcga acattatgag 7380
   ctctgtgtca actaatgcat taactgtagc ccattttgcc gagaatccag tcaatgccat 7440
   cattcagtat aactactttg gaacatttgc aaggctactg ctgatgatgc atgaccccgc 7500
   tctgaggatc tctctgtatg aagtccaatc aaaaattcca ggacttcaca gtttgacatt 7560
   taaatattct atgttgtatc tggatccttc gataggagga gtctccggaa tgtcactctc 7620
   gagattcctc ataagatcat ttccagatcc agtgacagaa agtttggcgt tctggaaatt 7680
   tatccactct catgcaagaa gcgattcatt aaaggagata tgtgcagttt ttggaaatcc 7740
   tgaaattgca agatttcggc taactcatgt cgataaattg gtggaagacc caacctcatt 7800
   gaacatagct atgggaatga gtcctgctaa tctattaaag acagaggtaa aaaaatgtct 7860
   actggaatca aggcagagca tcaagaacca gattgtaaga gatgctacta tttacctaca 7920
   ccatgaggaa gacaaacttc gtagtttctt atggtccata acaccactgt tccctcggtt 7980
   cttgagtgaa ttcaaatctg ggacattcat cggagtagca gatggcctga tcagcttatt 8040
   tcagaactct aggactattc gaaattcttt taaaaagcgt tatcacaggg aacttgatga 8100
   tttaataatc aagagcgaag tttcctcact tatgcatttg ggtaagctac atttgaggcg 8160
   aggctcagtt cgtatgtgga cttgctcttc tactcaggct gatcttctcc gattccggtc 8220
   atggggaaga tctgttatag gaaccacagt ccctcatccc ttagagatgt taggacaaca 8280
   ttttaaaaag gagactcctt gcagtgcttg caacatatcc ggattagact atgtatctgt 8340
   ccactgtccg aatgggattc atgacgtttt tgaatcacgt ggtccactcc ctgcatattt 8400
   gggttctaaa acatccgaat caacttcgat cttgcagccg tgggagagag agagtaaagt 8460
   accgttgatt aagcgtgcca caaggcttcg tgatgcaatt tcatggtttg tgtctcccga 8520
   ctctaacttg gcctcaacta tccttaagaa cataaatgca ttaacaggag aagaatggtc 8580
   aaagaagcag catggattta aaaggacggg atcggcgtta cacaggttct ccacatccag 8640
   gatgagtcat ggtggttttg cttctcagag tacggctgcc ttgactagat tgatggcaac 8700
   tactgacact atgagagatc tgggagaaca gaactatgat ttcctgtttc aggcgacatt 8760
   attgtatgct caaataacca caactgtagt caggaatgga tcatttcata gctgcacgga 8820
   ccattaccat ataacctgca aatcttgtct gagggccatt gatgagatta ccttggattc 8880
   agcgatggaa tatagccctc cagatgtatc atcagtttta caatcttgga ggaatggaga 8940
   aggctcttgg ggacatgaag tgaaacaaat atacccagtt gaaggtgact ggaggggact 9000
   atctcctgtt gaacaatctt atcaagtcgg acgctgtatc gggtttctgt tcggtgatct 9060
   ggcgtataga aaatcatccc atgcagatga tagctccatg tttccgttat ctatacaaaa 9120
   caaagtcaga ggaagaggct ttttaaaagg gcttatggat gggttaatga gagccagttg 9180
   ttgccaggtg atccatcgtc gaagcttagc ccatctgaag agaccggcta atgcagtcta 9240
   tggagggctg atttatttga tagacaaatt gagtgcatct gccccttttc tttcactgac 9300
   gagacatgga cctttaaggg aagaattaga aactgttcca cataagatac cgacttctta 9360
   tcctacgagc aaccgagata tgggggtgat agttcgtaat tattttaaat atcagtgcag 9420
   actggtagaa aaaggtcggt acaagacaca ttatcctcaa ttgtggcttt tctcagatgt 9480
   gctgtccatt gatttcttag gacccctgtc tatatcttca actctattgg gtattctgta 9540
   taaacagacg ttatcttctc gagacaaaaa tgagttgaga gaactcgcta acttgtcttc 9600
   attgttgaga tcaggagaag gatgggaaga tatccatgtc aaattcttct ctaaggacac 9660
   tttactctgc cctgaagaga tccgacatgc gtgcaaattt gggattgcta aggaatccgc 9720
   tgttttaagc tattatcctc cttggtctca agagtcttat ggaggcatca cctcgatccc 9780
   cgtatatttt tcgaccagga agtatcccaa aattttagat gtccctcctc gggttcaaaa 9840
   cccattggtc tcgggtctac gattggggca actccctact ggagcacatt ataagattag 9900
   gagcattgta aagaacaaga accttcgtta tagagatttc cttagttgtg gggatggatc 9960
   tggggggatg accgcggcac tattgagaga aaacagacaa agtaggggaa tcttcaacag 10020
   cctgttagag ttagccggat ctcttatgag aggagcatct ccagagcctc caagtgcact 10080
   ggagacgctc gggcaagaac gatctaggtg tgtgaatgga agcacatgtt gggagtactc 10140
   atctgaccta agccaaaaag agacatggga ttacttctta agattgaaga gaggcctggg 10200
   tttgaccgtg gacttaatca ccatggacat ggaggtcaga gaccctaata caagtttgat 10260
   gatagaaaag aacctcaaag tttatctgca tcagatatta gaaccaactg gtgtcttaat 10320
   atataaaaca tacgggaccc atattgcgac acaaacagat aatatcctga cgataatcgg 10380
   tcctttcttt gagacggttg acctagtcca gtccgaatac agcagctcac aaacgtccga 10440
   ggtctatttt gtaggacgag gcttgcgctc tcatgttgac gaaccctggg tggactggcc 10500
   atccttaatg gacaattgga gatccattta tgcttttcat gatcctacta cagaatttat 10560
   cagagcaaaa aaagtctgtg aaattgacag tcttataggc attccggctc aattcattcc 10620
   agacccattt gtaaatctcg agaccatgct acagatagtt ggtgttccaa caggagtttc 10680
   gcatgccgca gctctattat catcacaata tccaaatcaa ttggtcacaa cgtcaatatt 10740
   ttatatgaca ctcgtgtctt attataatgt aaaccatatt cgaagaagcc ccaagccttt 10800
   ctctcctccg tctgatggag tctcacagaa cattggttca gccatagtcg gactaagttt 10860
   ttgggtgagt ttgatggaga atgatctcgg attatacaaa caggctctag gtgcaataaa 10920
   gacgtcattc cctattagat ggtcctctgt ccagaccaag gatgggttta cacaagaatg 10980
   gagaactaaa ggaaacggaa ttcctaaaga ttgtcgtctc tcagactctt tggctcagat 11040
   aggaaactgg atcagagcga tggaattggt taggaacaaa acgaggcaat caggattttc 11100
   tgaaacccta tttgatcaat tctgcggact tgcagaccat cacctcaaat ggcggaagtt 11160
   gggaaacaga acaggaatta ttgattggct aaataataga atttcatcca ttgacaaatc 11220
   catcttggtg accaaaagtg atctgcatga cgagaactca tggagggagt gaagatgtat 11280
   tcttccacct ctcattgggt gatacccata tatgaaaaaa actataagta ctttaaactc 11340
   tctttgtttt ttaatgtata tctggttttg ttgtttccgt 11380
<210> 10
   <211> 11380
   <212> RNA
   <213> Artificial
<220>
   <223> Chimeric virus containing RNA from Maraba and Bahia Grande viruses
<400> 10
   acgaagacaa acaaaccauu gauagaauua agaggcucau gaaaauccuu aacagcguuc 60
   aaaaugucug uuacagucaa gagagucauu gaugauucac ucaucacccc caaauugccu 120
   gcgaaugagg acccugugga guacccugcu gauuauuuca aaaagucccg ugauauuccg 180
   guguacauaa acacgaccaa aaguuugucu gauuugcggg gcuauguuua ucaaggccua 240
   aagucaggca acaucucuau aauucauguc aacaguuauc uguaugcagc auuaaaagag 300
   aucagaggaa aauuggacag agauuggauc accuuuggua uccaaaucgg aaaaacagga 360
   gauagcgugg ggauauucga uuuacugacc cuaaaaccuc uagauggugu uuuaccagau 420
   ggggugucug augcuacucg aacuagcuca gacgaugcau ggcuuccacu guaucuauug 480
   ggguuauaca gaguuggucg aacacagaug ccagaauaca ggaagaagcu gauggauggu 540
   cugauuaauc aauguaagau gaucaaugag caguuugaac cacuguugcc agaaggaaga 600
   gaugucuuug augucugggg aaaugacagc aauuacacaa agauuguggc cgcuguagau 660
   auguucuucc auauguucaa aaagcaugag aaggccucuu ucagguaugg cacaauagug 720
   ucaagauuua aggauugugc agcauuggcu acauuugguc aucuguguaa gaucacuggu 780
   auguccacug aagaugugac aacuuggauu cuaaacaggg agguggcuga ugagaugguu 840
   caaaugaugu acccaggaca ggagauagau aaggcugauu cuuacaugcc uuaucuaauc 900
   gacuuagguc uguccucaaa aucuccauau ucaucaguua aaaauccagc uuuccauuuu 960
   uggggucaau ugaccgcauu guuacugaga ucaaccagag ccagaaaugc acgucagccg 1020
   gaugacaucg aguauacauc ccugaccacu gcugggcugu uguaugcaua ugccguuggu 1080
   ucgucugcag accuggcuca acaauucuac guuggggaca acaaguaugu gccagaaacu 1140
   ggagauggag gauuaaccac caaugcaccg ccacaagggc gagauguggu cgaguggcuu 1200
   aguugguuug aagaucaaaa cagaaaaccu accccagaca ugcucaugua ugcuaagaga 1260
   gcugucagug cuuuacaagg auugagggag aagacgauug gcaaguacgc caagucagag 1320
   uuugacaaau gacaacucac ucaccauaug uauuacuacc uuugcuucau augaaaaaaa 1380
   cuaacagcga ucauggauca gcuaucaaag gucaaggaau uccuuaagac uuacgcgcag 1440
   uuggaucaag caguacaaga gauggaugac auugagucuc agagagagga aaagacuaau 1500
   uuugauuugu uucaggaaga aggauuggag auuaaggaga agccuuccua uuaucgggca 1560
   gaugaagaag agauugauuc agaugaagac agcguggaug augcacaaga cuuagggaua 1620
   cguacaucaa caagucccau cgagggguau guggaugagg agcaggauga uuaugaggau 1680
   gaggaaguga acgugguguu uacaucggac uggaaacagc cugagcugga auccgacggg 1740
   gaugggaaaa cucuccgauu gacgauacca gauggauuga cuggggagca gaagucgcaa 1800
   uggcuugcca cgauuaaggc aguuguucag agugcuaaau auuggaacau cucagaaugu 1860
   ucauuugaga guuaugagca agggguuuug auuagagaga gacaaaugac uccugauguc 1920
   uacaaaguca cuccuguuuu aaaugcucca ccgguucaaa ugacagcuaa ucaagauguu 1980
   uggucucuca gcagcacucc auuuacauuu uugcccaaga aacaaggugu gacuccauug 2040
   accauguccu uagaagaacu cuucaacacc cgaggugaau ucauaucucu gggaggaaac 2100
   gggaaaauga gucaccggga ggccaucauu cuaggguuga gacacaagaa gcucuauaau 2160
   caagccagac uaaaguauaa cuuagcuuga auaugaaaaa aacuaacaga uaucaaaaga 2220
   uaucucuaac ucaguccauu guguucaguu caaucaugag cucucucaag aaaauuuugg 2280
   guauuaaagg gaaagggaag aaaucuaaga aauuagguau ggcuccccca cccuaugaag 2340
   aagagacucc aauggaauau ucuccaagug caccuuauga uaagucauug uuuggagucg 2400
   aagauaugga uuuccaugau caacgucaac uccgauauga gaaauuucac uucucauuga 2460
   agaugacugu gagaucaaac aaaccauuuc gaaauuauga ugacguugca gcagcggugu 2520
   ccaauuggga ucauauguac aucggcaugg caggaaaacg uccuuuuuau aagauauuag 2580
   cauucauggg uucuacucua uugaaggcua caccagcugu cuuggcugac caaggacagc 2640
   cagaauauca ugcucacugu gagggacgag cuuacuugcc gcaucgguua gggccgaccc 2700
   cuccgauguu gaaugucccu gaacauuuuc gccguccauu uaacaucgga uuauucagag 2760
   ggacaaucga cauaacccug guacuuuucg augaugaauc uguagauucu gccccgguca 2820
   uaugggauca uuuuaaugca uccagauuga gcagcuucag agaaaaggcu uuguuguuug 2880
   guuugauucu agaaaagaaa gccacuggga auuggguauu ggacucuauu agucauuuca 2940
   aguaauuauc acaaguguug aggugauggg cagacuauga aaaaaacuaa caggguucaa 3000
   acacucuuga ucgagguacc caguuauauu uguuacaaca augauuucga auauguuuuu 3060
   cuuguuucaa cucucauuau uucuacaguu uauagcagga gaugagucau uagaaacaau 3120
   aacagccccu gaaacuccug acccuauacu cuuaaaagga gauacaaaau aucuguucuu 3180
   agucccuucu ucugucaaaa auuggaaacc agcugaccug aaugaauuaa caugcccccc 3240
   ccuaaucucg aaaccagaua cuucugaaau gacuuauuuu uccacagaug ugauggaguu 3300
   acaaaaacau caugaauugg caccaguaga aggguauuua uguucggguu ugcguuacaa 3360
   aguaauaugu ucugaaggau uuuuuggaca aaaaacaaua gcaaaaaaga uugagaacau 3420
   ugaaccugau aguaaacaau gccuugauga cuugucaaaa uuuaagaaug augauuaccu 3480
   acucccauau uucccuucug aagauuguaa uuggaugaaa gagacuccca cccauaaaga 3540
   uuuuauaguu uuucaaaaac auuuuguuaa auaugaccca uacaauaaug guuuuuauga 3600
   uccuuuacuu aaaaaagacu acugugauac ucaagucugu gagacagaac augaucaaac 3660
   uauuuggaua acagaaaaga guauugaaaa ugaaugcauc uucaauuauc cgauuaaaaa 3720
   gcauauauuc cauacagcug acuuugggaa aaugauaaua gauuacgaau uaaaucaaug 3780
   gacuucagug gaagaugggu guuuaauuaa cuauugugga agagagggaa uaagguuauc 3840
   uaaugggaug uucuuuguag guaaguucua uaaaaaucuc aauaauuuac agaccuguag 3900
   ugcuggaaca aaggucaguu acaagccuuu aaccuccaag cuggaagaaa uugaaaauga 3960
   aaucauucua gaucaggaaa gauuauuaug ucuugauuca auuaggcaaa ugacagcaac 4020
   aaaaaaauua ucauuuuauu cuuuauccuu ucuagaacca aaaucuucua guaggcacaa 4080
   ggucuuuaga auucauaaua aaacacuaga auauaccgaa accgaauggc auccaaucau 4140
   gucguuuaau uuugaugaac caaacaaaau uggaauugac aagaauggua aaucaguuua 4200
   uuggaaugaa uggguuccua guggaauauc ugggcuguua ucaggguuca auggagucua 4260
   caaaaaagaa aaugaaacua aaguaacuau ugcccgauua gaaacaauaa aagaagauua 4320
   ugauagggag augaugauag aucacgaguu gguagaggua gaacauccua aaauuguaca 4380
   cuuaaaaaga gagaacauca caggaucuag agucgaaauu guuaauaaag aacauucuga 4440
   ugugaguggu uggcugucau caguauugag uaguuuuugg ggaaaaauca ugaugacaau 4500
   aauaaguaua aucuuaaucg uaauaauagg auuaguuuua auaaacugcu gcccaauuau 4560
   augcaaauca uguauuaaac guuauaaaac aaaggaagaa ucccgcaaua gacauagauu 4620
   ggauagagaa gauaacggua gauugaggag gcaacaucga guuauuuuua acaaucaauc 4680
   caaugaugaa gaaaaugcca uugaaauggu agaauauacu gacacuccca ggccauugcg 4740
   accgauuccu gaugccacaa caucagacac ugagucaaga ucccccacaa cagcccauag 4800
   uuuuuucaac cguuaagcgg ccgccagaug acgcaugagg gucagaucag auuuacagcg 4860
   uaagugugau auuuaggauu auaaagguuc cuucauuuua auuuguuaca gacuguauga 4920
   aaaaaacuca ucaacagcca ucauggaugu uaacgauuuu gaguugcaug aggacuuugc 4980
   auugucugaa gaugacuuug ucacuucaga auuucucaau ccggaagacc aaaugacaua 5040
   ccugaaucau gccgauuaua auuugaauuc ucccuuaauc agcgaugaua uugauuuccu 5100
   gaucaagaaa uauaaucaug agcaaauucc gaaaaugugg gaugucaaga auugggaggg 5160
   aguguuagag auguugacag ccugucaagc caguccaauu uuaucuagca cuaugcauaa 5220
   guggguggga aaguggcuca ugucugauga ucaugacgca agccaaggcu ucaguuuucu 5280
   ucaugaagug gacaaagaag cugaucugac guuugaggug guggagacau ucauuagagg 5340
   auggggaggu cgagaauugc aguacaagag gaaagacaca uuuccggacu ccuuuagagu 5400
   ugcagccuca uugugucaaa aauuccuuga uuugcacaaa cucacucuga uaaugaauuc 5460
   agucucugaa gucgaacuua ccaaccuagc aaagaauuuu aaaggaaaaa acaggaaagc 5520
   aaaaagcgga aaucugauaa ccagauugag gguucccagu uuagguccug cuuuugugac 5580
   ucagggaugg guguacauga agaaguugga aaugauuaug gaucggaauu uuuuguugau 5640
   guugaaagac guuaucaucg ggaggaugca gacgauccug uccaugaucu caagagauga 5700
   uaaucucuuc uccgagucug auaucuuuac uguauuaaag auauaccgga uaggggauaa 5760
   gauauuagaa aggcaaggga caaaggguua cgacuugauc aaaaugauug agccuauuug 5820
   uaacuuaaag augaugaauc uggcacguaa auaucguccu cucaucccua cauuuccuca 5880
   uuuugaaaaa cauauugcug acucuguuaa ggaaggaucg aaaauagaca aagggauuga 5940
   guuuauauau gaucacauua ugucaauccc ugguguggac uugaccuuag uuauuuacgg 6000
   aucauuucgg cacugggguc auccuuuuau caacuacuau gagggcuuag agaagcuaca 6060
   caagcagguu acaaugccca agacuauuga cagagaauau gcagaauguc uugcuaguga 6120
   ucuggcaaga aucguucuuc agcaacaauu caaugaacau aagaaauggu uuguugaugu 6180
   agauaaaguc ccacaauccc auccuuucaa aagccauaug aaagagaaua cuuggccuac 6240
   ugcagcccaa guucaggauu acggcgaucg cuggcaucag cucccacuca ucaaaugcuu 6300
   cgaaauccca gauuuguuag auccaucgau caucuacuca gacaaaaguc auuccaugaa 6360
   ccggucugaa guacuacgac auguaagacu uacaccucau gugcccauuc caagcaggaa 6420
   aguauugcag acaauguugg agacuaaggc aacagacugg aaagaguuuu uaaagaaaau 6480
   ugacgaagag ggguuagagg augaugaucu ugucauagga cucaaaggga aagagagaga 6540
   auuaaaaauu gcgggaagau ucuuuucuuu gauguccugg aagcucagag aguauuuugu 6600
   caucacugag uauuugauua agacgcacuu ugucccgaug uuuaaagggu ugaccauggc 6660
   ggaugacuug acagcgguga uaaagaagau gauggacaca ucuucaggac aaggcuuaga 6720
   uaauuaugaa uccauuugua uagccaacca uauugacuau gagaagugga acaaucauca 6780
   aagaaaagag ucgaacgggc ccguguucaa ggugaugggu caauucuugg gauauccacg 6840
   ucugauugag agaacucaug aauuuuuuga gaagagucug auauauuaca auggacgacc 6900
   agaucugaug cggguucgag gaaauucucu agucaacgcc ucaucuuuaa augucugcug 6960
   ggagggucaa gcugggggau uagaaggacu gcgacagaag ggauggagua uucuaaauuu 7020
   gcuugucauu cagagagaag caaaaauaag gaacaccgcc gugaaagugc uagcucaagg 7080
   ugacaaucag gugauaugua cucaguauaa aacgaagaaa ucccggaaug auauugagcu 7140
   uaaggcagcu cuaacacaga ugguaucuaa uaaugagaug auuaugucug cgauuaaauc 7200
   aggcaccgag aaacuggguc uuuugauuaa ugaugaugag acaaugcaau cugcugauua 7260
   ccucaauuac gggaagguuc ccauuuucag aggaguaauc agaggccuug agacaaaaag 7320
   auggucacgc gugaccugug ugacaaauga ucagauucca acgugugcga acauuaugag 7380
   cucuguguca acuaaugcau uaacuguagc ccauuuugcc gagaauccag ucaaugccau 7440
   cauucaguau aacuacuuug gaacauuugc aaggcuacug cugaugaugc augaccccgc 7500
   ucugaggauc ucucuguaug aaguccaauc aaaaauucca ggacuucaca guuugacauu 7560
   uaaauauucu auguuguauc uggauccuuc gauaggagga gucuccggaa ugucacucuc 7620
   gagauuccuc auaagaucau uuccagaucc agugacagaa aguuuggcgu ucuggaaauu 7680
   uauccacucu caugcaagaa gcgauucauu aaaggagaua ugugcaguuu uuggaaaucc 7740
   ugaaauugca agauuucggc uaacucaugu cgauaaauug guggaagacc caaccucauu 7800
   gaacauagcu augggaauga guccugcuaa ucuauuaaag acagagguaa aaaaaugucu 7860
   acuggaauca aggcagagca ucaagaacca gauuguaaga gaugcuacua uuuaccuaca 7920
   ccaugaggaa gacaaacuuc guaguuucuu augguccaua acaccacugu ucccucgguu 7980
   cuugagugaa uucaaaucug ggacauucau cggaguagca gauggccuga ucagcuuauu 8040
   ucagaacucu aggacuauuc gaaauucuuu uaaaaagcgu uaucacaggg aacuugauga 8100
   uuuaauaauc aagagcgaag uuuccucacu uaugcauuug gguaagcuac auuugaggcg 8160
   aggcucaguu cguaugugga cuugcucuuc uacucaggcu gaucuucucc gauuccgguc 8220
   auggggaaga ucuguuauag gaaccacagu cccucauccc uuagagaugu uaggacaaca 8280
   uuuuaaaaag gagacuccuu gcagugcuug caacauaucc ggauuagacu auguaucugu 8340
   ccacuguccg aaugggauuc augacguuuu ugaaucacgu gguccacucc cugcauauuu 8400
   ggguucuaaa acauccgaau caacuucgau cuugcagccg ugggagagag agaguaaagu 8460
   accguugauu aagcgugcca caaggcuucg ugaugcaauu ucaugguuug ugucucccga 8520
   cucuaacuug gccucaacua uccuuaagaa cauaaaugca uuaacaggag aagaaugguc 8580
   aaagaagcag cauggauuua aaaggacggg aucggcguua cacagguucu ccacauccag 8640
   gaugagucau ggugguuuug cuucucagag uacggcugcc uugacuagau ugauggcaac 8700
   uacugacacu augagagauc ugggagaaca gaacuaugau uuccuguuuc aggcgacauu 8760
   auuguaugcu caaauaacca caacuguagu caggaaugga ucauuucaua gcugcacgga 8820
   ccauuaccau auaaccugca aaucuugucu gagggccauu gaugagauua ccuuggauuc 8880
   agcgauggaa uauagcccuc cagauguauc aucaguuuua caaucuugga ggaauggaga 8940
   aggcucuugg ggacaugaag ugaaacaaau auacccaguu gaaggugacu ggaggggacu 9000
   aucuccuguu gaacaaucuu aucaagucgg acgcuguauc ggguuucugu ucggugaucu 9060
   ggcguauaga aaaucauccc augcagauga uagcuccaug uuuccguuau cuauacaaaa 9120
   caaagucaga ggaagaggcu uuuuaaaagg gcuuauggau ggguuaauga gagccaguug 9180
   uugccaggug auccaucguc gaagcuuagc ccaucugaag agaccggcua augcagucua 9240
   uggagggcug auuuauuuga uagacaaauu gagugcaucu gccccuuuuc uuucacugac 9300
   gagacaugga ccuuuaaggg aagaauuaga aacuguucca cauaagauac cgacuucuua 9360
   uccuacgagc aaccgagaua ugggggugau aguucguaau uauuuuaaau aucagugcag 9420
   acugguagaa aaaggucggu acaagacaca uuauccucaa uuguggcuuu ucucagaugu 9480
   gcuguccauu gauuucuuag gaccccuguc uauaucuuca acucuauugg guauucugua 9540
   uaaacagacg uuaucuucuc gagacaaaaa ugaguugaga gaacucgcua acuugucuuc 9600
   auuguugaga ucaggagaag gaugggaaga uauccauguc aaauucuucu cuaaggacac 9660
   uuuacucugc ccugaagaga uccgacaugc gugcaaauuu gggauugcua aggaauccgc 9720
   uguuuuaagc uauuauccuc cuuggucuca agagucuuau ggaggcauca ccucgauccc 9780
   cguauauuuu ucgaccagga aguaucccaa aauuuuagau gucccuccuc ggguucaaaa 9840
   cccauugguc ucgggucuac gauuggggca acucccuacu ggagcacauu auaagauuag 9900
   gagcauugua aagaacaaga accuucguua uagagauuuc cuuaguugug gggauggauc 9960
   uggggggaug accgcggcac uauugagaga aaacagacaa aguaggggaa ucuucaacag 10020
   ccuguuagag uuagccggau cucuuaugag aggagcaucu ccagagccuc caagugcacu 10080
   ggagacgcuc gggcaagaac gaucuaggug ugugaaugga agcacauguu gggaguacuc 10140
   aucugaccua agccaaaaag agacauggga uuacuucuua agauugaaga gaggccuggg 10200
   uuugaccgug gacuuaauca ccauggacau ggaggucaga gacccuaaua caaguuugau 10260
   gauagaaaag aaccucaaag uuuaucugca ucagauauua gaaccaacug gugucuuaau 10320
   auauaaaaca uacgggaccc auauugcgac acaaacagau aauauccuga cgauaaucgg 10380
   uccuuucuuu gagacgguug accuagucca guccgaauac agcagcucac aaacguccga 10440
   ggucuauuuu guaggacgag gcuugcgcuc ucauguugac gaacccuggg uggacuggcc 10500
   auccuuaaug gacaauugga gauccauuua ugcuuuucau gauccuacua cagaauuuau 10560
   cagagcaaaa aaagucugug aaauugacag ucuuauaggc auuccggcuc aauucauucc 10620
   agacccauuu guaaaucucg agaccaugcu acagauaguu gguguuccaa caggaguuuc 10680
   gcaugccgca gcucuauuau caucacaaua uccaaaucaa uuggucacaa cgucaauauu 10740
   uuauaugaca cucgugucuu auuauaaugu aaaccauauu cgaagaagcc ccaagccuuu 10800
   cucuccuccg ucugauggag ucucacagaa cauugguuca gccauagucg gacuaaguuu 10860
   uugggugagu uugauggaga augaucucgg auuauacaaa caggcucuag gugcaauaaa 10920
   gacgucauuc ccuauuagau gguccucugu ccagaccaag gauggguuua cacaagaaug 10980
   gagaacuaaa ggaaacggaa uuccuaaaga uugucgucuc ucagacucuu uggcucagau 11040
   aggaaacugg aucagagcga uggaauuggu uaggaacaaa acgaggcaau caggauuuuc 11100
   ugaaacccua uuugaucaau ucugcggacu ugcagaccau caccucaaau ggcggaaguu 11160
   gggaaacaga acaggaauua uugauuggcu aaauaauaga auuucaucca uugacaaauc 11220
   caucuuggug accaaaagug aucugcauga cgagaacuca uggagggagu gaagauguau 11280
   ucuuccaccu cucauugggu gauacccaua uaugaaaaaa acuauaagua cuuuaaacuc 11340
   ucuuuguuuu uuaauguaua ucugguuuug uuguuuccgu 11380
<210> 11
   <211> 11101
   <212> DNA
   <213> Artificial
<220>
   <223> DNA encoded by chimeric virus that contains RNA from Maraba and Lymphocytic choriomeningitis viruses
<400> 11
   acgaagacaa acaaaccatt gatagaatta agaggctcat gaaaatcctt aacagcgttc      60
   aaaatgtctg ttacagtcaa gagagtcatt gatgattcac tcatcacccc caaattgcct     120
   gcgaatgagg accctgtgga gtaccctgct gattatttca aaaagtcccg tgatattccg     180
   gtgtacataa acacgaccaa aagtttgtct gatttgcggg gctatgttta tcaaggccta     240
   aagtcaggca acatctctat aattcatgtc aacagttatc tgtatgcagc attaaaagag     300
   atcagaggaa aattggacag agattggatc acctttggta tccaaatcgg aaaaacagga     360
   gatagcgtgg ggatattcga tttactgacc ctaaaacctc tagatggtgt tttaccagat     420
   ggggtgtctg atgctactcg aactagctca gacgatgcat ggcttccact gtatctattg     480
   gggttataca gagttggtcg aacacagatg ccagaataca ggaagaagct gatggatggt     540
   ctgattaatc aatgtaagat gatcaatgag cagtttgaac cactgttgcc agaaggaaga     600
   gatgtctttg atgtctgggg aaatgacagc aattacacaa agattgtggc cgctgtagat     660
   atgttcttcc atatgttcaa aaagcatgag aaggcctctt tcaggtatgg cacaatagtg     720
   tcaagattta aggattgtgc agcattggct acatttggtc atctgtgtaa gatcactggt     780
   atgtccactg aagatgtgac aacttggatt ctaaacaggg aggtggctga tgagatggtt     840
   caaatgatgt acccaggaca ggagatagat aaggctgatt cttacatgcc ttatctaatc     900
   gacttaggtc tgtcctcaaa atctccatat tcatcagtta aaaatccagc tttccatttt     960
   tggggtcaat tgaccgcatt gttactgaga tcaaccagag ccagaaatgc acgtcagccg    1020
   gatgacatcg agtatacatc cctgaccact gctgggctgt tgtatgcata tgccgttggt    1080
   tcgtctgcag acctggctca acaattctac gttggggaca acaagtatgt gccagaaact    1140
   ggagatggag gattaaccac caatgcaccg ccacaagggc gagatgtggt cgagtggctt    1200
   agttggtttg aagatcaaaa cagaaaacct accccagaca tgctcatgta tgctaagaga    1260
   gctgtcagtg ctttacaagg attgagggag aagacgattg gcaagtacgc caagtcagag    1320
   tttgacaaat gacaactcac tcaccatatg tattactacc tttgcttcat atgaaaaaaa    1380
   ctaacagcga tcatggatca gctatcaaag gtcaaggaat tccttaagac ttacgcgcag 1440
   ttggatcaag cagtacaaga gatggatgac attgagtctc agagagagga aaagactaat 1500
   tttgatttgt ttcaggaaga aggattggag attaaggaga agccttccta ttatcgggca 1560
   gatgaagaag agattgattc agatgaagac agcgtggatg atgcacaaga cttagggata 1620
   cgtacatcaa caagtcccat cgaggggtat gtggatgagg agcaggatga ttatgaggat 1680
   gaggaagtga acgtggtgtt tacatcggac tggaaacagc ctgagctgga atccgacggg 1740
   gatgggaaaa ctctccgatt gacgatacca gatggattga ctggggagca gaagtcgcaa 1800
   tggcttgcca cgattaaggc agttgttcag agtgctaaat attggaacat ctcagaatgt 1860
   tcatttgaga gttatgagca aggggttttg attagagaga gacaaatgac tcctgatgtc 1920
   tacaaagtca ctcctgtttt aaatgctcca ccggttcaaa tgacagctaa tcaagatgtt 1980
   tggtctctca gcagcactcc atttacattt ttgcccaaga aacaaggtgt gactccattg 2040
   accatgtcct tagaagaact cttcaacacc cgaggtgaat tcatatctct gggaggaaac 2100
   gggaaaatga gtcaccggga ggccatcatt ctagggttga gacacaagaa gctctataat 2160
   caagccagac taaagtataa cttagcttga atatgaaaaa aactaacaga tatcaaaaga 2220
   tatctctaac tcagtccatt gtgttcagtt caatcatgag ctctctcaag aaaattttgg 2280
   gtattaaagg gaaagggaag aaatctaaga aattaggtat ggctccccca ccctatgaag 2340
   aagagactcc aatggaatat tctccaagtg caccttatga taagtcattg tttggagtcg 2400
   aagatatgga tttccatgat caacgtcaac tccgatatga gaaatttcac ttctcattga 2460
   agatgactgt gagatcaaac aaaccatttc gaaattatga tgacgttgca gcagcggtgt 2520
   ccaattggga tcatatgtac atcggcatgg caggaaaacg tcctttttat aagatattag 2580
   cattcatggg ttctactcta ttgaaggcta caccagctgt cttggctgac caaggacagc 2640
   cagaatatca tgctcactgt gagggacgag cttacttgcc gcatcggtta gggccgaccc 2700
   ctccgatgtt gaatgtccct gaacattttc gccgtccatt taacatcgga ttattcagag 2760
   ggacaatcga cataaccctg gtacttttcg atgatgaatc tgtagattct gccccggtca 2820
   tatgggatca ttttaatgca tccagattga gcagcttcag agaaaaggct ttgttgtttg 2880
   gtttgattct agaaaagaaa gccactggga attgggtatt ggactctatt agtcatttca 2940
   agtaattatc acaagtgttg aggtgatggg cagactatga aaaaaactaa cagggttcaa 3000
   acactcttga tcgaggtacc cagttatatt tgttacaaca atgggccaga tcgttactat 3060
   gtttgaagcc cttccacaca tcattgatga ggtaatcaat atagtgatca ttgtgcttat 3120
   tatcattaca tctattaagg ctgtctacaa tttcgcaacc tgcggcatcc tcgccctggt 3180
   tagattcttg ttcctggctg gccggtcatg cggtatgtac ggactgaacg gtcccgacat 3240
   atacaagggt gtataccaat ttaagagcgt agaatttgat atgtctcacc tgaacctgac 3300
   aatgcctaac gcttgcagcg ccaacaatag tcaccattat attagtatgg gttcatcagg 3360
   actggagctc actttcacaa acgactccat tcttaaccac aacttctgca acctgacaag 3420
   cgcctttaac aagaagacct tcgatcacac gctgatgtca atcgttagtt ccctccacct 3480
   gagtatcaga gggaattcta accataaagc cgtaagctgc gactttaaca acggcatcac 3540
   gatccagtac aacttgtctt tctccgaccc ccaatctgcc atctcccagt gccgcacctt 3600
   ccgagggcga gtgctcgata tgtttcgcac cgcttttggg ggcaagtata tgcgctctgg 3660
   atgggggtgg gcaggttcag atgggaaaac tacatggtgc agccagacca gttaccagta 3720
   tcttatcatt caaaatcgca cttgggaaaa ccattgtagg tatgccgggc ccttcggtat 3780
   gtcaagaata ctgtttgctc aggaaaagac taaattcctg accagaagac tcgccggcac 3840
   atttacatgg acactcagtg attcttctgg agtcgagaat ccaggaggtt attgtctgac 3900
   taagtggatg attctggctg ccgagctgaa gtgctttggc aacacagcgg ttgccaagtg 3960
   caatgtcaac cacgatgaag agttttgtga catgctgaga ctcatcgatt acaataaggc 4020
   tgcgctgtca aaatttaaac aggacgtcga aagcgcactc catgttttca aaactacagt 4080
   gaattcactg atatcagatc agcttctcat gcgaaaccac ctcagagatc tcatgggtgt 4140
   tccatactgc aattacagta aattctggta tctggaacat gccaagacgg gagagacgag 4200
   cgtgcctaag tgctggctgg tgactaacgg gtcatacctc aacgagacac atttttccga 4260
   ccagatagag caggaggcgg ataacatgat aacggagatg ctgcgcaagg attacattaa 4320
   aaggcagggc tctacccccc ttgcactgat ggatttgctc atgttttcca cctcagcgta 4380
   cctgatctct atcttccttc atctcgtgaa aataccgacc cacaggcaca tcaaaggggg 4440
   ttcatgccct aaaccacacc gccttaccaa taaggggatt tgtagttgtg gggcatttaa 4500
   ggtgcctgga gtcaaaacta tttggaagcg gagataagcg gccgccagat gacgcatgag 4560
   ggtcagatca gatttacagc gtaagtgtga tatttaggat tataaaggtt ccttcatttt 4620
   aatttgttac agactgtatg aaaaaaactc atcaacagcc atcatggatg ttaacgattt 4680
   tgagttgcat gaggactttg cattgtctga agatgacttt gtcacttcag aatttctcaa 4740
   tccggaagac caaatgacat acctgaatca tgccgattat aatttgaatt ctcccttaat 4800
   cagcgatgat attgatttcc tgatcaagaa atataatcat gagcaaattc cgaaaatgtg 4860
   ggatgtcaag aattgggagg gagtgttaga gatgttgaca gcctgtcaag ccagtccaat 4920
   tttatctagc actatgcata agtgggtggg aaagtggctc atgtctgatg atcatgacgc 4980
   aagccaaggc ttcagttttc ttcatgaagt ggacaaagaa gctgatctga cgtttgaggt 5040
   ggtggagaca ttcattagag gatggggagg tcgagaattg cagtacaaga ggaaagacac 5100
   atttccggac tcctttagag ttgcagcctc attgtgtcaa aaattccttg atttgcacaa 5160
   actcactctg ataatgaatt cagtctctga agtcgaactt accaacctag caaagaattt 5220
   taaaggaaaa aacaggaaag caaaaagcgg aaatctgata accagattga gggttcccag 5280
   tttaggtcct gcttttgtga ctcagggatg ggtgtacatg aagaagttgg aaatgattat 5340
   ggatcggaat tttttgttga tgttgaaaga cgttatcatc gggaggatgc agacgatcct 5400
   gtccatgatc tcaagagatg ataatctctt ctccgagtct gatatcttta ctgtattaaa 5460
   gatataccgg ataggggata agatattaga aaggcaaggg acaaagggtt acgacttgat 5520
   caaaatgatt gagcctattt gtaacttaaa gatgatgaat ctggcacgta aatatcgtcc 5580
   tctcatccct acatttcctc attttgaaaa acatattgct gactctgtta aggaaggatc 5640
   gaaaatagac aaagggattg agtttatata tgatcacatt atgtcaatcc ctggtgtgga 5700
   cttgacctta gttatttacg gatcatttcg gcactggggt catcctttta tcaactacta 5760
   tgagggctta gagaagctac acaagcaggt tacaatgccc aagactattg acagagaata 5820
   tgcagaatgt cttgctagtg atctggcaag aatcgttctt cagcaacaat tcaatgaaca 5880
   taagaaatgg tttgttgatg tagataaagt cccacaatcc catcctttca aaagccatat 5940
   gaaagagaat acttggccta ctgcagccca agttcaggat tacggcgatc gctggcatca 6000
   gctcccactc atcaaatgct tcgaaatccc agatttgtta gatccatcga tcatctactc 6060
   agacaaaagt cattccatga accggtctga agtactacga catgtaagac ttacacctca 6120
   tgtgcccatt ccaagcagga aagtattgca gacaatgttg gagactaagg caacagactg 6180
   gaaagagttt ttaaagaaaa ttgacgaaga ggggttagag gatgatgatc ttgtcatagg 6240
   actcaaaggg aaagagagag aattaaaaat tgcgggaaga ttcttttctt tgatgtcctg 6300
   gaagctcaga gagtattttg tcatcactga gtatttgatt aagacgcact ttgtcccgat 6360
   gtttaaaggg ttgaccatgg cggatgactt gacagcggtg ataaagaaga tgatggacac 6420
   atcttcagga caaggcttag ataattatga atccatttgt atagccaacc atattgacta 6480
   tgagaagtgg aacaatcatc aaagaaaaga gtcgaacggg cccgtgttca aggtgatggg 6540
   tcaattcttg ggatatccac gtctgattga gagaactcat gaattttttg agaagagtct 6600
   gatatattac aatggacgac cagatctgat gcgggttcga ggaaattctc tagtcaacgc 6660
   ctcatcttta aatgtctgct gggagggtca agctggggga ttagaaggac tgcgacagaa 6720
   gggatggagt attctaaatt tgcttgtcat tcagagagaa gcaaaaataa ggaacaccgc 6780
   cgtgaaagtg ctagctcaag gtgacaatca ggtgatatgt actcagtata aaacgaagaa 6840
   atcccggaat gatattgagc ttaaggcagc tctaacacag atggtatcta ataatgagat 6900
   gattatgtct gcgattaaat caggcaccga gaaactgggt cttttgatta atgatgatga 6960
   gacaatgcaa tctgctgatt acctcaatta cgggaaggtt cccattttca gaggagtaat 7020
   cagaggcctt gagacaaaaa gatggtcacg cgtgacctgt gtgacaaatg atcagattcc 7080
   aacgtgtgcg aacattatga gctctgtgtc aactaatgca ttaactgtag cccattttgc 7140
   cgagaatcca gtcaatgcca tcattcagta taactacttt ggaacatttg caaggctact 7200
   gctgatgatg catgaccccg ctctgaggat ctctctgtat gaagtccaat caaaaattcc 7260
   aggacttcac agtttgacat ttaaatattc tatgttgtat ctggatcctt cgataggagg 7320
   agtctccgga atgtcactct cgagattcct cataagatca tttccagatc cagtgacaga 7380
   aagtttggcg ttctggaaat ttatccactc tcatgcaaga agcgattcat taaaggagat 7440
   atgtgcagtt tttggaaatc ctgaaattgc aagatttcgg ctaactcatg tcgataaatt 7500
   ggtggaagac ccaacctcat tgaacatagc tatgggaatg agtcctgcta atctattaaa 7560
   gacagaggta aaaaaatgtc tactggaatc aaggcagagc atcaagaacc agattgtaag 7620
   agatgctact atttacctac accatgagga agacaaactt cgtagtttct tatggtccat 7680
   aacaccactg ttccctcggt tcttgagtga attcaaatct gggacattca tcggagtagc 7740
   agatggcctg atcagcttat ttcagaactc taggactatt cgaaattctt ttaaaaagcg 7800
   ttatcacagg gaacttgatg atttaataat caagagcgaa gtttcctcac ttatgcattt 7860
   gggtaagcta catttgaggc gaggctcagt tcgtatgtgg acttgctctt ctactcaggc 7920
   tgatcttctc cgattccggt catggggaag atctgttata ggaaccacag tccctcatcc 7980
   cttagagatg ttaggacaac attttaaaaa ggagactcct tgcagtgctt gcaacatatc 8040
   cggattagac tatgtatctg tccactgtcc gaatgggatt catgacgttt ttgaatcacg 8100
   tggtccactc cctgcatatt tgggttctaa aacatccgaa tcaacttcga tcttgcagcc 8160
   gtgggagaga gagagtaaag taccgttgat taagcgtgcc acaaggcttc gtgatgcaat 8220
   ttcatggttt gtgtctcccg actctaactt ggcctcaact atccttaaga acataaatgc 8280
   attaacagga gaagaatggt caaagaagca gcatggattt aaaaggacgg gatcggcgtt 8340
   acacaggttc tccacatcca ggatgagtca tggtggtttt gcttctcaga gtacggctgc 8400
   cttgactaga ttgatggcaa ctactgacac tatgagagat ctgggagaac agaactatga 8460
   tttcctgttt caggcgacat tattgtatgc tcaaataacc acaactgtag tcaggaatgg 8520
   atcatttcat agctgcacgg accattacca tataacctgc aaatcttgtc tgagggccat 8580
   tgatgagatt accttggatt cagcgatgga atatagccct ccagatgtat catcagtttt 8640
   acaatcttgg aggaatggag aaggctcttg gggacatgaa gtgaaacaaa tatacccagt 8700
   tgaaggtgac tggaggggac tatctcctgt tgaacaatct tatcaagtcg gacgctgtat 8760
   cgggtttctg ttcggtgatc tggcgtatag aaaatcatcc catgcagatg atagctccat 8820
   gtttccgtta tctatacaaa acaaagtcag aggaagaggc tttttaaaag ggcttatgga 8880
   tgggttaatg agagccagtt gttgccaggt gatccatcgt cgaagcttag cccatctgaa 8940
   gagaccggct aatgcagtct atggagggct gatttatttg atagacaaat tgagtgcatc     9000
   tgcccctttt ctttcactga cgagacatgg acctttaagg gaagaattag aaactgttcc     9060
   acataagata ccgacttctt atcctacgag caaccgagat atgggggtga tagttcgtaa     9120
   ttattttaaa tatcagtgca gactggtaga aaaaggtcgg tacaagacac attatcctca     9180
   attgtggctt ttctcagatg tgctgtccat tgatttctta ggacccctgt ctatatcttc     9240
   aactctattg ggtattctgt ataaacagac gttatcttct cgagacaaaa atgagttgag     9300
   agaactcgct aacttgtctt cattgttgag atcaggagaa ggatgggaag atatccatgt     9360
   caaattcttc tctaaggaca ctttactctg ccctgaagag atccgacatg cgtgcaaatt     9420
   tgggattgct aaggaatccg ctgttttaag ctattatcct ccttggtctc aagagtctta     9480
   tggaggcatc acctcgatcc ccgtatattt ttcgaccagg aagtatccca aaattttaga     9540
   tgtccctcct cgggttcaaa acccattggt ctcgggtcta cgattggggc aactccctac     9600
   tggagcacat tataagatta ggagcattgt aaagaacaag aaccttcgtt atagagattt     9660
   ccttagttgt ggggatggat ctggggggat gaccgcggca ctattgagag aaaacagaca     9720
   aagtagggga atcttcaaca gcctgttaga gttagccgga tctcttatga gaggagcatc     9780
   tccagagcct ccaagtgcac tggagacgct cgggcaagaa cgatctaggt gtgtgaatgg     9840
   aagcacatgt tgggagtact catctgacct aagccaaaaa gagacatggg attacttctt     9900
   aagattgaag agaggcctgg gtttgaccgt ggacttaatc accatggaca tggaggtcag     9960
   agaccctaat acaagtttga tgatagaaaa gaacctcaaa gtttatctgc atcagatatt    10020
   agaaccaact ggtgtcttaa tatataaaac atacgggacc catattgcga cacaaacaga    10080
   taatatcctg acgataatcg gtcctttctt tgagacggtt gacctagtcc agtccgaata    10140
   cagcagctca caaacgtccg aggtctattt tgtaggacga ggcttgcgct ctcatgttga    10200
   cgaaccctgg gtggactggc catccttaat ggacaattgg agatccattt atgcttttca    10260
   tgatcctact acagaattta tcagagcaaa aaaagtctgt gaaattgaca gtcttatagg    10320
   cattccggct caattcattc cagacccatt tgtaaatctc gagaccatgc tacagatagt    10380
   tggtgttcca acaggagttt cgcatgccgc agctctatta tcatcacaat atccaaatca    10440
   attggtcaca acgtcaatat tttatatgac actcgtgtct tattataatg taaaccatat    10500
   tcgaagaagc cccaagcctt tctctcctcc gtctgatgga gtctcacaga acattggttc    10560
   agccatagtc ggactaagtt tttgggtgag tttgatggag aatgatctcg gattatacaa    10620
   acaggctcta ggtgcaataa agacgtcatt ccctattaga tggtcctctg tccagaccaa    10680
   ggatgggttt acacaagaat ggagaactaa aggaaacgga attcctaaag attgtcgtct    10740
   ctcagactct ttggctcaga taggaaactg gatcagagcg atggaattgg ttaggaacaa    10800
   aacgaggcaa tcaggatttt ctgaaaccct atttgatcaa ttctgcggac ttgcagacca    10860
   tcacctcaaa tggcggaagt tgggaaacag aacaggaatt attgattggc taaataatag    10920
   aatttcatcc attgacaaat ccatcttggt gaccaaaagt gatctgcatg acgagaactc    10980
   atggagggag tgaagatgta ttcttccacc tctcattggg tgatacccat atatgaaaaa    11040
   aactataagt actttaaact ctctttgttt tttaatgtat atctggtttt gttgtttccg    11100
   t                                                                    11101
<210> 12
   <211> 11101
   <212> RNA
   <213> Artificial
<220>
   <223> Chimeric virus containing RNA from Maraba and Lymphocytic choriomeningitis viruses
<400> 12
   acgaagacaa acaaaccauu gauagaauua agaggcucau gaaaauccuu aacagcguuc        60
   aaaaugucug uuacagucaa gagagucauu gaugauucac ucaucacccc caaauugccu       120
   gcgaaugagg acccugugga guacccugcu gauuauuuca aaaagucccg ugauauuccg       180
   guguacauaa acacgaccaa aaguuugucu gauuugcggg gcuauguuua ucaaggccua       240
   aagucaggca acaucucuau aauucauguc aacaguuauc uguaugcagc auuaaaagag       300
   aucagaggaa aauuggacag agauuggauc accuuuggua uccaaaucgg aaaaacagga       360
   gauagcgugg ggauauucga uuuacugacc cuaaaaccuc uagauggugu uuuaccagau       420
   ggggugucug augcuacucg aacuagcuca gacgaugcau ggcuuccacu guaucuauug       480
   ggguuauaca gaguuggucg aacacagaug ccagaauaca ggaagaagcu gauggauggu       540
   cugauuaauc aauguaagau gaucaaugag caguuugaac cacuguugcc agaaggaaga       600
   gaugucuuug augucugggg aaaugacagc aauuacacaa agauuguggc cgcuguagau       660
   auguucuucc auauguucaa aaagcaugag aaggccucuu ucagguaugg cacaauagug       720
   ucaagauuua aggauugugc agcauuggcu acauuugguc aucuguguaa gaucacuggu       780
   auguccacug aagaugugac aacuuggauu cuaaacaggg agguggcuga ugagaugguu     840
   caaaugaugu acccaggaca ggagauagau aaggcugauu cuuacaugcc uuaucuaauc       900
   gacuuagguc uguccucaaa aucuccauau ucaucaguua aaaauccagc uuuccauuuu       960
   uggggucaau ugaccgcauu guuacugaga ucaaccagag ccagaaaugc acgucagccg      1020
   gaugacaucg aguauacauc ccugaccacu gcugggcugu uguaugcaua ugccguuggu      1080
   ucgucugcag accuggcuca acaauucuac guuggggaca acaaguaugu gccagaaacu      1140
   ggagauggag gauuaaccac caaugcaccg ccacaagggc gagauguggu cgaguggcuu    1200
   aguugguuug aagaucaaaa cagaaaaccu accccagaca ugcucaugua ugcuaagaga 1260
   gcugucagug cuuuacaagg auugagggag aagacgauug gcaaguacgc caagucagag 1320
   uuugacaaau gacaacucac ucaccauaug uauuacuacc uuugcuucau augaaaaaaa 1380
   cuaacagcga ucauggauca gcuaucaaag gucaaggaau uccuuaagac uuacgcgcag 1440
   uuggaucaag caguacaaga gauggaugac auugagucuc agagagagga aaagacuaau 1500
   uuugauuugu uucaggaaga aggauuggag auuaaggaga agccuuccua uuaucgggca 1560
   gaugaagaag agauugauuc agaugaagac agcguggaug augcacaaga cuuagggaua 1620
   cguacaucaa caagucccau cgagggguau guggaugagg agcaggauga uuaugaggau      1680
   gaggaaguga acgugguguu uacaucggac uggaaacagc cugagcugga auccgacggg 1740
   gaugggaaaa cucuccgauu gacgauacca gauggauuga cuggggagca gaagucgcaa 1800
   uggcuugcca cgauuaaggc aguuguucag agugcuaaau auuggaacau cucagaaugu 1860
   ucauuugaga guuaugagca agggguuuug auuagagaga gacaaaugac uccugauguc 1920
   uacaaaguca cuccuguuuu aaaugcucca ccgguucaaa ugacagcuaa ucaagauguu 1980
   uggucucuca gcagcacucc auuuacauuu uugcccaaga aacaaggugu gacuccauug 2040
   accauguccu uagaagaacu cuucaacacc cgaggugaau ucauaucucu gggaggaaac 2100
   gggaaaauga gucaccggga ggccaucauu cuaggguuga gacacaagaa gcucuauaau 2160
   caagccagac uaaaguauaa cuuagcuuga auaugaaaaa aacuaacaga uaucaaaaga 2220
   uaucucuaac ucaguccauu guguucaguu caaucaugag cucucucaag aaaauuuugg 2280
   guauuaaagg gaaagggaag aaaucuaaga aauuagguau ggcuccccca cccuaugaag 2340
   aagagacucc aauggaauau ucuccaagug caccuuauga uaagucauug uuuggagucg 2400
   aagauaugga uuuccaugau caacgucaac uccgauauga gaaauuucac uucucauuga 2460
   agaugacugu gagaucaaac aaaccauuuc gaaauuauga ugacguugca gcagcggugu 2520
   ccaauuggga ucauauguac aucggcaugg caggaaaacg uccuuuuuau aagauauuag 2580
   cauucauggg uucuacucua uugaaggcua caccagcugu cuuggcugac caaggacagc 2640
   cagaauauca ugcucacugu gagggacgag cuuacuugcc gcaucgguua gggccgaccc 2700
   cuccgauguu gaaugucccu gaacauuuuc gccguccauu uaacaucgga uuauucagag 2760
   ggacaaucga cauaacccug guacuuuucg augaugaauc uguagauucu gccccgguca 2820
   uaugggauca uuuuaaugca uccagauuga gcagcuucag agaaaaggcu uuguuguuug 2880
   guuugauucu agaaaagaaa gccacuggga auuggguauu ggacucuauu agucauuuca 2940
   aguaauuauc acaaguguug aggugauggg cagacuauga aaaaaacuaa caggguucaa 3000
   acacucuuga ucgagguacc caguuauauu uguuacaaca augggccaga ucguuacuau 3060
   guuugaagcc cuuccacaca ucauugauga gguaaucaau auagugauca uugugcuuau 3120
   uaucauuaca ucuauuaagg cugucuacaa uuucgcaacc ugcggcaucc ucgcccuggu 3180
   uagauucuug uuccuggcug gccggucaug cgguauguac ggacugaacg gucccgacau 3240
   auacaagggu guauaccaau uuaagagcgu agaauuugau augucucacc ugaaccugac 3300
   aaugccuaac gcuugcagcg ccaacaauag ucaccauuau auuaguaugg guucaucagg 3360
   acuggagcuc acuuucacaa acgacuccau ucuuaaccac aacuucugca accugacaag 3420
   cgccuuuaac aagaagaccu ucgaucacac gcugauguca aucguuaguu cccuccaccu 3480
   gaguaucaga gggaauucua accauaaagc cguaagcugc gacuuuaaca acggcaucac 3540
   gauccaguac aacuugucuu ucuccgaccc ccaaucugcc aucucccagu gccgcaccuu 3600
   ccgagggcga gugcucgaua uguuucgcac cgcuuuuggg ggcaaguaua ugcgcucugg 3660
   augggggugg gcagguucag augggaaaac uacauggugc agccagacca guuaccagua 3720
   ucuuaucauu caaaaucgca cuugggaaaa ccauuguagg uaugccgggc ccuucgguau 3780
   gucaagaaua cuguuugcuc aggaaaagac uaaauuccug accagaagac ucgccggcac 3840
   auuuacaugg acacucagug auucuucugg agucgagaau ccaggagguu auugucugac 3900
   uaaguggaug auucuggcug ccgagcugaa gugcuuuggc aacacagcgg uugccaagug 3960
   caaugucaac cacgaugaag aguuuuguga caugcugaga cucaucgauu acaauaaggc 4020
   ugcgcuguca aaauuuaaac aggacgucga aagcgcacuc cauguuuuca aaacuacagu 4080
   gaauucacug auaucagauc agcuucucau gcgaaaccac cucagagauc ucaugggugu 4140
   uccauacugc aauuacagua aauucuggua ucuggaacau gccaagacgg gagagacgag 4200
   cgugccuaag ugcuggcugg ugacuaacgg gucauaccuc aacgagacac auuuuuccga 4260
   ccagauagag caggaggcgg auaacaugau aacggagaug cugcgcaagg auuacauuaa 4320
   aaggcagggc ucuacccccc uugcacugau ggauuugcuc auguuuucca ccucagcgua 4380
   ccugaucucu aucuuccuuc aucucgugaa aauaccgacc cacaggcaca ucaaaggggg 4440
   uucaugcccu aaaccacacc gccuuaccaa uaaggggauu uguaguugug gggcauuuaa 4500
   ggugccugga gucaaaacua uuuggaagcg gagauaagcg gccgccagau gacgcaugag 4560
   ggucagauca gauuuacagc guaaguguga uauuuaggau uauaaagguu ccuucauuuu 4620
   aauuuguuac agacuguaug aaaaaaacuc aucaacagcc aucauggaug uuaacgauuu 4680
   ugaguugcau gaggacuuug cauugucuga agaugacuuu gucacuucag aauuucucaa 4740
   uccggaagac caaaugacau accugaauca ugccgauuau aauuugaauu cucccuuaau 4800
   cagcgaugau auugauuucc ugaucaagaa auauaaucau gagcaaauuc cgaaaaugug 4860
   ggaugucaag aauugggagg gaguguuaga gauguugaca gccugucaag ccaguccaau 4920
   uuuaucuagc acuaugcaua agugggUggg aaaguggcuc augucugaug aucaugacgc 4980
   aagccaaggc uucaguuuuc uucaugaagu ggacaaagaa gcugaucuga cguuugaggu 5040
   gguggagaca uucauuagag gauggggagg ucgagaauug caguacaaga ggaaagacac 5100
   auuuccggac uccuuuagag uugcagccuc auugugucaa aaauuccuug auuugcacaa 5160
   acucacucug auaaugaauu cagucucuga agucgaacuu accaaccuag caaagaauuu 5220
   uaaaggaaaa aacaggaaag caaaaagcgg aaaucugaua accagauuga ggguucccag 5280
   uuuagguccu gcuuuuguga cucagggaug gguguacaug aagaaguugg aaaugauuau 5340
   ggaucggaau uuuuuguuga uguugaaaga cguuaucauc gggaggaugc agacgauccu 5400
   guccaugauc ucaagagaug auaaucucuu cuccgagucu gauaucuuua cuguauuaaa 5460
   gauauaccgg auaggggaua agauauuaga aaggcaaggg acaaaggguu acgacuugau 5520
   caaaaugauu gagccuauuu guaacuuaaa gaugaugaau cuggcacgua aauaucgucc 5580
   ucucaucccu acauuuccuc auuuugaaaa acauauugcu gacucuguua aggaaggauc 5640
   gaaaauagac aaagggauug aguuuauaua ugaucacauu augucaaucc cuggugugga 5700
   cuugaccuua guuauuuacg gaucauuucg gcacuggggu cauccuuuua ucaacuacua 5760
   ugagggcuua gagaagcuac acaagcaggu uacaaugccc aagacuauug acagagaaua 5820
   ugcagaaugu cuugcuagug aucuggcaag aaucguucuu cagcaacaau ucaaugaaca 5880
   uaagaaaugg uuuguugaug uagauaaagu cccacaaucc cauccuuuca aaagccauau 5940
   gaaagagaau acuuggccua cugcagccca aguucaggau uacggcgauc gcuggcauca 6000
   gcucccacuc aucaaaugcu ucgaaauccc agauuuguua gauccaucga ucaucuacuc 6060
   agacaaaagu cauuccauga accggucuga aguacuacga cauguaagac uuacaccuca 6120
   ugugcccauu ccaagcagga aaguauugca gacaauguug gagacuaagg caacagacug 6180
   gaaagaguuu uuaaagaaaa uugacgaaga gggguuagag gaugaugauc uugucauagg 6240
   acucaaaggg aaagagagag aauuaaaaau ugcgggaaga uucuuuucuu ugauguccug 6300
   gaagcucaga gaguauuuug ucaucacuga guauuugauu aagacgcacu uugucccgau 6360
   guuuaaaggg uugaccaugg cggaugacuu gacagcggug auaaagaaga ugauggacac 6420
   aucuucagga caaggcuuag auaauuauga auccauuugu auagccaacc auauugacua 6480
   ugagaagugg aacaaucauc aaagaaaaga gucgaacggg cccguguuca aggugauggg 6540
   ucaauucuug ggauauccac gucugauuga gagaacucau gaauuuuuug agaagagucu 6600
   gauauauuac aauggacgac cagaucugau gcggguucga ggaaauucuc uagucaacgc 6660
   cucaucuuua aaugucugcu gggaggguca agcuggggga uuagaaggac ugcgacagaa 6720
   gggauggagu auucuaaauu ugcuugucau ucagagagaa gcaaaaauaa ggaacaccgc 6780
   cgugaaagug cuagcucaag gugacaauca ggugauaugu acucaguaua aaacgaagaa 6840
   aucccggaau gauauugagc uuaaggcagc ucuaacacag augguaucua auaaugagau 6900
   gauuaugucu gcgauuaaau caggcaccga gaaacugggu cuuuugauua augaugauga 6960
   gacaaugcaa ucugcugauu accucaauua cgggaagguu cccauuuuca gaggaguaau 7020
   cagaggccuu gagacaaaaa gauggucacg cgugaccugu gugacaaaug aucagauucc 7080
   aacgugugcg aacauuauga gcucuguguc aacuaaugca uuaacuguag cccauuuugc 7140
   cgagaaucca gucaaugcca ucauucagua uaacuacuuu ggaacauuug caaggcuacu 7200
   gcugaugaug caugaccccg cucugaggau cucucuguau gaaguccaau caaaaauucc 7260
   aggacuucac aguuugacau uuaaauauuc uauguuguau cuggauccuu cgauaggagg 7320
   agucuccgga augucacucu cgagauuccu cauaagauca uuuccagauc cagugacaga 7380
   aaguuuggcg uucuggaaau uuauccacuc ucaugcaaga agcgauucau uaaaggagau 7440
   augugcaguu uuuggaaauc cugaaauugc aagauuucgg cuaacucaug ucgauaaauu 7500
   gguggaagac ccaaccucau ugaacauagc uaugggaaug aguccugcua aucuauuaaa 7560
   gacagaggua aaaaaauguc uacuggaauc aaggcagagc aucaagaacc agauuguaag 7620
   agaugcuacu auuuaccuac accaugagga agacaaacuu cguaguuucu uaugguccau 7680
   aacaccacug uucccucggu ucuugaguga auucaaaucu gggacauuca ucggaguagc 7740
   agauggccug aucagcuuau uucagaacuc uaggacuauu cgaaauucuu uuaaaaagcg 7800
   uuaucacagg gaacuugaug auuuaauaau caagagcgaa guuuccucac uuaugcauuu 7860
   ggguaagcua cauuugaggc gaggcucagu ucguaugugg acuugcucuu cuacucaggc 7920
   ugaucuucuc cgauuccggu cauggggaag aucuguuaua ggaaccacag ucccucaucc 7980
   cuuagagaug uuaggacaac auuuuaaaaa ggagacuccu ugcagugcuu gcaacauauc 8040
   cggauuagac uauguaucug uccacugucc gaaugggauu caugacguuu uugaaucacg 8100
   ugguccacuc ccugcauauu uggguucuaa aacauccgaa ucaacuucga ucuugcagcc 8160
   gugggagaga gagaguaaag uaccguugau uaagcgugcc acaaggcuuc gugaugcaau 8220
   uucaugguuu gugucucccg acucuaacuu ggccucaacu auccuuaaga acauaaaugc 8280
   auuaacagga gaagaauggu caaagaagca gcauggauuu aaaaggacgg gaucggcguu 8340
   acacagguuc uccacaucca ggaugaguca uggugguuuu gcuucucaga guacggcugc 8400
   cuugacuaga uugauggcaa cuacugacac uaugagagau cugggagaac agaacuauga 8460
   uuuccuguuu caggcgacau uauuguaugc ucaaauaacc acaacuguag ucaggaaugg 8520
   aucauuucau agcugcacgg accauuacca uauaaccugc aaaucuuguc ugagggccau 8580
   ugaugagauu accuuggauu cagcgaugga auauagcccu ccagauguau caucaguuuu 8640
   acaaucuugg aggaauggag aaggcucuug gggacaugaa gugaaacaaa uauacccagu 8700
   ugaaggugac uggaggggac uaucuccugu ugaacaaucu uaucaagucg gacgcuguau 8760
   cggguuucug uucggugauc uggcguauag aaaaucaucc caugcagaug auagcuccau 8820
   guuuccguua ucuauacaaa acaaagucag aggaagaggc uuuuuaaaag ggcuuaugga 8880
   uggguuaaug agagccaguu guugccaggu gauccaucgu cgaagcuuag cccaucugaa 8940
   gagaccggcu aaugcagucu auggagggcu gauuuauuug auagacaaau ugagugcauc 9000
   ugccccuuuu cuuucacuga cgagacaugg accuuuaagg gaagaauuag aaacuguucc 9060
   acauaagaua ccgacuucuu auccuacgag caaccgagau auggggguga uaguucguaa 9120
   uuauuuuaaa uaucagugca gacugguaga aaaaggucgg uacaagacac auuauccuca 9180
   auuguggcuu uucucagaug ugcuguccau ugauuucuua ggaccccugu cuauaucuuc 9240
   aacucuauug gguauucugu auaaacagac guuaucuucu cgagacaaaa augaguugag 9300
   agaacucgcu aacuugucuu cauuguugag aucaggagaa ggaugggaag auauccaugu 9360
   caaauucuuc ucuaaggaca cuuuacucug cccugaagag auccgacaug cgugcaaauu 9420
   ugggauugcu aaggaauccg cuguuuuaag cuauuauccu ccuuggucuc aagagucuua 9480
   uggaggcauc accucgaucc ccguauauuu uucgaccagg aaguauccca aaauuuuaga 9540
   ugucccuccu cggguucaaa acccauuggu cucgggucua cgauuggggc aacucccuac 9600
   uggagcacau uauaagauua ggagcauugu aaagaacaag aaccuucguu auagagauuu 9660
   ccuuaguugu ggggauggau cuggggggau gaccgcggca cuauugagag aaaacagaca 9720
   aaguagggga aucuucaaca gccuguuaga guuagccgga ucucuuauga gaggagcauc 9780
   uccagagccu ccaagugcac uggagacgcu cgggcaagaa cgaucuaggu gugugaaugg 9840
   aagcacaugu ugggaguacu caucugaccu aagccaaaaa gagacauggg auuacuucuu 9900
   aagauugaag agaggccugg guuugaccgu ggacuuaauc accauggaca uggaggucag 9960
   agacccuaau acaaguuuga ugauagaaaa gaaccucaaa guuuaucugc aucagauauu 10020
   agaaccaacu ggugucuuaa uauauaaaac auacgggacc cauauugcga cacaaacaga 10080
   uaauauccug acgauaaucg guccuuucuu ugagacgguu gaccuagucc aguccgaaua 10140
   cagcagcuca caaacguccg aggucuauuu uguaggacga ggcuugcgcu cucauguuga 10200
   cgaacccugg guggacuggc cauccuuaau ggacaauugg agauccauuu augcuuuuca 10260
   ugauccuacu acagaauuua ucagagcaaa aaaagucugu gaaauugaca gucuuauagg 10320
   cauuccggcu caauucauuc cagacccauu uguaaaucuc gagaccaugc uacagauagu 10380
   ugguguucca acaggaguuu cgcaugccgc agcucuauua ucaucacaau auccaaauca 10440
   auuggucaca acgucaauau uuuauaugac acucgugucu uauuauaaug uaaaccauau 10500
   ucgaagaagc cccaagccuu ucucuccucc gucugaugga gucucacaga acauugguuc 10560
   agccauaguc ggacuaaguu uuugggugag uuugauggag aaugaucucg gauuauacaa 10620
   acaggcucua ggugcaauaa agacgucauu cccuauuaga ugguccucug uccagaccaa 10680
   ggauggguuu acacaagaau ggagaacuaa aggaaacgga auuccuaaag auugucgucu 10740
   cucagacucu uuggcucaga uaggaaacug gaucagagcg auggaauugg uuaggaacaa 10800
   aacgaggcaa ucaggauuuu cugaaacccu auuugaucaa uucugcggac uugcagacca 10860
   ucaccucaaa uggcggaagu ugggaaacag aacaggaauu auugauuggc uaaauaauag 10920
   aauuucaucc auugacaaau ccaucuuggu gaccaaaagu gaucugcaug acgagaacuc 10980
   auggagggag ugaagaugua uucuuccacc ucucauuggg ugauacccau auaugaaaaa 11040
   aacuauaagu acuuuaaacu cucuuuguuu uuuaauguau aucugguuuu guuguuuccg 11100
   u 11101
<210> 13
   <211> 11632
   <212> DNA
   <213> Artificial
<220>
   <223> DNA encoded by chimeric virus that contains RNA from Maraba and Ebola viruses
<400> 13
   acgaagacaa acaaaccatt gatagaatta agaggctcat gaaaatcctt aacagcgttc 60
   aaaatgtctg ttacagtcaa gagagtcatt gatgattcac tcatcacccc caaattgcct 120
   gcgaatgagg accctgtgga gtaccctgct gattatttca aaaagtcccg tgatattccg 180
   gtgtacataa acacgaccaa aagtttgtct gatttgcggg gctatgttta tcaaggccta 240
   aagtcaggca acatctctat aattcatgtc aacagttatc tgtatgcagc attaaaagag 300
   atcagaggaa aattggacag agattggatc acctttggta tccaaatcgg aaaaacagga 360
   gatagcgtgg ggatattcga tttactgacc ctaaaacctc tagatggtgt tttaccagat 420
   ggggtgtctg atgctactcg aactagctca gacgatgcat ggcttccact gtatctattg 480
   gggttataca gagttggtcg aacacagatg ccagaataca ggaagaagct gatggatggt 540
   ctgattaatc aatgtaagat gatcaatgag cagtttgaac cactgttgcc agaaggaaga 600
   gatgtctttg atgtctgggg aaatgacagc aattacacaa agattgtggc cgctgtagat 660
   atgttcttcc atatgttcaa aaagcatgag aaggcctctt tcaggtatgg cacaatagtg 720
   tcaagattta aggattgtgc agcattggct acatttggtc atctgtgtaa gatcactggt 780
   atgtccactg aagatgtgac aacttggatt ctaaacaggg aggtggctga tgagatggtt 840
   caaatgatgt acccaggaca ggagatagat aaggctgatt cttacatgcc ttatctaatc 900
   gacttaggtc tgtcctcaaa atctccatat tcatcagtta aaaatccagc tttccatttt 960
   tggggtcaat tgaccgcatt gttactgaga tcaaccagag ccagaaatgc acgtcagccg 1020
   gatgacatcg agtatacatc cctgaccact gctgggctgt tgtatgcata tgccgttggt 1080
   tcgtctgcag acctggctca acaattctac gttggggaca acaagtatgt gccagaaact 1140
   ggagatggag gattaaccac caatgcaccg ccacaagggc gagatgtggt cgagtggctt 1200
   agttggtttg aagatcaaaa cagaaaacct accccagaca tgctcatgta tgctaagaga 1260
   gctgtcagtg ctttacaagg attgagggag aagacgattg gcaagtacgc caagtcagag 1320
   tttgacaaat gacaactcac tcaccatatg tattactacc tttgcttcat atgaaaaaaa 1380
   ctaacagcga tcatggatca gctatcaaag gtcaaggaat tccttaagac ttacgcgcag 1440
   ttggatcaag cagtacaaga gatggatgac attgagtctc agagagagga aaagactaat 1500
   tttgatttgt ttcaggaaga aggattggag attaaggaga agccttccta ttatcgggca 1560
   gatgaagaag agattgattc agatgaagac agcgtggatg atgcacaaga cttagggata 1620
   cgtacatcaa caagtcccat cgaggggtat gtggatgagg agcaggatga ttatgaggat 1680
   gaggaagtga acgtggtgtt tacatcggac tggaaacagc ctgagctgga atccgacggg 1740
   gatgggaaaa ctctccgatt gacgatacca gatggattga ctggggagca gaagtcgcaa 1800
   tggcttgcca cgattaaggc agttgttcag agtgctaaat attggaacat ctcagaatgt 1860
   tcatttgaga gttatgagca aggggttttg attagagaga gacaaatgac tcctgatgtc 1920
   tacaaagtca ctcctgtttt aaatgctcca ccggttcaaa tgacagctaa tcaagatgtt 1980
   tggtctctca gcagcactcc atttacattt ttgcccaaga aacaaggtgt gactccattg 2040
   accatgtcct tagaagaact cttcaacacc cgaggtgaat tcatatctct gggaggaaac 2100
   gggaaaatga gtcaccggga ggccatcatt ctagggttga gacacaagaa gctctataat 2160
   caagccagac taaagtataa cttagcttga atatgaaaaa aactaacaga tatcaaaaga 2220
   tatctctaac tcagtccatt gtgttcagtt caatcatgag ctctctcaag aaaattttgg 2280
   gtattaaagg gaaagggaag aaatctaaga aattaggtat ggctccccca ccctatgaag 2340
   aagagactcc aatggaatat tctccaagtg caccttatga taagtcattg tttggagtcg 2400
   aagatgattt ccacgatcaa cgtcaactcc gatatgagaa atttcacttc tcattgaaga 2460
   tgactgtgag atcaaacaaa ccatttcgaa attatgatga cgttgcagca gcggtgtcca 2520
   attgggatca tatgtacatc ggcatggcag gaaaacgtcc tttttataag atattagcat 2580
   tcatgggttc tactctattg aaggctacac cagctgtctt ggctgaccaa ggacagccag 2640
   aatatcatgc tcactgtgag ggacgagctt acttgccgca tcggttaggg ccgacccctc 2700
   cgatgttgaa tgtccctgaa cattttcgcc gtccatttaa catcggatta ttcagaggga 2760
   caatcgacat aaccctggta cttttcgatg atgaatctgt agattctgcc ccggtcatat 2820
   gggatcattt taatgcatcc agattgagca gcttcagaga aaaggctttg ttgtttggtt 2880
   tgattctaga aaagaaagcc actgggaatt gggtattgga ctctattagt catttcaagt 2940
   aattatcaca agtgttgagg tgatgggcag actatgaaaa aaactaacag ggttcaaaca 3000
   ctcttgatcg aggtacccag ttatatttgt tacaacaatg ggcgttacag gaatattgca 3060
   gttacctcgt gatcgattca agaggacatc attctttctt tgggtaatta tccttttcca 3120
   aagaacattt tccatcccac ttggagtcat ccacaatagc acattacagg ttagtgatgt 3180
   cgacaaacta gtttgtcgtg acaaactgtc atccacaaat caattgagat cagttggact 3240
   gaatctcgaa gggaatggag tggcaactga cgtgccatct gcaactaaaa gatggggctt 3300
   caggtccggt gtcccaccaa aggtggtcaa ttatgaagct ggtgaatggg ctgaaaactg 3360
   ctacaatctt gaaatcaaaa aacctgacgg gagtgagtgt ctaccagcag cgccagacgg 3420
   gattcggggc ttcccccggt gccggtatgt gcacaaagta tcaggaacgg gaccgtgtgc 3480
   cggagacttt gccttccata aagagggtgc tttcttcctg tatgatcgac ttgcttccac 3540
   agttatctac cgaggaacga ctttcgctga aggtgtcgtt gcatttctga tactgcccca 3600
   agctaagaag gacttcttca gctcacaccc cttgagagag ccggtcaatg caacggagga 3660
   cccgtctagt ggctactatt ctaccacaat tagatatcag gctaccggtt ttggaaccaa 3720
   tgagacagag tacttgttcg aggttgacaa tttgacctac gtccaacttg aatcaagatt 3780
   cacaccacag tttctgctcc agctgaatga gacaatatat acaagtggga aaaggagcaa 3840
   taccacggga aaactaattt ggaaggtcaa ccccgaaatt gatacaacaa tcggggagtg 3900
   ggccttctgg gaaactaaaa aaaacctcac tagaaaaatt cgcagtgaag agttgtcttt 3960
   cacagttgta tcaaacggag ccaaaaacat cagtggtcag agtccggcgc gaacttcttc 4020
   cgacccaggg accaacacaa caactgaaga ccacaaaatc atggcttcag aaaattcctc 4080
   tgcaatggtt caagtgcaca gtcaaggaag ggaagctgca gtgtcgcatc taacaaccct 4140
   tgccacaatc tccacgagtc cccaatccct cacaaccaaa ccaggtccgg acaacagcac 4200
   ccataataca cccgtgtata aacttgacat ctctgaggca actcaagttg aacaacatca 4260
   ccgcagaaca gacaacgaca gcacagcctc cgacactccc tctgccacga ccgcagccgg 4320
   acccccaaaa gcagagaaca ccaacacgag caagagcact gacttcctgg accccgccac 4380
   cacaacaagt ccccaaaacc acagcgagac cgctggcaac aacaacactc atcaccaaga 4440
   taccggagaa gagagtgcca gcagcgggaa gctaggctta attaccaata ctattgctgg 4500
   agtcgcagga ctgatcacag gcgggagaag aactcgaaga gaagcaattg tcaatgctca 4560
   acccaaatgc aaccctaatt tacattactg gactactcag gatgaaggtg ctgcaatcgg 4620
   actggcctgg ataccatatt tcgggccagc agccgaggga atttacatag aggggctaat 4680
   gcacaatcaa gatggtttaa tctgtgggtt gagacagctg gccaacgaga cgactcaagc 4740
   tcttcaactg ttcctgagag ccacaactga gctacgcacc ttttcaatcc tcaaccgtaa 4800
   ggcaattgat ttcttgctgc agcgatgggg cggcacatgc cacattctgg gaccggactg 4860
   ctgtatcgaa ccacatgatt ggaccaagaa cataacagac aaaattgatc agattattca 4920
   tgattttgtt gataaaaccc ttccggacca gggggacaat gacaattggt ggacaggatg 4980
   gagacaatgg ataccggcag gtattggagt tacaggcgtt ataattgcag ttatcgcttt 5040
   attctgtata tgcaaatttg tcttttaggc ggccgccaga tgacgcatga gggtcagatc 5100
   agatttacag cgtaagtgtg atatttagga ttataaaggt tccttcattt taatttgtta 5160
   cagactgtat gaaaaaaact catcaacagc catcatggat gttaacgatt ttgagttgca 5220
   tgaggacttt gcattgtctg aagatgactt tgtcacttca gaatttctca atccggaaga 5280
   ccaaatgaca tacctgaatc atgccgatta taatttgaat tctcccttaa tcagcgatga 5340
   tattgatttc ctgatcaaga aatataatca tgagcaaatt ccgaaaatgt gggatgtcaa 5400
   gaattgggag ggagtgttag agatgttgac agcctgtcaa gccagtccaa ttttatctag 5460
   cactatgcat aagtgggtgg gaaagtggct catgtctgat gatcatgacg caagccaagg 5520
   cttcagtttt cttcatgaag tggacaaaga agctgatctg acgtttgagg tggtggagac 5580
   attcattaga ggatggggag gtcgagaatt gcagtacaag aggaaagaca catttccgga 5640
   ctcctttaga gttgcagcct cattgtgtca aaaattcctt gatttgcaca aactcactct 5700
   gataatgaat tcagtctctg aagtcgaact taccaaccta gcaaagaatt ttaaaggaaa 5760
   aaacaggaaa gcaaaaagcg gaaatctgat aaccagattg agggttccca gtttaggtcc 5820
   tgcttttgtg actcagggat gggtgtacat gaagaagttg gaaatgatta tggatcggaa 5880
   ttttttgttg atgttgaaag acgttatcat cgggaggatg cagacgatcc tgtccatgat 5940
   ctcaagagat gataatctct tctccgagtc tgatatcttt actgtattaa agatataccg 6000
   gataggggat aagatattag aaaggcaagg gacaaagggt tacgacttga tcaaaatgat 6060
   tgagcctatt tgtaacttaa agatgatgaa tctggcacgt aaatatcgtc ctctcatccc 6120
   tacatttcct cattttgaaa aacatattgc tgactctgtt aaggaaggat cgaaaataga 6180
   caaagggatt gagtttatat atgatcacat tatgtcaatc cctggtgtgg acttgacctt 6240
   agttatttac ggatcatttc ggcactgggg tcatcctttt atcaactact atgagggctt 6300
   agagaagcta cacaagcagg ttacaatgcc caagactatt gacagagaat atgcagaatg 6360
   tcttgctagt gatctggcaa gaatcgttct tcagcaacaa ttcaatgaac ataagaaatg 6420
   gtttgttgat gtagataaag tcccacaatc ccatcctttc aaaagccata tgaaagagaa 6480
   tacttggcct actgcagccc aagttcagga ttacggcgat cgctggcatc agctcccact 6540
   catcaaatgc ttcgaaatcc cagatttgtt agatccatcg atcatctact cagacaaaag 6600
   tcattccatg aaccggtctg aagtactacg acatgtaaga cttacacctc atgtgcccat 6660
   tccaagcagg aaagtattgc agacaatgtt ggagactaag gcaacagact ggaaagagtt 6720
   tttaaagaaa attgacgaag aggggttaga ggatgatgat cttgtcatag gactcaaagg 6780
   gaaagagaga gaattaaaaa ttgcgggaag attcttttct ttgatgtcct ggaagctcag 6840
   agagtatttt gtcatcactg agtatttgat taagacgcac tttgtcccga tgtttaaagg 6900
   gttgaccatg gcggatgact tgacagcggt gataaagaag atgatggaca catcttcagg 6960
   acaaggctta gataattatg aatccatttg tatagccaac catattgact atgagaagtg 7020
   gaacaatcat caaagaaaag agtcgaacgg gcccgtgttc aaggtgatgg gtcaattctt 7080
   gggatatcca cgtctgattg agagaactca tgaatttttt gagaagagtc tgatatatta 7140
   caatggacga ccagatctga tgcgggttcg aggaaattct ctagtcaacg cctcatcttt 7200
   aaatgtctgc tgggagggtc aagctggggg attagaagga ctgcgacaga agggatggag 7260
   tattctaaat ttgcttgtca ttcagagaga agcaaaaata aggaacaccg ccgtgaaagt 7320
   gctagctcaa ggtgacaatc aggtgatatg tactcagtat aaaacgaaga aatcccggaa 7380
   tgatattgag cttaaggcag ctctaacaca gatggtatct aataatgaga tgattatgtc 7440
   tgcgattaaa tcaggcaccg agaaactggg tcttttgatt aatgatgatg agacaatgca 7500
   atctgctgat tacctcaatt acgggaaggt tcccattttc agaggagtaa tcagaggcct 7560
   tgagacaaaa agatggtcac gcgtgacctg tgtgacaaat gatcagattc caacgtgtgc 7620
   gaacattatg agctctgtgt caactaatgc attaactgta gcccattttg ccgagaatcc 7680
   agtcaatgcc atcattcagt ataactactt tggaacattt gcaaggctac tgctgatgat 7740
   gcatgacccc gctctgagga tctctctgta tgaagtccaa tcaaaaattc caggacttca 7800
   cagtttgaca tttaaatatt ctatgttgta tctggatcct tcgataggag gagtctccgg 7860
   aatgtcactc tcgagattcc tcataagatc atttccagat ccagtgacag aaagtttggc 7920
   gttctggaaa tttatccact ctcatgcaag aagcgattca ttaaaggaga tatgtgcagt 7980
   ttttggaaat cctgaaattg caagatttcg gctaactcat gtcgataaat tggtggaaga 8040
   cccaacctca ttgaacatag ctatgggaat gagtcctgct aatctattaa agacagaggt 8100
   aaaaaaatgt ctactggaat caaggcagag catcaagaac cagattgtaa gagatgctac 8160
   tatttaccta caccatgagg aagacaaact tcgtagtttc ttatggtcca taacaccact 8220
   gttccctcgg ttcttgagtg aattcaaatc tgggacattc atcggagtag cagatggcct 8280
   gatcagctta tttcagaact ctaggactat tcgaaattct tttaaaaagc gttatcacag 8340
   ggaacttgat gatttaataa tcaagagcga agtttcctca cttatgcatt tgggtaagct 8400
   acatttgagg cgaggctcag ttcgtatgtg gacttgctct tctactcagg ctgatcttct 8460
   ccgattccgg tcatggggaa gatctgttat aggaaccaca gtccctcatc ccttagagat 8520
   gttaggacaa cattttaaaa aggagactcc ttgcagtgct tgcaacatat ccggattaga 8580
   ctatgtatct gtccactgtc cgaatgggat tcatgacgtt tttgaatcac gtggtccact 8640
   ccctgcatat ttgggttcta aaacatccga atcaacttcg atcttgcagc cgtgggagag 8700
   agagagtaaa gtaccgttga ttaagcgtgc cacaaggctt cgtgatgcaa tttcatggtt 8760
   tgtgtctccc gactctaact tggcctcaac tatccttaag aacataaatg cattaacagg 8820
   agaagaatgg tcaaagaagc agcatggatt taaaaggacg ggatcggcgt tacacaggtt 8880
   ctccacatcc aggatgagtc atggtggttt tgcttctcag agtacggctg ccttgactag 8940
   attgatggca actactgaca ctatgagaga tctgggagaa cagaactatg atttcctgtt 9000
   tcaggcgaca ttattgtatg ctcaaataac cacaactgta gtcaggaatg gatcatttca 9060
   tagctgcacg gaccattacc atataacctg caaatcttgt ctgagggcca ttgatgagat 9120
   taccttggat tcagcgatgg aatatagccc tccagatgta tcatcagttt tacaatcttg 9180
   gaggaatgga gaaggctctt ggggacatga agtgaaacaa atatacccag ttgaaggtga 9240
   ctggagggga ctatctcctg ttgaacaatc ttatcaagtc ggacgctgta tcgggtttct 9300
   gttcggtgat ctggcgtata gaaaatcatc ccatgcagat gatagctcca tgtttccgtt 9360
   atctatacaa aacaaagtca gaggaagagg ctttttaaaa gggcttatgg atgggttaat 9420
   gagagccagt tgttgccagg tgatccatcg tcgaagctta gcccatctga agagaccggc 9480
   taatgcagtc tatggagggc tgatttattt gatagacaaa ttgagtgcat ctgccccttt 9540
   tctttcactg acgagacatg gacctttaag ggaagaatta gaaactgttc cacataagat 9600
   accgacttct tatcctacga gcaaccgaga tatgggggtg atagttcgta attattttaa 9660
   atatcagtgc agactggtag aaaaaggtcg gtacaagaca cattatcctc aattgtggct 9720
   tttctcagat gtgctgtcca ttgatttctt aggacccctg tctatatctt caactctatt 9780
   gggtattctg tataaacaga cgttatcttc tcgagacaaa aatgagttga gagaactcgc 9840
   taacttgtct tcattgttga gatcaggaga aggatgggaa gatatccatg tcaaattctt 9900
   ctctaaggac actttactct gccctgaaga gatccgacat gcgtgcaaat ttgggattgc 9960
   taaggaatcc gctgttttaa gctattatcc tccttggtct caagagtctt atggaggcat 10020
   cacctcgatc cccgtatatt tttcgaccag gaagtatccc aaaattttag atgtccctcc 10080
   tcgggttcaa aacccattgg tctcgggtct acgattgggg caactcccta ctggagcaca 10140
   ttataagatt aggagcattg taaagaacaa gaaccttcgt tatagagatt tccttagttg 10200
   tggggatgga tctgggggga tgaccgcggc actattgaga gaaaacagac aaagtagggg 10260
   aatcttcaac agcctgttag agttagccgg atctcttatg agaggagcat ctccagagcc 10320
   tccaagtgca ctggagacgc tcgggcaaga acgatctagg tgtgtgaatg gaagcacatg 10380
   ttgggagtac tcatctgacc taagccaaaa agagacatgg gattacttct taagattgaa 10440
   gagaggcctg ggtttgaccg tggacttaat caccatggac atggaggtca gagaccctaa 10500
   tacaagtttg atgatagaaa agaacctcaa agtttatctg catcagatat tagaaccaac 10560
   tggtgtctta atatataaaa catacgggac ccatattgcg acacaaacag ataatatcct 10620
   gacgataatc ggtcctttct ttgagacggt tgacctagtc cagtccgaat acagcagctc 10680
   acaaacgtcc gaggtctatt ttgtaggacg aggcttgcgc tctcatgttg acgaaccctg 10740
   ggtggactgg ccatccttaa tggacaattg gagatccatt tatgcttttc atgatcctac 10800
   tacagaattt atcagagcaa aaaaagtctg tgaaattgac agtcttatag gcattccggc 10860
   tcaattcatt ccagacccat ttgtaaatct cgagaccatg ctacagatag ttggtgttcc 10920
   aacaggagtt tcgcatgccg cagctctatt atcatcacaa tatccaaatc aattggtcac 10980
   aacgtcaata ttttatatga cactcgtgtc ttattataat gtaaaccata ttcgaagaag 11040
   ccccaagcct ttctctcctc cgtctgatgg agtctcacag aacattggtt cagccatagt 11100
   cggactaagt ttttgggtga gtttgatgga gaatgatctc ggattataca aacaggctct 11160
   aggtgcaata aagacgtcat tccctattag atggtcctct gtccagacca aggatgggtt 11220
   tacacaagaa tggagaacta aaggaaacgg aattcctaaa gattgtcgtc tctcagactc 11280
   tttggctcag ataggaaact ggatcagagc gatggaattg gttaggaaca aaacgaggca 11340
   atcaggattt tctgaaaccc tatttgatca attctgcgga cttgcagacc atcacctcaa 11400
   atggcggaag ttgggaaaca gaacaggaat tattgattgg ctaaataata gaatttcatc 11460
   cattgacaaa tccatcttgg tgaccaaaag tgatctgcat gacgagaact catggaggga 11520
   gtgaagatgt attcttccac ctctcattgg gtgataccca tatatgaaaa aaactataag 11580
   tactttaaac tctctttgtt ttttaatgta tatctggttt tgttgtttcc gt 11632
<210> 14
   <211> 11632
   <212> RNA
   <213> Artificial
<220>
   <223> Chimeric virus containing RNA from Maraba and Ebola viruses
<400> 14
   acgaagacaa acaaaccauu gauagaauua agaggcucau gaaaauccuu aacagcguuc     60
   aaaaugucug uuacagucaa gagagucauu gaugauucac ucaucacccc caaauugccu    120
   gcgaaugagg acccugugga guacccugcu gauuauuuca aaaagucccg ugauauuccg    180
   guguacauaa acacgaccaa aaguuugucu gauuugcggg gcuauguuua ucaaggccua    240
   aagucaggca acaucucuau aauucauguc aacaguuauc uguaugcagc auuaaaagag    300
   aucagaggaa aauuggacag agauuggauc accuuuggua uccaaaucgg aaaaacagga    360
   gauagcgugg ggauauucga uuuacugacc cuaaaaccuc uagauggugu uuuaccagau    420
   ggggugucug augcuacucg aacuagcuca gacgaugcau ggcuuccacu guaucuauug    480
   ggguuauaca gaguuggucg aacacagaug ccagaauaca ggaagaagcu gauggauggu 540
   cugauuaauc aauguaagau gaucaaugag caguuugaac cacuguugcc agaaggaaga 600
   gaugucuuug augucugggg aaaugacagc aauuacacaa agauuguggc cgcuguagau 660
   auguucuucc auauguucaa aaagcaugag aaggccucuu ucagguaugg cacaauagug 720
   ucaagauuua aggauugugc agcauuggcu acauuugguc aucuguguaa gaucacuggu 780
   auguccacug aagaugugac aacuuggauu cuaaacaggg agguggcuga ugagaugguu 840
   caaaugaugu acccaggaca ggagauagau aaggcugauu cuuacaugcc uuaucuaauc 900
   gacuuagguc uguccucaaa aucuccauau ucaucaguua aaaauccagc uuuccauuuu 960
   uggggucaau ugaccgcauu guuacugaga ucaaccagag ccagaaaugc acgucagccg 1020
   gaugacaucg aguauacauc ccugaccacu gcugggcugu uguaugcaua ugccguuggu 1080
   ucgucugcag accuggcuca acaauucuac guuggggaca acaaguaugu gccagaaacu 1140
   ggagauggag gauuaaccac caaugcaccg ccacaagggc gagauguggu cgaguggcuu 1200
   aguugguuug aagaucaaaa cagaaaaccu accccagaca ugcucaugua ugcuaagaga 1260
   gcugucagug cuuuacaagg auugagggag aagacgauug gcaaguacgc caagucagag 1320
   uuugacaaau gacaacucac ucaccauaug uauuacuacc uuugcuucau augaaaaaaa 1380
   cuaacagcga ucauggauca gcuaucaaag gucaaggaau uccuuaagac uuacgcgcag 1440
   uuggaucaag caguacaaga gauggaugac auugagucuc agagagagga aaagacuaau 1500
   uuugauuugu uucaggaaga aggauuggag auuaaggaga agccuuccua uuaucgggca 1560
   gaugaagaag agauugauuc agaugaagac agcguggaug augcacaaga cuuagggaua 1620
   cguacaucaa caagucccau cgagggguau guggaugagg agcaggauga uuaugaggau 1680
   gaggaaguga acgugguguu uacaucggac uggaaacagc cugagcugga auccgacggg 1740
   gaugggaaaa cucuccgauu gacgauacca gauggauuga cuggggagca gaagucgcaa 1800
   uggcuugcca cgauuaaggc aguuguucag agugcuaaau auuggaacau cucagaaugu 1860
   ucauuugaga guuaugagca agggguuuug auuagagaga gacaaaugac uccugauguc 1920
   uacaaaguca cuccuguuuu aaaugcucca ccgguucaaa ugacagcuaa ucaagauguu 1980
   uggucucuca gcagcacucc auuuacauuu uugcccaaga aacaaggugu gacuccauug 2040
   accauguccu uagaagaacu cuucaacacc cgaggugaau ucauaucucu gggaggaaac 2100
   gggaaaauga gucaccggga ggccaucauu cuaggguuga gacacaagaa gcucuauaau 2160
   caagccagac uaaaguauaa cuuagcuuga auaugaaaaa aacuaacaga uaucaaaaga 2220
   uaucucuaac ucaguccauu guguucaguu caaucaugag cucucucaag aaaauuuugg 2280
   guauuaaagg gaaagggaag aaaucuaaga aauuagguau ggcuccccca cccuaugaag 2340
   aagagacucc aauggaauau ucuccaagug caccuuauga uaagucauug uuuggagucg 2400
   aagaugauuu ccacgaucaa cgucaacucc gauaugagaa auuucacuuc ucauugaaga 2460
   ugacugugag aucaaacaaa ccauuucgaa auuaugauga cguugcagca gcggugucca 2520
   auugggauca uauguacauc ggcauggcag gaaaacgucc uuuuuauaag auauuagcau 2580
   ucauggguuc uacucuauug aaggcuacac cagcugucuu ggcugaccaa ggacagccag      2640
   aauaucaugc ucacugugag ggacgagcuu acuugccgca ucgguuaggg ccgaccccuc 2700
   cgauguugaa ugucccugaa cauuuucgcc guccauuuaa caucggauua uucagaggga 2760
   caaucgacau aacccuggua cuuuucgaug augaaucugu agauucugcc ccggucauau 2820
   gggaucauuu uaaugcaucc agauugagca gcuucagaga aaaggcuuug uuguuugguu 2880
   ugauucuaga aaagaaagcc acugggaauu ggguauugga cucuauuagu cauuucaagu 2940
   aauuaucaca aguguugagg ugaugggcag acuaugaaaa aaacuaacag gguucaaaca 3000
   cucuugaucg agguacccag uuauauuugu uacaacaaug ggcguuacag gaauauugca 3060
   guuaccucgu gaucgauuca agaggacauc auucuuucuu uggguaauua uccuuuucca 3120
   aagaacauuu uccaucccac uuggagucau ccacaauagc acauuacagg uuagugaugu 3180
   cgacaaacua guuugucgug acaaacuguc auccacaaau caauugagau caguuggacu 3240
   gaaucucgaa gggaauggag uggcaacuga cgugccaucu gcaacuaaaa gauggggcuu     3300
   cagguccggu gucccaccaa agguggucaa uuaugaagcu ggugaauggg cugaaaacug 3360
   cuacaaucuu gaaaucaaaa aaccugacgg gagugagugu cuaccagcag cgccagacgg 3420
   gauucggggc uucccccggu gccgguaugu gcacaaagua ucaggaacgg gaccgugugc     3480
   cggagacuuu gccuuccaua aagagggugc uuucuuccug uaugaucgac uugcuuccac 3540
   aguuaucuac cgaggaacga cuuucgcuga aggugucguu gcauuucuga uacugcccca 3600
   agcuaagaag gacuucuuca gcucacaccc cuugagagag ccggucaaug caacggagga 3660
   cccgucuagu ggcuacuauu cuaccacaau uagauaucag gcuaccgguu uuggaaccaa 3720
   ugagacagag uacuuguucg agguugacaa uuugaccuac guccaacuug aaucaagauu 3780
   cacaccacag uuucugcucc agcugaauga gacaauauau acaaguggga aaaggagcaa 3840
   uaccacggga aaacuaauuu ggaaggucaa ccccgaaauu gauacaacaa ucggggagug 3900
   ggccuucugg gaaacuaaaa aaaaccucac uagaaaaauu cgcagugaag aguugucuuu 3960
   cacaguugua ucaaacggag ccaaaaacau caguggucag aguccggcgc gaacuucuuc 4020
   cgacccaggg accaacacaa caacugaaga ccacaaaauc auggcuucag aaaauuccuc 4080
   ugcaaugguu caagugcaca gucaaggaag ggaagcugca gugucgcauc uaacaacccu 4140
   ugccacaauc uccacgaguc cccaaucccu cacaaccaaa ccagguccgg acaacagcac 4200
   ccauaauaca cccguguaua aacuugacau cucugaggca acucaaguug aacaacauca 4260
   ccgcagaaca gacaacgaca gcacagccuc cgacacuccc ucugccacga ccgcagccgg 4320
   acccccaaaa gcagagaaca ccaacacgag caagagcacu gacuuccugg accccgccac 4380
   cacaacaagu ccccaaaacc acagcgagac cgcuggcaac aacaacacuc aucaccaaga 4440
   uaccggagaa gagagugcca gcagcgggaa gcuaggcuua auuaccaaua cuauugcugg 4500
   agucgcagga cugaucacag gcgggagaag aacucgaaga gaagcaauug ucaaugcuca 4560
   acccaaaugc aacccuaauu uacauuacug gacuacucag gaugaaggug cugcaaucgg 4620
   acuggccugg auaccauauu ucgggccagc agccgaggga auuuacauag aggggcuaau 4680
   gcacaaucaa gaugguuuaa ucuguggguu gagacagcug gccaacgaga cgacucaagc 4740
   ucuucaacug uuccugagag ccacaacuga gcuacgcacc uuuucaaucc ucaaccguaa 4800
   ggcaauugau uucuugcugc agcgaugggg cggcacaugc cacauucugg gaccggacug 4860
   cuguaucgaa ccacaugauu ggaccaagaa cauaacagac aaaauugauc agauuauuca 4920
   ugauuuuguu gauaaaaccc uuccggacca gggggacaau gacaauuggu ggacaggaug 4980
   gagacaaugg auaccggcag guauuggagu uacaggcguu auaauugcag uuaucgcuuu 5040
   auucuguaua ugcaaauuug ucuuuuaggc ggccgccaga ugacgcauga gggucagauc 5100
   agauuuacag cguaagugug auauuuagga uuauaaaggu uccuucauuu uaauuuguua 5160
   cagacuguau gaaaaaaacu caucaacagc caucauggau guuaacgauu uugaguugca 5220
   ugaggacuuu gcauugucug aagaugacuu ugucacuuca gaauuucuca auccggaaga 5280
   ccaaaugaca uaccugaauc augccgauua uaauuugaau ucucccuuaa ucagcgauga 5340
   uauugauuuc cugaucaaga aauauaauca ugagcaaauu ccgaaaaugu gggaugucaa 5400
   gaauugggag ggaguguuag agauguugac agccugucaa gccaguccaa uuuuaucuag 5460
   cacuaugcau aagugggugg gaaaguggcu caugucugau gaucaugacg caagccaagg 5520
   cuucaguuuu cuucaugaag uggacaaaga agcugaucug acguuugagg ugguggagac 5580
   auucauuaga ggauggggag gucgagaauu gcaguacaag aggaaagaca cauuuccgga 5640
   cuccuuuaga guugcagccu cauuguguca aaaauuccuu gauuugcaca aacucacucu 5700
   gauaaugaau ucagucucug aagucgaacu uaccaaccua gcaaagaauu uuaaaggaaa 5760
   aaacaggaaa gcaaaaagcg gaaaucugau aaccagauug aggguuccca guuuaggucc 5820
   ugcuuuugug acucagggau ggguguacau gaagaaguug gaaaugauua uggaucggaa 5880
   uuuuuuguug auguugaaag acguuaucau cgggaggaug cagacgaucc uguccaugau 5940
   cucaagagau gauaaucucu ucuccgaguc ugauaucuuu acuguauuaa agauauaccg 6000
   gauaggggau aagauauuag aaaggcaagg gacaaagggu uacgacuuga ucaaaaugau 6060
   ugagccuauu uguaacuuaa agaugaugaa ucuggcacgu aaauaucguc cucucauccc 6120
   uacauuuccu cauuuugaaa aacauauugc ugacucuguu aaggaaggau cgaaaauaga 6180
   caaagggauu gaguuuauau augaucacau uaugucaauc ccuggugugg acuugaccuu 6240
   aguuauuuac ggaucauuuc ggcacugggg ucauccuuuu aucaacuacu augagggcuu 6300
   agagaagcua cacaagcagg uuacaaugcc caagacuauu gacagagaau augcagaaug 6360
   ucuugcuagu gaucuggcaa gaaucguucu ucagcaacaa uucaaugaac auaagaaaug 6420
   guuuguugau guagauaaag ucccacaauc ccauccuuuc aaaagccaua ugaaagagaa 6480
   uacuuggccu acugcagccc aaguucagga uuacggcgau cgcuggcauc agcucccacu 6540
   caucaaaugc uucgaaaucc cagauuuguu agauccaucg aucaucuacu cagacaaaag 6600
   ucauuccaug aaccggucug aaguacuacg acauguaaga cuuacaccuc augugcccau 6660
   uccaagcagg aaaguauugc agacaauguu ggagacuaag gcaacagacu ggaaagaguu 6720
   uuuaaagaaa auugacgaag agggguuaga ggaugaugau cuugucauag gacucaaagg 6780
   gaaagagaga gaauuaaaaa uugcgggaag auucuuuucu uugauguccu ggaagcucag 6840
   agaguauuuu gucaucacug aguauuugau uaagacgcac uuugucccga uguuuaaagg 6900
   guugaccaug gcggaugacu ugacagcggu gauaaagaag augauggaca caucuucagg 6960
   acaaggcuua gauaauuaug aauccauuug uauagccaac cauauugacu augagaagug 7020
   gaacaaucau caaagaaaag agucgaacgg gcccguguuc aaggugaugg gucaauucuu 7080
   gggauaucca cgucugauug agagaacuca ugaauuuuuu gagaagaguc ugauauauua 7140
   caauggacga ccagaucuga ugcggguucg aggaaauucu cuagucaacg ccucaucuuu 7200
   aaaugucugc ugggaggguc aagcuggggg auuagaagga cugcgacaga agggauggag 7260
   uauucuaaau uugcuuguca uucagagaga agcaaaaaua aggaacaccg ccgugaaagu 7320
   gcuagcucaa ggugacaauc aggugauaug uacucaguau aaaacgaaga aaucccggaa 7380
   ugauauugag cuuaaggcag cucuaacaca gaugguaucu aauaaugaga ugauuauguc 7440
   ugcgauuaaa ucaggcaccg agaaacuggg ucuuuugauu aaugaugaug agacaaugca 7500
   aucugcugau uaccucaauu acgggaaggu ucccauuuuc agaggaguaa ucagaggccu 7560
   ugagacaaaa agauggucac gcgugaccug ugugacaaau gaucagauuc caacgugugc 7620
   gaacauuaug agcucugugu caacuaaugc auuaacugua gcccauuuug ccgagaaucc 7680
   agucaaugcc aucauucagu auaacuacuu uggaacauuu gcaaggcuac ugcugaugau 7740
   gcaugacccc gcucugagga ucucucugua ugaaguccaa ucaaaaauuc caggacuuca 7800
   caguuugaca uuuaaauauu cuauguugua ucuggauccu ucgauaggag gagucuccgg 7860
   aaugucacuc ucgagauucc ucauaagauc auuuccagau ccagugacag aaaguuuggc 7920
   guucuggaaa uuuauccacu cucaugcaag aagcgauuca uuaaaggaga uaugugcagu 7980
   uuuuggaaau ccugaaauug caagauuucg gcuaacucau gucgauaaau ugguggaaga 8040
   cccaaccuca uugaacauag cuaugggaau gaguccugcu aaucuauuaa agacagaggu 8100
   aaaaaaaugu cuacuggaau caaggcagag caucaagaac cagauuguaa gagaugcuac 8160
   uauuuaccua caccaugagg aagacaaacu ucguaguuuc uuauggucca uaacaccacu 8220
   guucccucgg uucuugagug aauucaaauc ugggacauuc aucggaguag cagauggccu 8280
   gaucagcuua uuucagaacu cuaggacuau ucgaaauucu uuuaaaaagc guuaucacag 8340
   ggaacuugau gauuuaauaa ucaagagcga aguuuccuca cuuaugcauu uggguaagcu 8400
   acauuugagg cgaggcucag uucguaugug gacuugcucu ucuacucagg cugaucuucu 8460
   ccgauuccgg ucauggggaa gaucuguuau aggaaccaca gucccucauc ccuuagagau 8520
   guuaggacaa cauuuuaaaa aggagacucc uugcagugcu ugcaacauau ccggauuaga 8580
   cuauguaucu guccacuguc cgaaugggau ucaugacguu uuugaaucac gugguccacu 8640
   cccugcauau uuggguucua aaacauccga aucaacuucg aucuugcagc cgugggagag 8700
   agagaguaaa guaccguuga uuaagcgugc cacaaggcuu cgugaugcaa uuucaugguu 8760
   ugugucuccc gacucuaacu uggccucaac uauccuuaag aacauaaaug cauuaacagg 8820
   agaagaaugg ucaaagaagc agcauggauu uaaaaggacg ggaucggcgu uacacagguu 8880
   cuccacaucc aggaugaguc auggugguuu ugcuucucag aguacggcug ccuugacuag 8940
   auugauggca acuacugaca cuaugagaga ucugggagaa cagaacuaug auuuccuguu 9000
   ucaggcgaca uuauuguaug cucaaauaac cacaacugua gucaggaaug gaucauuuca 9060
   uagcugcacg gaccauuacc auauaaccug caaaucuugu cugagggcca uugaugagau 9120
   uaccuuggau ucagcgaugg aauauagccc uccagaugua ucaucaguuu uacaaucuug 9180
   gaggaaugga gaaggcucuu ggggacauga agugaaacaa auauacccag uugaagguga 9240
   cuggagggga cuaucuccug uugaacaauc uuaucaaguc ggacgcugua ucggguuucu 9300
   guucggugau cuggcguaua gaaaaucauc ccaugcagau gauagcucca uguuuccguu 9360
   aucuauacaa aacaaaguca gaggaagagg cuuuuuaaaa gggcuuaugg auggguuaau 9420
   gagagccagu uguugccagg ugauccaucg ucgaagcuua gcccaucuga agagaccggc 9480
   uaaugcaguc uauggagggc ugauuuauuu gauagacaaa uugagugcau cugccccuuu 9540
   ucuuucacug acgagacaug gaccuuuaag ggaagaauua gaaacuguuc cacauaagau 9600
   accgacuucu uauccuacga gcaaccgaga uaugggggug auaguucgua auuauuuuaa 9660
   auaucagugc agacugguag aaaaaggucg guacaagaca cauuauccuc aauuguggcu 9720
   uuucucagau gugcugucca uugauuucuu aggaccccug ucuauaucuu caacucuauu 9780
   ggguauucug uauaaacaga cguuaucuuc ucgagacaaa aaugaguuga gagaacucgc 9840
   uaacuugucu ucauuguuga gaucaggaga aggaugggaa gauauccaug ucaaauucuu 9900
   cucuaaggac acuuuacucu gcccugaaga gauccgacau gcgugcaaau uugggauugc 9960
   uaaggaaucc gcuguuuuaa gcuauuaucc uccuuggucu caagagucuu auggaggcau 10020
   caccucgauc cccguauauu uuucgaccag gaaguauccc aaaauuuuag augucccucc 10080
   ucggguucaa aacccauugg ucucgggucu acgauugggg caacucccua cuggagcaca 10140
   uuauaagauu aggagcauug uaaagaacaa gaaccuucgu uauagagauu uccuuaguug 10200
   uggggaugga ucugggggga ugaccgcggc acuauugaga gaaaacagac aaaguagggg 10260
   aaucuucaac agccuguuag aguuagccgg aucucuuaug agaggagcau cuccagagcc 10320
   uccaagugca cuggagacgc ucgggcaaga acgaucuagg ugugugaaug gaagcacaug 10380
   uugggaguac ucaucugacc uaagccaaaa agagacaugg gauuacuucu uaagauugaa 10440
   gagaggccug gguuugaccg uggacuuaau caccauggac auggagguca gagacccuaa 10500
   uacaaguuug augauagaaa agaaccucaa aguuuaucug caucagauau uagaaccaac 10560
   uggugucuua auauauaaaa cauacgggac ccauauugcg acacaaacag auaauauccu 10620
   gacgauaauc gguccuuucu uugagacggu ugaccuaguc caguccgaau acagcagcuc 10680
   acaaacgucc gaggucuauu uuguaggacg aggcuugcgc ucucauguug acgaacccug 10740
   gguggacugg ccauccuuaa uggacaauug gagauccauu uaugcuuuuc augauccuac 10800
   uacagaauuu aucagagcaa aaaaagucug ugaaauugac agucuuauag gcauuccggc 10860
   ucaauucauu ccagacccau uuguaaaucu cgagaccaug cuacagauag uugguguucc 10920
   aacaggaguu ucgcaugccg cagcucuauu aucaucacaa uauccaaauc aauuggucac 10980
   aacgucaaua uuuuauauga cacucguguc uuauuauaau guaaaccaua uucgaagaag 11040
   ccccaagccu uucucuccuc cgucugaugg agucucacag aacauugguu cagccauagu 11100
   cggacuaagu uuuuggguga guuugaugga gaaugaucuc ggauuauaca aacaggcucu 11160
   aggugcaaua aagacgucau ucccuauuag augguccucu guccagacca aggauggguu 11220
   uacacaagaa uggagaacua aaggaaacgg aauuccuaaa gauugucguc ucucagacuc 11280
   uuuggcucag auaggaaacu ggaucagagc gauggaauug guuaggaaca aaacgaggca 11340
   aucaggauuu ucugaaaccc uauuugauca auucugcgga cuugcagacc aucaccucaa 11400
   auggcggaag uugggaaaca gaacaggaau uauugauugg cuaaauaaua gaauuucauc 11460
   cauugacaaa uccaucuugg ugaccaaaag ugaucugcau gacgagaacu cauggaggga 11520
   gugaagaugu auucuuccac cucucauugg gugauaccca uauaugaaaa aaacuauaag 11580
   uacuuuaaac ucucuuuguu uuuuaaugua uaucugguuu uguuguuucc gu 11632
<210> 15
   <211> 11719
   <212> DNA
   <213> Artificial
<220>
   <223> DNA encoded by chimeric virus that contains RNA from Maraba and Farmington viruses
<400> 15
   acgaagacaa acaaaccatt gatagaatta agaggctcat gaaaatcctt aacagcgttc      60
   aaaatgtctg ttacagtcaa gagagtcatt gatgattcac tcatcacccc caaattgcct     120
   gcgaatgagg accctgtgga gtaccctgct gattatttca aaaagtcccg tgatattccg     180
   gtgtacataa acacgaccaa aagtttgtct gatttgcggg gctatgttta tcaaggccta     240
   aagtcaggca acatctctat aattcatgtc aacagttatc tgtatgcagc attaaaagag     300
   atcagaggaa aattggacag agattggatc acctttggta tccaaatcgg aaaaacagga     360
   gatagcgtgg ggatattcga tttactgacc ctaaaacctc tagatggtgt tttaccagat     420
   ggggtgtctg atgctactcg aactagctca gacgatgcat ggcttccact gtatctattg     480
   gggttataca gagttggtcg aacacagatg ccagaataca ggaagaagct gatggatggt     540
   ctgattaatc aatgtaagat gatcaatgag cagtttgaac cactgttgcc agaaggaaga     600
   gatgtctttg atgtctgggg aaatgacagc aattacacaa agattgtggc cgctgtagat     660
   atgttcttcc atatgttcaa aaagcatgag aaggcctctt tcaggtatgg cacaatagtg     720
   tcaagattta aggattgtgc agcattggct acatttggtc atctgtgtaa gatcactggt     780
   atgtccactg aagatgtgac aacttggatt ctaaacaggg aggtggctga tgagatggtt     840
   caaatgatgt acccaggaca ggagatagat aaggctgatt cttacatgcc ttatctaatc     900
   gacttaggtc tgtcctcaaa atctccatat tcatcagtta aaaatccagc tttccatttt     960
   tggggtcaat tgaccgcatt gttactgaga tcaaccagag ccagaaatgc acgtcagccg    1020
   gatgacatcg agtatacatc cctgaccact gctgggctgt tgtatgcata tgccgttggt    1080
   tcgtctgcag acctggctca acaattctac gttggggaca acaagtatgt gccagaaact    1140
   ggagatggag gattaaccac caatgcaccg ccacaagggc gagatgtggt cgagtggctt    1200
   agttggtttg aagatcaaaa cagaaaacct accccagaca tgctcatgta tgctaagaga    1260
   gctgtcagtg ctttacaagg attgagggag aagacgattg gcaagtacgc caagtcagag    1320
   tttgacaaat gacaactcac tcaccatatg tattactacc tttgcttcat atgaaaaaaa    1380
   ctaacagcga tcatggatca gctatcaaag gtcaaggaat tccttaagac ttacgcgcag    1440
   ttggatcaag cagtacaaga gatggatgac attgagtctc agagagagga aaagactaat    1500
   tttgatttgt ttcaggaaga aggattggag attaaggaga agccttccta ttatcgggca    1560
   gatgaagaag agattgattc agatgaagac agcgtggatg atgcacaaga cttagggata    1620
   cgtacatcaa caagtcccat cgaggggtat gtggatgagg agcaggatga ttatgaggat    1680
   gaggaagtga acgtggtgtt tacatcggac tggaaacagc ctgagctgga atccgacggg    1740
   gatgggaaaa ctctccgatt gacgatacca gatggattga ctggggagca gaagtcgcaa 1800
   tggcttgcca cgattaaggc agttgttcag agtgctaaat attggaacat ctcagaatgt 1860
   tcatttgaga gttatgagca aggggttttg attagagaga gacaaatgac tcctgatgtc 1920
   tacaaagtca ctcctgtttt aaatgctcca ccggttcaaa tgacagctaa tcaagatgtt 1980
   tggtctctca gcagcactcc atttacattt ttgcccaaga aacaaggtgt gactccattg 2040
   accatgtcct tagaagaact cttcaacacc cgaggtgaat tcatatctct gggaggaaac 2100
   gggaaaatga gtcaccggga ggccatcatt ctagggttga gacacaagaa gctctataat 2160
   caagccagac taaagtataa cttagcttga atatgaaaaa aactaacaga tatcaaaaga 2220
   tatctctaac tcagtccatt gtgttcagtt caatcatgag ctctctcaag aaaattttgg 2280
   gtattaaagg gaaagggaag aaatctaaga aattaggtat ggctccccca ccctatgaag 2340
   aagagactcc aatggaatat tctccaagtg caccttatga taagtcattg tttggagtcg 2400
   aagatatgga tttccatgat caacgtcaac tccgatatga gaaatttcac ttctcattga 2460
   agatgactgt gagatcaaac aaaccatttc gaaattatga tgacgttgca gcagcggtgt 2520
   ccaattggga tcatatgtac atcggcatgg caggaaaacg tcctttttat aagatattag 2580
   cattcatggg ttctactcta ttgaaggcta caccagctgt cttggctgac caaggacagc 2640
   cagaatatca tgctcactgt gagggacgag cttacttgcc gcatcggtta gggccgaccc 2700
   ctccgatgtt gaatgtccct gaacattttc gccgtccatt taacatcgga ttattcagag 2760
   ggacaatcga cataaccctg gtacttttcg atgatgaatc tgtagattct gccccggtca 2820
   tatgggatca ttttaatgca tccagattga gcagcttcag agaaaaggct ttgttgtttg 2880
   gtttgattct agaaaagaaa gccactggga attgggtatt ggactctatt agtcatttca 2940
   agtaattatc acaagtgttg aggtgatggg cagactatga aaaaaactaa cagggttcaa 3000
   acactcttga tcgaggtacc cagttatatt tgttacaaca atgctcagga tccagatccc 3060
   tccgattgct atcattctgg taagtctcct cacactcgac ctgtccggtg caaggaggac 3120
   aaccacacaa agaatccctc tccttaatga ttcgtgggat ttgttctcga gctatggcga 3180
   cattcccgaa gaacttgtcg tataccagaa ctacagccac aattcctccg agttaccccc 3240
   tcctggcttc gagagatggt acataaaccg aagagtggca gacacttcca taccgtgcag 3300
   gggcccctgt ctagtgccct acatccttca tggcctcaat gacacaactg tctctcgacg 3360
   gggaggagga tggcgaaggt ccggaatgaa gtacccaacc cacgctgtca ggctaggccc 3420
   ttcaacagac gacgagagag ttgaggaaga catcggctac gtcaatgtct ccgcactatc 3480
   ctgcacaggg tcgcccgttg agatggcgat accaacaatc cccgactgca ccagtgctat 3540
   ccatccacga tccgaggtta ctgtgcccgt caagctcgat gtcatgagac gaaatcccaa 3600
   ctaccctccc attagagcgt ggtcgtgcat cggacagaaa atcaccaacc gatgtgattg 3660
   ggcactcttc ggcgagaacc tcatatatac tcaagttgaa gctagctctc tagcattcaa 3720
   gcacacaaga gcctctcttt tgaacgaatc caacgggata gacgctgaag gacgtgcagt 3780
   tccctatatc ctcggggata tcgaacccgg gtactgccga accctattca acacatgggt 3840
   ctctagtgag atcgtgtcat gcacgcccat cgaacttgtc ctagttgacc tgaacccttt 3900
   gtccccggga catggcggat atgctgtatt gctgccaaac ggagacaaag tggatgtaca 3960
   cgacaagcat gcatgggatg gggacaacaa aatgtggaga tgggtgtacg agaagaaaga 4020
   tccctgtgcg ttcgagctgg tatccaggga agtgtgtctt ttctcactga gtaggggtag 4080
   tagactgaga ggagcaaccc ctccccaagg agagctcctc acctgcccgc attcgggaaa 4140
   ggcatttgac ctgaaggggg cccgaaggat tacacccatt tcatgcaaaa tcgacatgga 4200
   atatgacttg ctgtcactac caaccggagt catcctaggc ctccacctat cagaactcgg 4260
   gacctccttt ggcaacctct caatgagtct tgaaatgtat gaacctgcca caactctgac 4320
   ccctgagcaa atcaacttct cgcttaaaga gctgggaagc tggaccgagg ctcaactgaa 4380
   gagcctgtct cactcaatct gcctctccac attctccata tgggaactat cggttgggat 4440
   gatcgatcta aaccctacca gggcagcaag ggccttgctc catgatgata acatactggc 4500
   aacattcgag aacggtcact tttccatcgt cagatgtcgt ccggaaatag ttcaagtccc 4560
   ttcgcatcct cgagcatgtc acatggatct ccgcccttat gacaagcaat cacgggcatc 4620
   aaccctggtg gttccccttg acaacagcac tgccctcctg gtccccgaca acatcgtggt 4680
   tgaaggagta gaggccagtc tatgcaacca ctccgttgcc atcacgctgt cgaagaacag 4740
   aactcactca tacagcctct atccccaggg tcgtcctgtg cttcgacaga aaggtgccgt 4800
   ggagctcccg acgatagggc ccctccagtt acatcctgcc actcgagtgg acctttatac 4860
   actgaaagag ttccaggagg accgaatagc gcgcagtcga gtcacagaca tcaaggctgc 4920
   cgttgacgat ctgcgtgcga agtggcgtaa aggcaaattt gaggcggaca ccacgggagg 4980
   gggactttgg tcggcgattg tgggagtctt cagttctctc ggggggttct tcatgaggcc 5040
   cttgattgct ctcgcggcga tagtgacctc aatcatcatc ctgtatatcc ttctgcgtgt 5100
   actgtgtgct gcctcatgtt cgacacaccg aagagtaagg caggactctt ggtaagcggc 5160
   cgccagatga cgcatgaggg tcagatcaga tttacagcgt aagtgtgata tttaggatta 5220
   taaaggttcc ttcattttaa tttgttacag actgtatgaa aaaaactcat caacagccat 5280
   catggatgtt aacgattttg agttgcatga ggactttgca ttgtctgaag atgactttgt 5340
   cacttcagaa tttctcaatc cggaagacca aatgacatac ctgaatcatg ccgattataa 5400
   tttgaattct cccttaatca gcgatgatat tgatttcctg atcaagaaat ataatcatga 5460
   gcaaattccg aaaatgtggg atgtcaagaa ttgggaggga gtgttagaga tgttgacagc 5520
   ctgtcaagcc agtccaattt tatctagcac tatgcataag tgggtgggaa agtggctcat 5580
   gtctgatgat catgacgcaa gccaaggctt cagttttctt catgaagtgg acaaagaagc 5640
   tgatctgacg tttgaggtgg tggagacatt cattagagga tggggaggtc gagaattgca 5700
   gtacaagagg aaagacacat ttccggactc ctttagagtt gcagcctcat tgtgtcaaaa 5760
   attccttgat ttgcacaaac tcactctgat aatgaattca gtctctgaag tcgaacttac 5820
   caacctagca aagaatttta aaggaaaaaa caggaaagca aaaagcggaa atctgataac 5880
   cagattgagg gttcccagtt taggtcctgc ttttgtgact cagggatggg tgtacatgaa 5940
   gaagttggaa atgattatgg atcggaattt tttgttgatg ttgaaagacg ttatcatcgg 6000
   gaggatgcag acgatcctgt ccatgatctc aagagatgat aatctcttct ccgagtctga 6060
   tatctttact gtattaaaga tataccggat aggggataag atattagaaa ggcaagggac 6120
   aaagggttac gacttgatca aaatgattga gcctatttgt aacttaaaga tgatgaatct 6180
   ggcacgtaaa tatcgtcctc tcatccctac atttcctcat tttgaaaaac atattgctga 6240
   ctctgttaag gaaggatcga aaatagacaa agggattgag tttatatatg atcacattat 6300
   gtcaatccct ggtgtggact tgaccttagt tatttacgga tcatttcggc actggggtca 6360
   tccttttatc aactactatg agggcttaga gaagctacac aagcaggtta caatgcccaa 6420
   gactattgac agagaatatg cagaatgtct tgctagtgat ctggcaagaa tcgttcttca 6480
   gcaacaattc aatgaacata agaaatggtt tgttgatgta gataaagtcc cacaatccca 6540
   tcctttcaaa agccatatga aagagaatac ttggcctact gcagcccaag ttcaggatta 6600
   cggcgatcgc tggcatcagc tcccactcat caaatgcttc gaaatcccag atttgttaga 6660
   tccatcgatc atctactcag acaaaagtca ttccatgaac cggtctgaag tactacgaca 6720
   tgtaagactt acacctcatg tgcccattcc aagcaggaaa gtattgcaga caatgttgga 6780
   gactaaggca acagactgga aagagttttt aaagaaaatt gacgaagagg ggttagagga 6840
   tgatgatctt gtcataggac tcaaagggaa agagagagaa ttaaaaattg cgggaagatt 6900
   cttttctttg atgtcctgga agctcagaga gtattttgtc atcactgagt atttgattaa 6960
   gacgcacttt gtcccgatgt ttaaagggtt gaccatggcg gatgacttga cagcggtgat 7020
   aaagaagatg atggacacat cttcaggaca aggcttagat aattatgaat ccatttgtat 7080
   agccaaccat attgactatg agaagtggaa caatcatcaa agaaaagagt cgaacggccc 7140
   cgtgttcaag gtgatgggtc aattcttggg atatccacgt ctgattgaga gaactcatga 7200
   attttttgag aagagtctga tatattacaa tggacgacca gatctgatgc gggttcgagg 7260
   aaattctcta gtcaacgcct catctttaaa tgtctgctgg gagggtcaag ctgggggatt 7320
   agaaggactg cgacagaagg gatggagtat tctaaatttg cttgtcattc agagagaagc 7380
   aaaaataagg aacaccgccg tgaaagtgct agctcaaggt gacaatcagg tgatatgtac 7440
   tcagtataaa acgaagaaat cccggaatga tattgagctt aaggcagctc taacacagat 7500
   ggtatctaat aatgagatga ttatgtctgc gattaaatca ggcaccgaga aactgggtct 7560
   tttgattaat gatgatgaga caatgcaatc tgctgattac ctcaattacg ggaaggttcc 7620
   cattttcaga ggagtaatca gaggccttga gacaaaaaga tggtcacgcg tgacctgtgt 7680
   gacaaatgat cagattccaa cgtgtgcgaa cattatgagc tctgtgtcaa ctaatgcatt 7740
   aactgtagcc cattttgccg agaatccagt caatgccatc attcagtata actactttgg 7800
   aacatttgca aggctactgc tgatgatgca tgaccccgct ctgaggatct ctctgtatga 7860
   agtccaatca aaaattccag gacttcacag tttgacattt aaatattcta tgttgtatct 7920
   ggatccttcg ataggaggag tctccggaat gtcactctcg agattcctca taagatcatt 7980
   tccagatcca gtgacagaaa gtttggcgtt ctggaaattt atccactctc atgcaagaag 8040
   cgattcatta aaggagatat gtgcagtttt tggaaatcct gaaattgcaa gatttcggct 8100
   aactcatgtc gataaattgg tggaagaccc aacctcattg aacatagcta tgggaatgag 8160
   tcctgctaat ctattaaaga cagaggtaaa aaaatgtcta ctggaatcaa ggcagagcat 8220
   caagaaccag attgtaagag atgctactat ttacctacac catgaggaag acaaacttcg 8280
   tagtttctta tggtccataa caccactgtt ccctcggttc ttgagtgaat tcaaatctgg 8340
   gacattcatc ggagtagcag atggcctgat cagcttattt cagaactcta ggactattcg 8400
   aaattctttt aaaaagcgtt atcacaggga acttgatgat ttaataatca agagcgaagt 8460
   ttcctcactt atgcatttgg gtaagctaca tttgaggcga ggctcagttc gtatgtggac 8520
   ttgctcttct actcaggctg atcttctccg attccggtca tggggaagat ctgttatagg 8580
   aaccacagtc cctcatccct tagagatgtt aggacaacat tttaaaaagg agactccttg 8640
   cagtgcttgc aacatatccg gattagacta tgtatctgtc cactgtccga atgggattca 8700
   tgacgttttt gaatcacgtg gtccactccc tgcatatttg ggttctaaaa catccgaatc 8760
   aacttcgatc ttgcagccgt gggagagaga gagtaaagta ccgttgatta agcgtgccac 8820
   aaggcttcgt gatgcaattt catggtttgt gtctcccgac tctaacttgg cctcaactat 8880
   ccttaagaac ataaatgcat taacaggaga agaatggtca aagaagcagc atggatttaa 8940
   aaggacggga tcggcgttac acaggttctc cacatccagg atgagtcatg gtggttttgc 9000
   ttctcagagt acggctgcct tgactagatt gatggcaact actgacacta tgagagatct 9060
   gggagaacag aactatgatt tcctgtttca ggcgacatta ttgtatgctc aaataaccac 9120
   aactgtagtc aggaatggat catttcatag ctgcacggac cattaccata taacctgcaa 9180
   atcttgtctg agggccattg atgagattac cttggattca gcgatggaat atagccctcc 9240
   agatgtatca tcagttttac aatcttggag gaatggagaa ggctcttggg gacatgaagt 9300
   gaaacaaata tacccagttg aaggtgactg gaggggacta tctcctgttg aacaatctta 9360
   tcaagtcgga cgctgtatcg ggtttctgtt cggtgatctg gcgtatagaa aatcatccca 9420
   tgcagatgat agctccatgt ttccgttatc tatacaaaac aaagtcagag gaagaggctt 9480
   tttaaaaggg cttatggatg ggttaatgag agccagttgt tgccaggtga tccatcgtcg 9540
   aagcttagcc catctgaaga gaccggctaa tgcagtctat ggagggctga tttatttgat 9600
   agacaaattg agtgcatctg ccccttttct ttcactgacg agacatggac ctttaaggga 9660
   agaattagaa actgttccac ataagatacc gacttcttat cctacgagca accgagatat 9720
   gggggtgata gttcgtaatt attttaaata tcagtgcaga ctggtagaaa aaggtcggta 9780
   caagacacat tatcctcaat tgtggctttt ctcagatgtg ctgtccattg atttcttagg 9840
   acccctgtct atatcttcaa ctctattggg tattctgtat aaacagacgt tatcttctcg 9900
   agacaaaaat gagttgagag aactcgctaa cttgtcttca ttgttgagat caggagaagg 9960
   atgggaagat atccatgtca aattcttctc taaggacact ttactctgcc ctgaagagat 10020
   ccgacatgcg tgcaaatttg ggattgctaa ggaatccgct gttttaagct attatcctcc 10080
   ttggtctcaa gagtcttatg gaggcatcac ctcgatcccc gtatattttt cgaccaggaa 10140
   gtatcccaaa attttagatg tccctcctcg ggttcaaaac ccattggtct cgggtctacg 10200
   attggggcaa ctccctactg gagcacatta taagattagg agcattgtaa agaacaagaa 10260
   ccttcgttat agagatttcc ttagttgtgg ggatggatct ggggggatga ccgcggcact 10320
   attgagagaa aacagacaaa gtaggggaat cttcaacagc ctgttagagt tagccggatc 10380
   tcttatgaga ggagcatctc cagagcctcc aagtgcactg gagacgctcg ggcaagaacg 10440
   atctaggtgt gtgaatggaa gcacatgttg ggagtactca tctgacctaa gccaaaaaga 10500
   gacatgggat tacttcttaa gattgaagag aggcctgggt ttgaccgtgg acttaatcac 10560
   catggacatg gaggtcagag accctaatac aagtttgatg atagaaaaga acctcaaagt 10620
   ttatctgcat cagatattag aaccaactgg tgtcttaata tataaaacat acgggaccca 10680
   tattgcgaca caaacagata atatcctgac gataatcggt cctttctttg agacggttga 10740
   cctagtccag tccgaataca gcagctcaca aacgtccgag gtctattttg taggacgagg 10800
   cttgcgctct catgttgacg aaccctgggt ggactggcca tccttaatgg acaattggag 10860
   atccatttat gcttttcatg atcctactac agaatttatc agagcaaaaa aagtctgtga 10920
   aattgacagt cttataggca ttccggctca attcattcca gacccatttg taaatctcga 10980
   gaccatgcta cagatagttg gtgttccaac aggagtttcg catgccgcag ctctattatc 11040
   atcacaatat ccaaatcaat tggtcacaac gtcaatattt tatatgacac tcgtgtctta 11100
   ttataatgta aaccatattc gaagaagccc caagcctttc tctcctccgt ctgatggagt 11160
   ctcacagaac attggttcag ccatagtcgg actaagtttt tgggtgagtt tgatggagaa    11220
   tgatctcgga ttatacaaac aggctctagg tgcaataaag acgtcattcc ctattagatg    11280
   gtcctctgtc cagaccaagg atgggtttac acaagaatgg agaactaaag gaaacggaat    11340
   tcctaaagat tgtcgtctct cagactcttt ggctcagata ggaaactgga tcagagcgat    11400
   ggaattggtt aggaacaaaa cgaggcaatc aggattttct gaaaccctat ttgatcaatt    11460
   ctgcggactt gcagaccatc acctcaaatg gcggaagttg ggaaacagaa caggaattat    11520
   tgattggcta aataatagaa tttcatccat tgacaaatcc atcttggtga ccaaaagtga    11580
   tctgcatgac gagaactcat ggagggagtg aagatgtatt cttccacctc tcattgggtg    11640
   atacccatat atgaaaaaaa ctataagtac tttaaactct ctttgttttt taatgtatat    11700
   ctggttttgt tgtttccgt                                                 11719
<210> 16
   <211> 11719
   <212> RNA
   <213> Artificial
<220>
   <223> Chimeric virus containing RNA from Maraba and Farmington viruses
<400> 16
   acgaagacaa acaaaccauu gauagaauua agaggcucau gaaaauccuu aacagcguuc      60
   aaaaugucug uuacagucaa gagagucauu gaugauucac ucaucacccc caaauugccu     120
   gcgaaugagg acccugugga guacccugcu gauuauuuca aaaagucccg ugauauuccg     180
   guguacauaa acacgaccaa aaguuugucu gauuugcggg gcuauguuua ucaaggccua     240
   aagucaggca acaucucuau aauucauguc aacaguuauc uguaugcagc auuaaaagag     300
   aucagaggaa aauuggacag agauuggauc accuuuggua uccaaaucgg aaaaacagga     360
   gauagcgugg ggauauucga uuuacugacc cuaaaaccuc uagauggugu uuuaccagau     420
   ggggugucug augcuacucg aacuagcuca gacgaugcau ggcuuccacu guaucuauug     480
   ggguuauaca gaguuggucg aacacagaug ccagaauaca ggaagaagcu gauggauggu     540
   cugauuaauc aauguaagau gaucaaugag caguuugaac cacuguugcc agaaggaaga     600
   gaugucuuug augucugggg aaaugacagc aauuacacaa agauuguggc cgcuguagau     660
   auguucuucc auauguucaa aaagcaugag aaggccucuu ucagguaugg cacaauagug     720
   ucaagauuua aggauugugc agcauuggcu acauuugguc aucuguguaa gaucacuggu     780
   auguccacug aagaugugac aacuuggauu cuaaacaggg agguggcuga ugagaugguu     840
   caaaugaugu acccaggaca ggagauagau aaggcugauu cuuacaugcc uuaucuaauc     900
   gacuuagguc uguccucaaa aucuccauau ucaucaguua aaaauccagc uuuccauuuu     960
   uggggucaau ugaccgcauu guuacugaga ucaaccagag ccagaaaugc acgucagccg    1020
   gaugacaucg aguauacauc ccugaccacu gcugggcugu uguaugcaua ugccguuggu 1080
   ucgucugcag accuggcuca acaauucuac guuggggaca acaaguaugu gccagaaacu 1140
   ggagauggag gauuaaccac caaugcaccg ccacaagggc gagauguggu cgaguggcuu 1200
   aguugguuug aagaucaaaa cagaaaaccu accccagaca ugcucaugua ugcuaagaga 1260
   gcugucagug cuuuacaagg auugagggag aagacgauug gcaaguacgc caagucagag 1320
   uuugacaaau gacaacucac ucaccauaug uauuacuacc uuugcuucau augaaaaaaa 1380
   cuaacagcga ucauggauca gcuaucaaag gucaaggaau uccuuaagac uuacgcgcag 1440
   uuggaucaag caguacaaga gauggaugac auugagucuc agagagagga aaagacuaau 1500
   uuugauuugu uucaggaaga aggauuggag auuaaggaga agccuuccua uuaucgggca 1560
   gaugaagaag agauugauuc agaugaagac agcguggaug augcacaaga cuuagggaua 1620
   cguacaucaa caagucccau cgagggguau guggaugagg agcaggauga uuaugaggau 1680
   gaggaaguga acgugguguu uacaucggac uggaaacagc cugagcugga auccgacggg 1740
   gaugggaaaa cucuccgauu gacgauacca gauggauuga cuggggagca gaagucgcaa 1800
   uggcuugcca cgauuaaggc aguuguucag agugcuaaau auuggaacau cucagaaugu 1860
   ucauuugaga guuaugagca agggguuuug auuagagaga gacaaaugac uccugauguc 1920
   uacaaaguca cuccuguuuu aaaugcucca ccgguucaaa ugacagcuaa ucaagauguu 1980
   uggucucuca gcagcacucc auuuacauuu uugcccaaga aacaaggugu gacuccauug 2040
   accauguccu uagaagaacu cuucaacacc cgaggugaau ucauaucucu gggaggaaac 2100
   gggaaaauga gucaccggga ggccaucauu cuaggguuga gacacaagaa gcucuauaau 2160
   caagccagac uaaaguauaa cuuagcuuga auaugaaaaa aacuaacaga uaucaaaaga 2220
   uaucucuaac ucaguccauu guguucaguu caaucaugag cucucucaag aaaauuuugg 2280
   guauuaaagg gaaagggaag aaaucuaaga aauuagguau ggcuccccca cccuaugaag 2340
   aagagacucc aauggaauau ucuccaagug caccuuauga uaagucauug uuuggagucg 2400
   aagauaugga uuuccaugau caacgucaac uccgauauga gaaauuucac uucucauuga 2460
   agaugacugu gagaucaaac aaaccauuuc gaaauuauga ugacguugca gcagcggugu 2520
   ccaauuggga ucauauguac aucggcaugg caggaaaacg uccuuuuuau aagauauuag 2580
   cauucauggg uucuacucua uugaaggcua caccagcugu cuuggcugac caaggacagc 2640
   cagaauauca ugcucacugu gagggacgag cuuacuugcc gcaucgguua gggccgaccc 2700
   cuccgauguu gaaugucccu gaacauuuuc gccguccauu uaacaucgga uuauucagag 2760
   ggacaaucga cauaacccug guacuuuucg augaugaauc uguagauucu gccccgguca 2820
   uaugggauca uuuuaaugca uccagauuga gcagcuucag agaaaaggcu uuguuguuug 2880
   guuugauucu agaaaagaaa gccacuggga auuggguauu ggacucuauu agucauuuca 2940
   aguaauuauc acaaguguug aggugauggg cagacuauga aaaaaacuaa caggguucaa 3000
   acacucuuga ucgagguacc caguuauauu uguuacaaca augcucagga uccagauccc 3060
   uccgauugcu aucauucugg uaagucuccu cacacucgac cuguccggug caaggaggac 3120
   aaccacacaa agaaucccuc uccuuaauga uucgugggau uuguucucga gcuauggcga 3180
   cauucccgaa gaacuugucg uauaccagaa cuacagccac aauuccuccg aguuaccccc 3240
   uccuggcuuc gagagauggu acauaaaccg aagaguggca gacacuucca uaccgugcag 3300
   gggccccugu cuagugcccu acauccuuca uggccucaau gacacaacug ucucucgacg 3360
   gggaggagga uggcgaaggu ccggaaugaa guacccaacc cacgcuguca ggcuaggccc 3420
   uucaacagac gacgagagag uugaggaaga caucggcuac gucaaugucu ccgcacuauc 3480
   cugcacaggg ucgcccguug agauggcgau accaacaauc cccgacugca ccagugcuau 3540
   ccauccacga uccgagguua cugugcccgu caagcucgau gucaugagac gaaaucccaa 3600
   cuacccuccc auuagagcgu ggucgugcau cggacagaaa aucaccaacc gaugugauug 3660
   ggcacucuuc ggcgagaacc ucauauauac ucaaguugaa gcuagcucuc uagcauucaa 3720
   gcacacaaga gccucucuuu ugaacgaauc caacgggaua gacgcugaag gacgugcagu 3780
   ucccuauauc cucggggaua ucgaacccgg guacugccga acccuauuca acacaugggu 3840
   cucuagugag aucgugucau gcacgcccau cgaacuuguc cuaguugacc ugaacccuuu 3900
   guccccggga cauggcggau augcuguauu gcugccaaac ggagacaaag uggauguaca 3960
   cgacaagcau gcaugggaug gggacaacaa aauguggaga uggguguacg agaagaaaga 4020
   ucccugugcg uucgagcugg uauccaggga agugugucuu uucucacuga guagggguag 4080
   uagacugaga ggagcaaccc cuccccaagg agagcuccuc accugcccgc auucgggaaa 4140
   ggcauuugac cugaaggggg cccgaaggau uacacccauu ucaugcaaaa ucgacaugga 4200
   auaugacuug cugucacuac caaccggagu cauccuaggc cuccaccuau cagaacucgg 4260
   gaccuccuuu ggcaaccucu caaugagucu ugaaauguau gaaccugcca caacucugac 4320
   cccugagcaa aucaacuucu cgcuuaaaga gcugggaagc uggaccgagg cucaacugaa 4380
   gagccugucu cacucaaucu gccucuccac auucuccaua ugggaacuau cgguugggau 4440
   gaucgaucua aacccuacca gggcagcaag ggccuugcuc caugaugaua acauacuggc 4500
   aacauucgag aacggucacu uuuccaucgu cagaugucgu ccggaaauag uucaaguccc 4560
   uucgcauccu cgagcauguc acauggaucu ccgcccuuau gacaagcaau cacgggcauc 4620
   aacccuggug guuccccuug acaacagcac ugcccuccug guccccgaca acaucguggu 4680
   ugaaggagua gaggccaguc uaugcaacca cuccguugcc aucacgcugu cgaagaacag 4740
   aacucacuca uacagccucu auccccaggg ucguccugug cuucgacaga aaggugccgu 4800
   ggagcucccg acgauagggc cccuccaguu acauccugcc acucgagugg accuuuauac      4860
   acugaaagag uuccaggagg accgaauagc gcgcagucga gucacagaca ucaaggcugc      4920
   cguugacgau cugcgugcga aguggcguaa aggcaaauuu gaggcggaca ccacgggagg      4980
   gggacuuugg ucggcgauug ugggagucuu caguucucuc gggggguucu ucaugaggcc      5040
   cuugauugcu cucgcggcga uagugaccuc aaucaucauc cuguauaucc uucugcgugu      5100
   acugugugcu gccucauguu cgacacaccg aagaguaagg caggacucuu gguaagcggc      5160
   cgccagauga cgcaugaggg ucagaucaga uuuacagcgu aagugugaua uuuaggauua      5220
   uaaagguucc uucauuuuaa uuuguuacag acuguaugaa aaaaacucau caacagccau      5280
   cauggauguu aacgauuuug aguugcauga ggacuuugca uugucugaag augacuuugu      5340
   cacuucagaa uuucucaauc cggaagacca aaugacauac cugaaucaug ccgauuauaa      5400
   uuugaauucu cccuuaauca gcgaugauau ugauuuccug aucaagaaau auaaucauga      5460
   gcaaauuccg aaaauguggg augucaagaa uugggaggga guguuagaga uguugacagc      5520
   cugucaagcc aguccaauuu uaucuagcac uaugcauaag ugggugggaa aguggcucau      5580
   gucugaugau caugacgcaa gccaaggcuu caguuuucuu caugaagugg acaaagaagc      5640
   ugaucugacg uuugaggugg uggagacauu cauuagagga uggggagguc gagaauugca      5700
   guacaagagg aaagacacau uuccggacuc cuuuagaguu gcagccucau ugugucaaaa      5760
   auuccuugau uugcacaaac ucacucugau aaugaauuca gucucugaag ucgaacuuac      5820
   caaccuagca aagaauuuua aaggaaaaaa caggaaagca aaaagcggaa aucugauaac      5880
   cagauugagg guucccaguu uagguccugc uuuugugacu cagggauggg uguacaugaa      5940
   gaaguuggaa augauuaugg aucggaauuu uuuguugaug uugaaagacg uuaucaucgg      6000
   gaggaugcag acgauccugu ccaugaucuc aagagaugau aaucucuucu ccgagucuga      6060
   uaucuuuacu guauuaaaga uauaccggau aggggauaag auauuagaaa ggcaagggac      6120
   aaaggguuac gacuugauca aaaugauuga gccuauuugu aacuuaaaga ugaugaaucu      6180
   ggcacguaaa uaucguccuc ucaucccuac auuuccucau uuugaaaaac auauugcuga      6240
   cucuguuaag gaaggaucga aaauagacaa agggauugag uuuauauaug aucacauuau      6300
   gucaaucccu gguguggacu ugaccuuagu uauuuacgga ucauuucggc acugggguca      6360
   uccuuuuauc aacuacuaug agggcuuaga gaagcuacac aagcagguua caaugcccaa      6420
   gacuauugac agagaauaug cagaaugucu ugcuagugau cuggcaagaa ucguucuuca      6480
   gcaacaauuc aaugaacaua agaaaugguu uguugaugua gauaaagucc cacaauccca      6540
   uccuuucaaa agccauauga aagagaauac uuggccuacu gcagcccaag uucaggauua      6600
   cggcgaucgc uggcaucagc ucccacucau caaaugcuuc gaaaucccag auuuguuaga      6660
   uccaucgauc aucuacucag acaaaaguca uuccaugaac cggucugaag uacuacgaca 6720
   uguaagacuu acaccucaug ugcccauucc aagcaggaaa guauugcaga caauguugga 6780
   gacuaaggca acagacugga aagaguuuuu aaagaaaauu gacgaagagg gguuagagga 6840
   ugaugaucuu gucauaggac ucaaagggaa agagagagaa uuaaaaauug cgggaagauu 6900
   cuuuucuuug auguccugga agcucagaga guauuuuguc aucacugagu auuugauuaa 6960
   gacgcacuuu gucccgaugu uuaaaggguu gaccauggcg gaugacuuga cagcggugau 7020
   aaagaagaug auggacacau cuucaggaca aggcuuagau aauuaugaau ccauuuguau 7080
   agccaaccau auugacuaug agaaguggaa caaucaucaa agaaaagagu cgaacgggcc 7140
   cguguucaag gugaugggug aauucuuggg auauccacgu cugauugaga gaacucauga 7200
   auuuuuugag aagagucuga uauauuacaa uggacgacca gaucugaugc ggguucgagg 7260
   aaauucucua gucaacgccu caucuuuaaa ugucugcugg gagggucaag cugggggauu 7320
   agaaggacug cgacagaagg gauggaguau ucuaaauuug cuugucauuc agagagaagc 7380
   aaaaauaagg aacaccgccg ugaaagugcu agcucaaggu gacaaucagg ugauauguac 7440
   ucaguauaaa acgaagaaau cccggaauga uauugagcuu aaggcagcuc uaacacagau 7500
   gguaucuaau aaugagauga uuaugucugc gauuaaauca ggcaccgaga aacugggucu 7560
   uuugauuaau gaugaugaga caaugcaauc ugcugauuac cucaauuacg ggaagguucc 7620
   cauuuucaga ggaguaauca gaggccuuga gacaaaaaga uggucacgcg ugaccugugu 7680
   gacaaaugau cagauuccaa cgugugcgaa cauuaugagc ucugugucaa cuaaugcauu 7740
   aacuguagcc cauuuugccg agaauccagu caaugccauc auucaguaua acuacuuugg 7800
   aacauuugca aggcuacugc ugaugaugca ugaccccgcu cugaggaucu cucuguauga 7860
   aguccaauca aaaauuccag gacuucacag uuugacauuu aaauauucua uguuguaucu 7920
   ggauccuucg auaggaggag ucuccggaau gucacucucg agauuccuca uaagaucauu 7980
   uccagaucca gugacagaaa guuuggcguu cuggaaauuu auccacucuc augcaagaag 8040
   cgauucauua aaggagauau gugcaguuuu uggaaauccu gaaauugcaa gauuucggcu 8100
   aacucauguc gauaaauugg uggaagaccc aaccucauug aacauagcua ugggaaugag 8160
   uccugcuaau cuauuaaaga cagagguaaa aaaaugucua cuggaaucaa ggcagagcau 8220
   caagaaccag auuguaagag augcuacuau uuaccuacac caugaggaag acaaacuucg 8280
   uaguuucuua ugguccauaa caccacuguu cccucgguuc uugagugaau ucaaaucugg 8340
   gacauucauc ggaguagcag auggccugau cagcuuauuu cagaacucua ggacuauucg 8400
   aaauucuuuu aaaaagcguu aucacaggga acuugaugau uuaauaauca agagcgaagu 8460
   uuccucacuu augcauuugg guaagcuaca uuugaggcga ggcucaguuc guauguggac 8520
   uugcucuucu acucaggcug aucuucuccg auuccgguca uggggaagau cuguuauagg 8580
   aaccacaguc ccucaucccu uagagauguu aggacaacau uuuaaaaagg agacuccuug 8640
   cagugcuugc aacauauccg gauuagacua uguaucuguc cacuguccga augggauuca 8700
   ugacguuuuu gaaucacgug guccacuccc ugcauauuug gguucuaaaa cauccgaauc 8760
   aacuucgauc uugcagccgu gggagagaga gaguaaagua ccguugauua agcgugccac 8820
   aaggcuucgu gaugcaauuu caugguuugu gucucccgac ucuaacuugg ccucaacuau 8880
   ccuuaagaac auaaaugcau uaacaggaga agaaugguca aagaagcagc auggauuuaa 8940
   aaggacggga ucggcguuac acagguucuc cacauccagg augagucaug gugguuuugc 9000
   uucucagagu acggcugccu ugacuagauu gauggcaacu acugacacua ugagagaucu 9060
   gggagaacag aacuaugauu uccuguuuca ggcgacauua uuguaugcuc aaauaaccac 9120
   aacuguaguc aggaauggau cauuucauag cugcacggac cauuaccaua uaaccugcaa 9180
   aucuugucug agggccauug augagauuac cuuggauuca gcgauggaau auagcccucc 9240
   agauguauca ucaguuuuac aaucuuggag gaauggagaa ggcucuuggg gacaugaagu 9300
   gaaacaaaua uacccaguug aaggugacug gaggggacua ucuccuguug aacaaucuua 9360
   ucaagucgga cgcuguaucg gguuucuguu cggugaucug gcguauagaa aaucauccca 9420
   ugcagaugau agcuccaugu uuccguuauc uauacaaaac aaagucagag gaagaggcuu 9480
   uuuaaaaggg cuuauggaug gguuaaugag agccaguugu ugccagguga uccaucgucg 9540
   aagcuuagcc caucugaaga gaccggcuaa ugcagucuau ggagggcuga uuuauuugau 9600
   agacaaauug agugcaucug ccccuuuucu uucacugacg agacauggac cuuuaaggga 9660
   agaauuagaa acuguuccac auaagauacc gacuucuuau ccuacgagca accgagauau 9720
   gggggugaua guucguaauu auuuuaaaua ucagugcaga cugguagaaa aaggucggua 9780
   caagacacau uauccucaau uguggcuuuu cucagaugug cuguccauug auuucuuagg 9840
   accccugucu auaucuucaa cucuauuggg uauucuguau aaacagacgu uaucuucucg 9900
   agacaaaaau gaguugagag aacucgcuaa cuugucuuca uuguugagau caggagaagg 9960
   augggaagau auccauguca aauucuucuc uaaggacacu uuacucugcc cugaagagau 10020
   ccgacaugcg ugcaaauuug ggauugcuaa ggaauccgcu guuuuaagcu auuauccucc 10080
   uuggucucaa gagucuuaug gaggcaucac cucgaucccc guauauuuuu cgaccaggaa 10140
   guaucccaaa auuuuagaug ucccuccucg gguucaaaac ccauuggucu cgggucuacg 10200
   auuggggcaa cucccuacug gagcacauua uaagauuagg agcauuguaa agaacaagaa 10260
   ccuucguuau agagauuucc uuaguugugg ggauggaucu ggggggauga ccgcggcacu 10320
   auugagagaa aacagacaaa guaggggaau cuucaacagc cuguuagagu uagccggauc 10380
   ucuuaugaga ggagcaucuc cagagccucc aagugcacug gagacgcucg ggcaagaacg 10440
   aucuaggugu gugaauggaa gcacauguug ggaguacuca ucugaccuaa gccaaaaaga 10500
   gacaugggau uacuucuuaa gauugaagag aggccugggu uugaccgugg acuuaaucac 10560
   cauggacaug gaggucagag acccuaauac aaguuugaug auagaaaaga accucaaagu 10620
   uuaucugcau cagauauuag aaccaacugg ugucuuaaua uauaaaacau acgggaccca 10680
   uauugcgaca caaacagaua auauccugac gauaaucggu ccuuucuuug agacgguuga 10740
   ccuaguccag uccgaauaca gcagcucaca aacguccgag gucuauuuug uaggacgagg 10800
   cuugcgcucu cauguugacg aacccugggu ggacuggcca uccuuaaugg acaauuggag 10860
   auccauuuau gcuuuucaug auccuacuac agaauuuauc agagcaaaaa aagucuguga 10920
   aauugacagu cuuauaggca uuccggcuca auucauucca gacccauuug uaaaucucga 10980
   gaccaugcua cagauaguug guguuccaac aggaguuucg caugccgcag cucuauuauc 11040
   aucacaauau ccaaaucaau uggucacaac gucaauauuu uauaugacac ucgugucuua 11100
   uuauaaugua aaccauauuc gaagaagccc caagccuuuc ucuccuccgu cugauggagu 11160
   cucacagaac auugguucag ccauagucgg acuaaguuuu ugggugaguu ugauggagaa 11220
   ugaucucgga uuauacaaac aggcucuagg ugcaauaaag acgucauucc cuauuagaug 11280
   guccucuguc cagaccaagg auggguuuac acaagaaugg agaacuaaag gaaacggaau 11340
   uccuaaagau ugucgucucu cagacucuuu ggcucagaua ggaaacugga ucagagcgau 11400
   ggaauugguu aggaacaaaa cgaggcaauc aggauuuucu gaaacccuau uugaucaauu 11460
   cugcggacuu gcagaccauc accucaaaug gcggaaguug ggaaacagaa caggaauuau 11520
   ugauuggcua aauaauagaa uuucauccau ugacaaaucc aucuugguga ccaaaaguga 11580
   ucugcaugac gagaacucau ggagggagug aagauguauu cuuccaccuc ucauugggug 11640
   auacccauau augaaaaaaa cuauaaguac uuuaaacucu cuuuguuuuu uaauguauau 11700
   cugguuuugu uguuuccgu 11719
<210> 17
   <211> 704
   <212> PRT
   <213> Farmington virus
<400> 17
   Met Leu Arg Ile Gln Ile Pro Pro Ile Ala Ile Ile Leu Val Ser Leu
   1               5                   10                  15
   Leu Thr Leu Asp Leu Ser Gly Ala Arg Arg Thr Thr Thr Gln Arg Ile
               20                  25                  30
   Pro Leu Leu Asn Asp Ser Trp Asp Leu Phe Ser Ser Tyr Gly Asp Ile
           35                  40                  45
   Pro Glu Glu Leu Val Val Tyr Gln Asn Tyr Ser His Asn Ser Ser Glu
       50                  55                  60
   Leu Pro Pro Pro Gly Phe Glu Arg Trp Tyr Ile Asn Arg Arg Val Ala
   65                  70                  75                  80
   Asp Thr Ser Ile Pro Cys Arg Gly Pro Cys Leu Val Pro Tyr Ile Leu
                   85                  90                  95
   His Gly Leu Asn Asp Thr Thr Val Ser Arg Arg Gly Gly Gly Trp Arg
               100                 105                 110
   Arg Ser Gly Met Lys Tyr Pro Thr His Ala Val Arg Leu Gly Pro Ser
           115                 120                 125
   Thr Asp Asp Glu Arg Val Glu Glu Asp Ile Gly Tyr Val Asn Val Ser
       130                 135                 140
   Ala Leu Ser Cys Thr Gly Ser Pro Val Glu Met Ala Ile Pro Thr Ile
   145                 150                 155                 160
   Pro Asp Cys Thr Ser Ala Ile His Pro Arg Ser Glu Val Thr Val Pro
                   165                 170                 175
   Val Lys Leu Asp Val Met Arg Arg Asn Pro Asn Tyr Pro Pro Ile Arg
               180                 185                 190
   Ala Trp Ser Cys Ile Gly Gln Lys Ile Thr Asn Arg Cys Asp Trp Ala
           195                 200                 205
   Leu Phe Gly Glu Asn Leu Ile Tyr Thr Gln Val Glu Ala Ser Ser Leu
       210                 215                 220
   Ala Phe Lys His Thr Arg Ala Ser Leu Leu Asn Glu Ser Asn Gly Ile
   225                 230                 235                 240
   Asp Ala Glu Gly Arg Ala Val Pro Tyr Ile Leu Gly Asp Ile Glu Pro
                   245                 250                 255
   Gly Tyr Cys Arg Thr Leu Phe Asn Thr Trp Val Ser Ser Glu Ile Val
               260                 265                 270
   Ser Cys Thr Pro Ile Glu Leu Val Leu Val Asp Leu Asn Pro Leu Ser
           275                 280                 285
   Pro Gly His Gly Gly Tyr Ala Val Leu Leu Pro Asn Gly Asp Lys Val
       290                 295                 300
   Asp Val His Asp Lys His Ala Trp Asp Gly Asp Asn Lys Met Trp Arg
   305 310 315 320
   Trp Val Tyr Glu Lys Lys Asp Pro Cys Ala Phe Glu Leu Val Ser Arg
   325 330 335
   Glu Val Cys Leu Phe Ser Leu Ser Arg Gly Ser Arg Leu Arg Gly Ala
   340 345 350
   Thr Pro Pro Gln Gly Glu Leu Leu Thr Cys Pro His Ser Gly Lys Ala
   355 360 365
   Phe Asp Leu Lys Gly Ala Arg Arg Ile Thr Pro Ile Ser Cys Lys Ile
   370 375 380
   Asp Met Glu Tyr Asp Leu Leu Ser Leu Pro Thr Gly Val Ile Leu Gly
   385 390 395 400
   Leu His Leu Ser Glu Leu Gly Thr Ser Phe Gly Asn Leu Ser Met Ser
   405 410 415
   Leu Glu Met Tyr Glu Pro Ala Thr Thr Leu Thr Pro Glu Gln Ile Asn
   420 425 430
   Phe Ser Leu Lys Glu Leu Gly Ser Trp Thr Glu Ala Gln Leu Lys Ser
   435 440 445
   Leu Ser His Ser Ile Cys Leu Ser Thr Phe Ser Ile Trp Glu Leu Ser
   450 455 460
   Val Gly Met Ile Asp Leu Asn Pro Thr Arg Ala Ala Arg Ala Leu Leu
   465 470 475 480
   His Asp Asp Asn Ile Leu Ala Thr Phe Glu Asn Gly His Phe Ser Ile
   485 490 495
   Val Arg Cys Arg Pro Glu Ile Val Gln Val Pro Ser His Pro Arg Ala
   500 505 510
   Cys His Met Asp Leu Arg Pro Tyr Asp Lys Gln Ser Arg Ala Ser Thr
   515 520 525
   Leu Val Val Pro Leu Asp Asn Ser Thr Ala Leu Leu Val Pro Asp Asn
   530 535 540
   Ile Val Val Glu Gly Val Glu Ala Ser Leu Cys Asn His Ser Val Ala
   545                 550                 555                 560
   Ile Thr Leu Ser Lys Asn Arg Thr His Ser Tyr Ser Leu Tyr Pro Gln
                   565                 570                 575
   Gly Arg Pro Val Leu Arg Gln Lys Gly Ala Val Glu Leu Pro Thr Ile
               580                 585                 590
   Gly Pro Leu Gln Leu His Pro Ala Thr Arg Val Asp Leu Tyr Thr Leu
           595                 600                 605
   Lys Glu Phe Gln Glu Asp Arg Ile Ala Arg Ser Arg Val Thr Asp Ile
       610                 615                 620
   Lys Ala Ala Val Asp Asp Leu Arg Ala Lys Trp Arg Lys Gly Lys Phe
   625                 630                 635                 640
   Glu Ala Asp Thr Thr Gly Gly Gly Leu Trp Ser Ala Ile Val Gly Val
                   645                 650                 655
   Phe Ser Ser Leu Gly Gly Phe Phe Met Arg Pro Leu Ile Ala Leu Ala
               660                 665                 670
   Ala Ile Val Thr Ser Ile Ile Ile Leu Tyr Ile Leu Leu Arg Val Leu
           675                 680                 685
   Cys Ala Ala Ser Cys Ser Thr His Arg Arg Val Arg Gln Asp Ser Trp
       690                 695                 700
<210> 18
   <211> 12695
   <212> DNA
   <213> Artificial
<220>
   <223> DNA encoded by chimeric virus that contains RNA from Maraba and Farmington viruses
<400> 18
   ttacgacgca taagctgaga aacataagag actatgttca tagtcaccct gtattcatta 60
   ttgactttta tgacctatta ttcgtgaggt catatgtgag gtaatgtcat ctgcttatgc 120
   gtttgcttat aagataaaac gatagaccct tcacgggtaa atccttctcc ttgcagttct 180
   cgccaagtac ctccaaagtc agaacgatgg ctcgtccgct agctgctgcg caacatctca 240
   taaccgagcg tcattccctt caggcgactc tgtcgcgggc gtccaagacc agagccgagg 300
   aattcgtcaa agatttctac cttcaagagc agtattctgt cccgaccatc ccgacggacg 360
   acattgccca gtctgggccc atgctgcttc aggccatcct gagcgaggaa tacacaaagg 420
   ccactgacat agcccaatcc atcctctgga acactcccac acccaacggg ctcctcagag 480
   agcatctaga tgccgatggg ggaggctcat tcacagcgct gcccgcgtct gcaatcagac 540
   ccagcgacga ggcgaatgca tgggccgctc gcatctccga ctcagggttg gggcctgtct 600
   tctatgcagc cctcgctgct tacatcatcg gctggtcagg aagaggagag actagccgcg 660
   tgcagcagaa cataggtcag aaatggctga tgaacctgaa cgcaatcttc ggcaccacga 720
   tcacccatcc aacaaccgtg cgtctgccaa tcaacgtcgt caacaacagc ctcgcagtga 780
   ggaacggact tgctgccaca ctctggctat actaccgttc atcacctcag agtcaggacg 840
   cgttcttcta tgggctcatc cgtccctgtt gcagtggata tctcggcctg ctacatcggg 900
   tgcaggagat tgatgagatg gagccggact tcctcagtga cccccggatc atccaggtga 960
   atgaggtcta cagtgcactc agagccctgg ttcaactggg aaacgacttc aagaccgccg 1020
   atgatgagcc catgcaggtc tgggcgtgca ggggaatcaa caacggatat ctgacatatc 1080
   tctcagaaac tcctgcgaag aaaggagctg ttgtgcttat gtttgcccaa tgcatgctga 1140
   agggcgactc tgaggcctgg aacagctacc gcactgcaac ctgggtgatg ccctattgcg 1200
   acaatgtggc cctaggagcg atggcaggct acatccaagc ccgccagaac accagggcat 1260
   atgaggtctc agcccagaca ggtctcgacg tcaacatggc cgcggtcaag gactttgagg 1320
   ccagttcaaa acccaaggct gctccaatct cgctgatccc acgccccgct gatgtcgcat 1380
   cccgcacctc tgagcgccca tctattcctg aggttgacag cgacgaagag ctcggaggaa 1440
   tgtaaaccaa taagcttcac tgccggtagt ttaggcatac acacgcagtt ccgttatcca 1500
   tcacacccgt cccttctttt atgctgctat tatttcagtt gctaagcttc ctgatttgat 1560
   taacaaaaaa ccgtagacct cctacgtgag gtatagctag aaattggttc tatcggttga 1620
   gagtctttgt actattagcc atggaggact atttgtctag cttagaggcc gcgagagagc 1680
   tcgtccggac ggagctggag cccaagcgta acctcatagc cagcttagag tccgacgatc 1740
   ccgatccggt aatagcgcca gcggtaaaac caaaacatcc caagccatgc ctgagcacta 1800
   aagaagagga tcatctcccc tctcttcgcc tactattcgg cgcaaaacga gacacctcgg 1860
   tgggcgtaga gcagactctc cacaagcgtc tctgcgcttg tctcgacggt tacctgacca 1920
   tgacgaagaa agaggccaat gcctttaagg ccgcggctga agcagcagca ttagcagtca 1980
   tggacattaa gatggagcat cagcgccagg atctagagga tctgaccgct gctatcccta 2040
   ggatagaatt caaactcaat gccatcctgg aaaacaacaa ggagatagcc aaggctgtaa 2100
   ctgctgctaa ggagatggag cgggagatgt cgtgggggga aagcgccgcc agctcgctca 2160
   agtctgtcac cctagatgag tcgtttaggg gccctgaaga gctttcagag tcatttggca 2220
   tccgatataa ggtcagaacc tggaatgagt tcaagaaggc gctggaaacc agcattgtgg 2280
   acctgaggcc tagccctgtt tcatttaggg aattacggac tatgtggctg tctcttgaca 2340
   cctcctttag gctcattggg tttgccttca ttcccacatg cgagcgcctg gagaccaaag 2400
   ccaaatgcaa ggagacaagg actctactcc cccttgcaga gtcgatcatg cgaagatggg 2460
   acctgcggga tccaaccatc ttggagaaag cctgcgtagt aatgatgatc cgtgggaatg 2520
   agattgcatc gctgaatcag gtaaaagatg ttctcccgac cacaattcgt gggtggaaga 2580
   tcgcttatta gtcactgctc ccattagtcc cactagacgg catacttcca ttccgccctt 2640
   taattcccct gtcagacact catgctccga aatcactaac catccttgtc caccaagcaa 2700
   tacgcatatt cagtagcact gcatctcgcc ctccccctat caagccccag cgctgcagat 2760
   cttcaccaca tatatacatg catcaactac atgtgattta gaaaaaacca gacccttcac 2820
   gggtaatagc ctaactcacg aacgttcctc tcgtttcgta tgataaggcc ttaagcattg 2880
   tcgatacggt cgttatgcgt cggttctttt taggagagag cagtgcccct gcgagggact 2940
   gggagtccga gcgacctccc ccctatgctg ttgaggtccc tcaaagtcac gggataagag 3000
   tcaccgggta cttccagtgc aacgagcgtc cgaaatccaa gaagaccctc cacagcttcg 3060
   ccgtaaaact ctgcgacgca attaagccgg ttcgagcgga tgctcccagc ttgaagatag 3120
   caatatggac ggctctagat ctggccttcg tgaaacctcc caatggaact gtaacaatag 3180
   atgcggcggt gaaagctaca ccgctaatcg ggaacaccca gtacaccgta ggcgatgaaa 3240
   tcttccagat gctagggaga aggggtggcc tgatcgtcat caggaactta ccccatgatt 3300
   atcctcgaac gttgattgag ttcgcctctc ccgagccttg agcaccaggg catcggtccg 3360
   cccgccctgt gatctcccgt agccgggctc agcgatcaag ccggcccggg tcggggggga 3420
   ctggtgcaac acaaggggcg gcagtggacg ctgattaaca aaaaaccacc tatatagacc 3480
   cctcacggtc ttagactctg ttgccagctg acaaccaaca cacaagacat ctctctgatt 3540
   cagccgaccc gatcgattcc tccccaccca attcctacca acgcactcct cacaagctcc 3600
   accggcgcgc catgttgaga ctttttctct tttgtttctt ggccttagga gcccactcca 3660
   aatttactat agtattccct catcatcaaa aagggaattg gaagaatgtg ccttccacat 3720
   atcattattg cccttctagt tctgaccaga attggcataa tgatttgact ggagttagtc 3780
   ttcatgtgaa aattcccaaa agtcacaaag ctatacaagc agatggctgg atgtgccacg 3840
   ctgctaaatg ggtgactact tgtgacttca gatggtacgg acccaaatac atcacgcatt 3900
   ccatacactc tatgtcaccc accctagaac agtgcaagac cagtattgag cagacaaagc 3960
   aaggagtttg gattaatcca ggctttcccc ctcaaagctg cggatatgct acagtgacgg 4020
   atgcagaggt ggttgttgta caagcaacac ctcatcatgt gttggttgat gagtacacag 4080
   gagaatggat tgactcacaa ttggtggggg gcaaatgttc caaggaggtt tgtcaaacgg 4140
   ttcacaactc gaccgtgtgg catgctgatt acaagattac agggctgtgc gagtcaaatc 4200
   tggcatcagt ggatatcacc ttcttctctg aggatggtca aaagacgtct ttgggaaaac 4260
   cgaacactgg attcaggagt aatcactttg cttacgaaag tggagagaag gcatgccgta 4320
   tgcagtactg cacacaatgg gggatccgac taccttctgg agtatggttt gaattagtgg 4380
   acaaagatct cttccaggcg gcaaaattgc ctgaatgtcc tagaggatcc agtatctcag 4440
   ctccttctca gacttctgtg gatgttagtt tgatacaaga cgtagagagg atcttagatt 4500
   actctctatg ccaggagacg tggagtaaga tacgagccaa gcttcctgta tctccagtag 4560
   atctgagtta tctcgcccca aaaaatccag ggagcggacc ggccttcact atcattaatg 4620
   gcactttgaa atatttcgaa acaagataca tcagagttga cataagtaat cccatcatcc 4680
   ctcacatggt gggaacaatg agtggaacca cgactgagcg tgaattgtgg aatgattggt 4740
   atccatatga agacgtagag attggtccaa atggggtgtt gaaaactccc actggtttca 4800
   agtttccgct gtacatgatt gggcacggaa tgttggattc cgatctccac aaatcctccc 4860
   aggctcaagt cttcgaacat ccacacgcaa aggacgctgc atcacagctt cctgatgatg 4920
   agactttatt ttttggtgac acaggactat caaaaaaccc agtagagtta gtagaaggct 4980
   ggttcagtag ctggaagagc acattggcat cgttctttct gattataggc ttgggggttg 5040
   cattaatctt catcattcga attattgttg cgattcgcta taaatacaag gggaggaaga 5100
   cccaaaaaat ttacaatgat gtcgagatga gtcgattggg aaataaataa ggcgcgccag 5160
   aggactgcga ttgttgagtg gagccggcaa accctaggcc tattccgatt tagaaaaaac 5220
   cagacctctc acgaggtctt ttctactagc tgggttttcc tcattctatc cagagccgcg 5280
   gccgcatggt gagcaagggc gaggagctgt tcaccggggt ggtgcccatc ctggtcgagc 5340
   tggacggcga cgtaaacggc cacaagttca gcgtgtccgg cgagggcgag ggcgatgcca 5400
   cctacggcaa gctgaccctg aagttcatct gcaccaccgg caagctgccc gtgccctggc 5460
   ccaccctcgt gaccaccctg acctacggcg tgcagtgctt cagccgctac cccgaccaca 5520
   tgaagcagca cgacttcttc aagtccgcca tgcccgaagg ctacgtccag gagcgcacca 5580
   tcttcttcaa ggacgacggc aactacaaga cccgcgccga ggtgaagttc gagggcgaca 5640
   ccctggtgaa ccgcatcgag ctgaagggca tcgacttcaa ggaggacggc aacatcctgg 5700
   ggcacaagct ggagtacaac tacaacagcc acaacgtcta tatcatggcc gacaagcaga 5760
   agaacggcat caaggtgaac ttcaagatcc gccacaacat cgaggacggc agcgtgcagc 5820
   tcgccgacca ctaccagcag aacaccccca tcggcgacgg ccccgtgctg ctgcccgaca 5880
   accactacct gagcacccag tccgccctga gcaaagaccc caacgagaag cgcgatcaca 5940
   tggtcctgct ggagttcgtg accgccgccg ggatcactct cggcatggac gagctgtaca 6000
   agtaagcggc cgccaaaccc taggcctatt ccgatttaga aaaaaccaga cctctcacga 6060
   ggtcttttct actagctggg ttttcctcat tctatccaga gccatggcct tcgacccgaa 6120
   ctggcagaga gaaggttatg aatgggatcc gtcaagtgag ggcagaccga ccgatgagaa    6180
   cgaagacgac agaggtcatc ggccaaaaac gagacttcgt acattccttg cccgcacgtt    6240
   aaatagccct atccgagccc tattctacac aatattccta ggaattcgag cggtttggga    6300
   cgggttcaaa agactcctac ctgtgaggac cgaaaagggt tatgcgaggt tttctgagtg    6360
   cgtcacatat ggaatgatcg gatgtgatga gtgtgtaata gacccggtga gggttgtcat    6420
   tgagctgacc gagatgcagt taccgattaa aggcaaaggc tctacgaggt tgagagcaat    6480
   gataactgaa gaccttctca cggggatgcg cacagccgtg cctcagatca gagtgagatc    6540
   gaagatccta gcagagcggt tagggagagc aatcggccga gagaccttgc cggcaatgat    6600
   ccatcatgag tgggcatttg tgatggggaa gattctcact ttcatggcag acaatgtggg    6660
   tatgaacgct gacacggtcg agggcgttct atcactatca gaggtcacac ggcgatggga    6720
   tatcggcaac tctgtgtccg cagtgttcaa tcctgatggc cttactatca gagtagaaaa    6780
   cacgggttac atcatgacca gagagactgc ctgcatgatc ggagacattc atgctcaatt    6840
   tgcaatccaa tacctagctg catacctaga cgaggtgatc ggcacaagga cgtctctctc    6900
   acccgccgaa ctgacctctc tcaaactatg gggacttaac gtcctgaaac tcctaggacg    6960
   gaacggttat gaggtgatcg cctgcatgga gcccataggg tacgctgtcc tgatgatggg    7020
   aagagacagg agtcctgatc cctatgtcaa tgacacctat ttaaacagca tcctctcaga    7080
   attccctgtc gactctgacg ctcgagcctg cgttgaagcc ctcttaacta tctatatgag    7140
   cttcggcaca ccccataaag tctcggacgc attcggcctc ttcagaatgt tgggacatcc    7200
   gatggttgat ggagctgacg ggattgaaaa gatgcgaagg ttaagcaaga aggtcaagat    7260
   cccagaccag tctacagcga tcgacctcgg ggctatcatg gccgaactgt ttgtgcggag    7320
   tttcgtaaag aagcacaaaa ggtggcccaa ctgctccatc aatctcccgc cacgacaccc    7380
   cttccaccac gcccgcctat gtgggtatgt cccggctgaa acccatcccc taaacaacac    7440
   tgcatcctgg gcggctgtgg agttcaacca ggaattcgag ccgccgagac agtacaacct    7500
   tgcagacatc attgatgaca agtcgtgctc tcccaacaag catgagctat atggtgcttg    7560
   gatgaagtca aaaacagctg ggtggcagga acaaaagaag ctcatactcc gatggttcac    7620
   tgagaccatg gttaaacctt cggagctcct ggaagagatt gatgcacacg gcttccgaga    7680
   agaggataag ttgattggat taacaccaaa ggagagagag ctgaaattaa caccaagaat    7740
   gttctccttg atgacattca agttcagaac ctaccaagtc ctcactgaga gtatggtcgc    7800
   cgatgagatc ctcccgcact tcccccagat caccatgacc atgtccaacc acgaactcac    7860
   aaagaggttg attagcagaa cgagacctca atctggagga gggcgtgatg ttcacatcac    7920
   cgtgaacata gatttccaga aatggaacac aaacatgaga cacggactgg tcaaacatgt    7980
   cttcgagcga ctggacaacc tctttggctt caccaactta atcagacgaa ctcatgaata 8040
   cttccaggag gcgaaatact atctggctga agatggaact aatctgtcgt tcgacaggaa 8100
   cggggagtta atagatggcc catacgttta caccggatca tacgggggga acgaggggtt 8160
   acgacagaag ccctggacaa tagttaccgt gtgtggaata tacaaggtag ctagagacct 8220
   gaaaatcaaa catcagatca ccggtcaggg agataatcag gtggtcaccc taatatttcc 8280
   ggatcgagag ttgccttcag atccggtgga gaggagcaag tactgtagag acaagagcag 8340
   tcagttcctg acacgtctca gtcaatattt cgctgaggtt ggtttgcccg tcaagactga 8400
   agagacatgg atgtcatcac gtctctatgc ttacggtaag cgcatgttct tagagggagt 8460
   tccacttaag atgtttctca agaagatagg cagagctttc gccctctcga atgagtttgt 8520
   cccgtccctc gaggaagatc tggccagagt ctggagtgcc accagcgcag cggtagagct 8580
   tgacctaact ccctacgtag gatatgtcct cgggtgctgc ttgtctgcgc aggcgatcag 8640
   aaatcacctc atctactccc ctgttctgga gggccctctg ctggttaagg cctacgagcg 8700
   taagttcatt aactacgacg gaggaacaaa gcggggggcg atgcccggcc tacgtccaac 8760
   ctttgagagc ctagtcaaaa gtatctgctg gaagccaaag gccatcggag ggtggccggt 8820
   attgatgtta gaagatctca tcatcaaagg gttccctgat ccggcgacta gcgccctggc 8880
   tcaattgaag tcaatggtgc catatacctc tggtatcgac cgggagatca tactttcctg 8940
   tctcaacctt cccttatcgt cggtggtatc tccgtcaatg ttgttaaagg acccggcggc 9000
   catcaacacc atcacaaccc cgtccgcggg cgacatcctg caagaggtcg ccagagacta 9060
   tgttaccgat tacccactcc aaaacccgca gctcagagca gtggtcaaga acgtgaagac 9120
   cgagctagac acattggcca gtgacttatt caaatgtgaa cctttctttc ctcctttaat 9180
   gagcgatatc ttctcggcat ctctcccggc atatcaagac aggattgttc gcaagtgctc 9240
   cacgacttct acaatcagga gaaaagctgc cgagaggggc tccgactctc tcctcaaccg 9300
   gatgaaaagg aatgagatca ataagatgat gttacatctt tgggctacct ggggaaggag 9360
   ccctctggcc agattagaca ccagatgtct cacaacctgc accaagcaat tagcccaaca 9420
   gtatcggaac cagtcttggg gaaagcagat ccatggagtc tcagtcggcc accccttaga 9480
   actgttcggt cgaataacac ccagccatag atgcctacat gaggaggacc acggagattt 9540
   cctgcaaacc ttcgccagcg agcatgtgaa ccaagtggac accgacatca ccacaactct 9600
   ggggccgttc tacccttaca taggctcgga gacgcgagaa cgggcagtca aggttcgaaa 9660
   aggagtgaat tacgtagttg agccgcttct gaaacccgca gttcgactac taagagccat 9720
   taattggttc attcccgagg agtcagatgc gtcccatttg ctgagcaatc tattagcgtc 9780
   tgttaccgac atcaatcctc aagaccacta ctcatctacc gaagtagggg ggggcaacgc 9840
   cgtccatcgc tacagctgcc gactatccga caaattgagc agagtcaaca acttatatca 9900
   gttgcatact tatttatctg tcacaacaga gcggttgacc aagtacagtc gaggatcaaa 9960
   aaacactgac gcacacttcc agagcatgat gatttatgca caaagccgtc atatagacct 10020
   catcttggag tctctgcaca ccggagagat ggtaccgttg gagtgtcatc atcacattga 10080
   gtgcaatcac tgtatagagg atatacccga cgagccaatc acgggggacc cggcttggac 10140
   tgaagtcaag tttccttcaa gtcctcagga gccctttctt tacatcaggc aacaagatct 10200
   gccggtcaaa gacaaactcg agcctgtgcc tcgcatgaac atcgtccgtc ttgccggatt 10260
   gggtccggag gcgattagtg agctagcgca ctactttgtt gcattccgag ttatccgggc 10320
   gtcagagacg gatgtcgacc ctaacgatgt tctctcgtgg acctggctga gccgaattga 10380
   tcctgacaaa ttggttgagt atatcgtgca tgtgttcgct tcactggaat ggcatcatgt 10440
   attaatgtca ggcgtgagtg tgagcgtcag agatgcattc tttaagatgc tagtgtctaa 10500
   aagaatctca gagactccgc taagttcatt ctattatctg gccaacctgt tcgttgaccc 10560
   tcagactcgc gaagcactaa tgagctctaa atacgggttc agcccccccg ccgagacagt 10620
   ccccaacgca aatgccgccg cagccgaaat aagaagatgc tgtgcgaaca gtgcgccgtc 10680
   gatcttagaa tcagcccttc acagccgtga ggttgtttgg atgccaggaa cgaacaatta 10740
   tggagacgtt gtcatctggt ctcattacat tagattacgg ttcagcgaag ttaaactagt 10800
   tgacattaca cgatatcagc agtggtggag acagtctgag cgagacccct acgatttggt 10860
   cccggacatg caggttcttg agagcgacct agatacgctg atgaaacgga taccgaggct 10920
   catgcgcaag gcgagacgtc cccctcttca ggtaattcga gaggacctgg atgtcgcagt 10980
   catcaatgct gatcatcccg ctcactctgt gcttcagaac aaatacagga aattgatttt 11040
   cagagagccg aagattatca cgggagctgt gtacaagtac ctctccctaa aatcagagtt 11100
   gacagagttc acctcagcaa tggtgatcgg agacggaact ggaggtatca ccgccgccat 11160
   gatggccgat gggatagatg tgtggtatca gacgctcgtc aactatgacc acgtgacaca 11220
   acagggatta tccgtacaag ccccggcagc attggatctt ctgcgcgggg caccctctgg 11280
   taggctcttg aatccgggaa gattcgcatc atttgggtct gacctaactg accctcgatt 11340
   tacagcctac tttgatcaat atcccccgtt caaggtggac actctatggt ctgacgcaga 11400
   gggcgacttt tgggacaagc cttccaagtt gaatcaatac tttgagaaca tcattgcttt 11460
   gagacatcgg ttcgtgaaga caaatggaca gcttgtcgtg aaggtgtatc tgactcaaga 11520
   cactgctacc acaattgaag cattcagaaa gaagctgtcc ccatgcgcca tcatcgtgtc 11580
   tctcttctcg acggaaggct ccacagaatg cttcgtccta agcaatctca tcgcaccaga 11640
   cacccctgtc gaccttgaga tggtggagaa tatccctaaa ctaacatccc ttgttcccca 11700
   gaggacgaca gtgaaatgct attcccgacg agtagcgtgc atcagtaaaa ggtggggact 11760
   tttcagatct ccgagcatag cccttgaagt ccaaccgttc cttcactaca tcacaaaggt 11820
   catctcagac aaaggaacac aactgagtct catggcggta gctgacacaa tgatcaacag 11880
   ttacaagaag gctatctcac cccgagtgtt cgatctacac cggcataggg ccgcactggg 11940
   tttcgggagg agatccttgc atctcatctg ggggatgatc atctcaccaa tcgcttacca 12000
   gcattttgag aatccggcca agttgatgga tgtcctggac atgttgacca ataacatctc 12060
   agctttctta tcgatatcgt cgtcaggatt tgacctgtca tttagtgtca gtgcagaccg 12120
   agatgtccgg attgacagca aacttgtcag actcccgcta ttcgaaggat cagacctaaa 12180
   attcatgaaa accatcatgt ctaccctcgg atctgtgttc aaccaggtcg agccttttaa 12240
   ggggatcgcc ataaaccctt ctaaactaat gactgtcaag aggacacagg agttacgtta 12300
   caacaaccta atttacacta aggatgccat cctattcccc aatgaagcgg caaaaaacac 12360
   tgccccgctt cgagccaaca tggtataccc cgtccgggga gatctattcg cccctaccga 12420
   tcgcatacca atcatgactc tagtcagcga tgagacaaca cctcagcact ctcctccaga 12480
   ggatgaggca taactgaatc ctccctgaag gctcacatgt cccacgcgac gcaagatata 12540
   acgacaagca actcgcccta ttaactgtga ttaataaaaa accgattatt cagttgcttg 12600
   agggagtttc aatccgttca gtgtatgata ggaagtttct gagatggtgg ggattagggg 12660
   gcacctagag tatgtttgtt cgttttatgc gtcgt 12695
<210> 19
   <211> 12695
   <212> RNA
   <213> Artificial
<220>
   <223> Chimeric virus containing RNA from Maraba and Farmington viruses
<400> 19
   uuacgacgca uaagcugaga aacauaagag acuauguuca uagucacccu guauucauua      60
   uugacuuuua ugaccuauua uucgugaggu cauaugugag guaaugucau cugcuuaugc     120
   guuugcuuau aagauaaaac gauagacccu ucacggguaa auccuucucc uugcaguucu     180
   cgccaaguac cuccaaaguc agaacgaugg cucguccgcu agcugcugcg caacaucuca     240
   uaaccgagcg ucauucccuu caggcgacuc ugucgcgggc guccaagacc agagccgagg     300
   aauucgucaa agauuucuac cuucaagagc aguauucugu cccgaccauc ccgacggacg     360
   acauugccca gucugggccc augcugcuuc aggccauccu gagcgaggaa uacacaaagg     420
   ccacugacau agcccaaucc auccucugga acacucccac acccaacggg cuccucagag     480
   agcaucuaga ugccgauggg ggaggcucau ucacagcgcu gcccgcgucu gcaaucagac     540
   ccagcgacga ggcgaaugca ugggccgcuc gcaucuccga cucaggguug gggccugucu     600
   ucuaugcagc ccucgcugcu uacaucaucg gcuggucagg aagaggagag acuagccgcg     660
   ugcagcagaa cauaggucag aaauggcuga ugaaccugaa cgcaaucuuc ggcaccacga 720
   ucacccaucc aacaaccgug cgucugccaa ucaacgucgu caacaacagc cucgcaguga 780
   ggaacggacu ugcugccaca cucuggcuau acuaccguuc aucaccucag agucaggacg 840
   cguucuucua ugggcucauc cgucccuguu gcaguggaua ucucggccug cuacaucggg 900
   ugcaggagau ugaugagaug gagccggacu uccucaguga cccccggauc auccagguga 960
   augaggucua cagugcacuc agagcccugg uucaacuggg aaacgacuuc aagaccgccg 1020
   augaugagcc caugcagguc ugggcgugca ggggaaucaa caacggauau cugacauauc 1080
   ucucagaaac uccugcgaag aaaggagcug uugugcuuau guuugcccaa ugcaugcuga 1140
   agggcgacuc ugaggccugg aacagcuacc gcacugcaac cugggugaug cccuauugcg 1200
   acaauguggc ccuaggagcg auggcaggcu acauccaagc ccgccagaac accagggcau 1260
   augaggucuc agcccagaca ggucucgacg ucaacauggc cgcggucaag gacuuugagg 1320
   ccaguucaaa acccaaggcu gcuccaaucu cgcugauccc acgccccgcu gaugucgcau 1380
   cccgcaccuc ugagcgccca ucuauuccug agguugacag cgacgaagag cucggaggaa 1440
   uguaaaccaa uaagcuucac ugccgguagu uuaggcauac acacgcaguu ccguuaucca 1500
   ucacacccgu cccuucuuuu augcugcuau uauuucaguu gcuaagcuuc cugauuugau 1560
   uaacaaaaaa ccguagaccu ccuacgugag guauagcuag aaauugguuc uaucgguuga 1620
   gagucuuugu acuauuagcc auggaggacu auuugucuag cuuagaggcc gcgagagagc 1680
   ucguccggac ggagcuggag cccaagcgua accucauagc cagcuuagag uccgacgauc 1740
   ccgauccggu aauagcgcca gcgguaaaac caaaacaucc caagccaugc cugagcacua 1800
   aagaagagga ucaucucccc ucucuucgcc uacuauucgg cgcaaaacga gacaccucgg 1860
   ugggcguaga gcagacucuc cacaagcguc ucugcgcuug ucucgacggu uaccugacca 1920
   ugacgaagaa agaggccaau gccuuuaagg ccgcggcuga agcagcagca uuagcaguca 1980
   uggacauuaa gauggagcau cagcgccagg aucuagagga ucugaccgcu gcuaucccua 2040
   ggauagaauu caaacucaau gccauccugg aaaacaacaa ggagauagcc aaggcuguaa 2100
   cugcugcuaa ggagauggag cgggagaugu cgugggggga aagcgccgcc agcucgcuca 2160
   agucugucac ccuagaugag ucguuuaggg gcccugaaga gcuuucagag ucauuuggca 2220
   uccgauauaa ggucagaacc uggaaugagu ucaagaaggc gcuggaaacc agcauugugg 2280
   accugaggcc uagcccuguu ucauuuaggg aauuacggac uauguggcug ucucuugaca 2340
   ccuccuuuag gcucauuggg uuugccuuca uucccacaug cgagcgccug gagaccaaag 2400
   ccaaaugcaa ggagacaagg acucuacucc cccuugcaga gucgaucaug cgaagauggg 2460
   accugcggga uccaaccauc uuggagaaag ccugcguagu aaugaugauc cgugggaaug 2520
   agauugcauc gcugaaucag guaaaagaug uucucccgac cacaauucgu ggguggaaga 2580
   ucgcuuauua gucacugcuc ccauuagucc cacuagacgg cauacuucca uuccgcccuu 2640
   uaauuccccu gucagacacu caugcuccga aaucacuaac cauccuuguc caccaagcaa 2700
   uacgcauauu caguagcacu gcaucucgcc cucccccuau caagccccag cgcugcagau 2760
   cuucaccaca uauauacaug caucaacuac augugauuua gaaaaaacca gacccuucac 2820
   ggguaauagc cuaacucacg aacguuccuc ucguuucgua ugauaaggcc uuaagcauug 2880
   ucgauacggu cguuaugcgu cgguucuuuu uaggagagag cagugccccu gcgagggacu 2940
   gggaguccga gcgaccuccc cccuaugcug uugagguccc ucaaagucac gggauaagag 3000
   ucaccgggua cuuccagugc aacgagcguc cgaaauccaa gaagacccuc cacagcuucg 3060
   ccguaaaacu cugcgacgca auuaagccgg uucgagcgga ugcucccagc uugaagauag 3120
   caauauggac ggcucuagau cuggccuucg ugaaaccucc caauggaacu guaacaauag 3180
   augcggcggu gaaagcuaca ccgcuaaucg ggaacaccca guacaccgua ggcgaugaaa 3240
   ucuuccagau gcuagggaga agggguggcc ugaucgucau caggaacuua ccccaugauu 3300
   auccucgaac guugauugag uucgccucuc ccgagccuug agcaccaggg caucgguccg 3360
   cccgcccugu gaucucccgu agccgggcuc agcgaucaag ccggcccggg ucggggggga 3420
   cuggugcaac acaaggggcg gcaguggacg cugauuaaca aaaaaccacc uauauagacc 3480
   ccucacgguc uuagacucug uugccagcug acaaccaaca cacaagacau cucucugauu 3540
   cagccgaccc gaucgauucc uccccaccca auuccuacca acgcacuccu cacaagcucc 3600
   accggcgcgc cauguugaga cuuuuucucu uuuguuucuu ggccuuagga gcccacucca 3660
   aauuuacuau aguauucccu caucaucaaa aagggaauug gaagaaugug ccuuccacau 3720
   aucauuauug cccuucuagu ucugaccaga auuggcauaa ugauuugacu ggaguuaguc 3780
   uucaugugaa aauucccaaa agucacaaag cuauacaagc agauggcugg augugccacg 3840
   cugcuaaaug ggugacuacu ugugacuuca gaugguacgg acccaaauac aucacgcauu 3900
   ccauacacuc uaugucaccc acccuagaac agugcaagac caguauugag cagacaaagc 3960
   aaggaguuug gauuaaucca ggcuuucccc cucaaagcug cggauaugcu acagugacgg 4020
   augcagaggu gguuguugua caagcaacac cucaucaugu guugguugau gaguacacag 4080
   gagaauggau ugacucacaa uugguggggg gcaaauguuc caaggagguu ugucaaacgg 4140
   uucacaacuc gaccgugugg caugcugauu acaagauuac agggcugugc gagucaaauc 4200
   uggcaucagu ggauaucacc uucuucucug aggaugguca aaagacgucu uugggaaaac 4260
   cgaacacugg auucaggagu aaucacuuug cuuacgaaag uggagagaag gcaugccgua 4320
   ugcaguacug cacacaaugg gggauccgac uaccuucugg aguaugguuu gaauuagugg 4380
   acaaagaucu cuuccaggcg gcaaaauugc cugaaugucc uagaggaucc aguaucucag 4440
   cuccuucuca gacuucugug gauguuaguu ugauacaaga cguagagagg aucuuagauu     4500
   acucucuaug ccaggagacg uggaguaaga uacgagccaa gcuuccugua ucuccaguag     4560
   aucugaguua ucucgcccca aaaaauccag ggagcggacc ggccuucacu aucauuaaug     4620
   gcacuuugaa auauuucgaa acaagauaca ucagaguuga cauaaguaau cccaucaucc     4680
   cucacauggu gggaacaaug aguggaacca cgacugagcg ugaauugugg aaugauuggu     4740
   auccauauga agacguagag auugguccaa augggguguu gaaaacuccc acugguuuca     4800
   aguuuccgcu guacaugauu gggcacggaa uguuggauuc cgaucuccac aaauccuccc     4860
   aggcucaagu cuucgaacau ccacacgcaa aggacgcugc aucacagcuu ccugaugaug     4920
   agacuuuauu uuuuggugac acaggacuau caaaaaaccc aguagaguua guagaaggcu     4980
   gguucaguag cuggaagagc acauuggcau cguucuuucu gauuauaggc uuggggguug    5040
   cauuaaucuu caucauucga auuauuguug cgauucgcua uaaauacaag gggaggaaga     5100
   cccaaaaaau uuacaaugau gucgagauga gucgauuggg aaauaaauaa ggcgcgccag     5160
   aggacugcga uuguugagug gagccggcaa acccuaggcc uauuccgauu uagaaaaaac     5220
   cagaccucuc acgaggucuu uucuacuagc uggguuuucc ucauucuauc cagagccgcg     5280
   gccgcauggu gagcaagggc gaggagcugu ucaccggggu ggugcccauc cuggucgagc     5340
   uggacggcga cguaaacggc cacaaguuca gcguguccgg cgagggcgag ggcgaugcca     5400
   ccuacggcaa gcugacccug aaguucaucu gcaccaccgg caagcugccc gugcccuggc     5460
   ccacccucgu gaccacccug accuacggcg ugcagugcuu cagccgcuac cccgaccaca     5520
   ugaagcagca cgacuucuuc aaguccgcca ugcccgaagg cuacguccag gagcgcacca     5580
   ucuucuucaa ggacgacggc aacuacaaga cccgcgccga ggugaaguuc gagggcgaca     5640
   cccuggugaa ccgcaucgag cugaagggca ucgacuucaa ggaggacggc aacauccugg     5700
   ggcacaagcu ggaguacaac uacaacagcc acaacgucua uaucauggcc gacaagcaga     5760
   agaacggcau caaggugaac uucaagaucc gccacaacau cgaggacggc agcgugcagc     5820
   ucgccgacca cuaccagcag aacaccccca ucggcgacgg ccccgugcug cugcccgaca     5880
   accacuaccu gagcacccag uccgcccuga gcaaagaccc caacgagaag cgcgaucaca     5940
   ugguccugcu ggaguucgug accgccgccg ggaucacucu cggcauggac gagcuguaca     6000
   aguaagcggc cgccaaaccc uaggccuauu ccgauuuaga aaaaaccaga ccucucacga     6060
   ggucuuuucu acuagcuggg uuuuccucau ucuauccaga gccauggccu ucgacccgaa     6120
   cuggcagaga gaagguuaug aaugggaucc gucaagugag ggcagaccga ccgaugagaa     6180
   cgaagacgac agaggucauc ggccaaaaac gagacuucgu acauuccuug cccgcacguu     6240
   aaauagcccu auccgagccc uauucuacac aauauuccua ggaauucgag cgguuuggga     6300
   cggguucaaa agacuccuac cugugaggac cgaaaagggu uaugcgaggu uuucugagug 6360
   cgucacauau ggaaugaucg gaugugauga guguguaaua gacccgguga ggguugucau 6420
   ugagcugacc gagaugcagu uaccgauuaa aggcaaaggc ucuacgaggu ugagagcaau 6480
   gauaacugaa gaccuucuca cggggaugcg cacagccgug ccucagauca gagugagauc 6540
   gaagauccua gcagagcggu uagggagagc aaucggccga gagaccuugc cggcaaugau 6600
   ccaucaugag ugggcauuug ugauggggaa gauucucacu uucauggcag acaauguggg 6660
   uaugaacgcu gacacggucg agggcguucu aucacuauca gaggucacac ggcgauggga 6720
   uaucggcaac ucuguguccg caguguucaa uccugauggc cuuacuauca gaguagaaaa 6780
   cacggguuac aucaugacca gagagacugc cugcaugauc ggagacauuc augcucaauu 6840
   ugcaauccaa uaccuagcug cauaccuaga cgaggugauc ggcacaagga cgucucucuc 6900
   acccgccgaa cugaccucuc ucaaacuaug gggacuuaac guccugaaac uccuaggacg 6960
   gaacgguuau gaggugaucg ccugcaugga gcccauaggg uacgcugucc ugaugauggg 7020
   aagagacagg aguccugauc ccuaugucaa ugacaccuau uuaaacagca uccucucaga 7080
   auucccuguc gacucugacg cucgagccug cguugaagcc cucuuaacua ucuauaugag 7140
   cuucggcaca ccccauaaag ucucggacgc auucggccuc uucagaaugu ugggacaucc 7200
   gaugguugau ggagcugacg ggauugaaaa gaugcgaagg uuaagcaaga aggucaagau 7260
   cccagaccag ucuacagcga ucgaccucgg ggcuaucaug gccgaacugu uugugcggag 7320
   uuucguaaag aagcacaaaa gguggcccaa cugcuccauc aaucucccgc cacgacaccc 7380
   cuuccaccac gcccgccuau guggguaugu cccggcugaa acccaucccc uaaacaacac 7440
   ugcauccugg gcggcugugg aguucaacca ggaauucgag ccgccgagac aguacaaccu 7500
   ugcagacauc auugaugaca agucgugcuc ucccaacaag caugagcuau auggugcuug 7560
   gaugaaguca aaaacagcug gguggcagga acaaaagaag cucauacucc gaugguucac 7620
   ugagaccaug guuaaaccuu cggagcuccu ggaagagauu gaugcacacg gcuuccgaga 7680
   agaggauaag uugauuggau uaacaccaaa ggagagagag cugaaauuaa caccaagaau 7740
   guucuccuug augacauuca aguucagaac cuaccaaguc cucacugaga guauggucgc 7800
   cgaugagauc cucccgcacu ucccccagau caccaugacc auguccaacc acgaacucac 7860
   aaagagguug auuagcagaa cgagaccuca aucuggagga gggcgugaug uucacaucac 7920
   cgugaacaua gauuuccaga aauggaacac aaacaugaga cacggacugg ucaaacaugu 7980
   cuucgagcga cuggacaacc ucuuuggcuu caccaacuua aucagacgaa cucaugaaua 8040
   cuuccaggag gcgaaauacu aucuggcuga agauggaacu aaucugucgu ucgacaggaa 8100
   cggggaguua auagauggcc cauacguuua caccggauca uacgggggga acgagggguu 8160
   acgacagaag cccuggacaa uaguuaccgu guguggaaua uacaagguag cuagagaccu 8220
   gaaaaucaaa caucagauca ccggucaggg agauaaucag guggucaccc uaauauuucc 8280
   ggaucgagag uugccuucag auccggugga gaggagcaag uacuguagag acaagagcag 8340
   ucaguuccug acacgucuca gucaauauuu cgcugagguu gguuugcccg ucaagacuga 8400
   agagacaugg augucaucac gucucuaugc uuacgguaag cgcauguucu uagagggagu 8460
   uccacuuaag auguuucuca agaagauagg cagagcuuuc gcccucucga augaguuugu 8520
   cccgucccuc gaggaagauc uggccagagu cuggagugcc accagcgcag cgguagagcu 8580
   ugaccuaacu cccuacguag gauauguccu cgggugcugc uugucugcgc aggcgaucag 8640
   aaaucaccuc aucuacuccc cuguucugga gggcccucug cugguuaagg ccuacgagcg 8700
   uaaguucauu aacuacgacg gaggaacaaa gcggggggcg augcccggcc uacguccaac 8760
   cuuugagagc cuagucaaaa guaucugcug gaagccaaag gccaucggag gguggccggu 8820
   auugauguua gaagaucuca ucaucaaagg guucccugau ccggcgacua gcgcccuggc 8880
   ucaauugaag ucaauggugc cauauaccuc ugguaucgac cgggagauca uacuuuccug 8940
   ucucaaccuu cccuuaucgu cggugguauc uccgucaaug uuguuaaagg acccggcggc 9000
   caucaacacc aucacaaccc cguccgcggg cgacauccug caagaggucg ccagagacua 9060
   uguuaccgau uacccacucc aaaacccgca gcucagagca guggucaaga acgugaagac 9120
   cgagcuagac acauuggcca gugacuuauu caaaugugaa ccuuucuuuc cuccuuuaau 9180
   gagcgauauc uucucggcau cucucccggc auaucaagac aggauuguuc gcaagugcuc 9240
   cacgacuucu acaaucagga gaaaagcugc cgagaggggc uccgacucuc uccucaaccg 9300
   gaugaaaagg aaugagauca auaagaugau guuacaucuu ugggcuaccu ggggaaggag 9360
   cccucuggcc agauuagaca ccagaugucu cacaaccugc accaagcaau uagcccaaca 9420
   guaucggaac cagucuuggg gaaagcagau ccauggaguc ucagucggcc accccuuaga 9480
   acuguucggu cgaauaacac ccagccauag augccuacau gaggaggacc acggagauuu 9540
   ccugcaaacc uucgccagcg agcaugugaa ccaaguggac accgacauca ccacaacucu 9600
   ggggccguuc uacccuuaca uaggcucgga gacgcgagaa cgggcaguca agguucgaaa 9660
   aggagugaau uacguaguug agccgcuucu gaaacccgca guucgacuac uaagagccau 9720
   uaauugguuc auucccgagg agucagaugc gucccauuug cugagcaauc uauuagcguc 9780
   uguuaccgac aucaauccuc aagaccacua cucaucuacc gaaguagggg ggggcaacgc 9840
   cguccaucgc uacagcugcc gacuauccga caaauugagc agagucaaca acuuauauca 9900
   guugcauacu uauuuaucug ucacaacaga gcgguugacc aaguacaguc gaggaucaaa 9960
   aaacacugac gcacacuucc agagcaugau gauuuaugca caaagccguc auauagaccu 10020
   caucuuggag ucucugcaca ccggagagau gguaccguug gagugucauc aucacauuga 10080
   gugcaaucac uguauagagg auauacccga cgagccaauc acgggggacc cggcuuggac 10140
   ugaagucaag uuuccuucaa guccucagga gcccuuucuu uacaucaggc aacaagaucu 10200
   gccggucaaa gacaaacucg agccugugcc ucgcaugaac aucguccguc uugccggauu 10260
   ggguccggag gcgauuagug agcuagcgca cuacuuuguu gcauuccgag uuauccgggc 10320
   gucagagacg gaugucgacc cuaacgaugu ucucucgugg accuggcuga gccgaauuga 10380
   uccugacaaa uugguugagu auaucgugca uguguucgcu ucacuggaau ggcaucaugu 10440
   auuaauguca ggcgugagug ugagcgucag agaugcauuc uuuaagaugc uagugucuaa 10500
   aagaaucuca gagacuccgc uaaguucauu cuauuaucug gccaaccugu ucguugaccc 10560
   ucagacucgc gaagcacuaa ugagcucuaa auacggguuc agcccccccg ccgagacagu 10620
   ccccaacgca aaugccgccg cagccgaaau aagaagaugc ugugcgaaca gugcgccguc 10680
   gaucuuagaa ucagcccuuc acagccguga gguuguuugg augccaggaa cgaacaauua 10740
   uggagacguu gucaucuggu cucauuacau uagauuacgg uucagcgaag uuaaacuagu 10800
   ugacauuaca cgauaucagc agugguggag acagucugag cgagaccccu acgauuuggu 10860
   cccggacaug cagguucuug agagcgaccu agauacgcug augaaacgga uaccgaggcu 10920
   caugcgcaag gcgagacguc ccccucuuca gguaauucga gaggaccugg augucgcagu 10980
   caucaaugcu gaucaucccg cucacucugu gcuucagaac aaauacagga aauugauuuu 11040
   cagagagccg aagauuauca cgggagcugu guacaaguac cucucccuaa aaucagaguu 11100
   gacagaguuc accucagcaa uggugaucgg agacggaacu ggagguauca ccgccgccau 11160
   gauggccgau gggauagaug ugugguauca gacgcucguc aacuaugacc acgugacaca 11220
   acagggauua uccguacaag ccccggcagc auuggaucuu cugcgcgggg cacccucugg 11280
   uaggcucuug aauccgggaa gauucgcauc auuugggucu gaccuaacug acccucgauu 11340
   uacagccuac uuugaucaau aucccccguu caagguggac acucuauggu cugacgcaga 11400
   gggcgacuuu ugggacaagc cuuccaaguu gaaucaauac uuugagaaca ucauugcuuu 11460
   gagacaucgg uucgugaaga caaauggaca gcuugucgug aagguguauc ugacucaaga 11520
   cacugcuacc acaauugaag cauucagaaa gaagcugucc ccaugcgcca ucaucguguc 11580
   ucucuucucg acggaaggcu ccacagaaug cuucguccua agcaaucuca ucgcaccaga 11640
   caccccuguc gaccuugaga ugguggagaa uaucccuaaa cuaacauccc uuguucccca 11700
   gaggacgaca gugaaaugcu auucccgacg aguagcgugc aucaguaaaa gguggggacu 11760
   uuucagaucu ccgagcauag cccuugaagu ccaaccguuc cuucacuaca ucacaaaggu 11820
   caucucagac aaaggaacac aacugagucu cauggcggua gcugacacaa ugaucaacag 11880
   uuacaagaag gcuaucucac ccgagguguu cgaucuacac cggcauaggg ccgcacuggg 11940
   uuucgggagg agauccuugc aucucaucug ggggaugauc aucucaccaa ucgcuuacca 12000
   gcauuuugag aauccggcca aguugaugga uguccuggac auguugacca auaacaucuc 12060
   agcuuucuua ucgauaucgu cgucaggauu ugaccuguca uuuaguguca gugcagaccg 12120
   agauguccgg auugacagca aacuugucag acucccgcua uucgaaggau cagaccuaaa 12180
   auucaugaaa accaucaugu cuacccucgg aucuguguuc aaccaggucg agccuuuuaa 12240
   ggggaucgcc auaaacccuu cuaaacuaau gacugucaag aggacacagg aguuacguua 12300
   caacaaccua auuuacacua aggaugccau ccuauucccc aaugaagcgg caaaaaacac 12360
   ugccccgcuu cgagccaaca ugguauaccc cguccgggga gaucuauucg ccccuaccga 12420
   ucgcauacca aucaugacuc uagucagcga ugagacaaca ccucagcacu cuccuccaga 12480
   ggaugaggca uaacugaauc cucccugaag gcucacaugu cccacgcgac gcaagauaua 12540
   acgacaagca acucgcccua uuaacuguga uuaauaaaaa accgauuauu caguugcuug 12600
   agggaguuuc aauccguuca guguaugaua ggaaguuucu gagauggugg ggauuagggg 12660
   gcaccuagag uauguuuguu cguuuuaugc gucgu 12695
<210> 20
   <211> 412
   <212> PRT
   <213> Farmington virus
<400> 20
   Met Ala Arg Pro Leu Ala Ala Ala Gln His Leu Ile Thr Glu Arg His
   1               5                   10                  15
   Ser Leu Gln Ala Thr Leu Ser Arg Ala Ser Lys Thr Arg Ala Glu Glu
               20                  25                  30
   Phe Val Lys Asp Phe Tyr Leu Gln Glu Gln Tyr Ser Val Pro Thr Ile
           35                  40                  45
   Pro Thr Asp Asp Ile Ala Gln Ser Gly Pro Met Leu Leu Gln Ala Ile
       50                  55                  60
   Leu Ser Glu Glu Tyr Thr Lys Ala Thr Asp Ile Ala Gln Ser Ile Leu
   65                  70                  75                  80
   Trp Asn Thr Pro Thr Pro Asn Gly Leu Leu Arg Glu His Leu Asp Ala
                   85                  90                  95
   Asp Gly Gly Gly Ser Phe Thr Ala Leu Pro Ala Ser Ala Ile Arg Pro
               100                 105                 110
   Ser Asp Glu Ala Asn Ala Trp Ala Ala Arg Ile Ser Asp Ser Gly Leu
           115                 120                 125
   Gly Pro Val Phe Tyr Ala Ala Leu Ala Ala Tyr Ile Ile Gly Trp Ser
       130                 135                 140
   Gly Arg Gly Glu Thr Ser Arg Val Gln Gln Asn Ile Gly Gln Lys Trp
   145                 150                 155                 160
   Leu Met Asn Leu Asn Ala Ile Phe Gly Thr Thr Ile Thr His Pro Thr
                   165                 170                 175
   Thr Val Arg Leu Pro Ile Asn Val Val Asn Asn Ser Leu Ala Val Arg
               180                 185                 190
   Asn Gly Leu Ala Ala Thr Leu Trp Leu Tyr Tyr Arg Ser Ser Pro Gln
           195                 200                 205
   Ser Gln Asp Ala Phe Phe Tyr Gly Leu Ile Arg Pro Cys Cys Ser Gly
       210                 215                 220
   Tyr Leu Gly Leu Leu His Arg Val Gln Glu Ile Asp Glu Met Glu Pro
   225                 230                 235                 240
   Asp Phe Leu Ser Asp Pro Arg Ile Ile Gln Val Asn Glu Val Tyr Ser
                   245                 250                 255
   Ala Leu Arg Ala Leu Val Gln Leu Gly Asn Asp Phe Lys Thr Ala Asp
               260                 265                 270
   Asp Glu Pro Met Gln Val Trp Ala Cys Arg Gly Ile Asn Asn Gly Tyr
           275                 280                 285
   Leu Thr Tyr Leu Ser Glu Thr Pro Ala Lys Lys Gly Ala Val Val Leu
       290                 295                 300
   Met Phe Ala Gln Cys Met Leu Lys Gly Asp Ser Glu Ala Trp Asn Ser
   305                 310                 315                 320
   Tyr Arg Thr Ala Thr Trp Val Met Pro Tyr Cys Asp Asn Val Ala Leu
                   325                 330                 335
   Gly Ala Met Ala Gly Tyr Ile Gln Ala Arg Gln Asn Thr Arg Ala Tyr
               340                 345                 350
   Glu Val Ser Ala Gln Thr Gly Leu Asp Val Asn Met Ala Ala Val Lys
           355                 360                 365
   Asp Phe Glu Ala Ser Ser Lys Pro Lys Ala Ala Pro Ile Ser Leu Ile
   370 375 380
   Pro Arg Pro Ala Asp Val Ala Ser Arg Thr Ser Glu Arg Pro Ser Ile
   385 390 395 400
   Pro Glu Val Asp Ser Asp Glu Glu Leu Gly Gly Met
   405 410
<210> 21
   <211> 316
   <212> PRT
   <213> Farmington virus
<400> 21
   Met Glu Asp Tyr Leu Ser Ser Leu Glu Ala Ala Arg Glu Leu Val Arg
   1               5                   10                  15
   Thr Glu Leu Glu Pro Lys Arg Asn Leu Ile Ala Ser Leu Glu Ser Asp
               20                  25                  30
   Asp Pro Asp Pro Val Ile Ala Pro Ala Val Lys Pro Lys His Pro Lys
           35                  40                  45
   Pro Cys Leu Ser Thr Lys Glu Glu Asp His Leu Pro Ser Leu Arg Leu
       50                  55                  60
   Leu Phe Gly Ala Lys Arg Asp Thr Ser Val Gly Val Glu Gln Thr Leu
   65                  70                  75                  80
   His Lys Arg Leu Cys Ala Cys Leu Asp Gly Tyr Leu Thr Met Thr Lys
                   85                  90                  95
   Lys Glu Ala Asn Ala Phe Lys Ala Ala Ala Glu Ala Ala Ala Leu Ala
               100                 105                 110
   Val Met Asp Ile Lys Met Glu His Gln Arg Gln Asp Leu Glu Asp Leu
           115                 120                 125
   Thr Ala Ala Ile Pro Arg Ile Glu Phe Lys Leu Asn Ala Ile Leu Glu
       130                 135                 140
   Asn Asn Lys Glu Ile Ala Lys Ala Val Thr Ala Ala Lys Glu Met Glu
   145                 150                 155                 160
   Arg Glu Met Ser Trp Gly Glu Ser Ala Ala Ser Ser Leu Lys Ser Val
                   165                 170                 175
   Thr Leu Asp Glu Ser Phe Arg Gly Pro Glu Glu Leu Ser Glu Ser Phe
                   180                 185                 190
   Gly Ile Arg Tyr Lys Val Arg Thr Trp Asn Glu Phe Lys Lys Ala Leu
           195                 200                 205
   Glu Thr Ser Ile Val Asp Leu Arg Pro Ser Pro Val Ser Phe Arg Glu
       210                 215                 220
   Leu Arg Thr Met Trp Leu Ser Leu Asp Thr Ser Phe Arg Leu Ile Gly
   225                 230                 235                 240
   Phe Ala Phe Ile Pro Thr Cys Glu Arg Leu Glu Thr Lys Ala Lys Cys
                   245                 250                 255
   Lys Glu Thr Arg Thr Leu Leu Pro Leu Ala Glu Ser Ile Met Arg Arg
               260                 265                 270
   Trp Asp Leu Arg Asp Pro Thr Ile Leu Glu Lys Ala Cys Val Val Met
           275                 280                 285
   Met Ile Arg Gly Asn Glu Ile Ala Ser Leu Asn Gln Val Lys Asp Val
       290                 295                 300
   Leu Pro Thr Thr Ile Arg Gly Trp Lys Ile Ala Tyr
   305                 310                 315
<210> 22
   <211> 2129
   <212> PRT
   <213> Farmington virus
<400> 22
   Met Ala Phe Asp Pro Asn Trp Gln Arg Glu Gly Tyr Glu Trp Asp Pro
   1               5                   10                  15
   Ser Ser Glu Gly Arg Pro Thr Asp Glu Asn Glu Asp Asp Arg Gly His
               20                  25                  30
   Arg Pro Lys Thr Arg Leu Arg Thr Phe Leu Ala Arg Thr Leu Asn Ser
           35                  40                  45
   Pro Ile Arg Ala Leu Phe Tyr Thr Ile Phe Leu Gly Ile Arg Ala Val
       50                  55                  60
   Trp Asp Gly Phe Lys Arg Leu Leu Pro Val Arg Thr Glu Lys Gly Tyr
   65                  70                  75                  80
   Ala Arg Phe Ser Glu Cys Val Thr Tyr Gly Met Ile Gly Cys Asp Glu
                   85                  90                  95
   Cys Val Ile Asp Pro Val Arg Val Val Ile Glu Leu Thr Glu Met Gln
               100                 105                 110
   Leu Pro Ile Lys Gly Lys Gly Ser Thr Arg Leu Arg Ala Met Ile Thr
           115                 120                 125
   Glu Asp Leu Leu Thr Gly Met Arg Thr Ala Val Pro Gln Ile Arg Val
       130                 135                 140
   Arg Ser Lys Ile Leu Ala Glu Arg Leu Gly Arg Ala Ile Gly Arg Glu
   145                 150                 155                 160
   Thr Leu Pro Ala Met Ile His His Glu Trp Ala Phe Val Met Gly Lys
                   165                 170                 175
   Ile Leu Thr Phe Met Ala Asp Asn Val Gly Met Asn Ala Asp Thr Val
               180                 185                 190
   Glu Gly Val Leu Ser Leu Ser Glu Val Thr Arg Arg Trp Asp Ile Gly
           195                 200                 205
   Asn Ser Val Ser Ala Val Phe Asn Pro Asp Gly Leu Thr Ile Arg Val
       210                 215                 220
   Glu Asn Thr Gly Tyr Ile Met Thr Arg Glu Thr Ala Cys Met Ile Gly
   225                 230                 235                 240
   Asp Ile His Ala Gln Phe Ala Ile Gln Tyr Leu Ala Ala Tyr Leu Asp
                   245                 250                 255
   Glu Val Ile Gly Thr Arg Thr Ser Leu Ser Pro Ala Glu Leu Thr Ser
               260                 265                 270
   Leu Lys Leu Trp Gly Leu Asn Val Leu Lys Leu Leu Gly Arg Asn Gly
           275                 280                 285
   Tyr Glu Val Ile Ala Cys Met Glu Pro Ile Gly Tyr Ala Val Leu Met
       290                 295                 300
   Met Gly Arg Asp Arg Ser Pro Asp Pro Tyr Val Asn Asp Thr Tyr Leu
   305                 310                 315                 320
   Asn Ser Ile Leu Ser Glu Phe Pro Val Asp Ser Asp Ala Arg Ala Cys
                   325                 330                 335
   Val Glu Ala Leu Leu Thr Ile Tyr Met Ser Phe Gly Thr Pro His Lys
               340                 345                 350
   Val Ser Asp Ala Phe Gly Leu Phe Arg Met Leu Gly His Pro Met Val
           355                 360                 365
   Asp Gly Ala Asp Gly Ile Glu Lys Met Arg Arg Leu Ser Lys Lys Val
       370                 375                 380
   Lys Ile Pro Asp Gln Ser Thr Ala Ile Asp Leu Gly Ala Ile Met Ala
   385                 390                 395                 400
   Glu Leu Phe Val Arg Ser Phe Val Lys Lys His Lys Arg Trp Pro Asn
                   405                 410                 415
   Cys Ser Ile Asn Leu Pro Pro Arg His Pro Phe His His Ala Arg Leu
               420                 425                 430
   Cys Gly Tyr Val Pro Ala Glu Thr His Pro Leu Asn Asn Thr Ala Ser
           435                 440                 445
   Trp Ala Ala Val Glu Phe Asn Gln Glu Phe Glu Pro Pro Arg Gln Tyr
       450                 455                 460
   Asn Leu Ala Asp Ile Ile Asp Asp Lys Ser Cys Ser Pro Asn Lys His
   465                 470                 475                 480
   Glu Leu Tyr Gly Ala Trp Met Lys Ser Lys Thr Ala Gly Trp Gln Glu
                   485                 490                 495
   Gln Lys Lys Leu Ile Leu Arg Trp Phe Thr Glu Thr Met Val Lys Pro
               500                 505                 510
   Ser Glu Leu Leu Glu Glu Ile Asp Ala His Gly Phe Arg Glu Glu Asp
           515                 520                 525
   Lys Leu Ile Gly Leu Thr Pro Lys Glu Arg Glu Leu Lys Leu Thr Pro
       530                 535                 540
   Arg Met Phe Ser Leu Met Thr Phe Lys Phe Arg Thr Tyr Gln Val Leu
   545                 550                 555                 560
   Thr Glu Ser Met Val Ala Asp Glu Ile Leu Pro His Phe Pro Gln Ile
                   565                 570                 575
   Thr Met Thr Met Ser Asn His Glu Leu Thr Lys Arg Leu Ile Ser Arg
               580                 585                 590
   Thr Arg Pro Gln Ser Gly Gly Gly Arg Asp Val His Ile Thr Val Asn
           595                 600                 605
   Ile Asp Phe Gln Lys Trp Asn Thr Asn Met Arg His Gly Leu Val Lys
       610                 615                 620
   His Val Phe Glu Arg Leu Asp Asn Leu Phe Gly Phe Thr Asn Leu Ile
   625                 630                 635                 640
   Arg Arg Thr His Glu Tyr Phe Gln Glu Ala Lys Tyr Tyr Leu Ala Glu
                   645                 650                 655
   Asp Gly Thr Asn Leu Ser Phe Asp Arg Asn Gly Glu Leu Ile Asp Gly
               660                 665                 670
   Pro Tyr Val Tyr Thr Gly Ser Tyr Gly Gly Asn Glu Gly Leu Arg Gln
           675                 680                 685
   Lys Pro Trp Thr Ile Val Thr Val Cys Gly Ile Tyr Lys Val Ala Arg
       690                 695                 700
   Asp Leu Lys Ile Lys His Gln Ile Thr Gly Gln Gly Asp Asn Gln Val
   705                 710                 715                 720
   Val Thr Leu Ile Phe Pro Asp Arg Glu Leu Pro Ser Asp Pro Val Glu
                   725                 730                 735
   Arg Ser Lys Tyr Cys Arg Asp Lys Ser Ser Gln Phe Leu Thr Arg Leu
               740                 745                 750
   Ser Gln Tyr Phe Ala Glu Val Gly Leu Pro Val Lys Thr Glu Glu Thr
           755                 760                 765
   Trp Met Ser Ser Arg Leu Tyr Ala Tyr Gly Lys Arg Met Phe Leu Glu
       770                 775                 780
   Gly Val Pro Leu Lys Met Phe Leu Lys Lys Ile Gly Arg Ala Phe Ala
   785                 790                 795                 800
   Leu Ser Asn Glu Phe Val Pro Ser Leu Glu Glu Asp Leu Ala Arg Val
                   805                 810                 815
   Trp Ser Ala Thr Ser Ala Ala Val Glu Leu Asp Leu Thr Pro Tyr Val
               820                 825                 830
   Gly Tyr Val Leu Gly Cys Cys Leu Ser Ala Gln Ala Ile Arg Asn His
           835                 840                 845
   Leu Ile Tyr Ser Pro Val Leu Glu Gly Pro Leu Leu Val Lys Ala Tyr
       850                 855                 860
   Glu Arg Lys Phe Ile Asn Tyr Asp Gly Gly Thr Lys Arg Gly Ala Met
   865                 870                 875                 880
   Pro Gly Leu Arg Pro Thr Phe Glu Ser Leu Val Lys Ser Ile Cys Trp
                   885                 890                 895
   Lys Pro Lys Ala Ile Gly Gly Trp Pro Val Leu Met Leu Glu Asp Leu
               900                 905                 910
   Ile Ile Lys Gly Phe Pro Asp Pro Ala Thr Ser Ala Leu Ala Gln Leu
           915                 920                 925
   Lys Ser Met Val Pro Tyr Thr Ser Gly Ile Asp Arg Glu Ile Ile Leu
       930                 935                 940
   Ser Cys Leu Asn Leu Pro Leu Ser Ser Val Val Ser Pro Ser Met Leu
   945                 950                 955                 960
   Leu Lys Asp Pro Ala Ala Ile Asn Thr Ile Thr Thr Pro Ser Ala Gly
                   965                 970                 975
   Asp Ile Leu Gln Glu Val Ala Arg Asp Tyr Val Thr Asp Tyr Pro Leu
               980                 985                 990
   Gln Asn Pro Gln Leu Arg Ala Val  Val Lys Asn Val Lys  Thr Glu Leu
           995                 1000                1005
   Asp Thr  Leu Ala Ser Asp Leu  Phe Lys Cys Glu Pro  Phe Phe Pro
       1010                1015                1020
   Pro Leu  Met Ser Asp Ile Phe  Ser Ala Ser Leu Pro  Ala Tyr Gln
       1025                1030                1035
   Asp Arg  Ile Val Arg Lys Cys  Ser Thr Thr Ser Thr  Ile Arg Arg
       1040                1045                1050
   Lys Ala  Ala Glu Arg Gly Ser  Asp Ser Leu Leu Asn  Arg Met Lys
       1055                1060                1065
   Arg Asn  Glu Ile Asn Lys Met  Met Leu His Leu Trp  Ala Thr Trp
       1070                1075                1080
   Gly Arg  Ser Pro Leu Ala Arg  Leu Asp Thr Arg Cys  Leu Thr Thr
   1085 1090 1095
   Cys Thr Lys Gln Leu Ala Gln Gln Tyr Arg Asn Gln Ser Trp Gly
   1100 1105 1110
   Lys Gln Ile His Gly Val Ser Val Gly His Pro Leu Glu Leu Phe
   1115 1120 1125
   Gly Arg Ile Thr Pro Ser His Arg Cys Leu His Glu Glu Asp His
   1130 1135 1140
   Gly Asp Phe Leu Gln Thr Phe Ala Ser Glu His Val Asn Gln Val
   1145 1150 1155
   Asp Thr Asp Ile Thr Thr Thr Leu Gly Pro Phe Tyr Pro Tyr Ile
   1160 1165 1170
   Gly Ser Glu Thr Arg Glu Arg Ala Val Lys Val Arg Lys Gly Val
   1175 1180 1185
   Asn Tyr Val Val Glu Pro Leu Leu Lys Pro Ala Val Arg Leu Leu
   1190 1195 1200
   Arg Ala Ile Asn Trp Phe Ile Pro Glu Glu Ser Asp Ala Ser His
   1205 1210 1215
   Leu Leu Ser Asn Leu Leu Ala Ser Val Thr Asp Ile Asn Pro Gln
   1220 1225 1230
   Asp His Tyr Ser Ser Thr Glu Val Gly Gly Gly Asn Ala Val His
   1235 1240 1245
   Arg Tyr Ser Cys Arg Leu Ser Asp Lys Leu Ser Arg Val Asn Asn
   1250 1255 1260
   Leu Tyr Gln Leu His Thr Tyr Leu Ser Val Thr Thr Glu Arg Leu
   1265 1270 1275
   Thr Lys Tyr Ser Arg Gly Ser Lys Asn Thr Asp Ala His Phe Gln
   1280 1285 1290
   Ser Met Met Ile Tyr Ala Gln Ser Arg His Ile Asp Leu Ile Leu
   1295 1300 1305
   Glu Ser Leu His Thr Gly Glu Met Val Pro Leu Glu Cys His His
   1310 1315 1320
   His Ile  Glu Cys Asn His Cys  Ile Glu Asp Ile Pro  Asp Glu Pro
       1325                 1330                 1335
   Ile Thr  Gly Asp Pro Ala Trp  Thr Glu Val Lys Phe  Pro Ser Ser
       1340                 1345                 1350
   Pro Gln  Glu Pro Phe Leu Tyr  Ile Arg Gln Gln Asp  Leu Pro Val
       1355                 1360                 1365
   Lys Asp  Lys Leu Glu Pro Val  Pro Arg Met Asn Ile  Val Arg Leu
       1370                 1375                 1380
   Ala Gly  Leu Gly Pro Glu Ala  Ile Ser Glu Leu Ala  His Tyr Phe
       1385                 1390                 1395
   Val Ala  Phe Arg Val Ile Arg  Ala Ser Glu Thr Asp  Val Asp Pro
       1400                 1405                 1410
   Asn Asp  Val Leu Ser Trp Thr  Trp Leu Ser Arg Ile  Asp Pro Asp
       1415                 1420                 1425
   Lys Leu  Val Glu Tyr Ile Val  His Val Phe Ala Ser  Leu Glu Trp
       1430                 1435                 1440
   His His  Val Leu Met Ser Gly  Val Ser Val Ser Val  Arg Asp Ala
       1445                 1450                 1455
   Phe Phe  Lys Met Leu Val Ser  Lys Arg Ile Ser Glu  Thr Pro Leu
       1460                 1465                 1470
   Ser Ser  Phe Tyr Tyr Leu Ala  Asn Leu Phe Val Asp  Pro Gln Thr
       1475                 1480                 1485
   Arg Glu  Ala Leu Met Ser Ser  Lys Tyr Gly Phe Ser  Pro Pro Ala
       1490                 1495                 1500
   Glu Thr  Val Pro Asn Ala Asn  Ala Ala Ala Ala Glu  Ile Arg Arg
       1505                 1510                 1515
   Cys Cys  Ala Asn Ser Ala Pro  Ser Ile Leu Glu Ser  Ala Leu His
       1520                 1525                 1530
   Ser Arg  Glu Val Val Trp Met  Pro Gly Thr Asn Asn  Tyr Gly Asp
       1535                 1540                 1545
   Val Val  Ile Trp Ser His Tyr  Ile Arg Leu Arg Phe  Ser Glu Val
       1550                 1555                 1560
   Lys Leu Val Asp Ile Thr Arg Tyr Gln Gln Trp Trp Arg Gln Ser
       1565                1570                1575
   Glu Arg Asp Pro Tyr Asp Leu Val Pro Asp Met Gln Val Leu Glu
       1580                1585                1590
   Ser Asp Leu Asp Thr Leu Met Lys Arg Ile Pro Arg Leu Met Arg
       1595                1600                1605
   Lys Ala Arg Arg Pro Pro Leu Gln Val Ile Arg Glu Asp Leu Asp
       1610                1615                1620
   Val Ala Val Ile Asn Ala Asp His Pro Ala His Ser Val Leu Gln
       1625                1630                1635
   Asn Lys Tyr Arg Lys Leu Ile Phe Arg Glu Pro Lys Ile Ile Thr
       1640                1645                1650
   Gly Ala Val Tyr Lys Tyr Leu Ser Leu Lys Ser Glu Leu Thr Glu
       1655                1660                1665
   Phe Thr Ser Ala Met Val Ile Gly Asp Gly Thr Gly Gly Ile Thr
       1670                1675                1680
   Ala Ala Met Met Ala Asp Gly Ile Asp Val Trp Tyr Gln Thr Leu
       1685                1690                1695
   Val Asn Tyr Asp His Val Thr Gln Gln Gly Leu Ser Val Gln Ala
       1700                1705                1710
   Pro Ala Ala Leu Asp Leu Leu Arg Gly Ala Pro Ser Gly Arg Leu
       1715                1720                1725
   Leu Asn Pro Gly Arg Phe Ala Ser Phe Gly Ser Asp Leu Thr Asp
       1730                1735                1740
   Pro Arg Phe Thr Ala Tyr Phe Asp Gln Tyr Pro Pro Phe Lys Val
       1745                1750                1755
   Asp Thr Leu Trp Ser Asp Ala Glu Gly Asp Phe Trp Asp Lys Pro
       1760                1765                1770
   Ser Lys Leu Asn Gln Tyr Phe Glu Asn Ile Ile Ala Leu Arg His
       1775                1780                1785
   Arg Phe Val Lys Thr Asn Gly Gln Leu Val Val Lys Val Tyr Leu
       1790                1795                1800
   Thr Gln  Asp Thr Ala Thr Thr  Ile Glu Ala Phe Arg  Lys Lys Leu
       1805                 1810                 1815
   Ser Pro  Cys Ala Ile Ile Val  Ser Leu Phe Ser Thr  Glu Gly Ser
       1820                 1825                 1830
   Thr Glu  Cys Phe Val Leu Ser  Asn Leu Ile Ala Pro  Asp Thr Pro
       1835                 1840                 1845
   Val Asp  Leu Glu Met Val Glu  Asn Ile Pro Lys Leu  Thr Ser Leu
       1850                 1855                 1860
   Val Pro  Gln Arg Thr Thr Val  Lys Cys Tyr Ser Arg  Arg Val Ala
       1865                 1870                 1875
   Cys Ile  Ser Lys Arg Trp Gly  Leu Phe Arg Ser Pro  Ser Ile Ala
       1880                 1885                 1890
   Leu Glu  Val Gln Pro Phe Leu  His Tyr Ile Thr Lys  Val Ile Ser
       1895                 1900                 1905
   Asp Lys  Gly Thr Gln Leu Ser  Leu Met Ala Val Ala  Asp Thr Met
       1910                 1915                 1920
   Ile Asn  Ser Tyr Lys Lys Ala  Ile Ser Pro Arg Val  Phe Asp Leu
       1925                 1930                 1935
   His Arg  His Arg Ala Ala Leu  Gly Phe Gly Arg Arg  Ser Leu His
       1940                 1945                 1950
   Leu Ile  Trp Gly Met Ile Ile  Ser Pro Ile Ala Tyr  Gln His Phe
       1955                 1960                 1965
   Glu Asn  Pro Ala Lys Leu Met  Asp Val Leu Asp Met  Leu Thr Asn
       1970                 1975                 1980
   Asn Ile  Ser Ala Phe Leu Ser  Ile Ser Ser Ser Gly  Phe Asp Leu
       1985                 1990                 1995
   Ser Phe  Ser Val Ser Ala Asp  Arg Asp Val Arg Ile  Asp Ser Lys
       2000                 2005                 2010
   Leu Val  Arg Leu Pro Leu Phe  Glu Gly Ser Asp Leu  Lys Phe Met
       2015                 2020                 2025
   Lys Thr  Ile Met Ser Thr Leu  Gly Ser Val Phe Asn  Gln Val Glu
   2030 2035 2040
   Pro Phe Lys Gly Ile Ala Ile Asn Pro Ser Lys Leu Met Thr Val
   2045 2050 2055
   Lys Arg Thr Gln Glu Leu Arg Tyr Asn Asn Leu Ile Tyr Thr Lys
   2060 2065 2070
   Asp Ala Ile Leu Phe Pro Asn Glu Ala Ala Lys Asn Thr Ala Pro
   2075 2080 2085
   Leu Arg Ala Asn Met Val Tyr Pro Val Arg Gly Asp Leu Phe Ala
   2090 2095 2100
   Pro Thr Asp Arg Ile Pro Ile Met Thr Leu Val Ser Asp Glu Thr
   2105 2110 2115
   Thr Pro Gln His Ser Pro Pro Glu Asp Glu Ala
   2120 2125
<210> 23
   <211> 148
   <212> PRT
   <213> Farmington virus
<400> 23
   Met Arg Arg Phe Phe Leu Gly Glu Ser Ser Ala Pro Ala Arg Asp Trp
   1               5                   10                  15
   Glu Ser Glu Arg Pro Pro Pro Tyr Ala Val Glu Val Pro Gln Ser His
               20                  25                  30
   Gly Ile Arg Val Thr Gly Tyr Phe Gln Cys Asn Glu Arg Pro Lys Ser
           35                  40                  45
   Lys Lys Thr Leu His Ser Phe Ala Val Lys Leu Cys Asp Ala Ile Lys
       50                  55                  60
   Pro Val Arg Ala Asp Ala Pro Ser Leu Lys Ile Ala Ile Trp Thr Ala
   65                  70                  75                  80
   Leu Asp Leu Ala Phe Val Lys Pro Pro Asn Gly Thr Val Thr Ile Asp
                   85                  90                  95
   Ala Ala Val Lys Ala Thr Pro Leu Ile Gly Asn Thr Gln Tyr Thr Val
               100                 105                 110
   Gly Asp Glu Ile Phe Gln Met Leu Gly Arg Arg Gly Gly Leu Ile Val
           115                 120                 125
   Ile Arg Asn Leu Pro His Asp Tyr Pro Arg Thr Leu Ile Glu Phe Ala
       130                 135                 140
   Ser Pro Glu Pro
   145
<210> 24
   <211> 512
   <212> PRT
   <213> Maraba virus
<400> 24
   Met Leu Arg Leu Phe Leu Phe Cys Phe Leu Ala Leu Gly Ala His Ser
   1               5                   10                  15
   Lys Phe Thr Ile Val Phe Pro His His Gln Lys Gly Asn Trp Lys Asn
               20                  25                  30
   Val Pro Ser Thr Tyr His Tyr Cys Pro Ser Ser Ser Asp Gln Asn Trp
           35                  40                  45
   His Asn Asp Leu Thr Gly Val Ser Leu His Val Lys Ile Pro Lys Ser
       50                  55                  60
   His Lys Ala Ile Gln Ala Asp Gly Trp Met Cys His Ala Ala Lys Trp
   65                  70                  75                  80
   Val Thr Thr Cys Asp Phe Arg Trp Tyr Gly Pro Lys Tyr Ile Thr His
                   85                  90                  95
   Ser Ile His Ser Met Ser Pro Thr Leu Glu Gln Cys Lys Thr Ser Ile
               100                 105                 110
   Glu Gln Thr Lys Gln Gly Val Trp Ile Asn Pro Gly Phe Pro Pro Gln
           115                 120                 125
   Ser Cys Gly Tyr Ala Thr Val Thr Asp Ala Glu Val Val Val Val Gln
       130                 135                 140
   Ala Thr Pro His His Val Leu Val Asp Glu Tyr Thr Gly Glu Trp Ile
   145                 150                 155                 160
   Asp Ser Gln Leu Val Gly Gly Lys Cys Ser Lys Glu Val Cys Gln Thr
                   165                 170                 175
   Val His Asn Ser Thr Val Trp His Ala Asp Tyr Lys Ile Thr Gly Leu
               180                 185                 190
   Cys Glu Ser Asn Leu Ala Ser Val Asp Ile Thr Phe Phe Ser Glu Asp
           195                 200                 205
   Gly Gln Lys Thr Ser Leu Gly Lys Pro Asn Thr Gly Phe Arg Ser Asn
       210                 215                 220
   His Phe Ala Tyr Glu Ser Gly Glu Lys Ala Cys Arg Met Gln Tyr Cys
   225                 230                 235                 240
   Thr Gln Trp Gly Ile Arg Leu Pro Ser Gly Val Trp Phe Glu Leu Val
                   245                 250                 255
   Asp Lys Asp Leu Phe Gln Ala Ala Lys Leu Pro Glu Cys Pro Arg Gly
               260                 265                 270
   Ser Ser Ile Ser Ala Pro Ser Gln Thr Ser Val Asp Val Ser Leu Ile
           275                 280                 285
   Gln Asp Val Glu Arg Ile Leu Asp Tyr Ser Leu Cys Gln Glu Thr Trp
       290                 295                 300
   Ser Lys Ile Arg Ala Lys Leu Pro Val Ser Pro Val Asp Leu Ser Tyr
   305                 310                 315                 320
   Leu Ala Pro Lys Asn Pro Gly Ser Gly Pro Ala Phe Thr Ile Ile Asn
                   325                 330                 335
   Gly Thr Leu Lys Tyr Phe Glu Thr Arg Tyr Ile Arg Val Asp Ile Ser
               340                 345                 350
   Asn Pro Ile Ile Pro His Met Val Gly Thr Met Ser Gly Thr Thr Thr
           355                 360                 365
   Glu Arg Glu Leu Trp Asn Asp Trp Tyr Pro Tyr Glu Asp Val Glu Ile
       370                 375                 380
   Gly Pro Asn Gly Val Leu Lys Thr Pro Thr Gly Phe Lys Phe Pro Leu
   385                 390                 395                 400
   Tyr Met Ile Gly His Gly Met Leu Asp Ser Asp Leu His Lys Ser Ser
                   405                 410                 415
   Gln Ala Gln Val Phe Glu His Pro His Ala Lys Asp Ala Ala Ser Gln
               420                 425                 430
   Leu Pro Asp Asp Glu Thr Leu Phe Phe Gly Asp Thr Gly Leu Ser Lys
           435                 440                 445
   Asn Pro Val Glu Leu Val Glu Gly Trp Phe Ser Ser Trp Lys Ser Thr
       450                 455                 460
   Leu Ala Ser Phe Phe Leu Ile Ile Gly Leu Gly Val Ala Leu Ile Phe
   465                 470                 475                 480
   Ile Ile Arg Ile Ile Val Ala Ile Arg Tyr Lys Tyr Lys Gly Arg Lys
                   485                 490                 495
   Thr Gln Lys Ile Tyr Asn Asp Val Glu Met Ser Arg Leu Gly Asn Lys
               500                 505                 510